# EUROPÄISCHE PATENTSCHRIFT

(11) **EP 2 576 539 B1**
(45) Veröffentlichungstag und Bekanntmachung des Hinweises auf die Patenterteilung: **13.12.2017**
(21) Anmeldenummer: 11720550.0
(22) Anmeldetag: 23.05.2011
(51) Int. Cl.: C07D 401/12, C07D 401/14, C07D 409/12, C07D 409/14, A01N 43/40, A01N 43/48, A01P 3/00

(54) **PYRIDINYLCARBONSÄURE DERIVATE ALS FUNGIZIDE**
PYRIDINYL CARBONIC ACID DERIVATIVES AS FUNGICIDES
DÉRIVÉS D'ACIDE CARBONIQUE DE PYRIDINYLE EN TANT QUE FONGICIDES

(30) Priorität: 27.05.2010 US 348981 P; 27.05.2010 EP 10164099
(43) Veröffentlichungstag der Anmeldung: 10.04.2013
(73) Patentinhaber: Bayer CropScience AG, 40789 Monheim am Rhein (DE)
(72) Erfinder: CRISTAU, Pierre, F-69009 Lyon (FR); HOFFMANN, Sebastian, 41470 Neuss (DE); KLUTH, Joachim, 40764 Langenfeld (DE); RAHN, Nicola, 40589 Düsseldorf (DE); TSUCHIYA, Tomoki, 69009 Lyon (FR); WASNAIRE, Pierre, 40225 Düsseldorf (DE); BENTING, Jürgen, 42799 Leichlingen (DE); WACHENDORFF-NEUMANN, Ulrike, 56566 Neuwied (DE)
(74) Vertreter: BIP Patents
(86) Internationale Anmeldenummer: PCT/EP2011/058330
(87) Internationale Veröffentlichungsnummer: WO 2011/147765

(56) Entgegenhaltungen:
- WO-A1-2009/098576
- WO-A2-2007/014290
- WO-A2-2011/018415

## Beschreibung

Die Erfindung betrifft Pyridinylcarbonsäure Derivate, sowie deren Salze, Metallkomplexe und N-Oxide, deren Verwendung sowie Verfahren und Mittel zur Bekämpfung von pflanzenpathogenen Schadpilzen in und/oder auf Pflanzen oder in und/oder auf Saatgut von Pflanzen, Verfahren zur Herstellung solcher Mittel und behandeltes Saatgut sowie deren Verwendung zur Bekämpfung von pflanzenpathogenen Schadpilzen in der Land-, Garten- und Forstwirtschaft, im Materialschutz sowie im Bereich Haushalt und Hygiene. Die vorliegende Erfindung betrifft weiterhin ein Verfahren zur Herstellung von Pyridinylcarbonsäure Derivaten.

WO-A-2010/008739 beschreibt bestimmte heterocyclisch substituierte Piperidine für die Behandlung von diabetischen Krankheiten.

US-A-2009/0197859 offenbart bestimmte Pyperidinyl-Derivate zur Behandlung von Krankheiten des Nervensystems.

Darüberhinaus ist es bereits bekannt, dass bestimmte heterocyclisch substituierte Thiazole als fungizide Pflanzenschutzmittel benutzt werden können (siehe WO-A-07/014290, WO-A-08/013925, WO-A-08/013622, WO-A-08/091594, WO-A-08/091580, WO-A-09/055514, WO-A-09/094407, WO-A-09/094445, WO-A-09/132785, WO-A-10/037479, WO 2010/065579, WO 2010/149275, WO 2010/066353, WO 2011/018401, WO-2011/018415. WO2007014290 betrifft fungizide Carboxamide. Die fungizide Wirksamkeit dieser Verbindungen ist jedoch gerade bei niedrigeren Aufwandmengen nicht immer ausreichend. Da sich die ökologischen und ökonomischen Anforderungen an moderne Pflanzenschutzmittel laufend erhöhen, beispielsweise was Wirkspektrum, Toxizität, Selektivität, Aufwandmenge, Rückstandsbildung und günstige Herstellbarkeit angeht, und außerdem z.B. Probleme mit Resistenzen auftreten können, besteht die ständige Aufgabe, neue Pflanzenschutzmittel, insbesondere Fungizide zu entwickeln, die zumindest in Teilbereichen Vorteile gegenüber den bekannten aufweisen.

Überraschenderweise wurde nun gefunden, dass die vorliegenden Pyridinylcarbonsäure Derivate die genannten Aufgaben zumindest in Teilaspekten lösen und sich als Pflanzenschutzmittel, insbesondere als Fungizide eignen. Jeglicher Gegenstand, welcher nicht vom Anspruchsumfang gedeckt ist, ist nicht Teil der Erfindung. Gegenstand der Erfindung sind Verbindungen der Formel **(I),** 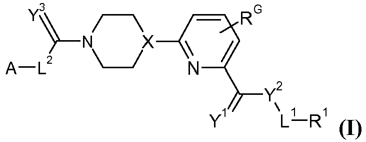 in welcher die Restedefinitionen folgende Bedeutungen haben:
- A: steht für Phenyl, das bis zu drei Substituenten enthalten kann, wobei die Substituenten unabhängig voneinander ausgewählt aus Z^{A-1} sind,
oder
- A: steht für ein gegebenenfalls benzokondensiertes, unsubstituiertes oder substituiertes 5-oder 6-gliedriges Heteroaryl, wobei die Substituenten am Kohlenstoff unabhängig voneinander ausgewählt aus Z^{A-2} sind und die Substituenten am Stickstoff unabhängig voneinander ausgewählt sind aus Z^{A-3},
- L¹: steht für (C(R¹¹)₂)ₚ, p steht für 1, 2 oder 3,
- R¹¹: ist gleich oder verschieden unabhängig voneinander Wasserstoff, Halogen, C₁-C₄-Alkyl, C₂-C₄-Alkenyl, C₂-C₄-Alkinyl, C₁-C₄-Alkoxy, C₃-C₆-Cycloalkyl, C₁-C₄-Halogenalkyl, oder Phenyl
unter der Voraussetzung, dass L¹ maximal zwei R¹¹ enthalten kann, die von Wasserstoff verschieden sind,
- L²: steht für NR¹²¹ oder C(R¹²²)₂,
- R¹²¹: steht für Wasserstoff, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂₋C₄-Halogenalkylcarbonyl, C₂-C₅-Alkoxycarbonyl, C₃-C₅-Alkoxycalbonylalkyl, C₂-C₅-Alkylaminocarbonyl, C₃-C₅-Dialkylaminocarbonyl, C₁-C₄-Alkylsulfonyl oder C₁-C₄-Halogenalkylsulfonyl,
- R¹²²: steht gleich oder verschieden unabhängig voneinander für Wasserstoff, C₁-C₄-Alkyl, C₁-C₄-Halogenalkyl, Cyclopropyl, Halogen,
oder die beiden Reste R¹²² bilden zusammen mit dem Kohlenstoffatom, an das sie gebunden sind, einen Cyclopropylring,
- Y¹ und Y³: stehen gleich oder verschieden unabhängig voneinander für Schwefel oder Sauerstoff,
- Y²: steht für -(NR^{Y2})-, Schwefel oder Sauerstoff,
- R^{Y2}: steht für Wasserstoff, C₁-C₆-Alkyl, C₂-C₆-Alkenyl, C₂-C₆-Alkinyl, C₁-C₆-Halogenalkyl, C₃-C₆-Cycloalkyl, C₃-C₆-Halogencycloalkyl, Benzyl, Phenyl, NR³R⁴, C₁-C₄-Alkoxy, C₁-C₄-Halogenalkoxy, Phenoxy oder Benzyloxy,
- oder: die Reste R^{Y2}, L¹ und R¹ bilden zusammen mit dem Stickstoffatom von Y² ein 5- bis 15-gliedriges, unsubstituiertes oder substituiertes, gesättigtes oder teilweise gesättigtes oder ungesättigtes mono-, bi- oder tricyclisches Ringsystem, das bis zu zwei weitere Heteroatome, ausgewählt aus N, O und S enthalten kann, wobei zwei Sauerstoffatome nicht benachbart sind und wobei mögliche Substituenten am Kohlenstoff unabhängig voneinander ausgewählt sind aus Z^{Y-1} und wobei die Substituenten am Stickstoff unabhängig voneinander ausgewählt sind aus Z^{Y-2},
- R³ und R⁴: stehen gleich oder verschieden unabhängig voneinander für Wasserstoff, C₁-C₄-Alkyl, C₃-C₆-Alkenyl, C₃-C₆-Alkinyl, C₁-C₃-Halogenalkyl, C₃-C₆-Cycloalkyl, Benzyl oder Phenyl,
- X: steht für -CR^{X1}- oder Stickstoff,
- R^{X1}: steht für Wasserstoff, Halogen, Cyano, Hydroxy, C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₂-C₄-Carbonylalkoxy, OC(=O)H, C(=O)H, C(=O)OH, C₂-C₄-Alkoxycarbonyl oder C₁-C₃-Alkylcarbonyl,
- R^{G}: steht für Wasserstoff, Halogen oder C₁-C₃-Alkyl,
- R¹: steht für Wasserstoff, C₁-C₆-Alkyl, C₁-C₆-Halogenalkyl, C₂-C₆-Alkenyl, C₂-C₆-Alkinyl, C₂-C₈-Alkoxyalkyl oder C₅-C₉-Cycloalkoxyalkyl,
oder
- R¹: steht für unsubstituiertes oder substituiertes C₃-C₁₀-Cycloalkyl, wobei die Substituenten unabhängig voneinander ausgewählt sind aus Q oder aus Z¹,
oder
- R¹: steht für unsubstituiertes oder substituiertes C₅-C₁₀-Cycloalkenyl, wobei die Substituenten unabhängig voneinander ausgewählt sind aus Z²,
oder
- R¹: steht für substituiertes Phenyl, wobei die Substituenten unabhängig voneinander ausgewählt sind aus L³-Q oder aus Z³,
oder
- R¹: steht für unsubstituiertes oder substituiertes, Naphthalen-1-yl, Naphthalen-2-yl, 1,2,3,4-Tetrahydronaphthalen-1-yl, 1, 2 , 3 , 4-Tetrahydronaphthalen-2-yl, 5, 6, 7 , 8-Tetrahydro-naphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, wobei die Substituenten unabhängig voneinander ausgewählt sind aus Z⁴,
oder
- R¹: steht für einen unsubstituierten oder substituierten 5- oder 6-gliedrigen Heteroarylrest, wobei die Substituenten am Kohlenstoff unabhängig voneinander ausgewählt sind aus L³-Q oder aus Z⁵ und die Substituenten am Stickstoff unanhängig voneinander ausgewählt sind aus Z⁶,
oder
- R¹: steht für benzokondensiertes unsubstituiertes oder substituiertes 5- oder 6- gliedriges Heteroaryl, wobei die Substituenten am Kohlenstoff unabhängig voneinander ausgewählt sind aus Z⁷ sind und die Substituenten am Stickstoff unanhängig voneinander ausgewählt aus Z⁸ sind,
oder
- R¹: steht für unsubstituiertes oder substituiertes C₅-C₁₅-Heterocyclyl, wobei die Substituenten am Kohlenstoff unabhängig voneinander ausgewählt sind aus Z⁹ sind und die Substituenten am Stickstoff unanhängig voneinander ausgewählt sind aus Z¹⁰,
- L³: steht für eine direkte Bindung, -O, -C(=O), -S(O)ₘ, -CHR^{L31} oder -NR^{L32},
- m: steht für 0, 1 oder 2,
- R^{L31}: steht für Wasserstoff, C₁-C₄-Alkyl oder C₁-C₄-Halogenalkyl,
- R¹³²: steht für Wasserstoff, C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
- Q: steht für ein Phenyl, das bis zu zwei Substituenten enthalten kann, wobei die Substituenten gleich oder verschieden unabhängig voneinander ausgewählt sind aus folgender Liste: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenaₗkylsulfinyl, C₁-C₄-Alkylsulfonyl, C₁-C₄-Halogenalkylsulfonyl oder Phenyl,
oder
- Q: steht für ein 5- oder 6-gliedrigen Heteroarylrest, das bis zu zwei Substituenten enthalten kann, wobei die Substituenten gleich oder verschieden unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe oder Phenyl,
- Z^{A-1} und Z³: stehen gleich oder verschieden unabhängig voneinander für Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₈-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-Cycloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamⁱno oder C₁-C₆-Halogenalkylsulfonylamⁱno oder SF₅,
- Z¹ und Z²: stehen gleich oder verschieden unabhängig voneinander für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio, C₂-C₄-Alkylcarbonyl, oder C₂-C₆-Alkylcarbonyloxy,
- Z⁴ und Z⁷: stehen gleich oder verschieden unabhängig voneinander für Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
- Z^{A-2} und Z⁵: stehen gleich oder verschieden unabhängig voneinander für Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₃-C₆-Alkenyloxy, C₃-C₆-Alkinyloxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
- Z^{A-3}, Z¹², Z⁶, Z⁸ und Z¹⁰: stehen gleich oder verschieden unabhängig voneinander für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, Phenyl, Benzyl, C₁-C₄-Alkylsulfonyl, C(=O)H, C₂-C₄-Alkylcarbonylalkoxy oder C₁-C₃-Alkylcarbonyl,
- Z^{Y-1} und Z⁹: stehen gleich oder verschieden unabhängig voneinander für Hydroxy, Cyano, Halogen, SH, Amino, Nitro, Oxo, NR³R⁴, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Phenyl, , C -C₆-A|koxy, C₁-C₆-Halogenalkoxy, C₂-C₆-Alkenyloxy, C₂-C₆-Alkinyloxy, C₁-C₆-Alkylthio oder C₁-C₆-Halogenalkylthio, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₂-C₆-Alkylcarbonyloxy, C₂-C₆-Alkylcarbonylthio, C₁-C₄-Alkylsulfinyl, C₁-C₄-Halogenalkylsulfinyl, C₁-C₄-Alkylsulfonyl oder C₁-C₄-Halogenalkylsulfonyl,
sowie Salze, Metallkomplexe und N-Oxide der Verbindungen der Formel (I).

Ein weiterer Gegenstand ist die Verwendung der Verbindungen der Formel (I) als Fungizide.

Erfindungsgemäße Pyridinylcarbonsäure Derivate der Formel (I) sowie deren Salze, Metallkomplexe und N-Oxide eignen sich sehr gut als zur Bekämpfung pflanzenpathogener Schadpilze. Die vorgenannten erfindungsgemäßen Verbindungen zeigen vor allem eine starke fungizide Wirksamkeit und lassen sich sowohl im Pflanzenschutz, im Bereich Haushalt und Hygiene als auch im Materialschutz verwenden.

Die Verbindungen der Formel (I) können sowohl in reiner Form als auch als Mischungen verschiedener möglicher isomerer Formen, insbesondere von Stereoisomeren, wie E- und Z-, threo- und erythro-, sowie optischen Isomeren, wie R- und S-Isomeren oder Atropisomeren, gegebenenfalls aber auch von Tautomeren vorliegen. Es werden sowohl die E- als auch die Z-Isomeren, wie auch die threo- und erythro-, sowie die optischen Isomeren, beliebige Mischungen dieser Isomeren, sowie die möglichen tautomeren Formen beansprucht.

Die Restedefinitionen der erfindungsgemäßen Verbindungen der Formel (I) haben bevorzugt, besonders bevorzugt und ganz besonders bevorzugt folgende Bedeutungen:
- A: steht bevorzugt für Phenyl, das bis zu zwei Substituenten enthalten kann, wobei die Substituenten unabhängig voneinander ausgewählt aus Z^{A-1} sind, oder
- A: steht bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-l-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-l-yl, Pyrazol-3-yl, Pyrazol-4-yl, Imidazol-l-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrazin-2-yl, Pyrazin-3-yl, Pyrimidin-2-yl, Pyrimidin-4-yl oder Pyrimidin-5-yl, der bis zu zwei Substituenten enthalten kann, wobei die Substituenten am Kohlenstoff unabhängig voneinander ausgewählt aus Z^{A-2} sind, und die Substituenten am Stickstoff unabhängig voneinander ausgewählt sind aus Z^{A-3},
- A: steht besonders bevorzugt für Phenyl, das bis zu zwei Substituenten enthalten kann, wobei die Substituenten unabhängig voneinander ausgewählt sind aus folgender Liste:
Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio oder Trifluormethylthio, oder
- A: steht besonders bevorzugt für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-l-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-l-yl, Pyrazol-3-yl, Pyrazol-4-yl, Imidazol-l-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden unabhängig voneinander ausgewählt sind aus folgender Liste:
Substituenten am Kohlenstoff:
   Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio Trifluormethylthio oder Phenyl,
Substituenten am Stickstoff:
   Methyl, Ethyl, Propyl, 1-Methylethyl, 2,2-Trifluorethyl, 2,2-Difluorethyl, 2,2-Dichlor-2-fluorethyl, 2-Chlor-2-difluorethyl oder 2-Chlor-2-fluorethyl,
- A: steht ganz besonders bevorzugt für Pyrazol-1-yl, der bis zu zwei Substituenten enthalten kann, wobei die Substituenten unabhängig voneinander ausgewählt sind aus folgender Liste:
Methyl, Difluormethyl oder Trifluormethyl, oder
- A: steht ganz besonders bevorzugt für Phenyl, der bis zu zwei Substituenten enthalten kann, wobei die Substituenten unabhängig voneinander ausgewählt sind aus folgender Liste: Methyl, Ethyl, Iod, Chlor, Brom, Fluor, Methoxy, Ethoxy, Difluormethyl oder Trifluormethyl,
- L¹: steht bevorzugt für -CH₂-, -CHCH₃-, CH₂CH₂CH₂- oder -CH₂C≡C-,
- L²: steht bevorzugt für, CHR^{L22}, NR^{L21} und besonders bevorzugt für CH₂,
- R^{L21}: steht bevorzugt für Wasserstoff, Methyl, Ethyl oder Cyclopropyl und besonders bevorzugt für Wasserstoff oder Methyl,
- R^{L22}: steht bevorzugt für Wasserstoff oder Methyl und besonders bevorzugt für Wasserstoff,
- Y¹: steht bevorzugt für Sauerstoff oder Schwefel und besonders bevorzugt für Sauerstoff,
- R^{Y2}: steht bevorzugt für Wasserstoff, C₁-C₆-Alkyl, C₂-C₆-Alkenyl, C₂-C₆-Alkinyl, C₁-C₆-Halogenalkyl, C₃-C₆-Cycloalkyl, C₃-C₆-Halogencycloalkyl, Benzyl oder Phenyl,
- oder: die Reste R ^{Y2}, L¹ und R¹ bilden zusammen mit dem Stickstoffatom von Y² ein 5 bis 15-gliedriges, unsubstituiertes, gesättigtes oder teilweise gesättigtes oder ungesättigtes mono-, bi- oder tricyclisches Ringsystem, das bis zu zwei weitere Heteroatome, ausgewählt aus N, O und S enthalten kann, wobei zwei Sauerstoffatome nicht benachbart sind und wobei mögliche Substituenten am Kohlenstoff voneinander ausgewählt sind aus Z^{Y-1}, und wobei die Substituenten am Stickstoff voneinander ausgewählt sind aus Z^{Y-2},
- R^{Y2}: steht besonders bevorzugt für Wasserstoff, Methyl, Ethyl, Propyl, 1-Methylethyl, Prop-2-enyl, 1-Mehylprop-2-enyl, Ethinyl, Prop-2-inyl, 2,2,2-Trifluorethyl, Cyclopropyl, 1-Chlorcyclopropyl, Benzyl oder Phenyl,
oder die Reste R^{Y2}, L¹ und R¹ bilden besonders bevorzugt zusammen mit dem Stickstoffatom von Y² Piperidin, Morpholin, Thiomorpholin, 2,3-Dihydro-4H-1,4-oxazin, 2,3-Dihydro-4H-1,4-benzoxazin, oder 1,2,3,4-Tetrahydrochinolin,
- R^{Y2}: steht ganz besonders bevorzugt für Wasserstoff, Methyl oder Cyclopropyl,
oder die Reste R^{Y2}, L¹ und R¹ bilden ganz besonders bevorzugt zusammen mit dem Stickstoffatom von Y² 2,3-Dihydro-4H-1,4-benzoxazin-4-yl,
- R³, R⁴: stehen bevorzugt gleich oder verschieden unabhängig voneinander für Wasserstoff, Methyl, Ethyl, Propyl, iso-Propyl, Butyl, iso-Butyl oder *tert*-Butyl,
- X: steht bevorzugt für -CR^{X1-} oder Stickstoff und besonders bevorzugt für -CH-, -CF- oder Stickstoff und ganz besonders bevorzugt für -CH- oder Stickstoff,
- R^{X1}: steht bevorzugt für Wasserstoff, Halogen, Cyano, Hydroxy, C₁-C₄-Alkoxy, C₁-C₄-Halogenalkoxy oder C₂-C₄-Carbonylalkoxy,
- R^{G}: steht bevorzugt für Wasserstoff oder Halogen und besonders bevorzugt für Wasserstoff,
- R1: steht bevorzugt für Wasserstoff, C₁-C₆-Alkyl, C₁-C₆-Halogenalkyl, C₂-C₆-Alkenyl oder C₂-C₆-Alkinyl und besonders bevorzugt für Wasserstoff, 1,1-Dimethylethyl, 3,3-Dimethylbutyl, 2,2-Dimethylpropyl, 1,2,2-Trimethylpropyl, n-Pentyl, 1-Ethylpropyl, n-Butyl, 2-Methylpropyl, 1-Methylethyl, Ethyl, n-Propyl, 4-Methylpentyl, n-Hexyl, Trifluoromethyl, Methoxymethyl, Ethoxymethyl, Ethenyl, Prop-2-en-1-yl oder But-3-en-1-yl,
oder
- R¹: steht bevorzugt für unsubstituiertes oder substituiertes C₃-Cₗ₀-Cycloalkyl, wobei die Substituenten unabhängig voneinander ausgewählt sind aus -Q oder aus Z¹, und besonders bevorzugt für Cyclopentyl, Cyclohexyl, Cycloheptyl oder Cyclooctyl, welche jeweils bis zu zwei Substituenten enthalten kann, wobei die Substituenten unabhängig voneinander ausgewählt sind aus folgender Liste:
Cyano, Chlor, Fluor, Brom, Iod, Methyl, Ethyl, Propyl, 1-Methylethyl, 1,1-Dimethylethyl, Ethenyl, 2-Propenyl, 2-Propinyloxy, Phenyl, Methoxy, Ethoxy, Propyloxy, Trifluormethoxy, Ethinyl, 2-Propinyloxy, Methylthio, Ethylthio oder Trifluomethylthio,
- R¹: steht ganz besonders bevorzugt für Cyclopentyl, Cyclohexyl, Cycloheptyl oder Cyclooctyl, das bis zu zwei Substituenten enthalten kann, wobei die Substituenten unabhängig voneinander ausgewählt sind aus folgender Liste: Methyl oder Fluoro,
oder
- R¹: steht bevorzugt für unsubstituiertes oder substituiertes C₅-C₆-Cycloalkenyl, wobei die Substituenten unabhängig voneinander ausgewählt sind aus Z², und besonders bevorzugt für Cyclopentenyl, Cyclohexenyl oder Cycloheptenyl, welche jeweils bis zu zwei Substituenten enthalten kann, wobei die Substituenten unabhängig voneinander ausgewählt sind aus folgender Liste: Methyl, Ethyl, Methoxy, Ethoxy, Trifluormethoxy, Ethinyl, 2-Propinyloxy, Methylthio, Ethylthio oder Trifluormethylthio und ganz besonders bevorzugt für Cyclopent-2-en-1-yl, Cyclopent-3-en-1-yl, Cyclohex-1-en-1-yl, Cyclohex-2-en-1-yl, Cyclohex-3-en-1-yl, Cyclohept-l-en-l-yl, Cyclohept-2-en-1-yl, Cyclohept-3-en-1-yl oder Cyclohept-4-en-1-yl, oder
- R¹: steht bevorzugt für substituiertes Phenyl, wobei die Substituenten unabhängig voneinander ausgewählt sind aus L³-Q oder aus Z³ und besonders bevorzugt für Phenyl, das bis zu drei Substituenten enthalten kann, wobei die Substituenten unabhängig voneinander ausgewählt sind aus folgender Liste: Fluor, Chlor, Brom, Iod, Cyano, Nitro, Methyl, Ethyl, Propyl, 1-Methylethyl, Butyl, 1,1-Dimethylethyl, 1,2-Dimethylethyl, Ethenyl, Ethinyl, Trifluoromethyl, Difluormethyl, Trichlormethyl, Dichlormethyl, Cyclopropyl, Methoxy, Ethoxy, Propoxy, 1-Methylethoxyl, 1,1-Dimethylethoxyl, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 2-Propinyloxy, Methylthio, Ethylthio, Methylsulfinyl oder Methylsulfonyl oder -L3Q, ganz besonders bevorzugt für Phenyl, das bis zu drei Substituenten enthalten kann, wobei die Substituenten unabhängig voneinander ausgewählt sind aus folgender Liste: Fluor, Chlor, Brom, Iod, Cyano, Nitro, Methyl, Ethyl, Propyl, 1-Methylethyl, Butyl, 1,1-Dimethylethyl, 1,2-Dimethylethyl, Ethenyl, Ethinyl, Trifluoromethyl, Difluormethyl, Trichlormethyl, Dichlormethyl, Cyclopropyl, Methoxy, Ethoxy, Propoxy, 1-Methylethoxyl, 1,1-Dimethylethoxyl, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1-Dimethylethoxycarbonyl, 2-Propinyloxy, Methylthio, Ethylthio, Methylsulfinyl oder Methylsulfonyl, oder
- R¹: steht bevorzugt für unsubstituiertes oder substituiertes Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
wobei die Substituenten unabhängig voneinander ausgewählt sind aus Z⁴ sind und besonders bevorzugt für unsubstituiertes oder substituiertes Naphthalen-1-yl, Naphthalen-2-yl, 5,6,7,8-Tetrahydronaphthalen-1-yl oder 5,6,7,8-Tetrahydronaphthalen-2-yl, wobei die Substituenten unabhängig voneinander aus folgender Liste ausgewählt sind: Methyl, Methoxy, Cyano, Fluor, Chlor, Brom, Iod wobei bei der besonders bevorzugten Variante höchstens drei Substituenten enthalten sind und ganz besonders bevorzugt keine Substitutenten vorliegen, oder
- R¹: steht bevorzugt für einen unsubstituierten oder substituierten 5- oder 6-gliedrigen Heteroarylrest, wobei die Substituenten am Kohlenstoff unabhängig voneinander ausgewählt sind aus L³-Q oder aus Z⁵, und die Substituenten am Stickstoff unabhängig voneinander ausgewählt sind aus Z⁶, und besonders bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-l-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-l-yl, Pyrazol-3-yl, Pyrazol-4-yl, Imidazol-l-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus folgender Liste ausgewählt sind:
Substituenten am Kohlenstoff: Chlor, Fluor, Brom, Iod, Cyano, Nitro, Methyl, Ethyl, Propyl, 1-Methylethyl, Butyl, 1,1-Dimethylethyl, Ethenyl, Ethinyl, Trifluormethyl, Difluormethyl, Cyclopropyl, Cyclopentyl, Cyclohexyl, Methylcarbonyl, Ethylcarbonyl, Methoxylcarbonyl, Ethoxycarbonyl, Methoxy, Ethoxy, Propoxy, 1-Methylethoxy, 2-Propinyoxy, Trifluormethoxyl, Methylcabonyloxy, Methylcarbonylthio, Methylthio, Ethylthio, Trifluomethylthio, Methylsulfinyl, Ethylsulfinyl, Trifluoromethylsulfinyl, Methylsulfonyl, Ethylsulfonyl oder Trifluormethylsulfonyl,
Substituenten am Stickstoff: Methyl, Ethyl, Propyl, Cyclopropyl, Cyclohexyl, Phenyl oder 2-Propinyl
und ganz besonders bevorzugt keine Substitutenten an den Heteroarylresten vorliegen, oder
- R¹: steht bevorzugt für benzokondensiertes unsubstituiertes oder substituiertes 5- oder 6-gliedriges Heteroaryl, wobei die Substituenten am Kohlenstoff unabhängig voneinander ausgewählt aus Z⁷ sind, und die Substituenten am Stickstoff unabhängig voneinander ausgewählt sind aus Z⁸ und besonders bevorzugt für Indol-1-yl, Indol-2-yl, Indol-3-yl, Indol-4-yl, Indol-5-yl, Indol-6-yl, Indol-7-yl, Benzimidazol-l-yl, Benzimidazol-2-yl, Benzimidazol-4-yl, Benzimidazol-5-yl, Indazol-l-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, I-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-l-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, Cyclopropyl, Cyclohexyl, Phenyl oder 2-Propinyl,
und ganz besonders bevorzugt keine Substitutenten an den benzokondensierten Heteroarylresten vorliegen, oder
- R¹: steht bevorzugt für unsubstituiertes oder substituiertes C₅-C₁₅-Heterocyclyl, wobei die Substituenten am Kohlenstoff unabhängig voneinander ausgewählt sind aus Z⁹ und die Substituenten am Stickstoff unabhängig voneinander ausgewählt aus Z¹⁰ sind und besonders bevorzugt für Piperidin-l-yl, Piperidin-2-yl, Piperidin-3-yl, Piperidin-4-yl, Piperazin-1-yl, Piperazin-2-yl, Piperazin-3-yl, Morpholin-l-yl, Morpholin-2-yl, Morpholin-3-yl, Tetrahydropyran-2-yl, Tetrahydropyran-3-yl, Tetrahydropyran-4-yl, 1,2,3,4-Tetrahydrochinolin-l -yl, 1,2,3,4-Tetrahydroisochinolin-2-yl, 1,2,3,4-Tetrahydrochinoxalin-1-yl, Indolin-l-yl, Isoindolin-2-yl, Decahydrochinolin-1-yl oder Decahydroisochinolin-2-yl, welche jeweils bis zu zwei Substituenten enthalten kann, wobei die Substituenten unabhängig voneinander ausgewählt sind aus folgender Liste:
Substituenten am Kohlenstoff: Fluor, Chlor, Brom, Iod, Methyl, Methoxy, 2-Propinyloxy, 2-Propenyloxy,
Substituenten am Stickstoff: Methyl, Ethyl, Propyl, Cyclopropyl, Cyclohexyl, Phenyl oder 2-Propinyl,
und ganz besonders bevorzugt keine Substitutenten an den Heterocyclylresten vorliegen,
- Q: steht bevorzugt für Phenyl, das bis zu zwei Substituenten enthalten kann, wobei die Substituenten unabhängig voneinander gleich oder verschieden ausgewählt sind aus folgender Liste:
Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio, und
- Q: steht besonders bevorzugt für unsubstituiertes Phenyl, und Q steht ganz besonders bevorzugt nur für unsubstituiertes Phenyl, oder
- Q: steht bevorzugt für Furan-2-yl, Furan-3-yl, Thiophen-2-yl, Thiophen-3-yl, Isoxazol-3-yl, Isoxazol-4-yl, Isoxazol-5-yl, Pyrrol-l-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-l-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, Tetrazol-5-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Tetrazol-5-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl oder 1,2,4-Triazin-3-yl, das bis zu zwei Substituenten enthalten kann, wobei die Substituenten unabhängig voneinander ausgewählt sind aus folgender Liste:
Substituenten am Kohlenstoff:
   Fluor, Chlor, Brom, Iod, Cyano, Hydroxy, SH, Nitro, C₁-C₆-Alkyl, C₁-C₆-Halogenalkyl, C₃-C₆-Cycloalkyl, C₁-C₄-Alkoxy, C₁-C₄-Halogenalkoxy, C₁-C₄-Alkylthio oder C₁-C₄-Halogenalkylthio,
Substituenten am Stickstoff: C₁-C₆-Alkyl, C₂-C₆-Alkenyl, C₂-C₆-Alkinyl, C₃-C₆-Cycloalkyl oder Phenyl,
- Z^{A-1}: steht bevorzugt für Halogen, Cyano, Hydroxy, Amino, Nitro, C₁-C₆-Alkyl, C₂-C₆-Alkenyl, C₂-C₆-Alkinyl, C₃-C₈-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₁-C₄-Hydroxyalkyl, C₂-C₅-Alkylcarbonyl, C₂-C₆-Alkoxycarbonyl, C₂-C₆-Alkylcarbonyloxy oder C(=O)H,
- Z^{A-2}: steht bevorzugt für Halogen, Cyano, Hydroxy, Amino, Nitro, C₁-C₆-Alkyl, C₂-C₆-Alkenyl, C₂-C₆-Alkinyl, C₃-C₆-Cycloalkyl, C₁-C₆-Halogenalkyl, C₂-C₆-Halogenalkenyl, C₂-C₆-Halogenalkinyl, C₃-C₆-Halogencycloalkyl, C₁-C₄-Alkoxy, C₁-C₄-Halogenalkoxy, C₁-C₄-Alkylthio, C₁-C₄-Alkylsulfonyl, C₁-C₄-Halogenalkylthio, C₁-C₄-Halogenalkylsulfonyl, C₂-C₄-Alkoxyalkyl, C₁-C₄-Hydroxyalkyl, C₂-C₄-Alkylcarbonyl, C₂-C₆-Alkoxycarbonyl oder C₂-C₆- Alkylcarbonyloxy,
- Z^{A-3}, Z^{Y-2}, Z⁶, Z⁸ und Z¹⁰: stehen gleich oder verschieden unabhängig voneinander bevorzugt für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₄-C₁₀-Cycloalkylalkyl, Phenyl, Benzyl, C₁-C₄-Alkylsulfonyl, C(=O)H, oder C₁-C₃-Alkylcarbonyl,
- Z^{Y-1} und Z⁹: stehen gleich oder verschieden unabhängig voneinander bevorzugt für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Phenyl, C₁-C₆-Alkoxy, C₁-C₆-Halogenalkoxy, C₂-C₆-Alkenyloxy, C₂-C₆-Alkinyloxy, C₁-C₆-Alkylthio, NR³R⁴, C₂-C₄-Alkylcarbonyl, C₂-C₆-Alkoxycarbonyl, C₃-C₈-Dialkylaminocarbonyl, C₂-C₆-Alkylcarbonyloxy, oder C₁-C₆-Halogenalkylthio,
- Z¹ und Z²: stehen gleich oder verschieden unabhängig voneinander bevorzugt für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Hydroxy, Oxo, C₁-C₆-Alkoxy, C₁-C₆-Halogenalkoxy, C₃-C₆-Alkenyloxy, C₃-C₆-Alkinyloxy, C₁-C₆-Alkylthio oder C₁-C₆-Halogenalkylthio,
- Z³: steht bevorzugt für Halogen, Cyano, Hydroxy, Nitro, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloakyl, C₃-C₈-Halogencycloalkyl, C₃-C₈-Cycloalkenyl, C₃-C₈-Halogencycloalkenyl, C₂-C₆-Alkoxyalkyl, C₂-C₆-Alkylarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₂-C₆-Alkylaminocarbonyl, C₃-C₈-Dialkylaminocarbonyl, C₄-C₈-Cycloalkylaminocarbonyl, C₁-C₆-Alkoxy, C₁-C₆-Halogenalkoxy, C₃-C₈-Cycloalkoxy, C₃-C₈-Halogencycloalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfonyl, C₁-C₆-Halogenalkylsulfonyl, C₃-C₈-Cycloalkylsulfonyl, C₁-C₆-Alkylsulfonylamino, C₁-C₆-Halogenalkylsulfonylamino,
- Z⁴ und Z⁷: stehen gleich oder verschieden unabhängig voneinander bevorzugt für Halogen, Cyano, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfonyl, C₁-C₄-Halogenalkylsulfonyl,
- Z⁵: steht bevorzugt für Halogen, Cyano, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₂-C₄-Alkoxyalkyl, C₃-C₆-Alkenyloxy, C₃-C₆-Alkinyloxy, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfbnyl, C₁-C₄- Halogenalkylsulfonyl.

Die erfindungsgemäß verwendbaren Pyridinylcarbonsäure Derivate sind durch die Formel (I) allgemein definiert. Die Restedefinitionen der vorstehenden und nachfolgend genannten Restedefinitionen der Formel (I) gelten für die Endprodukte der Formel (I) wie für alle Zwischenprodukte (siehe auch unten unter "Erläuterungen der Verfahren und Zwischenprodukte") gleichermaßen.

Die oben aufgeführten bzw. nachfolgenden allgemeinen oder in Vorzugsbereichen aufgeführten Restedefinitionen bzw. Erläuterungen können auch untereinander, also zwischen den jeweiligen Bereichen und Vorzugsbereichen beliebig kombiniert werden. Sie gelten für die Endprodukte sowie für die Vor- und Zwischenprodukte entsprechend. Außerdem können einzelne Definitionen entfallen.

Bevorzugt sind solche Verbindungen der Formel (I), in welcher alle Reste jeweils die oben genannten bevorzugten Bedeutungen haben.

Besonders bevorzugt sind solche Verbindungen der Formel (I), in welcher alle Reste jeweils die oben genannten besonders bevorzugten Bedeutungen haben.

Ganz besonders bevorzugt sind solche Verbindungen der Formel (I), in welcher alle Reste jeweils die oben genannten ganz besonders bevorzugten Bedeutungen haben.

Weiterhin bevorzugt sind Verbindungen der Formel (I), in welcher A für 3,5-Bis(difluormethyl)-1H-pyrazol-1-yl steht.

Weiterhin bevorzugt sind Verbindungen der Formel (I), in welcher A für 5-Methyl-3-(trifluormethyl)-1H-pyrazol-1-yl steht.

Weiterhin bevorzugt sind Verbindungen der Formel (I), in welcher Y¹ für Sauerstoff steht.

Weiterhin bevorzugt sind Verbindungen der Formel (I), in welcher Y² für Sauerstoff steht.

Weiterhin bevorzugt sind Verbindungen der Formel (I), in welcher Y² für Schwefel steht.

Weiterhin bevorzugt sind Verbindungen der Formel (I), in welcher Y³ für Sauerstoff steht.

Weiterhin bevorzugt sind Verbindungen der Formel (I), in welcher X für CH steht.

Weiterhin bevorzugt sind Verbindungen der Formel (I), in welcher X für Stickstoff steht.

Weiterhin bevorzugt sind Verbindungen der Formel (I), in welcher R^{G} für Wasserstoff steht.

Weiterhin bevorzugt sind Verbindungen der Formel (I), in welcher L¹ für -CH₂- steht.

Weiterhin bevorzugt sind Verbindungen der Formel (I), in welcher L² für -CH₂- steht.

Weiterhin bevorzugt sind Verbindungen der Formel (I), in welcher R¹ für Cyclohexyl steht.

Weiterhin bevorzugt sind Verbindungen der Formel (I), in welcher R¹ für 1,2,3,4-Tetrahydronaphthalen-1-yl steht.

Weiterhin bevorzugt sind Verbindungen der Formel (I), in welcher R¹ für (1R)-1,2,3,4-Tetrahydronaphthalen-1-yl steht.

Weiterhin bevorzugt sind Verbindungen der Formel (I), in welcher R¹ für Naphthalen-1 -yl steht.

Weiterhin bevorzugt sind Verbindungen der Formel (I), in welcher R¹ für Naphthalen-2-yl steht.

Weiterhin bevorzugt sind Verbindungen der Formel (I), in welcher R¹ für 2,3-Dihydro-1H-inden-1-yl steht.

Weiterhin bevorzugt sind Verbindungen der Formel (I), in welcher R¹ für 2-Bromphenyl steht.

Weiterhin bevorzugt sind Verbindungen der Formel (I), in welcher R¹ für 2,6-Difluorphenyl steht.

Weiterhin bevorzugt sind Verbindungen der Formel (I), in welcher R¹ für 2-(Trifluormethoxy)phenyl steht.

Weiterhin bevorzugt sind Verbindungen der Formel (I), in welcher R¹ für 2-(Trifluormethyl)phenyl steht.

Weiterhin bevorzugt sind Verbindungen der Formel (I), in welcher R¹ für 2-Chlorphenyl steht.

Weiterhin bevorzugt sind Verbindungen der Formel (I), in welcher R¹ für 2,4-Dichlorphenyl steht.

Weiterhin bevorzugt sind Verbindungen der Formel (I), in welcher R¹ für Pyridin-2-yl steht.

Weiterhin bevorzugt sind Verbindungen der Formel (I), in welcher R¹ für Thiophen-2-yl steht.

Weiterhin bevorzugt sind Verbindungen der Formel (I), in welcher R¹ für Chinolin-8-yl steht.

Weiterhin bevorzugt sind Verbindungen der Formel (I), in welcher R¹ für Cyclohex-2-en-1-yl steht.

Weiterhin bevorzugt sind Verbindungen der Formel (I), in welcher R¹ für 2,4-Difluorphenyl steht.

Weiterhin bevorzugt sind Verbindungen der Formel (I), in welcher R¹ für 2,4,6-Trifluorphenyl steht.

Weiterhin bevorzugt sind Verbindungen der Formel (I), in welcher R¹ für 2-Methylphenyl steht.

Weiterhin bevorzugt sind Verbindungen der Formel (I), in welcher R¹ für 2-(Trifluormethyl)phenyl steht.

Weiterhin bevorzugt sind Verbindungen der Formel (I), in welcher R¹ für 2-Fluorphenyl steht.

Weiterhin bevorzugt sind Verbindungen der Formel (I), in welcher R¹ für 4-Fluorphenyl steht.

Weiterhin bevorzugt sind Verbindungen der Formel (I), in welcher R¹ für 1,2,3,4-Tetrahydronaphthalen-8-yl steht.

Weiterhin bevorzugt sind Verbindungen der Formel (I), in welcher R¹ für 2-Fluor cyclohexyl steht.

Weiterhin bevorzugt sind Verbindungen der Formel (I), in welcher R¹ für 3-Methylpyridin-2-yl steht.

Weiterhin bevorzugt sind Verbindungen der Formel (I), in welcher R¹ für 4-Mathylthiophen-2-yl steht.

Weiterhin bevorzugt sind Verbindungen der Formel (I), in welcher R¹ für 2-Methylcyclohexyl steht.

Weiterhin bevorzugt sind Verbindungen der Formel (I), in welcher Y²L¹R¹ für 2,3-Dihydro-4H-1,4-benzoxazin-4-yl steht.

Weiterhin bevorzugt sind Verbindungen der Formel (I), in welcher R¹ für CF₃ und L¹ für -(CH₂)₃-stehen.

Weiterhin bevorzugt sind Verbindungen der Formel (I), in welcher R¹ für Pentyl und L¹ für - CH(CH₃)- stehen.

Weiterhin bevorzugt sind Verbindungen der Formel (I), in welcher R¹ für Butyl und L¹ für - (CH₂)₃- stehen.

Weiterhin bevorzugt sind Verbindungen der Formel (I), in welcher R¹ für *tert-*Butyl und L¹ für - CH₂- stehen.

Weiterhin bevorzugt sind Verbindungen der Formel (I), in welcher R¹ für But-3-en-1-yl und L¹ für -(CH₂)₂- stehen.

Weiterhin bevorzugt sind Verbindungen der Formel (I), in welcher R¹ für Pentyl und L¹ für - (CH₂)₂- stehen.

Weiterhin bevorzugt sind Verbindungen der Formel (I), in welcher R¹ für Methoxymethyl und L¹ für -CH₂- stehen.

Weiterhin bevorzugt sind Verbindungen der Formel (Ix): 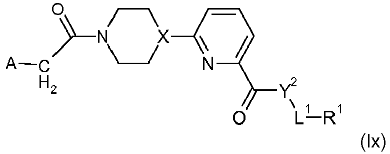 in welcher A, X, Y², L¹ und R¹ jeweils unabhängig voneinander die oben die oben bei der Verbindung der Formel (I) angegebenen allgemeinen, bevorzugten, besonders bevorzugten oder ganz besonders bevorzugten Bedeutungen haben,

Die zuvor genannten Reste-Definitionen können untereinander in beliebiger Weise kombiniert werden. Außerdem können einzelne Definitionen entfallen.

Je nach Art der oben definierten Substituenten weisen die Verbindungen der Formel (I) saure oder basische Eigenschaften auf und können mit anorganischen oder organischen Säuren oder mit Basen oder mit Metallionen Salze, gegebenenfalls auch innere Salze oder Addukte bilden. Tragen die Verbindungen der Formel **(I)** Amino, Alkylamino oder andere, basische Eigenschaften induzierende Gruppen, so können diese Verbindungen mit Säuren zu Salzen umgesetzt werden oder fallen durch die Synthese direkt als Salze an. Tragen die Verbindungen der Formel **(I)** Hydroxy, Carboxy oder andere, saure Eigenschaften induzierende Gruppen, so können diese Verbindungen mit Basen zu Salzen umgesetzt werden. Geeignete Basen sind beispielsweise Hydroxide, Carbonate, Hydrogencarbonate der Alkali- und Erdalkalimetalle, insbesondere die von Natrium, Kalium, Magnesium und Calcium, weiterhin Ammoniak, primäre, sekundäre und tertiäre Amine mit C₁-C₄-Alkyl-Gruppen, Mono-, Di- und Trialkanolamine von C₁-C₄-Alkanolen, Cholin sowie Chlorcholin.

Die so erhältlichen Salze weisen ebenfalls fungizide Eigenschaften auf.

Beispiele für anorganische Säuren sind Halogenwasserstoffsäuren wie Fluorwasserstoff, Chlorwasserstoff, Bromwasserstoff und Iodwasserstoff, Schwefelsäure, Phosphorsäure und Salpetersäure und saure Salze wie NaHSO₄ und KHSO₄. Als organische Säuren kommen beispielsweise Ameisensäure, Kohlensäure und Alkansäuren wie Essigsäure, Trifluoressigsäure, Trichloressigsäure und Propionsäure sowie Glycolsäure, Thiocyansäure, Milchsäure, Bernsteinsäure, Zitronensäure, Benzoesäure, Zimtsäure, Oxal-säure, gesättigte oder einfach oder doppelt ungesättigte C₆-C₂₀-Fettsäuren, Alkylschwefelsäuremonoester, Alkylsulfonsäuren (Sulfonsäuren mit geradkettigen oder verzweigten Alkylresten mit 1 bis 20 Kohlenstoffatomen), Arylsulfonsäuren oder Aryldisulfonsäuren (aromatische Reste wie Phenyl und Naphthyl welche ein oder zwei Sulfonsäuregruppen tragen), Alkylphosphonsäuren (Phosphonsäuren mit geradkettigen oder verzweigten Alkylresten mit 1 bis 20 Kohlenstoffatomen), Arylphosphonsäuren oder Aryldiphosphonsäuren (aromatische Reste wie Phenyl und Naphthyl welche ein oder zwei Phosphonsäurereste tragen), wobei die Alkyl- bzw. Arylreste weitere Substituenten tragen können, z.B. p-Toluolsulfonsäure, Salicylsäure, p-Aminosalicylsäure, 2-Phenoxybenzoesäure, 2-Acetoxybenzoesäure etc.

Als Metallionen kommen insbesondere die Ionen der Elemente der zweiten Hauptgruppe, insbesondere Calzium und Magnesium, der dritten und vierten Hauptgruppe, insbesondere Aluminium, Zinn und Blei, sowie der ersten bis achten Nebengruppe, insbesondere Chrom, Mangan, Eisen, Kobalt, Nickel, Kupfer, Zink und andere in Betracht. Besonders bevorzugt sind die Metallionen der Elemente der vierten Periode. Die Metalle können dabei in den verschiedenen ihnen zukommenden Wertigkeiten vorliegen.

Gegebenenfalls substituierte Gruppen können einfach oder mehrfach substituiert sein, wobei bei Mehrfachsubstitutionen die Substituenten gleich oder verschieden sein können.

Bei den in den vorstehenden Formeln angegebenen Definitionen der Symbole wurden Sammelbegriffe verwendet, die allgemein repräsentativ für die folgenden Substituenten stehen:
Halogen: Fluor, Chlor, Brom und Iod;
Alkyl: gesättigte, geradkettige oder verzweigte Kohlenwasserstoffreste mit 1 bis 8 Kohlenstoffatomen, z.B. (aber nicht beschränkt auf) C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl,1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und l-Ethyl-2-methylpropyl;
**Alkenyl:** ungesättigte, geradkettige oder verzweigte Kohlenwasserstoffreste mit 2 bis 8 Kohlenstoffatomen und einer Doppelbindung in einer beliebigen Position, z.B. (aber nicht beschränkt auf) C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, l-Ethyl-2-methyl-1-propenyl und 1-Ethyl-2-methyl-2-propenyl;
**Alkinyl:** geradkettige oder verzweigte Kohlenwasserstoffgruppen mit 2 bis 8 Kohlenstoffatomen und einer Dreifachbindung in einer beliebigen Position, z.B. (aber nicht beschränkt auf) C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1 -Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Alkoxy:** gesättigte, geradkettige oder verzweigte Alkoxyreste mit 1 bis 8 Kohlenstoffatomen, z.B. (aber nicht beschränkt auf) C₁-C₆-Alkoxy wie Methoxy, Ethoxy, Propoxy, 1-Methylethoxy, Butoxy, 1-Methyl-propoxy, 2-Methylpropoxy, 1,1-Dimethylethoxy, Pentoxy, 1-Methylbutoxy, 2-Methylbutoxy, 3-Methylbutoxy, 2,2-Di-methylpropoxy, 1-Ethylpropoxy, Hexoxy, 1,1-Dimethylpropoxy, 1,2-Dimethylpropoxy,1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methyl-propoxy;
**Alkylthio:** gesättigte, geradkettige oder verzweigte Alkylthioreste mit 1 bis 8 Kohlenstoffatomen, z.B. (aber nicht beschränkt auf) C₁-C₆-Alkylthio wie Methylthio, Ethylthio, Propylthio, 1-Methylethylthio, Butylthio, 1-Methyl-propylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio,1-Methylpentylthio, 2-Methylpentylthio, 3-Methyl-pentylthio, 4-Methylpentylthio, 1,1-Dimethylbutylthio, 1,2-Dimethylbutylthio, 1,3-Dimethyl-butylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropyl-thio und l-Ethyl-2-methylpropylthio;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Alkylsulfinyl:** gesättigte, geradkettige oder verzweigte Alkylsulfinylreste mit 1 bis 8 Kohlenstoffatomen, z.B. (aber nicht beschränkt auf) C₁-C₆-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Di-methylpropylsulfinyl, 1-Ethylpropylsulfinyl, Hexylsulfinyl, 1,1-Dimethylpropylsulfinyl, 1,2-Dimethylpropylsulfinyl,1-Methylpentylsulfinyl, 2-Methylpentyl-sulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
**Alkylsulfonyl:** gesättigte, geradkettige oder verzweigte Alkylsulfonylreste mit 1 bis 8 Kohlenstoffatomen, z.B. (aber nicht beschränkt auf) C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Di-methylpropylsulfonyl, 1-Ethylpropylsulfonyl, Hexylsulfonyl, 1,1-Dimethylpropylsulfonyl, 1,2-Dimethylpropylsulfonyl, 1 -Methylpentylsulfonyl, 2-Methylpentyl-sulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
**Cycloalkyl:** monocyclische, gesättigte Kohlenwasserstoffgruppen mit 3 bis 10 Kohlenstoffringgliedern, z.B. (aber nicht beschränkt auf) Cyclopropyl, Cyclopentyl und Cyclohexyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor,2-difluorethyl, 2,2-Dichlor-2-fluorethyl, 2,2,2-Trichlorethyl, Pentafluorethyl und 1,1,1-Trifluorprop-2-yl;
**Halogenalkoxy:** geradkettige oder verzweigte Alkoxygruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkoxy wie Chlormethoxy, Brommethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor,2-difluorethoxy, 2,2-Dichlor-2-fluorethoxy, 2,2,2-Trichlorethoxy, Pentafluor-ethoxy und 1,1,1-Trifluorprop-2-oxy;
**Halogenalkylthio:** geradkettige oder verzweigte Alkylthiogruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. (aber nicht beschränkt auf) C₁-C₃-Halogenalkylthio wie Chlormethylthio, Brommethylthio, Dichlormethylthio, Trichlormethylthio, Fluormethylthio, Difluormethylthio, Trifluormethylthio, Chlorfluormethylthio, Dichlorfluor-methylthio, Chlordifluormethylthio, 1-Chlorethylthio, 1-Bromethylthio, 1-Fluorethylthio, 2-Fluorethylthio, 2,2-Difluorethylthio, 2,2,2-Trifluorethylthio, 2-Chlor-2-fluorethylthio, 2-Chlor,2-difluorethylthio, 2,2-Dichlor-2-fluorethylthio, 2,2,2-Trichlorethylthio, Pentafluorethylthio und 1,1,1-Trifluorprop-2-ylthio;
**Heteroaryl:** 5 oder 6-gliedriges, vollständig ungesättigtes monocyclisches Ringsystem, enthaltend ein bis vier Heteroatome aus der Gruppe Sauerstoff, Stickstoff oder Schwefel, enthält der Ring mehrere Sauerstoffatome, so stehen diese nicht direkt benachbart;
**5-gliedriges Heteroaryl: enthaltend ein bis vier Stickstoffatome oder ein bis drei Stickstoffatome und ein Schwefel- oder Sauerstoffatom:** 5-Ring Heteroarylgruppen, welche neben Kohlenstoffatomen ein bis vier Stickstoffatome oder ein bis drei Stickstoffatome und ein Schwefel-oder Sauerstoffatom als Ringglieder enthalten können, z.B. (aber nicht beschränkt auf) 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5- Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
**über Stickstoff gebundenes 5-gliedriges Heteroaryl, enthaltend ein bis vier Stickstoffatome, oder über Stickstoff gebundenes benzokondensiertes 5-gliederiges Heteroaryl, enthaltend ein bis drei Stickstoffatome:** 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, in der ein oder zwei C-Atome durch N-Atome ersetzt sein können, in der ein oder zwei C-Atome durch N-Atome ersetzt sein können, wobei diese Ringe über eines der Stickstoffringglieder an das Gerüst gebunden sind, z.B. (aber nicht beschränkt auf) 1-Pyrrolyl, 1-Pyrazolyl, 1,2,4-Trazol-1-yl, 1-Imidazolyl, 1,2,3-Triazol-1-yl und 1,3,4-Triazol-1-yl;
**6-gliedriges Heteroaryl, enthaltend ein bis vier Stickstoffatome:** 6-Ring Heteroarylgruppen, welche neben Kohlenstoffatomen ein bis drei bzw. ein bis vier Stickstoffatome als Ringglieder enthalten können, beispielsweise (aber nicht beschränkt auf) 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
**Benzokondensiertes 5-gliedriges Heteroaryl, enthaltend ein bis drei Stickstoffatome oder ein Stickstoffatom und ein Sauerstoff- oder Schwefelatom:** z.B. (aber nicht beschränkt auf) Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1,3-Benzothiazol-2-yl, 1,3-Benzothiazol-4-yl, 1,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl und 1,3-Benzoxazol-7-yl,
**Benzokondensiertes 6-gliedriges Heteroaryl, enthaltend ein bis drei Stickstoffatome:** : z.B. (aber nicht beschränkt auf) Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl, und Isochinolin-8-yl;
**Heterocyclyl:** drei- bis fünfzehngliedriger gesättigter oder partiell ungesättigter Heterocyclus, enthaltend ein bis vier Heteroatome aus der Gruppe Sauerstoff, Stickstoff oder Schwefel: mono-, bi- oder tricyclische Heterocyclen enthaltend neben Kohlenstoffringgliedern ein bis drei Stickstoffatome und/oder ein Sauerstoff- oder Schwefelatom oder ein oder zwei Sauerstoff- und/oder Schwefelatome; enthält der Ring mehrere Sauerstoffatome, so stehen diese nicht direkt benachbart; wie z.B. (aber nicht beschränkt auf) Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2, 3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3 ,4-Dihydropyrazol-4-yl, 3,4-Dihydropyrazol-5-yl, 4,5-Dihydroopyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
**Abgangsgruppe:** S_{N}1 oder S_{N}2-Abgangsgruppe, beispielsweise Chlor, Brom, Iod, Alkylsulfonate (-OSO₂-Alkyl, z.B. -OSO₂CH₃, -OSO₂CF₃) oder Arylsulfonate (-OSO₂-Aryl, z.B. -OSO₂Ph, - OSO₂PhMe );
Bei der Nennung von Kombinationen von mehreren Resten, wie zum Beispiel Cx-Cy-Alkylcarbonyl oder Cx-Cy-Alkoxyalkyl, bezeichnet der Ausdruck Cx-Cy jeweils die Summe aller Kohlenstoffatome, die jeweils in dem gesamten Fragment vorhanden sind. X und Y stehen dabei jeweils für eine ganze Zahl, wobei die Zahl Y größer ist als diejenige von X.

Nicht umfasst sind solche Kombinationen, die den Naturgesetzen widersprechen und die der Fachmann daher aufgrund seines Fachwissens ausgeschlossen hätte. Beispielsweise sind Ringstrukturen mit drei oder mehreren benachbarten O-Atomen ausgeschlossen.

### Erläuterung der Herstellverfahren und Zwischenprodukte

Die Pyridinylcarbonsäuere Derivate der Formel (I) lassen sich auf unterschiedliche Weise herstellen. Im Folgenden sind die möglichen Verfahren zunächst schematisch dargestellt. Wenn nicht anders angegeben haben die angegebenen Reste die oben angegebenen Bedeutungen.

Die erfindungsgemäßen Verfahren zur Herstellung von Verbindungen der Formel (I) werden gegebenenfalls unter Verwendung eines oder mehrerer Reaktionshilfsmittel durchgeführt.

Als Reaktionshilfsmittel kommen gegebenenfalls anorganische oder organische Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calciumhydroxid, Natrium- oder Kalium-methanolat, -ethanolat, -n- oder -i-propanolat, -n-, -i-, -s- oder - tButanolat; weiterhin auch basische organische Stickstoffverbindungen, wie beispielsweise Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl-und 3,5-Dimethyl-pyridin, 5-Ethyl-2-methyl-pyridin, 4-Dimethylamino-pyridin, N-Methyl-piperidin, 1,4-Diazabicyclo[2,2,2]-octan (DABCO), 1,5-Diazabicyclo[4,3,0]-non-5-en (DBN), oder 1,8 Diazabicyclo[5,4,0]-undec-7-en (DBU).

Die erfindungsgemäßen Verfahren werden gegebenenfalls unter Verwendung eines oder mehrerer Verdünnungsmittel durchgeführt. Als Verdünnungsmittel kommen praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-und Dibutylether, Glykoldimethylether und Diglykoldimethylether, Tetrahydrofuran und Dioxan, Ketone wie Aceton, Methylethyl-, Methyl-isopropyl-oder Methyl-isobutyl-keton, Ester wie Essigsäuremethylester oder - ethylester, Nitrile wie z.B. Acetonitril oder Propionitril, Amide wie z.B. Dimethylformamid, Dimethylacetamid und N-Methyl-pyrrolidon, sowie Dimethylsulfoxid, Tetramethylensulfon und Hexamethylphosphorsäuretriamid und DMPU.

Die Reaktionstemperaturen können bei den erfindungsgemäßen Verfahren in einem größeren Bereich variiert werden. Im Allgemeinen arbeitet man bei Temperaturen zwischen 0 °C und 250 °C, vorzugsweise bei Temperaturen zwischen 10 °C und 185 °C.

Die Reaktionszeit variiert in Abhängigkeit vom Maßstab der Reaktion und der Reaktionstemperatur, liegt aber im Allgemeinen zwischen einigen Minuten und 48 Stunden.

Die erfindungsgemäßen Verfahren werden im Allgemeinen unter Normaldruck durchgeführt. Es ist jedoch auch möglich, unter erhöhtem oder vermindertem Druck zu arbeiten.

Zur Durchführung der erfindungsgemäßen Verfahren werden die jeweils benötigten Ausgangsstoffe im Allgemeinen in angenähert äquimolaren Mengen eingesetzt. Es ist jedoch auch möglich, eine der jeweils eingesetzten Komponenten in einem größeren Überschuss zu verwenden.

### Verfahren A

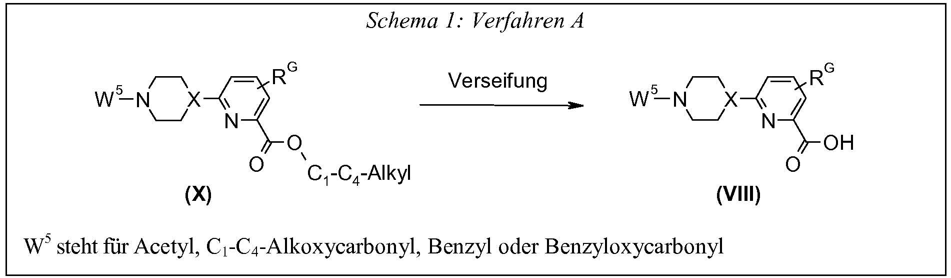

Eine Möglichkeit das Zwischenprodukt **(VIII)** aus entsprechenden Verbindungen **(X)** darzustellen ist in Schema 1 gezeigt.

Die Verbindungen der Fomeln **(X)** mit können aus kommerziell erhältlichen Vorstufen nach literaturbeschriebenen Vorschriften hergestellt werden (s. z.B. US-A-2009/0197859, WO-A-2010/008739).

Die Carbonsäure der Formel **(VIII)** kann durch Verseifung des entsprechenden C₁-C₄-Alkylesters der Formel **(X)** dargestellt werden. Zum Beispiel kann die Methode, die in WO-A-2007/014290 beschrieben wird, verwendet werden.

Geeignete Alkalimetallhydroxide sind beispielsweise LiOH, NaOH oder KOH, gewöhnlich in der Gegenwart von Wasser zusammen mit einem Cosolvens, bevorzugt THF und/oder Methanol, um das Lösen des Esters zu vereinfachen. Das entstehende Carboxylatsalz wird in die freie Säure durch das Behandeln mit einem geringen Überschuss an Mineralsäuren, wie zum Beispiel Salzsäure oder Schwefelsäure überführt.

Die Reaktion wird normalerweise bei Temperaturen von 0 °C - 60 °C durchgeführt, aber sie kann auch bei Rückflusstemperatur der Reaktionsmischung durchgeführt werden. Die Reaktionszeit variiert in Abhängigkeit vom Maßstab der Reaktion und der Reaktionstemperatur, aber liegt im Allgemeinen zwischen einigen Minuten und 48 Stunden.

Nach Beendigung der Reaktion werden die Verbindungen **(VIII)** von der Reaktionsmischung durch eine der üblichen Trenntechniken getrennt. Falls notwendig werden die Verbindungen durch Umkristallisation gereinigt oder können gegebenenfalls auch im nächsten Schritt ohne vorhergehende Reinigung eingesetzt werden.

### Verfahren B

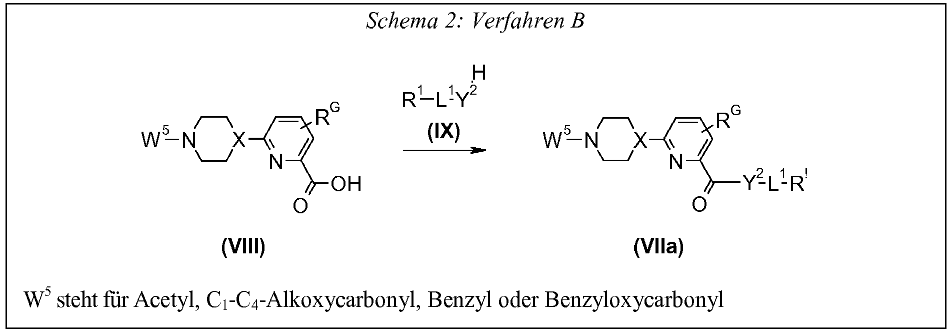

Eine Möglichkeit Verbindungen der Formel **(VIIa)** aus entsprechenden Verbindungen **(VIII)** darzustellen ist in Schema 2 gezeigt.

Eine Verbindung der Formel **(VIIa)** kann aus der entsprechenden Verbindung der Formel **(VIII)** mit einem Substrat der Formel **(IX)** in Gegenwart eines Kupplungsreagenzes synthetisiert werden, analog zu in der Literatur beschriebenen Vorschriften (z.B. Tetrahedron 2005, 61, 10827-10852, und darin zitierte Referenzen).

Geeignete Kupplungsreagenzien sind beispielsweise Peptidkupplungsreagenzien (wie etwa N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyluroniumhexafluorophosphat, etc.)

Gegebenenfalls kann eine Base, wie z.B. Triethylamin oder Hünig-Base in der Reaktion verwendet werden.

Die bevorzugten Lösungsmittel sind *N,N*-Dimethylformamid und Dichlormethan.

Alternativ kann eine Verbindung der Formel **(VIIa)** auch ausgehend von der Verbindung der Formel **(VIII)** durch eine zweistufige Transformation unter Verwendung literaturbekannter Prozesse (z.B. Tetrahedron 2005, 61, 10827-10852, und darin zitierte Literatur) synthetisiert werden, gegebenenfalls in der Gegenwart eines Säurefängers / Base. Typischerweise wird eine Verbindung der Formel **(VIII)** zunächst in das korrespondierende Säurehalogenid oder -sulfonat überführt, dann erfolgt eine Kupplungsreaktion mit einem Substrat der Formel **(IX).**

Substrate mit der allgemeinen Formel **(IX)** sind kommerziell erhältlich oder durch in der Literatur beschriebene Prozesse darstellbar (siehe z.B. "The Chemistry of Functional groups"; "The Chemistry of the Thiol Group"; John Wiley & Sons, 1974, 163-269, und darin zitierte Referenzen; "The Chemistry of Functional groups"; "Supplement F2: The Chemistry of amino, nitroso, nitro and related groups"; John Wiley & Sons, und darin zitierte Referenzen; "Science of Synthesis"; "Alcohols", Volume 36, Thieme, 2008 und darin zitierte Referenzen; "Science of Synthesis"; "Amines and Ammonium Salts", Volume 40a, Thieme, 2008, und darin zitierte Referenzen).

Wenigstens ein Äquivalent eines Säurefängers / einer Base (z.B. Hünig-Base, Triethylamin oder kommerziell erhältliche polymere Säurefänger) im Verhältnis zum Startmaterial der allgemeinen Formel **(IX)** wird verwendet. Ist das Startmaterial ein Salz, werden wenigstens zwei Äquivalente des Säurefängers benötigt.

Nach Beendigung der Reaktion werden die Verbindungen **(VIIa)** von der Reaktionsmischung durch eine der üblichen Trenntechniken getrennt. Falls notwendig werden die Verbindungen durch Umkristallisation oder Chromatographie gereinigt oder können gegebenenfalls auch im nächsten Schritt ohne vorhergehende Reinigung eingesetzt werden.

### Verfahren C

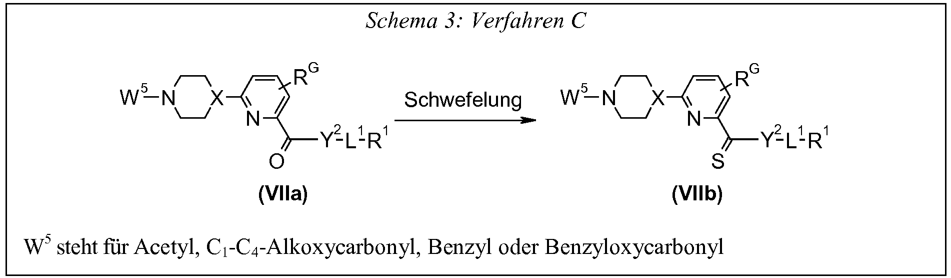

Eine Möglichkeit, Verbindungen der Formel **(VIIb)** aus entsprechenden Verbindungen **(VIIa)** darzustellen, ist in Schema 3 gezeigt.

Das erfindungsgemäße Verfahren C wird vorzugsweise unter Verwendung eines oder mehrerer Verdünnungsmittel durchgeführt. Die bevorzugten Lösungsmittel sind Toluene, Tetrahydrofuran und 1,2-Dimethoxyethan.

Geeignete Schwefelungsreagenzien sind beispielsweise Lawesson's Reagenz (s. Tetrahedron 1986, 42, 6555-6564, Tetrahedron Lett. 1993, 46, 7459-7462) und Phosphorpentasulfid.

Nach Beendigung der Reaktion werden die Verbindungen **(VIIb)** von der Reaktionsmischung durch eine der üblichen Trenntechniken getrennt. Falls notwendig werden die Verbindungen durch Umkristallisation oder Chromatographie gereinigt, oder können gegebenenfalls auch im nächsten Schritt ohne vorhergehende Reinigung eingesetzt werden.

### Verfahren D

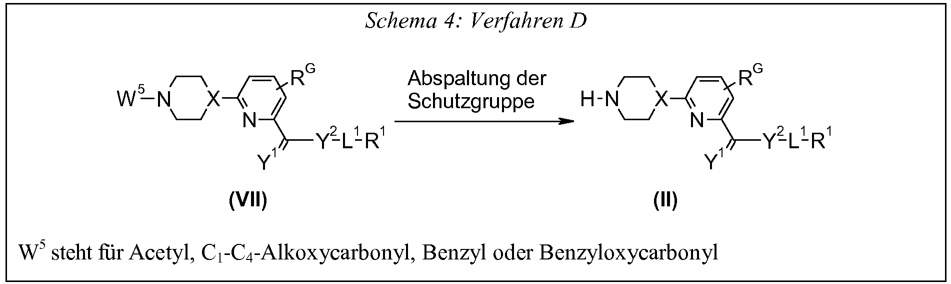

Eine Möglichkeit Verbindungen der Formel **(II)** aus entsprechenden Verbindungen **(VII)** darzustellen ist in Schema 4 gezeigt.

Eine Verbindung der Formel **(VII)** wird in eine Verbindung der Formel **(II)** durch geeignete Methoden zur Entfernung von Schutzgruppen, die in der Literatur beschrieben sind ("Protective Groups in Organic Synthesis"; Third Edition; 494-653, und dort zitierte Literatur), überführt.

tert-Butoxycarbonyl und Benzyloxycarbonyl Schutzgruppen können im sauren Medium entfernt werden (z.B mit Salzsäure oder der Trifluoressigsäure). Acetylschutzgruppen können unter basischen Bedingungen (z.B. mit Kaliumcarbonat oder Cäsiumcarbonat) entfernt werden. Benzylische Schutzgruppen können hydrogenolytisch mit Wasserstoff in Gegenwart eines Katalysators (z.B. Palladium auf Aktivkohle) entfernt werden.

Säuren, die für diese Reaktion, der Entschützung von *tert*-Butoxycarbonyl und Benzyloxycarbonyl Gruppen verwendet werden können, sind z.B. Trifluoressigsäure, Salzsäure oder andere Säuren, wie sie in der Literatur beschrieben sind (z.B. "Protective Groups in Organic Synthesis"; Third Edition; S. 494-653).

Nach Beendigung der Reaktion werden die Verbindungen **(II)** von der Reaktionsmischung durch eine der üblichen Trenntechniken getrennt. Falls notwendig werden die Verbindungen durch Umkristallisation oder Chromatographie gereinigt oder können, wenn gewünscht, auch im nächsten Schritt ohne vorhergehende Reinigung eingesetzt werden. Es ist außerdem möglich, die Verbindung der allgemeinen Formel **(II)** als Salz zu isolieren, z.B. als Salz der Salzsäure oder der Trifluoressigsäure.

### Verfahren E

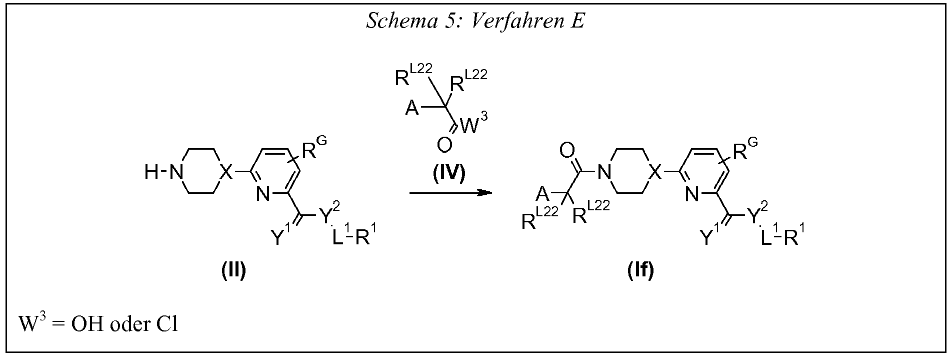

Eine Möglichkeit, Verbindungen der Formel (**If)** aus entsprechenden Verbindungen **(II)** mit den Verbindungen **(IV)** darzustellen, ist in Schema 5 gezeigt

Verbindungen **(IV)** sind durch in der Literatur beschriebene Prozesse darstellbar (s. z.B. WO -A-2008/013622 und WO -A-2008/013925).

Eine Verbindung mit der allgemeinen Formel **(If)** kann analog zu in der Literatur beschriebenen Vorschriften (s. z.B. WO -A-2007/147336) durch eine Kupplungsreaktion einer Verbindung mit der entsprechenden allgemeinen Formel **(II)** mit einem Substrat der allgemeinen Formel **(IV),** wobei W³ für Chlor steht, gegebenenfalls in der Gegenwart eines Säurefängers / Base synthetisiert werden.

Wenigstens ein Äquivalent eines Säurefängers/einer Base (z.B. Hünig Base, Triethylamin oder kommerziell erhältliche polymere Säurefänger) wird im Verhältnis zum Startmaterial der allgemeinen Formel **(II)** verwendet. Ist das Startmaterial ein Salz, werden wenigstens zwei Äquivalente des Säurefängers benötigt.

Alternativ kann eine Verbindung der Formel **(If)**, auch aus der entsprechenden Verbindung der Formel **(II)** mit einem Substrat der Formel **(IV),** wobei W³ für Hydroxy steht, in Gegenwart eines Kupplungsreagenzes analog zu in der Literatur beschriebenen Vorschriften synthetisiert werden (z.B. Tetrahedron 2005, 61, 10827-10852, und darin zitierte Referenzen).

Geeignete Kupplungsreagenzien sind beispielsweise Peptidkupplungsreagenzien zum Beispiel, N-(3-Dimethylaminopropyl)-N'-ethyl-carbodiimid gemischt mit 4-Dimethylamino-pyridin, N-(3-Dimethylaminopropyl)-N'-ethyl-carbodiimid gemischt mit 1-Hydroxy-benzotriazol, Bromtripyrrolidinophosphonium-hexafluorophosphat, O-(7-Azabenzotriazol-1-yl)-N,N,N',N'-tetramethyl-uroniumhexafluorophosphat, etc.

Nach Beendigung der Reaktion, werden die Verbindungen **(If)** von der Reaktionsmischung durch eine der üblichen Trenntechniken getrennt. Falls notwendig werden die Verbindungen durch Umkristallisation oder Chromatographie gereinigt.

### Verfahren F

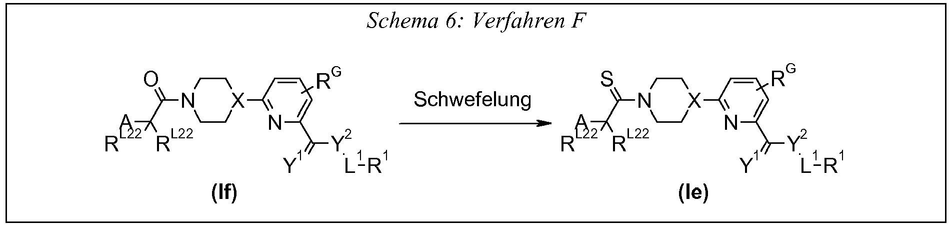

Die bei der Durchführung des erfindungsgemäßen Verfahrens F (Schema 6) erhaltenen Amide **(Ie)** lassen sich mittels literaturbeschriebener Methoden in die korrespondierenden Thioamide überführen (z.B. Bioorganic & Medicinal Chemistry Letters (2009), 19(2), 462-468). Hierbei werden die Verbindungen der Formel **(Ie)** typischerweise mit Phosphorpentasulfid oder 2,4-Bis(4-Methoxyphenyl)-1,3-dithia-2,4-diphosphetane-2,4-disulfid (Lawesson Reagenz) umgesetzt.

Das erfindungsgemäße Verfahren C wird vorzugsweise unter Verwendung eines oder mehrerer Verdünnungsmittel durchgeführt. Die bevorzugten Lösungsmittel sind Toluene, Tetrahydrofuran und 1,2-Dimethoxyethan.

Nach Beendigung der Reaktion werden die Verbindungen **(Ie)** von der Reaktionsmischung durch eine der üblichen Trenntechniken getrennt. Falls notwendig werden die Verbindungen durch Umkristallisation oder Chromatographie gereinigt.

### Verfahren G

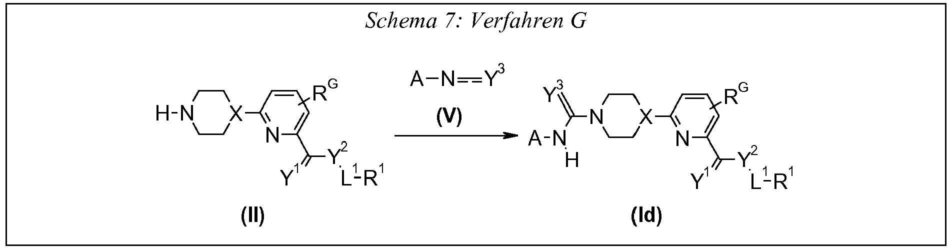

Eine Möglichkeit, Verbindungen der Formel **(Id)** aus entsprechenden Verbindungen **(II)** mit den Verbindungen **(V)** darzustellen, ist in Schema 7 gezeigt.

Eine Verbindung mit der allgemeinen Formel **(Id)** kann analog zu in der Literatur beschriebenen Vorschriften (s. z.B. WO -A-2009/055514) durch eine Kupplungsreaktion einer Verbindung mit der entsprechenden allgemeinen Formel **(II)** mit einem Substrat der allgemeinen Formel **(V),** gegebenenfalls in der Gegenwart eines Säurefängers / Base, wie z.B. Triethylamin, 1,8-Diazabicyclo[5.4.0]undec-7-en oder Hünig-Base synthetisiert werden.

Nach Beendigung der Reaktion, werden die Verbindungen **(Id)** von der Reaktionsmischung durch eine der üblichen Trenntechniken getrennt. Falls notwendig werden die Verbindungen durch Umkristallisation oder Chromatographie gereinigt.

### Verfahren H

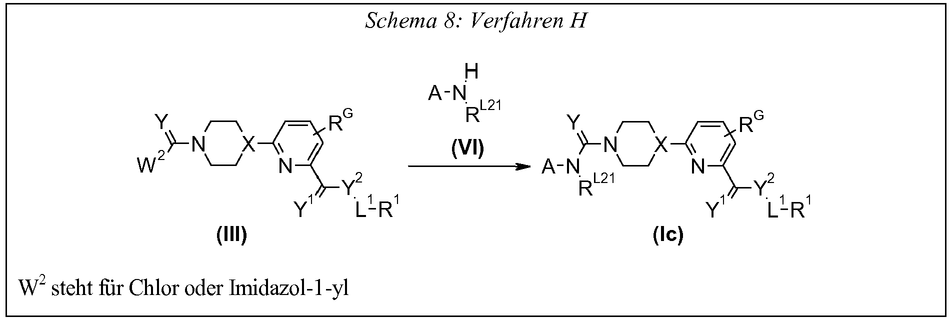

Die bei der Durchführung des erfindungsgemäßen Verfahren H (Schema 8) als Ausgangstoffe benötigten Carbamoyl- und Thiocarbamoylchloride der Formel **(IIIa,** W² = Chlor) lassen sich mittels literaturbeschriebener Methoden herstellen (s. z.B. Tetrahedron, 2008, 7605; Journal of Organic Chemistry, 2004, 3787; Journal of Organic Chemistry, 1983, 4750; European Journal of Organic Chemistry, 2006, 1177). Typischerweise werden die Verbindungen der Formel **(IIIa,** W² = Chlor) ausgehend von Aminen der Formel **(II)** und Phosgen, Thiophosgen oder deren Äquivalenten hergestellt.

Die bei der Durchführung des erfindungsgemäßen Verfahrens als Ausgangstoffe benötigten Carbamoyl- und Thiocarbamoylimidazole der Formel **(IIIb,** W² = imidazol-1-yl) lassen sich mittels literaturbeschriebener Methoden herstellen (s. z.B. Tetrahedron Letters, 2008, 5279; Tetrahedron, 2005, 7153). Typischerweise werden die Verbindungen der Formel **(IIIb,** W² = imidazol-1-yl) ausgehend von Aminen der Formel **(II)** und 1,1'-Carbonyldiimidazole oder 1,1'-Thiocarbonyldimidazole hergestellt.

Das Schema 8 beschreibt die Herstellung von Verbindungen der Struktur **(Ic)** durch Reaktion von Verbindungen der Struktur **(III)** und Aminen **(VI).**

Das Verfahren H wird gegebenenfalls in Gegenwart eines geeigneten Säureakzeptors durchgeführt.

Die bei der Durchführung des erfindungsgemäßen Verfahren H erhaltenen Verbindungen **(Ic)** können alternativ teilweise auch ohne Verwendung eines Säureakzeptors als korrespondierende Säurechloride [**(Ic)**-HCl] erhalten werden. Bei Bedarf erfolgt die Freisetzung der Verbindungen **(Ic)** nach üblichen Methoden.

Nach Beendigung der Reaktion, werden die Verbindungen **(Ic)** von der Reaktionsmischung durch eine der üblichen Trenntechniken getrennt. Falls notwendig werden die Verbindungen durch Umkristallisation oder Chromatographie gereinigt.

### Verfahren I

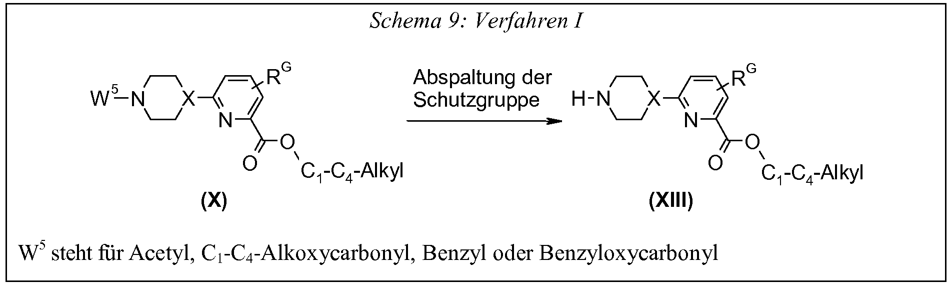

Eine Möglichkeit, Verbindungen der Formel **(XIII)** aus entsprechenden Verbindungen **(X)** herzustellen, ist in Schema 9 gezeigt.

Verfahren I wird analog Verfahren D durchgeführt.

### Verfahren J

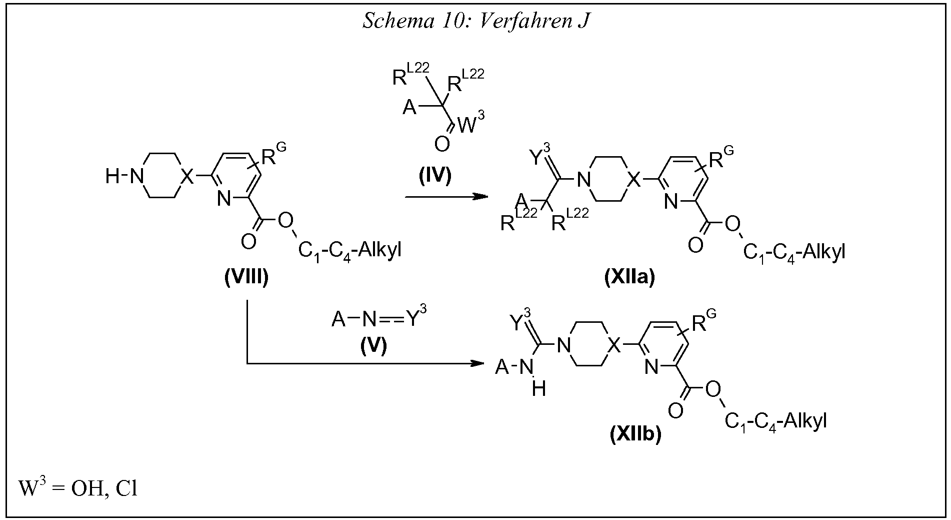

Eine Möglichkeit, Verbindungen der Formel **(XIIa)** und **(XIIb)** aus entsprechenden Verbindungen **(VIII)** herzustellen, ist in Schema 10 gezeigt.

Verfahren J wird analog Verfahren E und Verfahren G durchgeführt.

### Verfahren K

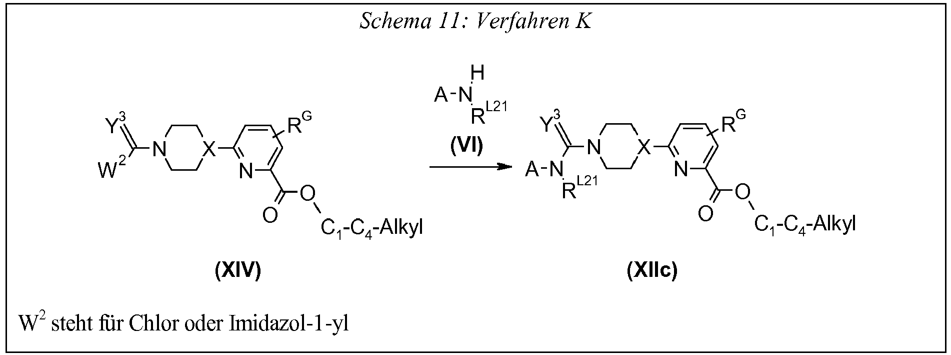

Eine Möglichkeit, Verbindungen der Formel (XIIc) aus entsprechenden Verbindungen (XIV) herzustellen, ist in Schema 11 gezeigt.

Verfahren K wird analog Verfahren H durchgeführt.

### Verfahren L

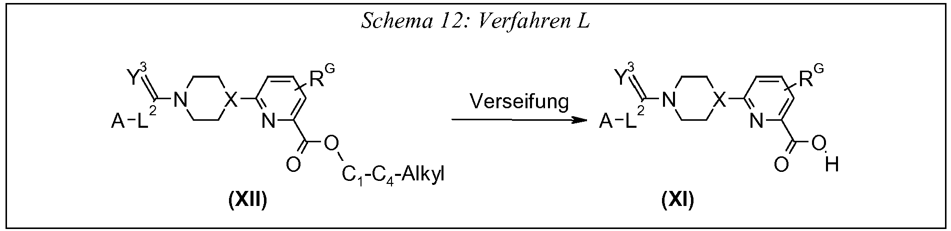

Eine Möglichkeit, Verbindungen der Formel **(XI)** aus entsprechenden Verbindungen **(XII)** herzustellen, ist in Schema 12 gezeigt.

Verfahren L wird analog Verfahren A durchgeführt.

### Verfahren M

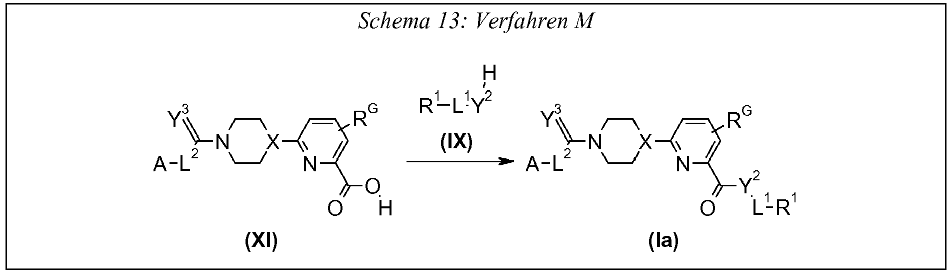

Eine Möglichkeit, Verbindungen der Formel **(Ia)** aus entsprechenden Verbindungen **(XI)** herzustellen, ist in Schema 13 gezeigt.

Verfahren M wird analog Verfahren B durchgeführt.

### Verfahren N

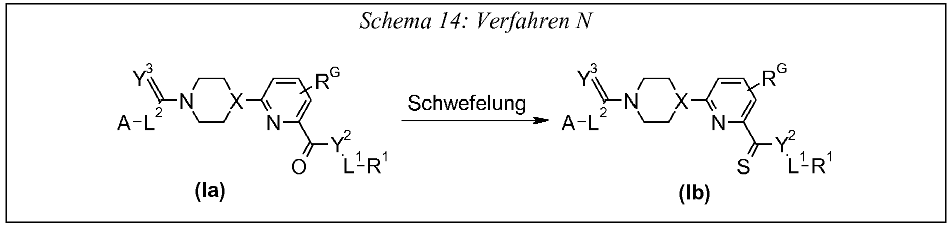

Eine Möglichkeit, Verbindungen der Formel **(Ib)** aus entsprechenden Verbindungen **(Ia)** herzustellen, ist in Schema 14 gezeigt.

Verfahren N wird analog Verfahren C durchgeführt.

Ein weiterer Gegenstand der Erfindung betrifft die nichtmedizinische Verwendung der erfindungsgemäßen Pyridinylcarbonsäure Derivate der Formel **(I)** zum Bekämpfen unerwünschter Mikroorganismen.

Ein weiterer Gegenstand der Erfindung betrifft ein Mittel zum Bekämpfen unerwünschter Mikroorganismen, umfassend wenigstens ein Pyridinylcarbonsäure Derivate gemäß der vorliegenden Erfindung.

Außerdem betrifft die Erfindung ein Verfahren zum Bekämpfen unerwünschter Mikroorganismen, dadurch gekennzeichnet, dass die erfindungsgemäßen Pyridinylcarbonsäure Derivate auf die Mikroorganismen und/oder in deren Lebensraum ausgebracht werden.

Ein letzter Gegenstand der Erfindung betrifft ein Verfahren zum Schutz von Saatgut vor unerwünschten Mikroorganismen durch Verwendung eines mit wenigstens einem Pyridinylcarbonsäure Derivate gemäß der vorliegenden Erfindung behandelten Saatgutes.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Die erfindungsgemäßen Pyridinylcarbonsäure Derivate der Formel **(I)** besitzen sehr gute fungizide Eigenschaften und lassen sich im Pflanzenschutz beispielsweise zur Bekämpfung von Plasmodiophoromyceten, Oomyceten, Chytridiomyceten, Zygomyceten, Ascomyceten, Basidiomyceten und Deuteromyceten einsetzen.

Bakterizide lassen sich im Pflanzenschutz beispielsweise zur Bekämpfung von Pseudomonadaceae, Rhizobiaceae, Enterobacteriaceae, Corynebacteriaceae und Streptomycetaceae einsetzen.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen..

Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Erkrankungen, hervorgerufen durch bakterielle Erreger wie z.B. Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora;
Bevorzugt können die folgenden Krankheiten von Soja-Bohnen bekämpft werden:
   Pilzkrankheiten an Blättern, Stängeln, Schoten und Samen verursacht durch z.B. Alternaria leaf spot (Alternaria spec. atrans tenuissima), Anthracnose (Colletotrichum gloeosporoides dematium var. truncatum), Brown spot (Septoria glycines), Cercospora leaf spot and blight (Cercospora kikuchii), Choanephora leaf blight (Choanephora infundibulifera trispora (Syn.)), Dactuliophora leaf spot (Dactuliophora glycines), Downy Mildew (Peronospora manshurica), Drechslera blight (Drechslera glycini), Frogeye Leaf spot (Cercospora sojina), Leptosphaerulina Leaf Spot (Leptosphaerulina trifolii), Phyllostica Leaf Spot (Phyllosticta sojaecola), Pod and Stem Blight (Phomopsis sojae), Powdery Mildew (Microsphaera diffusa), Pyrenochaeta Leaf Spot (Pyrenochaeta glycines), Rhizoctonia Aerial, Foliage, and Web Blight (Rhizoctonia solani), Rust (Phakopsora pachyrhizi, Phakopsora meibomiae), Scab (Sphaceloma glycines), Stemphylium Leaf Blight (Stemphylium botryosum), Target Spot (Corynespora cassiicola).

Pilzkrankheiten an Wurzeln und der Stängelbasis verursacht durch z.B. Black Root Rot (Calonectria crotalariae), Charcoal Rot (Macrophomina phaseolina), Fusarium Blight or Wilt, Root Rot, and Pod and Collar Rot (Fusarium oxysporum, Fusarium orthoceras, Fusarium semitectum, Fusarium equiseti), Mycoleptodiscus Root Rot (Mycoleptodiscus terrestris), Neocosmospora (Neocosmopspora vasinfecta), Pod and Stem Blight (Diaporthe phaseolorum), Stem Canker (Diaporthe phaseolorum var. caulivora), Phytophthora Rot (Phytophthora megasperma), Brown Stem Rot (Phialophora gregata), Pythium Rot (Pythium aphanidermatum, Pythium irreguläre, Pythium debaryanum, Pythium myriotylum, Pythium ultimum), Rhizoctonia Root Rot, Stem Decay, and Damping-Off (Rhizoctonia solani), Sclerotinia Stem Decay (Sclerotinia sclerotiorum), Sclerotinia Southern Blight (Sclerotinia rolfsii), Thielaviopsis Root Rot (Thielaviopsis basicola).

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Dabei lassen sich die erfindungsgemäßen Wirkstoffe mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Insektizide verwenden. Sie lassen sich gegebenenfalls auch als Zwischen- oder Vorprodukte für die Synthese weiterer Wirkstoffe einsetzen.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes in der Landwirtschaft, im Gartenbau, bei der Tierzucht, in Forsten, in Gärten und Freizeiteinrichtungen, im Vorrats- und Materialschutz sowie auf dem Hygienesektor. Sie können vorzugsweise als Pflanzenschutzmittel eingesetzt werden. Sie sind gegen normal sensible und resistente Arten sowie gegen alle oder einzelne Entwicklungsstadien wirksam.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Die erfindungsgemäßen Wirkstoffe bzw. Mittel können außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von sogenannten Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die vorliegende Erfindung betrifft weiterhin ein Mittel zum Bekämpfen unerwünschter Mikroorganismen, umfassend wenigstens eines der erfindungsgemäßen Pyridinylcarbonsäure Derivate. Vorzugsweise handelt es sich um fungizide Mittel, welche landwirtschaftlich verwendbare Hilfsmittel, Solventien, Trägerstoffe, oberflächenaktive Stoffe oder Streckmittel enthalten.

Erfindungsgemäß bedeutet Trägerstoff eine natürliche oder synthetische, organische oder anorganische Substanz, mit welchen die Wirkstoffe zur besseren Anwendbarkeit, v.a. zum Aufbringen auf Pflanzen oder Pflanzenteile oder Saatgut, gemischt oder verbunden sind. Der Trägerstoff, welcher fest oder flüssig sein kann, ist im Allgemeinen inert und sollte in der Landwirtschaft verwendbar sein.

Als feste Trägerstoffe kommen infrage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-FettsäureEster, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP-POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Die Wirkstoffe können als solche, in Form ihrer Formulierungen oder den daraus bereiteten Anwendungsformen, wie gebrauchsfertige Lösungen, Emulsionen, wasser- oder ölbasierte Suspensionen, Pulver, Spritzpulver, Pasten, lösliche Pulver, Stäubemittel, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die genannten Formulierungen können in an sich bekannter Weise hergestellt werden, z.B. durch Vermischen der Wirkstoffe mit mindestens einem üblichen Streckmittel, Lösungs- bzw. Verdünnungsmittel, Emulgator, Dispergier- und/oder Binde- oder Fixiermittels, Netzmittel, Wasser-Repellent, gegebenenfalls Sikkative und UV-Stabilisatoren und gegebenenfalls Farbstoffen und Pigmenten, Entschäumer, Konservierungsmittel, sekundäre Verdickungsmittel, Kleber, Gibberelline sowie weiteren Verarbeitungshilfsmitteln.

Die vorliegende Erfindung umfasst nicht nur Formulierungen, welche bereits anwendungsfertig sind und mit einer geeigneten Apparatur auf die Pflanze oder das Saatgut ausgebracht werden können, sondern auch kommerzielle Konzentrate, welche vor Gebrauch mit Wasser verdünnt werden müssen.

Die erfindungsgemäßen Wirkstoffe können als solche oder in ihren (handelsüblichen) Formulierungen sowie in den aus diesen Formulierungen bereiteten Anwendungsformen in Mischung mit anderen (bekannten) Wirkstoffen, wie Insektiziden, Lockstoffen, Sterilantien, Bakteriziden, Akariziden, Nematiziden, Fungiziden, Wachstumsregulatoren, Herbiziden, Düngemitteln, Safener bzw. Semiochemicals vorliegen.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Im Falle der Benutzung von Wasser als Streckmittel können z.B. auch organische Lösungsmittel als Hilfslösungsmittel verwendet werden. Als flüssige Lösungsmittel kommen im Wesentlichen infrage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Die erfindungsgemäßen Mittel können zusätzlich weitere Bestandteile enthalten, wie z.B. oberflächenaktive Stoffe. Als oberflächenaktive Stoffe kommen Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe infrage. Beispiele hierfür sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen, und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist notwendig, wenn einer der Wirkstoff und/oder einer der inerten Trägerstoffe nicht in Wasser löslich ist und wenn die Anwendung in Wasser erfolgt. Der Anteil an oberflächenaktiven Stoffen liegt zwischen 5 und 40 Gewichtsprozent des erfindungsgemäßen Mittels.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Gegebenenfalls können auch andere zusätzliche Komponenten enthalten sein, z.B. schützende Kolloide, Bindemittel, Klebstoffe, Verdicker, thixotrope Stoffe, Penetrationsförderer, Stabilisatoren, Sequestiermittel, Komplexbildner. Im Allgemeinen können die Wirkstoffe mit jedem festen oder flüssigen Additiv, welches für Formulierungszwecke gewöhnlich verwendet wird, kombiniert werden.

Die Formulierungen enthalten im Allgemeinen zwischen 0,05 und 99 Gew.-%, 0,01 und 98 Gew.-%, vorzugsweise zwischen 0,1 und 95 Gew.-%, besonders bevorzugt zwischen 0,5 und 90 % Wirkstoff, ganz besonders bevorzugt zwischen 10 und 70 Gewichtsprozent.

Die zuvor beschriebenen Formulierungen können in einem erfindungsgemäßen Verfahren zum Bekämpfen unerwünschter Mikroorganismen verwendet werden, bei dem die erfindungsgemäßen Pyridinylcarbonsäure Derivate auf die Mikroorganismen und/oder in deren Lebensraum ausgebracht werden.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Als Mischpartner kommen zum Beispiel bekannte Fungizide, Insektizide, Akarizide, Nematizide oder auch Bakterizide (siehe auch Pesticide Manual, 14th ed.) infrage.

Auch eine Mischung mit anderen bekannten Wirkstoffen, wie Herbiziden, oder mit Düngemitteln und Wachstumsregulatoren, Safenern bzw. Semiochemicals ist möglich.

Die Anwendung geschieht in einer den Anwendungsformen angepaßten üblichen Weise.

Die Erfindung umfasst weiterhin ein Verfahren zur Behandlung von Saatgut.

Die erfindungsgemäßen Wirkstoffe bzw. Mittel sind auch geeignet für die Behandlung von Saatgut. Ein großer Teil des durch Schadorganismen hervorgerufenen Schadens an Kulturpflanzen wird durch den Befall des Saatguts während der Lagerung oder nach der Aussaat sowie während und nach der Keimung der Pflanze ausgelöst. Diese Phase ist besonders kritisch, weil die Wurzeln und Schösslinge der wachsenden Pflanze besonders empfindlich sind und auch nur eine kleine Schädigung zum Tod der Pflanze führen kann. Es besteht daher ein großes Interesse daran, das Saatgut und die keimende Pflanze durch Einsatz geeigneter Mittel zu schützen.

Die Bekämpfung von pflanzenpathogenen Schadpilzen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt werden, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher auch auf ein Verfahren zum Schutz von Saatgut und keimenden Pflanzen vor dem Befall von tierischen Schädlingen und/oder pflanzenpathogenen Schadpilzen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der keimenden Pflanze vor phytopathogenen Pilzen. Weiterhin bezieht sich die Erfindung auf Saatgut, welches zum Schutz vor phytopathogenen Pilzen mit einem erfindungsgemäßen Mittel behandelt wurde.

Die Bekämpfung von tierischen Schädlingen und/oder pflanzenpathogenen Schadpilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen und/oder pflanzenpathogenen Schadpilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe bzw. Mittel insbesondere auch bei transgenem Saatgut eingesetzt werden können, wobei die aus diesem Saatgut wachsende Pflanze in der Lage ist, ein Protein zu exprimieren, welches gegen Schädlinge wirkt. Durch die Behandlung solchen Saatguts mit den erfindungsgemäßen Wirkstoffen bzw. Mitteln können bereits durch die Expression des beispielsweise insektiziden Proteins bestimmte Schädlinge bekämpft werden. Überraschenderweise kann dabei ein weiterer synergistischer Effekt beobachtet werden, welcher zusätzlich die Effektivität zum Schutz gegen den Schädlingsbefall vergrößert.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Wie auch weiter unten beschrieben, ist die Behandlung von transgenem Saatgut mit den erfindungsgemäßen Wirkstoffen bzw. Mitteln von besonderer Bedeutung. Dies betrifft das Saatgut von Pflanzen, die wenigstens ein heterologes Gen enthalten, das die Expression eines Polypeptids oder Proteins mit insektiziden Eigenschaften ermöglicht. Das heterologe Gen in transgenem Saatgut kann z.B. aus Mikroorganismen der Arten Bacillus, Rhizobium, Pseudomonas, Serratia, Trichoderma, Clavibacter, Glomus or Gliocladium stammen. Bevorzugt stammt dieses heterologe Gen aus Bacillus sp., wobei das Genprodukt eine Wirkung gegen den Maiszünsler (European corn borer) und/oder Western Corn Rootworm besitzt. Besonders bevorzugt stammt das heterologe Gen aus Bacillus thuringiensis.

Im Rahmen der vorliegenden Erfindung wird das erfindungsgemäße Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US-A- 4,272,417, US-A- 4,245,432, US-A-4,808,430, US-A-5,876,739, US-A-2003/0176428, WO-A-2002/080675, WO-A-2002/028186.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstoffkombination liegen im Allgemeinen zwischen 0,001 und 50 g pro Kilogramm Saatgut, vorzugsweise zwischen 0,01 und 15 g pro Kilogramm Saatgut.

Darüber hinaus weisen die erfindungsgemäßen Verbindungen der Formel **(I)** auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die erfindungsgemäßen Wirkstoffe der Formel **(I)** können daher sowohl in medizinischen als auch in nicht-medizinischen Anwendungen eingesetzt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Die Aufwandmenge der erfindungsgemäßen Wirkstoffe beträgt
- bei der Behandlung von Pflanzenteilen, z.B. Blättern: von 0,1 bis 10 000 g/ha, bevorzugt von 10 bis 1 000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden);
- bei der Saatgutbehandlung: von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut;
- bei der Bodenbehandlung: von 0,1 bis 10 000 g/ha, bevorzugt von 1 bis 5 000 g/ha.

Diese Aufwandmengen seien nur beispielhaft und nicht limitierend im Sinne der Erfindung genannt. Etwaige im folgenden beschriebene Anwendungen an Tieren oder Menschen sind nicht Teil der Erfindung. Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiterhin können die erfindungsgemäßen Verbindungen allein oder in Kombinationen mit anderen Wirkstoffen als Antifouling-Mittel eingesetzt werden.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

In Abhängigkeit von den Pflanzenarten oder Pflanzensorten, ihrem Standort und ihren Wachstumsbedingungen (Böden, Klima, Vegetationsperiode, Ernährung) kann die erfindungsgemäße Behandlung auch zu überadditiven ("synergistischen") Effekten führen. So sind zum Beispiel die folgenden Effekte möglich, die über die eigentlich zu erwartenden Effekte hinausgehen: verringerte Aufwandmengen und/oder erweitertes Wirkungsspektrum und/oder erhöhte Wirksamkeit der Wirkstoffe und Zusammensetzungen, die erfindungsgemäß eingesetzt werden können, besseres Pflanzenwachstum, erhöhte Toleranz gegenüber hohen oder niedrigen Temperaturen, erhöhte Toleranz gegenüber Trockenheit oder Wasser- oder Bodensalzgehalt, erhöhte Blühleistung, Ernteerleichterung, Reifebeschleunigung, höhere Erträge, größere Früchte, größere Pflanzenhöhe, intensiver grüne Farbe des Blatts, frühere Blüte, höhere Qualität und/oder höherer Nährwert der Ernteprodukte, höhere Zuckerkonzentration in den Früchten, bessere Lagerfähigkeit und/oder Verarbeitbarkeit der Ernteprodukte.

In gewissen Aufwandmengen können die erfindungsgemäßen Wirkstoffkombinationen auch eine stärkende Wirkung auf Pflanzen ausüben. Sie eignen sich daher für die Mobilisierung des pflanzlichen Abwehrsystems gegen Angriff durch unerwünschte phytopathogene Pilze und/oder Mikroorganismen und/oder Viren. Dies kann gegebenenfalls einer der Gründe für die erhöhte Wirksamkeit der erfindungsgemäßen Kombinationen sein, zum Beispiel gegen Pilze. Pflanzenstärkende (resistenzinduzierende) Substanzen sollen im vorliegenden Zusammenhang auch solche Substanzen oder Substanzkombinationen bedeuten, die fähig sind, das pflanzliche Abwehrsystem so zu stimulieren, daß die behandelten Pflanzen, wenn sie im Anschluß daran mit unerwünschten phytopathogenen Pilzen und/oder Mikroorganismen und/oder Viren inokkuliert werde, einen beträchtlichen Resistenzgrad gegen diese unerwünschten phytopathogenen Pilze und/oder Mikroorganismen und/oder Viren aufweisen. Im vorliegenden Fall versteht man unter unerwünschten phytopathogenen Pilzen und/oder Mikroorganismen und/oder Viren phytopathogene Pilze, Bakterien und Viren. Die erfindungsgemäßen Substanzen lassen sich daher zum Schutz von Pflanzen gegen Angriff durch die erwähnten Pathogene innerhalb eines gewissen Zeitraums nach der Behandlung einsetzen. Der Zeitraum, über den eine Schutzwirkung erzielt wird, erstreckt sich im Allgemeinen von 1 bis 10 Tagen, vorzugsweise 1 bis 7 Tagen, nach der Behandlung der Pflanzen mit den Wirkstoffen.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphomährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben. Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden.

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium *Salmonella typhimurium,* das CP4-Gen des Bakteriums *Agrobacterium sp.,* die Gene, die für eine EPSPS aus der Petunie, für eine EPSPS aus der Tomate oder für eine EPSPS aus Eleusine kodieren. Es kann sich auch um eine mutierte EPSPS handeln. Glyphosatetolerante Pflanzen können auch dadurch erhalten werden, daß man ein Gen exprimiert, das für ein Glyphosate-Oxidoreduktase-Enzym kodiert. Glyphosatetolerante Pflanzen können auch dadurch erhalten werden, daß man ein Gen exprimiert, das für ein Glyphosate-acetyltransferase-Enzym kodiert. Glyphosatetolerante Pflanzen können auch dadurch erhalten werden, daß man Pflanzen, die natürlich vorkommende Mutationen der oben erwähnten Gene selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert, oder einem Gen, das für ein mutiertes HPPD-Enzym kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Die Toleranz von Pflanzen gegenüber HPPD-Hemmern kann auch dadurch verbessert werden, daß man Pflanzen zusätzlich zu einem Gen, das für ein HPPDtolerantes Enzym kodiert, mit einem Gen transformiert, das für ein Prephenatdehydrogenase-Enzym kodiert.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen. Die Herstellung von sulfonylharnstofftoleranten Pflanzen und imidazolinontoleranten Pflanzen ist in der internationalen Veröffentlichung WO 1996/033270 beschrieben. Weitere sulfonylharnstoff- und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus *Bacillus thuringiensis* oder einen insektiziden Teil davon, wie die insektiziden Kristallproteine, die online bei: http://www.lifesci.sussex.ac.uk/Home/Neil_Crickmore/Bt/ beschrieben sind, zusammengestellt wurden, oder insektizide Teile davon, z.B. Proteine der Cry-Proteinklassen CrylAb, CrylAc, CrylF, Cry2Ab, Cry3Ae oder Cry3Bb oder insektizide Teile davon; oder
2) ein Kristallprotein aus *Bacillus thuringiensis* oder einen Teil davon, der in Gegenwart eines zweiten, anderen Kristallproteins als *Bacillus thuringiensis* oder eines Teils davon insektizid wirkt, wie das binäre Toxin, das aus den Kristallproteinen Cy34 und Cy35 besteht; oder
3) ein insektizides Hybridprotein, das Teile von zwei unterschiedlichen insektiziden Kristallproteinen aus *Bacillus thuringiensis* umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden, wie das Protein Cry3Bb1 in Mais-Events MON863 oder MON88017 oder das Protein Cry3A im Mais-Event MIR 604;
5) ein insektizides sezerniertes Protein aus *Bacillus thuringiensis* oder *Bacillus cereus* oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus *Bacillus thuringiensis* oder *Bacillus cereus,* das in Gegenwart eines zweiten sezernierten Proteins aus *Bacillus thuringiensis* oder *B. cereus* insektizid wirkt, wie das binäre Toxin, das aus den Proteinen VIP1A und VIP2A besteht.
7) ein insektizides Hybridprotein, das Teile von verschiedenen sezernierten Proteinen von *Bacillus thuringiensis* oder *Bacillus cereus* umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier- DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag.
b. Pflanzen, die ein streßtoleranzförderndes Transgen enthalten, das die Expression und/oder Aktivität der für PARG kodierenden Gene der Pflanzen oder Pflanzenzellen zu reduzieren vermag;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremononukleotidadenyltransferase, Nicotinamidadenindinukleotidsynthetase oder Nicotinamidphosphoribosyltransferase.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekommorphologie im Vergleich mit der synthetisierten Stärke in Wildtyppflanzenzellen oder -pflanzen verändert ist, so daß sich diese modifizierte Stärke besser für bestimmte Anwendungen eignet.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, Pflanzen, die alpha-1,4-Glucane produzieren, Pflanzen, die alpha-1,6-verzweigte alpha-1,4-Glucane produzieren und Pflanzen, die Alternan produzieren.
3) Transgene Pflanzen, die Hyaluronan produzieren.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten,
b) Pflanzen wie Baumwollpflanzen, die eine veränderte Form von rsw2- oder rsw3-homologen Nukleinsäuren enthalten;
c) Pflanzen wie Baumwollpflanzen mit einer erhöhten Expression der Saccharosephosphatsynthase;
d) Pflanzen wie Baumwollpflanzen mit einer erhöhten Expression der Saccharosesynthase;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren;
b) Pflanzen wie Rapspflanzen, die Öl mit einem niedrigen Linolensäuregehalt produzieren.
c) Pflanzen wie Rapspflanzen, die Öl mit einem niedrigen gesättigten Fettsäuregehalt produzieren.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die aufgeführten Pflanzen können besonders vorteilhaft erfindungsgemäß mit den Verbindungen der allgemeinen Formel **(I)** bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Mittel können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die Herstellung und die Verwendung der erfindungsgemäßen Wirkstoffe der Formeln **(I)** geht aus den folgenden Beispielen hervor. Die Erfindung ist jedoch nicht auf diese Beispiele beschränkt. Beispiele, welche nicht unter den Anspruchsumfang fallen, sind nicht Teil der Erfindung.

### Beispiele

**Allgemeines:** Wenn nicht anders angegeben, werden alle chromatografischen Reinigungs- bzw. Trennungsschritte an Kieselgel und mit einem Lösungsmittelgradienten von 0:100 Essigsäureethylester/Cyclohexan zu 100:0 Essigsäure-ethylester/Cyclohexan durchgeführt.

### Herstellung von (I-12):

### Schritt 1

### tert-Butyl-4-(6-brompyridin-2-yl)-4-hydroxypiperidin-1-carboxylat

Zu einer Lösung von 2,6-Dibrompyridin (34 g) in Dichloromethan (740 mL) wird bei -78 °C unter Argon n-Butyllithium (1.6M in Tetrahydrofuran, 100 mL) getropft. Das Reaktionsgemisch wird 20 Minuten bei -78 °C gerührt und dann wird *tert*-Butyl-4-oxopiperidin-1-carboxylat zugegeben. Das Gemisch wird bei Raumtemperatur für 20 Minuten gerührt. Das Reaktionsgemisch wird anschließend mit gesättigter Ammoniumchloridlösung bei -30°C versetzt und die wässrige Phase abgetrennt. Nach Extraktion der wässrigen Phase mit Dichloromethan werden die vereinigten organischen Phasen über Natriumsulfat getrocknet und unter vermindertem Druck eingeengt. Der Rückstand wird chromatographisch gereinigt. Man erhält *tert*-Butyl-4-(6-brompyridin-2-yl)-4-hydroxypiperidin-1-carboxylat (60 g).
logP (pH2.7): 3.05
¹H NMR (DMSO-d₆, 400 MHz): δₚₚₘ : 1.43 (s, 9H), 1.95 (td, 2H), 2.35 (t, 2H), 3.15 (t, 2H), 3.84 (bd, 2H), 5.24 (bs, 1H), 7.46 (dd, 1H), 7.68 (dd, 1H), 7.73 (t, 1H)
MS (ESI): 301 und 303 ([M-COOC(CH₃)₃+2H]⁺)

### Schritt 2

### tert-Butyl-6-brom-3',6'-dihydro-2,4'-bipyridin-1'(2'H)-carboxylat

Zu einer Lösung von *tert*-Butyl-4-(6-brompyridin-2-yl)-4-hydroxypiperidin-1-carboxylat (100 mg) in Pyridin (2.5 mL) wird bei 0 °C unter Argon POCl₃ (0.26 mL mL) gegeben. Das Gemisch wird bei 0 °C gerührt und danach langsam auf Raumtemperatur erwärmt. Das Gemisch wird bei Raumtemperatur übernacht gerührt. Das Reaktionsgemisch wird anschließend mit gesättigter Natriumhydrogencarbonatlösung versetzt und die wässrige Phase abgetrennt. Nach Extraktion der wässrigen Phase mit *tert*-Butylmethylether werden die vereinigten organischen Phasen über Natriumsulfat getrocknet und unter vermindertem Druck eingeengt. Der Rückstand wird chromatographisch gereinigt. Man erhält *tert*-Butyl-6-brom-3',6'-dihydro-2,4'-bipyridin-1'(2'H)-carboxylat (60 mg).
logP (pH2.7): 4.14
¹H NMR (DMSO-d₆, 400 MHz): δₚₚₘ : 1.43 (s, 9H), 2.53-2.49 (m, 2H), 3.54 (t, 2H), 4.06 (td, 2H), 6.70 (m, 1H), 7.46 (d, 1H), 7.53 (d, 1H), 7.70 (t, 1H)
MS (ESI): 283 und 285 ([M-C(CH₃)₃+2H]⁺)

### Schritt 3

### 1'-tert-Butyl-6-ethyl-3',6'-dihydro-2,4'-bipyridin-1',6(2'H)-dicarboxylat

*tert*-Butyl-6-brom-3',6'-dihydro-2,4'-bipyridin-1'(2'H)-carboxylat (500 mg) wird in Ethanol (10 mL) gelöst und bei 70 °C unter 3 bar CO für 20 Stunden in Gegenwart von PdCl₂(PPh)₂ (52 mg) und Triethylamin (1.44 mL) gerührt. Der Katalysator wird durch Filtration über Celite entfernt und unter vermindertem Druck eingeengt. Nach chromatografischer Reinigung erhält man 1 '-tert-Butyl-6-ethyl-3',6'-dihydro-2,4'-bipyridin-l',6(2'H)-dicarboxylat (380 mg).
logP (pH2.7): 3.48
¹H NMR (DMSO-d₆, 400 MHz): δₚₚₘ : 1.34 (t, 3H), 1.44 (s, 9H), 2.63-2.57 (m, 2H), 3.56 (t, 2H), 4.07 (td, 2H), 4.36 (q, 2H), 6.74 (m, 1H), 7.75 (dd, 1H), 7.88 (dd, 1H), 7.94 (t, 1H)
MS (ESI): 333 ([M+H]⁺)

### Schritt 4

### Ethyl-6-[1-(tert-butoxycarbonyl)piperidin-4-yl]pyridin-2-carboxylat

1'-*tert*-Butyl-6-ethyl-3',6'-dihydro-2,4'-bipyridin-1',6(2'H)-dicarboxylat (31.5 g) wird in Ethanol (315 mL) gelöst und bei Raumtempratur unter 1 bar H₂ für 12 Stunden in Gegenwart von Pd/C (10%, 4.5 g) hydrogeniert. Nach Filtration und Entfernung des Lösungsmittels unter vermindertem Druck erhält man Ethyl-6-[1-(*tert*-butoxycarbonyl)piperidin-4-yl]pyridin-2-carboxylat (30.5 g).
logP (pH2.7): 3.29
¹H NMR (DMSO-d₆, 400 MHz): δₚₚₘ : 1.33 (t, 3H), 1.42 (s, 9H), 1.62 (tdd, 2H), 1.86 (d, 2H), 2.87 (dd, 2H), 2.95 (m, 1H), 4.07 (bd, 2H), 4.35 (q, 2H), 7.51 (dd, 1H), 7.85 (dd, 1H), 7.89 (t, 1H)
MS (ESI): 335 ([M+H]⁺)

### Schritt 5

### 6-[1-(tert-Butoxycarbonyl)piperidin-4-yl]pyridin-2-carbonsäure

Zu einer Lösung von Ethyl-6-[1-(*tert*-butoxycarbonyl)piperidin-4-yl]pyridin-2-carboxylat (500 mg) in Tetrahydrofuran (5 mL) und Wasser (1.3 mL) wird bei Raumtemperatur Lithiumhydroxidmonohydrat (125 mg) gegeben. Das Gemisch wird 2 h bei Raumtemperatur gerührt und dann mit eiskalter IN HCl-Lösung versetzt. Die wässrige Phase wird mit Ethylacetat extrahiert und dann die vereinigten organischen Phasen mit Natriumsulfat getrocknet. Der Feststoff wird abfiltriert und das Lösungsmittel abdestilliert. Man erhält *6-*[1*-*(*tert-*Butoxycarbonyl)piperidin-4-yl]pyridin-2-carbonsäure (310 mg).
logP (pH2.7): 1.78
¹H NMR (DMSO-d₆, 400 MHz): δₚₚₘ 1.42 (s, 9H), 1.64 (tdd, 2H), 1.86 (d, 2H), 2.87 (dd, 2H), 2.95 (m, 1H), 4.08 (bd, 2H), 7.50 (dd, 1H), 7.85 (dd, 1H), 7.89 (t, 1H)
MS (ESI): 251 ([M-C(CH₃)₃+2H]⁺)

### Schritt 6

### tert-Butyl-4-{6-[methyl(1,2,3,4-tetrahydronaphthalen-1-yl)carbamoyl]pyridin-2-yl}piperidin-1-carboxylat

Zu einer Lösung von 6-[1-(*tert*-Butoxycarbonyl)piperidin-4-yl]pyridin-2-carbonsäure (247 mg) in Dichlormethan (5 mL) wird bei Raumtemperatur N-Methyl-1,2,3,4-tetrahydronaphthalen-1-amin (130 mg), 4-Dimethylaminopyridin (10 mg) und 1-Ethyl-3-(3'-dimethylaminopropyl)carbodiimide (162 mg) gegeben. Das Gemisch wird über Nacht bei Raumtemperatur gerührt und dann mit Wasser versetzt. Die wässrige Phase wird abgetrennt und mit Ethylacetat extrahiert. Die vereinigten organischen Phasen werden über Natriumsulfat getrocknet und unter vermindertem Druck eingeengt. Der Rückstand wird chromatographisch gereinigt. Man erhält tert-Butyl-4-{6-[methyl(1,2,3,4-tetrahydronaphthalen-1-yl)carbamoyl]pyridin-2-yl}piperidin-1-carboxylat (219 mg).
logP (pH2.7): 5.09
MS (ESI): 393 ([M-C(CH₃)₃+2H]⁺)

### Schritt 7

### N-Methyl-6-(1-{[5-methyl-3-(trifluormethyl)-1H-pyrazol-1-yl]acetyl}piperidin-4-yl)-N-(1,2,3,4-tetrahydronaphthalen-1-yl)pyridin-2-carboxamid

Zu *tert*-Butyl-4-{6-[methyl(1,2,3,4-tetrahydronaphthalen-1-yl)carbamoyl]pyridin-2-yl}piperidin-1-carboxylat (302 mg), wird tropfenweise bei Raumtemperatur eine Lösung von Trifluoressigsäure (0.52 mL) zugegeben. Die Reaktionsmischung wird 30 Minuten lang gerührt, dann wird das Lösungsmittel und überschüssiger Trifluoressigsäure entfernt. Man erhält 4-{6-[Methyl(1,2,3,4-tetrahydronaphthalen-1-yl)carbamoyl]pyridin-2-yl}piperidiniumtrifluoracetat.

Zu einer Lösung von [5-Methyl-3-(trifluormethyl)-1H-pyrazol-1-yl]essigsäure (140 mg) in Dichlormethan (5 mL) wird Oxalylchlorid (256 mg) und ein Tropfen N,N-Dimethylformamid gegeben. Dann wird das Reaktionsgemisch für 30 Minuten gerührt. Dann wird der Überschuss Oxalylchlorid unter vermindertem Druck entfernt und der Rückstand wird wieder in Dichlormethan (1 mL) gelöst. Die Lösung wird dann zu der ersterer Lösung aus 4-{6-[Methyl(1,2,3,4-tetrahydronaphthalen-1-yl)carbamoyl]pyridin-2-yl}piperidiniumtrifluoracetat in Dichlormethan (5 mL) und Diisopropylethylamin (869 mg) gegeben Die Reaktionsmischung wird 2 Stunden lang gerührt. Nach Zugabe von konz. Ammoniumchloridlösung wird die wässrige Phase abgetrennt und mit Essigsäureethylester extrahiert. Die vereinigten organischen Phasen werden über Natriumsulfat getrocknet und unter vermindertem Druck eingeengt. Der Rückstand wird chromatographisch gereinigt. Man erhält N-Methyl-6-(1-{[5-methyl-3-(trifluormethyl)-1H-pyrazol-1-yl]acetyl}piperidin-4-yl)-N-(1-yl)pyridin-2-carboxamid (150 mg).
logP (pH2.7): 3.61
¹H NMR (DMSO-d₆, 400 MHz): δₚₚₘ : 1.45-2.15 (m, 8H), 2.22 (s, 3H), 2.61 und 2.68 (s, 3H), 2.65-2.88 (m, 3H), 2.95-3.31 (m, 2H), 3.98 (bs, 1H), 4.38 (bs, 1H), 4.99 und 5.83 (m, 1H), 5.17 (bs, 2H), 6.45 (s, 1H), 7.05-7.25 (m, 4H), 7.38 (m, 1H), 7.50 (m, 1H), 7.86 (m, 1H)
MS (ESI): 410 ([M-1,2-Dihydronaphthalen+H]⁺)

### Herstellung von Verbindung (I-99)

### Schritt 1

### 4-[6-(Ethoxycarbonyl)pyridin-2-yl]piperidiniumchlorid

Zu einer Lösung von Ethyl-6-[1-(tert-butoxycarbonyl)piperidin-4-yl]pyridin-2-carboxylat (2.0 g) wurde bei 0 °C eine 4-molare Lösung von Salzsäure in 1,4-Dioxan getropft (45 mL). Das Reaktionsgemisch wurde bei 0 °C gerührt und dann langsam auf Raumtemperatur erwärmt. Nach Rühren über Nacht wurden das Lösungsmittel und überschüssiger Chlorwasserstoff entfernt. Man erhielt 4-[6-(Ethoxycarbonyl)pyridin-2-yl]piperidiniumchlorid (1.75 g).
logP (pH2.7): 0.55
¹H NMR (DMSO-d₆, 400 MHz): δₚₚₘ : 1.34 (t, 3H), 2.10-1.95 (m, 4H), 3.07-2.96 (m, 2H), 3.12 (m, 1H), 3.48-3.33 (m, 2H), 4.21 (bs, 2H), 4.36 (q, 2H), 7.53 (dd, 1H), 7.89 (dd, 1H), 7.95 (t, 1H), 8.94 (bs, 1H), 9.32 (bs, 1H)
MS (ESI): 235 ([M-Cl]⁺)

### Schritt 2

### Ethyl-6-(1-{[3,5-bis-1H-pyrazol-1-yl]acetyl}piperidin-4-yl)pyridin-2-carboxylat

Zu einer Lösung von [1,5-Bis-(difluormethyl)-1H-pyrazol-1-yl]essigsäure (11.4 g) in Dichlormethan (50 mL) wird Oxalylchlorid (17.5 g) langsam getropft und wird ein Tropfen N,N-Dimethylformamid gegeben. Dann wird das Reaktionsgemisch übernacht gerührt. Dann wird der Überschuss Oxalylchlorid unter vermindertem Druck entfernt und der Rückstand wird wieder in Dichlormethan (20 mL) gelöst. Die Lösung wird dann zu der Lösung aus 4-[6-(Ethoxycarbonyl)pyridin-2-yl]piperidiniumchlorid in Dichlormethan (30 mL) und Diisopropylethylamin (17.8 g) gegeben Die Reaktionsmischung wird übernacht gerührt. Nach Zugabe von konz. Ammoniumchloridlösung wird die wässrige Phase abgetrennt und mit Essigsäureethylester extrahiert. Die vereinigten organischen Phasen werden über Natriumsulfat getrocknet und unter vermindertem Druck eingeengt. Der Rückstand wird chromatographisch gereinigt. Man erhält Ethyl-6-(1-{[3,5-bis(difluormethyl)-1H-pyrazol-1-yl]acetyl}piperidin-4-yl)pyridin-2-carboxylat (15.8 g).
logP (pH2.7): 2.68
¹H NMR (DMSO-d₆, 400 MHz): δₚₚₘ : 1.34 (t, 3H), 1.65 (bs, 1H), 1.80 (bs, 1H), 1.99-1.90 (m, 2H), 2.83 (bs, 1H), 3.10 (m, 1H), 3.25 (bs, 1H), 4.03 (bs, 1H), 4.36 (q, 2H), 4.40 (bs, 1H), 5.38-4.33 (m, 2H), 6.86 (s, 1H), 6.98 (t, 1H), 7.15 (t, 1H), 7.54 (dd, 1H), 7.87 (dd, 1H), 7.92 (t, 1H)
MS (ESI): 443 ([M+H]⁺)

### Schritt 3

### 6-(1-{[3,5-Bis H-pyrazol-1-yl]acetyl}piperidin-4-yl)pyridin-2-carbonsäure

Zu einer Lösung von Ethyl-6-(1-{[3,5-bis(difluormethyl)-1H-pyrazol-1-yl]acetyl}piperidin-4-yl)pyridin-2-carboxylat (15.5 g) in Tetrahydrofuran (160 mL) und Wasser (40 mL) wird bei Raumtemperatur Lithiumhydroxidmonohydrat (1.99 g) gegeben. Das Gemisch wird 1 Stunde bei Raumtemperatur gerührt und dann mit eiskalter IN HCl-Lösung versetzt. Die wässrige Phase wird mit Ethylacetat extrahiert. Die vereinigten organischen Phasen werden über Natriumsulfat getrocknet und unter vermindertem Druck eingeengt. Man erhält 6-(1-{[3,5-Bis(difluormethyl)-1H-pyrazol-1-yl]acetyl}piperidin-4-yl)pyridin-2-carbonsäure (11.5 g).
logP (pH2.7): 1.55
¹H NMR (DMSO-d₆, 400 MHz): δₚₚₘ : 1.70 (bs, 1H), 1.82 (bs, 1H), 1.99-1.90 (m, 2H), 2.82 (bs, 1H), 3.08 (m, 1H), 3.25 (bs, 1H), 4.02 (bs, 1H), 4.43 (bs, 1H), 5.36 (bs, 2H), 6.86 (s, 1H), 6.98 (t, 1H), 7.16 (t, 1H), 7.52 (dd, 1H), 7.93-7.86 (m, 2H)
MS (ESI): 415 ([M+H]⁺)

### Schritt 4

### Cyclohexyl-6-(1-{[3,5-bis)-1H-pyrazol-1-yl]acetyl}piperidin-4-yl)pyridin-2-carboxylat

Zu einer Lösung von 6-(1-{[3,5-Bis(difluormethyl)-1H-pyrazol-1-yl]acetyl}piperidin-4-yl)pyridin-2-carbonsäure (157 mg) in Dichlormethan (5 mL) wird bei Raumtemperatur Cyclohexanol (42 mg), 4-Dimethylaminopyridin (4.6 mg) und 1-Ethyl-3-(3'-dimethylaminopropyl)carbodiimide (80 mg) gegeben. Das Gemisch wird über Nacht bei Raumtemperatur gerührt und dann mit Wasser versetzt. Die wässrige Phase wird abgetrennt und mit Ethylacetat extrahiert. Die vereinigten organischen Phasen werden über Natriumsulfat getrocknet und unter vermindertem Druck eingeengt. Der Rückstand wird chromatographisch gereinigt. Man erhält Cyclohexyl-6-(1-{[3,5-bis(difluormethyl)-1H-pyrazol-1-yl]acetyl}piperidin-4-yl)pyridin-2-carboxylat (87 mg).
logP (pH2.7): 3.83
¹H NMR (DMSO-d₆, 400 MHz): δₚₚₘ : 1.27-1.98 (m, 14H), 2.76-2.84 (m, 1H), 3.02-3.11 (m, 1H), 3.25 (m, 1H), 3.98-4.03 (m, 1H), 4.38-4.43 (m, 1H), 4.91-4.98 (m, 1H), 5.34 (d, 1H), 5.44 (d, 1H), 6.90 (s, 1H), 7.03 (t, 1H), 7.18 (t, 1H), 7.56 (dd, 1H), 7.87-7.95 (m, 2H)
MS (ESI): 497 ([M+H]⁺)

### Herstellung von Verbindung (I-117)

### Schritt 1

### tert-Butyl-4-(6-brompyridin-2-yl)piperazin-1-carboxylat

2.6-Dibrompyridin (1.0 g) und *tert*-Butylpiperazin-1-carboxylat (870 mg) werden in N,N-Dimethylformamid (10 mL) gelöst und bei 80 °C für 5 Stunden in Gegenwart von Kalilumcarbonat (1.17 g) gerührt. Zum Reaktiongemisch wird *tert*-Butylpiperazin-1-carboxylat (200 mg) gegeben. Das Gemisch wird über Nacht bei Raumtemperatur gerührt und dann mit Wasser versetzt. Die wässrige Phase wird abgetrennt und mit Dichlromethan extrahiert. Die vereinigten organischen Phasen werden über Natriumsulfat getrocknet und unter vermindertem Druck eingeengt. Der Rückstand wird chromatographisch gereinigt. Man erhält *tert*-Butyl-4-(6-brompyridin-2-yl)piperazin-1-carboxylat (900 mg).
logP (pH2.7): 4.08
¹H NMR (DMSO-d₆, 400 MHz): δₚₚₘ : 1.42 (s, 9H), 3.44-3.40 (m, 4H), 3.50-3.46 (m, 4H), 6.78 (dd, 2H), 7.43 (dd, 1H)
MS (ESI): 342 und 344 ([M+H]⁺)

### Schritt 2

### tert-Butyl-4-[6-(ethoxycarbonyl)pyridin-2-yl]piperazin-1-carboxylat

*tert*-Butyl-4-(6-brompyridin-2-yl)piperazin-1-carboxylat (40 g) wird in Ethanol (300 mL) gelöst und bei 70 °C unter 3 bar CO für 48 Stunden in Gegenwart von PdCl₂(PPh)₂ (4.10 g) und Triethylamin (114 mL) gerührt. Der Katalysator wird durch Filtration über Celite entfernt und unter vermindertem Druck eingeengt. Nach chromatografischer Reinigung erhält man tert-Butyl-4-[6-(ethoxycarbonyl)pyridin-2-yl]piperazin-1-carboxylat (43 g).
logP (pH2.7): 3.32
¹H NMR (DMSO-d₆, 400 MHz): δₚₚₘ : 1.30 (t, 3H), 1.42 (s, 9H), 3.44-3.41 (m, 4H), 3.56-3.52 (m, 4H), 4.29 (q, 2H), 7.08 (d, 1H), 7.32 (d, 1H), 7.70 (dd, 1H)
MS (ESI): 336 ([M+H]⁺)

### Schritt 3

### Ethyl-6-(4-{[3,5-bis(difluormethyl)-1H-pyrazol-1-yl]acetyl}piperazin-1-yl)pyridin-2-carboxylat

Zu *tert*-Butyl-4-[6-(ethoxycarbonyl)pyridin-2-yl]piperazin-1-carboxylat (2.36 g) wird unter Argon und bei 0 °C eine Lösung von Chlorwasserstoff in Dioxan (4 M, 10.5 mL) gegeben. Das Gemisch wird bei 0 °C gerührt und danach langsam auf Raumtemperatur erwärmt. Nach Rühren für 2 Stunden werden die überschüssige Säure und das Lösungsmittel unter vermindertem Druck entfernt. Man erhält 4-[6-(Ethoxycarbonyl)pyridin-2-yl]piperazin-1-iumchlorid.

Zu einer Lösung von [3,5-Bis-(difluormethyl)-1H-pyrazol-1-yl]essigsäure (1.67 g) in Dichlormethan werden bei 0 °C Oxalylchlorid (2.68 g) und ein Tropfen N,N-Dimethylformamid gegeben. Das Reaktionsgemisch wird bei Raumtemperatur für 40 Minuten gerührt. Das Lösungsmittel und das überschüssige Reagenz werden unter vermindertem Druck entfernt. Der feste Rückstand wird dann erneut in Dichlormethan gelöst und tropfenweise bei 0 °C zu einer Lösung von 4-[6-(Ethoxycarbonyl)pyridin-2-yl]piperazin-1-iumchlorid und Triethylamine (9.8 mL) in Dichlormethan (10 mL) gegeben. Daraufhin wird die Reaktionslösung mit konzentrierter Natriumhydrogencarbonatlösung versetzt, die wässrige Phase abgetrennt und mit Essigsäureethylester extrahiert. Die vereinigten organischen Phasen werden über wasserfreiem Natriumsulfat getrocknet und eingeengt. Man erhält Ethyl-6-(4-{[3,5-bis(difluormethyl)-1H-pyrazol-1-yl]acetyl}piperazin-1-yl)pyridin-2-carboxylat (1.3 g).
logP (pH2.7): 2.71
¹H NMR (DMSO-d₆, 400 MHz): δₚₚₘ : 1.31 (t, 3H), 3.59 (bs, 4H), 3.72-3.62 (m, 4H), 4.30 (q, 2H), 5.42 (bs, 2H), 6.91 (s, 1H), 7.03 (t, 1H), 7.13 (d, 1H), 7.18 (t, 1H), 7.35 (d, 1H), 7.73 (dd, 1H)
MS (ESI): 444 ([M+H]⁺)

### Schritt 4

### 6-(4-{[3,5-Bis(difluormethyl)-1H-pyrazol-1-yl]acetyl}piperazin-1-yl)pyridin-2-carbonsäure

Zu einer Lösung von Ethyl-6-(4-{[3,5-bis(difluormethyl)-1H-pyrazol-1-yl]acetyl}piperazin-1-yl)pyridin-2-carboxylat (1.3 g) in Tetrahydrofuran (20 mL) und Wasser (5 mL) wird bei Raumtemperatur Lithiumhydroxidmonohydrat (185 mg) gegeben. Das Gemisch wird 5 Stunde bei Raumtemperatur gerührt und dann mit eiskalter IN HCl-Lösung versetzt. Die wässrige Phase wird mit Ethylacetat extrahiert und dann die vereinigten organischen Phasen mit Natriumsulfat getrocknet. Der Feststoff wird abfiltriert und das Lösungsmittel abdestilliert. Man erhält 6-(4-{[3,5-Bis(difluormethyl)-1H-pyrazol-1-yl]acetyl}piperazin-1-yl)pyridin-2-carbonsäure (700 mg).
logP (pH2.7): 0.68
¹H NMR (DMSO-d₆, 400 MHz): δₚₚₘ : 3.67-3.55 (m, 6H), 3.74-3.69 (m, 2H), 5.14 (bs, 1H), 5.42 (bs, 2H), 6.91 (s, 1H), 7.03 (t, 1H), 7.11 (d, 1H), 7.18 (t, 1H), 7.35 (d, 1H), 7.73 (dd, 1H)
MS (ESI): 416 ([M+H]⁺)

### Schritt 5

### 1,2,3,4-Tetrahydronaphthalen-1-yl-6-(4-{[3,5-bis(difluormethyl)-1H-pyrazol-1-yl]acetyl} piperazin-1-yl)pyridin-2-carboxylat

Zu einer Lösung von 6-(4-{[3,5-Bis(difluormethyl)-1H-pyrazol-1-yl]acetyl}piperazin-1-yl)pyridin-2-carbonsäure (150 mg) in Dichlormethan (5 mL) wird bei Raumtemperatur 1,2,3,4-Tetrahydronaphthalen-1-ol (70 mg), 4-Dimethylaminopyridin (4.4 mg) und 1-Ethyl-3-(3'-dimethylaminopropyl)carbodiimide-HCl (104 mg) gegeben. Das Gemisch wird über Nacht bei Raumtemperatur gerührt und dann mit Wasser versetzt. Die wässrige Phase wird abgetrennt und mit Ethylacetat extrahiert und dann die vereinigten organischen Phasen mit Natriumsulfat getrocknet. Der Feststoff wird abfiltriert und das Lösungsmittel abdestilliert. Der Rückstand wird chromatographisch gereinigt. Man erhält 1,2,3,4-Tetrahydronaphthalen-1-yl-6-(4-{[3,5-bis(difluormethyl)-1H-pyrazol-1yl]-acetyl}piperazin-1-yl)pyridin-2-carboxylat (70 mg).
logP (pH2.7): 4.02
¹H NMR (DMSO-d₆, 400 MHz): δₚₚₘ : 1.86 (m, 1H), 2.11-1.90 (m, 3H), 2.81-2.73 (m, 1H), 2.92-2.84 (m, 1H), 3.57 (s, 4H), 3.65-3.60 (m, 2H), 3.70-3.66 (m, 2H), 5.41 (bs, 2H), 6.13 (t, 1H), 6.91 (s, 1H), 7.02 (t, 1H), 7.28-7.10 (m, 5H), 7.31 (d, 2H), 7.70 (dd, 1H)
MS (ESI): 546 ([M+H]⁺)

### Beispiele

Analog zu den zuvor angegebenen Methoden können die in der nachstehenden Tabelle 1 aufgeführten Verbindungen der Formel **(I)** erhalten werden.

**Tabelle I**

| | | | | | | | |
|---|---|---|---|---|---|---|---|
| 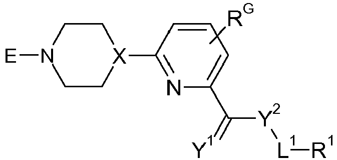 | | | | | | | |
| R^{G} = H | | | | | | | |

| **Bsp.** | **E** | **X** | **Y¹** | **Y²** | **L¹** | **R¹** | **Log p** |
|---|---|---|---|---|---|---|---|
| 1 | [3,5-Bis(difluormethyl)-1H-pyrazol-1-yl]acetyl | CH | O | NCH₃ | CH₂ | 2-Bromphenyl | 3,32^{[b]}; 3,37^{[c]} |
| 2 | [5-Methyl-3-(trifluormethyl)-1H-pyrazol-1-yl]acetyl | CH | O | NCH₃ | CH₂ | 2-Bromphenyl | 3,47^{[b]}; 3,49^{[c]} |
| 3 | [5-Methyl-3-(trifluormethyl)-1H-pyrazol-1-yl]acetyl | N | O | NCH₃ | CH₂ | 2-Bromphenyl | 3,44^{[c]}; 3,46^{\|c]} |
| 4 | [3,5-Bis(difluormethyl)-1H-pyrazol-1-yl]acetyl | CH | O | NH | CH₂ | 2-Bromphenyl | 3,44^{[b]}; 3,47^{[c]} |
| 5 | [5-Methyl-3-(trifluormethyl)-1H-pyrazol-1-yl]acetyl | CH | O | NH | CH₂ | 2-Bromphenyl | 3,59^{[b]}; 3,59^{[c]} |
| 6 | [5-Methyl-3-(trifluormethyl)-1H-pyrazol-1-yl]acetyl | CH | O | O | CH₂CH₂CH₂ | CF₃ | 3,37^{[b]} |
| 7 | [3,5-Bis(difluormethyl)-1H-pyrazol-1-yl]acetyl | CH | O | O | CH₂ | 2-(Trifluormethoxy)phenyl | 3,98^{[b]} |
| 8 | [5-Methyl-3-(trifluormethyl)-1H-pyrazol-1-yl]acetyl | CH | O | O | CH₂ | 2-(Trifluormethoxy)phenyl | 4,06^{[b]} |
| 9 | [3,5-Bis(difluormethyl)-1H-pyrazol-1-yl]acetyl | CH | O | O | CH₂ | 2-(Trifluormethyl )phenyl | 3,89^{[b]} |
| 10 | [5-Methyl-3-(trifluormethyl)-1H-pyrazol-1-yl]acetyl | CH | O | NCH₃ | Bindung | 2-Chlorphenyl | 3,18^{[b]}; 3,2^{[c]} |
| 11 | [5-Methyl-3-(trifluormethyl)-1H-pyrazol-1-yl]acetyl | CH | O | NCH₃ | Bindung | Cyclohexyl | 3,15^{[b]}; 3,18^{[c]} |
| 12 | [5-Methyl-3-(trifluormethyl)-1H-pyrazol-1-yl]acetyl | CH | O | NCH₃ | Bindung | 1,2,3,4-Tetrahydronaphthalen-1-yl | 3,61^{[b]} |
| 13 | [5-Methyl-3-(trifluormethyl)-1H-pyrazol-1-yl]acetyl | CH | O | NCH₃ | CH₂ | 2,4-Dichlorphenyl | 3,85^{[b]}; 3,85^{[c]} |
| 14 | [5-Methyl-3-(trifluormethyl)-1H-pyrazol-1-yl]acetyl | CH | O | NCH₃ | CH₂ | Pyridin-2-yl | 2,23^{[c]} |
| 15 | [5-Methyl-3-(trifluormethyl)-1H-pyrazol-1-yl]acetyl | CH | O | Y²-L¹-R¹= 2,3 -Dihydro-4H-1,4-benzoxazin-4-yl | | | 3,04^{[b]}; 3,04^{[c]} |
| 16 | [5-Methyl-3-(trifluormethyl)-1H-pyrazol-1-yl]acetyl | CH | O | NH | CH(CH₃) | 2-Chlorphenyl | 3,82[^{b]}; 3,75^{[c]} |
| 17 | [5-Methyl-3-(trifluormethyl)-1H-pyrazol-1-yl]acetyl | CH | O | NH | CH(CH₃) | Pentyl | 4,17^{[b]}; 4,13^{[c]} |
| 18 | [5-Methyl-3-(trifluormethyl)-1H-pyrazol-1-yl]acetyl | CH | O | NH | Bindung | Cyclohexyl | 3,46^{[c]} |
| 19 | [5-Methyl-3-(trifluormethyl)-1H-pyrazol-1-yl]acetyl | CH | O | NH | Bindung | 2,3-Dihydro-1H-inden-1-yl | 3,56^{[b]}; 3,56^{[c]} |
| 20 | [5-Methyl-3-(trifluormethyl)-1H-pyrazol-1-yl]acetyl | CH | O | NH | CH₂CH₂CH₂ | Butyl | 4,25^{[b]}; 4,24^{[c]} |
| 21 | [5-Methyl-3-(trifluormethyl)-1H-pyrazol-1-yl]acetyl | CH | O | NH | CH₂ | 2,4-Dichlorphenyl | 4,1^{[b]}; 4^{[c]} |
| 22 | [5-Methyl-3-(trifluormethyl)-1H-pyrazol-1-yl]acetyl | CH | O | NH | CH₂ | 2,4-Difluorphenyl | 3,36^{[b]}; 3,36^{[c]} |
| 23 | [5-Methyl-3-(trifluormethyl)-1H-pyrazol-1-yl]acetyl | CH | O | NH | CH₂ | 2-Chlorphenyl | 3,49^{[b]}; 3,52^{[c]} |
| 24 | [5-Methyl-3-(trifluormethyl)-1H-pyrazol-1-yl]acetyl | CH | O | NH | CH₂ | 2,6-Difluorphenyl | 3,33^{[b]}; 3,26^{[c]} |
| 25 | [5-Methyl-3-(trifluormethyl)-1H-pyrazol-1-yl]acetyl | CH | O | NH | CH₂ | Pyridin-2-yl | 1,97^{[b]}; 2,44^{[c]} |
| 26 | [5-Methyl-3-(trifluormethyl)-1H-pyrazol-1-yl]acetyl | CH | O | NH | CH₂ | Thiophen-2-yl | 3,15^{[b]}; 3,1^{[c]} |
| 27 | [5-Methyl-3-(trifluormethyl)-1H-pyrazol-1-yl]acetyl | CH | O | NH | CH₂ | 3-Methylthiophen-2-yl | 3,29^{[b]}; 3,34^{[c]} |
| 28 | [5-Methyl-3-(trifluormethyl)-1H-pyrazol-1-yl]acetyl | CH | O | NH | Bindung | 2,6-Difluorphenyl | 3,13^{[b]}; |
| 29 | [5-Methyl-3-(trifluormethyl)-1H-pyrazol-1-yl]acetyl | CH | O | NH | Bindung | Naphthalen-1-yl | 3,94^{[b]}; 3,91^{[c]} |
| 30 | [5-Methyl-3-(trifluormethyl)-1H-pyrazol-1-yl]acetyl | CH | O | NH | Bindung | Naphthalen-2-yl | 4,0^{[b]}; 4,11^{[c]} |
| 31 | [5-Methyl-3-(trifluormethyl)-1H-pyrazol-1-yl]acetyl | CH | O | NH | Bindung | Chinolin-8-yl | 4,02^{[b]}; 4,04^{[c]} |
| 32 | [5-Methyl-3-(trifluormethyl)-1H-pyrazol-1-yl]acetyl | CH | O | O | CH(CH₃) | 2-Chlorphenyl | 4,12^{[b]} |
| 33 | [5-Methyl-3-(trifluormethyl)-1H-pyrazol-1-yl]acetyl | CH | O | O | Bindung | Cyclohex-2-en-1-yl | 3,64^{[b]} |
| 34 | [5-Methyl-3-(trifluormethyl)-1H-pyrazol-1-yl]acetyl | CH | O | O | Bindung | Cyclohexyl | 3,9^{[b]} |
| 35 | [5-Methyl-3-(trifluormethyl)-1H-pyrazol-1-yl]acetyl | CH | O | O | Bindung | 1,2,3,4-Tetrahydronaphthalen-1-yl | 4,15^{[b]} |
| 36 | [5-Methyl-3-(trifluormethyl)-1H-pyrazol-1-yl]acetyl | CH | O | O | CH₂C≡C | 2-Chlorphenyl | 4,03^{[b]} |
| 37 | [5-Methyl-3-(trifluormethyl)-1H-pyrazol-1-yl]acetyl | CH | O | O | CH₂ | tert-Butyl | 3,86^{[b]} |
| 38 | [5-Methyl-3-(trifluormethyl)-1H-pyrazol-1-yl]acetyl | CH | O | O | CH₂ | Cyclohexyl | 4,38^{[b]} |
| 39 | [5-Methyl-3-(trifluormethyl)-1H-pyrazol-1-yl]acetyl | CH | O | O | CH₂CH₂ | But-3-en-1-yl | 3,86^{[b]} |
| 40 | [5-Methyl-3-(trifluormethyl)-1H-pyrazol-1-yl]acetyl | CH | O | O | CH₂CH₂ | Pentyl | 4,73^{[b]} |
| 41 | [5-Methyl-3-(trifluormethyl)-1H-pyrazol-1-yl]acetyl | CH | O | O | CH₂CH₂ | Methoxy | 2,46^{[b]} |
| 42 | [5-Methyl-3-(trifluormethyl)-1H-pyrazol-1-yl]acetyl | CH | O | O | CH₂ | 2,4-Difluorphenyl | 3,6^{[b]} |
| 43 | [5-Methyl-3-(trifluormethyl)-1H-pyrazol-1-yl]acetyl | CH | O | O | CH₂ | 2,4,6-Trifluorphenyl | 3,69^{[b]} |
| 44 | [5-Methyl-3-(trifluormethyl)-1H-pyrazol-1-yl]acetyl | CH | O | O | CH₂ | 2-Methylphenyl | 3,8^{[b]} |
| 45 | [5-Methyl-3-(trifluormethyl)-1H-pyrazol-1-yl]acetyl | CH | O | O | CH₂ | 2-(Trifluormethyl)phenyl | 3,97^{[b]} |
| 46 | [5-Methyl-3-(trifluormethyl)-1H-pyrazol-1-yl]acetyl | CH | O | O | CH₂ | 2-Chlorphenyl | 3,84^{[b]} |
| 47 | [5-Methyl-3-(trifluormethyl)-1H-pyrazol-1-yl]acetyl | CH | O | O | CH₂ | 2-Fluorphenyl | 3,52^{[b]} |
| 48 | [5-Methyl-3-(trifluormethyl)-1H-pyrazol-1-yl]acetyl | CH | O | O | CH₂ | 2,6-Difluorphenyl | 3,51^{[b]} |
| 49 | [5-Methyl-3-(trifluormethyl)-1H-pyrazol-1-yl]acetyl | CH | O | O | CH₂ | 3-Methylpyridin-2-yl | 2,23^{[b]} |
| 50 | [5-Methyl-3-(trifluormethyl)-1H-pyrazol-1-yl]acetyl | CH | O | O | CH₂ | 4-Methylthiophen-2-yl | 3,67^{[b]} |
| 51 | [5-Methyl-3-(trifluormethyl)-1H-pyrazol-1-yl]acetyl | CH | O | O | Bindung | Naphthalen-1-yl | 3,97^{[b]} |
| 52 | [5-Methyl-3-(trifluormethyl)-1H-pyrazol-1-yl]acetyl | CH | O | O | Bindung | Naphthalen-2-yl | 3,9^{[b]} |
| 53 | [5-Methyl-3-(trifluormethyl)-1H-pyrazol-1-yl]acetyl | CH | O | S | Bindung | Cyclohexyl | 4,83^{[c]} |
| 54 | [5-Methyl-3-(trifluormethyl)-1H-pyrazol-1-yl]acetyl | CH | O | S | CH₂CH₂CH₂ | Butyl | |
| 55 | [5-Methyl-3-(trifluormethyl)-1H-pyrazol-1-yl]acetyl | CH | O | S | CH₂ | 2,4-Dichlorphenyl | 5,17^{[b]}; 5,21^{[c]} |
| 56 | [5-Methyl-3-(trifluormethyl)-1H-pyrazol-1-yl]acetyl | CH | O | S | CH₂ | 2-Methylphenyl | 4,57^{[b]}; 4,58^{[c]} |
| 57 | [5-Methyl-3-(trifluormethyl)-1H-pyrazol-1-yl]acetyl | CH | O | S | CH₂ | 2-Chlorphenyl | 4,61^{[b]}; 4,61^{[c]} |
| 58 | [5-Methyl-3-(trifluormethyl)-1H-pyrazol-1-yl]acetyl | CH | O | S | CH₂ | 2,6-Difluorphenyl | 4,27^{[b]}; 4,29^{[c]} |
| 59 | [5-Methyl-3-(trifluormethyl)-1H-pyrazol-1-yl]acetyl | CH | O | S | CH₂ | 4-Fluorphenyl | 4,29^{[b]}; 4,31^{[c]} |
| 60 | [5-Methyl-3-(trifluormethyl)-1H-pyrazol-1-yl]acetyl | CH | O | S | Bindung | Naphthalen-1-yl | 4,41^{[b]}; 4,46^{[c]} |
| 61 | [5-Methyl-3-(trifluormethyl)-1H-pyrazol-1-yl]acetyl | CH | O | S | Bindung | Naphthalen-2-yl | 4,57^{[b]}; 4,61^{[c]} |
| 62 | [5-Methyl-3-(trifluormethyl)-1H-pyrazol-1-yl]acetyl | CH | O | S | Bindung | Phenyl | 3,95^{[b]}; 3,95^{[c]} |
| 63 | [5-Methyl-3-(trifluormethyl)-1H-pyrazol-1-yl]acetyl | CH | O | S | Bindung | Thiophen-2-yl | 3,85^{[b]}; 3,88^{[c]} |
| 64 | [5-Methyl-3-(trifluormethyl)-1H-pyrazol-1-yl]acetyl | N | O | NCH₃ | Bindung | Cyclohexyl | 3,19^{[b]}; 3,21^{[c]} |
| 65 | [5-Methyl-3-(trifluormethyl)-1H-pyrazol-1-yl]acetyl | N | O | NH | CH₂ | 2-Chlorphenyl | 3,34^{[b]}; 3,36^{[c]} |
| 66 | [5-Methyl-3-(trifluormethyl)-1H-pyrazol-1-yl]acetyl | N | O | NH | CH₂ | 2,6-Difluorphenyl | 3,11^{[b]}; 3,12^{[c]} |
| 67 | [5-Methyl-3-(trifluormethyl)-1H-pyrazol-1-yl]acetyl | N | O | NH | CH₂ | Thiophen-2-yl | 2,92^{[b]}_{;} 2,93^{[c]} |
| 68 | [5-Methyl-3-(trifluormethyl)-1H-pyrazol-1-yl]acetyl | N | O | O | Bindung | Cyclohex-2-en-1-yl | 3,67^{[b]}; 3,7^{[c]} |
| 69 | [5-Methyl-3-(trifluormethyl)-1H-pyrazol-1-yl]acetyl | N | O | O | Bindung | Cyclohexyl | 3,96^{[b]}; 3,98^{[c]} |
| 70 | [5-Methyl-3-(trifluormethyl)-1H-pyrazol-1-yl]acetyl | N | O | O | Bindung | 1,2,3,4-Tetrahydronaphthalen-1-yl | 4,19^{[b]}; 4,2^{[c]} |
| 71 | [5-Methyl-3-(trifluormethyl)-1H-pyrazol-1-yl]acetyl | N | O | O | CH₂CH₂ | But-3-en-1-yl | 3,92^{[b]}; 3,95^{[c]} |
| 72 | [5-Methyl-3-(trifluormethyl)-1H-pyrazol-1-yl]acetyl | N | O | O | CH₂ | 2-Chlorphenyl | 3,89^{[b]}; 3,9^{[c]} |
| 73 | [5-Methyl-3-(trifluormethyl)-1H-pyrazol-1-yl]acetyl | N | O | O | CH₂ | 2-Fluorphenyl | 3,58^{[b]}; 3,6^{[c]} |
| 74 | [5-Methyl-3-(trifluormethyl)-1H-pyrazol-1-yl]acetyl | N | O | O | CH₂ | 2,6-Difluorphenyl | 3,54^{[b]}; 3,55^{[c]} |
| 75 | [5-Methyl-3-(trifluormethyl)-1H-pyrazol-1-yl]acetyl | N | O | O | Bindung | Naphthalen-1-yl | 3,99^{[b]}; 4^{[c]} |
| 76 | [5-Methyl-3-(trifluormethyl)-1H-pyrazol-1-yl]acetyl | N | O | O | Bindung | Naphthalen-2-yl | 4,01^{[b]}; 4,02^{[c]} |
| 77 | [3,5-Bis(difluormethyl)-1H-pyrazol-1-yl]acetyl | CH | O | NCH₃ | Bindung | 2-Chlorphenyl | 3,01^{[b]}; 3,01^{[c]} |
| 78 | [3,5-Bis(difluormethyl)-1H-pyrazol-1-yl]acetyl | CH | O | NCH₃ | Bindung | Cyclohexyl | 3,02^{[b]}; 3,08^{[c]} |
| 79 | [3,5-Bis(difluormethyl)-1H-pyrazol-1-yl]acetyl | CH | O | NCH₃ | CH₂ | 2,4-Dichlorphenyl | 3,66^{[b]} 3,73^{[c]} |
| 80 | [3,5-Bis(difluormethyl)-1H-pyrazol-1-yl]acetyl | CH | O | NCH₃ | CH₂ | Pyridin-2-yl | 1,81^{[b]}; 2,18^{[c]} |
| 81 | [3,5-Bis(difluormethyl)-1H-pyrazol-1-yl]acetyl | CH | O | Y²⁻L¹-R¹= 2,3-Dihydro-4H-1,4-benzoxazin-4-yl | | | 2,98^{[b]}; 2,97^{[c]} |
| 82 | [3,5-Bis(difluormethyl)-1H-pyrazol-1-yl]acetyl | CH | O | NH | CH(CH₃) | 2-Chlorphenyl | 3,53^{[b]}; 3,61^{[c]} |
| 83 | [3,5-Bis(difluormethyl)-1H-pyrazol-1-yl]acetyl | CH | O | NH | CH(CH₃) | Pentyl | 3,99^{[b]}; 3,95^{[c]} |
| 84 | [3,5-Bis(difluormethyl)-1H-pyrazol-1-yl]acetyl | CH | O | NH | Bindung | 2,3-Dihydro-1H-inden-1-yl | 3,4^{[b]}; 3,44^{[c]} |
| 85 | [3,5-Bis(difluormethyl)-1H-pyrazol-1-yl]acetyl | CH | O | NH | CH₂CH₂CH₂ | Butyl | 3,96^{[b]}; 4,05^{[c]} |
| 86 | [3,5-Bis(difluormethyl)-1H-pyrazol-1-yl]acetyl | CH | O | NH | CH₂ | 2,4-Dichlorphenyl | 3,86^{[b]}; 3,86^{[c]} |
| 87 | [3,5-Bis(difluormethyl)-1H-pyrazol-1-yl]acetyl | CH | O | NH | CH₂ | 2,4-Difluorphenyl | 3,22^{[b]}; 3,24^{[c]} |
| 88 | [3,5-Bis(difluormethyl)-1H-pyrazol-1-yl]acetyl | CH | O | NH | CH₂ | 2-Chlorphenyl | 3,35^{[b]}; 3,39^{[c]} |
| 89 | [3,5-Bis(difluormethyl)-1H-pyrazol-1-yl]acetyl | CH | O | NH | CH₂ | 2,6-Difluorphenyl | 3,11^{[b]}; 3,14^{[c]} |
| 90 | [3,5-Bis(difluormethyl)-1H-pyrazol-1-yl]acetyl | CH | O | NH | CH₂ | Pyridin-2-yl | 1,83^{[b]}; 2,37^{[c]} |
| 91 | [3,5-Bis(difluormethyl)-1H-pyrazol-1-yl]acetyl | CH | O | NH | CH₂ | Thiophen-2-yl | 2,98^{[b]}; 2,97^{[c]} |
| 92 | [3,5-Bis(difluormethyl)-1H-pyrazol-1-yl]acetyl | CH | O | NH | CH₂ | 3-Methylthiophen-2-yl | 3,19^{[b]}; 3,18^{[c]} |
| 93 | [3,5-Bis(difluormethyl)-1H-pyrazol-1-yl]acetyl | CH | O | NH | Bindung | 2,6-Difluorphenyl | 3,06^{[b]} 3,04^{[c]} |
| 94 | [3,5-Bis(difluormethyl)-1H-pyrazol-1-yl]acetyl | CH | O | NH | Bindung | Naphthalen-1-yl | 3,79^{[b]}; 3,79^{[c]} |
| 95 | [3,5-Bis(difluormethyl)-1H-pyrazol-1-yl]acetyl | CH | O | NH | Bindung | Naphthalen-2-yl | 4,03^{[b]}; 3,98^{[c]} |
| 96 | [3,5-Bis(difluormethyl)-1H-pyrazol-1-yl]acetyl | CH | O | NH | Bindung | Chinolin-8-yl | 3,91^{[b]}; 3,92^{[c]} |
| 97 | [3,5-Bis(difluormethyl)-1H-pyrazol-1-yl]acetyl | CH | O | O | Bindung | 2-Methylcyclohexyl | 4,17^{[b]} |
| 98 | [3,5-Bis(difluormethyl)-1H-pyrazol-1-yl]acetyl | CH | O | O | Bindung | Cyclohex-2-en-1-yl | 3,87^{[b]} |
| 99 | [3,5-Bis(difluormethyl)-1H-pyrazol-1-yl]acetyl | CH | O | O | Bindung | Cyclohexyl | 3,83^{[b]} |
| 100 | [3,5-Bis(difluormethyl)-1H-pyrazol-1-yl]acetyl | CH | O | O | Bindung | (1R)-1,2,3,4-Tetrahydronaphthalen-1-yl | 4,07^{[b]} |
| 101 | [3,5-Bis(difluormethyl)-1H-pyrazol-1-yl]acetyl | CH | O | O | CH₂CH₂ | But-3-en-1-yl | 3,8^{[b]} |
| 102 | [3,5-Bis(difluormethyl)-1H-pyrazol-1-yl]acetyl | CH | O | O | CH₂CH₂ | Pentyl | 4,59^{[b]} |
| 103 | [3,5-Bis(difluormethyl)-1H-pyrazol-1-yl]acetyl | CH | O | O | CH₂ | 2,4-Difluorphenyl | 3,54^{[b]} |
| 104 | [3,5-Bis(difluormethyl)-1H-pyrazol-1-yl]acetyl | CH | O | O | CH₂ | 2,4,6-Trifluorphenyl | 3,57^{[b]} |
| 105 | [3,5-Bis(difluormethyl)-1H-pyrazol-1-yl]acetyl | CH | O | O | CH₂ | 2-Chlorphenyl | 3,77^{[b]} |
| 106 | [3,5-Bis(difluormethyl)-1H-pyrazol-1-yl]acetyl | CH | O | O | CH₂ | 2-Fluorphenyl | 3,45^{[b]} |
| 107 | [3,5-Bis(difluormethyl)-1H-pyrazol-1-yl]acetyl | CH | O | O | CH₂ | 2,6-Difluorphenyl | 3,44^{LBJ} |
| 108 | [3,5-Bis(difluormethyl)-1H-pyrazol-1-yl]acetyl | CH | O | O | CH₂ | 3-Methylpyridin-2-yl | 2,14^{[b]} |
| 109 | [3,5-Bis(difluormethyl)-1H-pyrazol-1-yl]acetyl | CH | O | O | CH₂ | 4-Methylthiophen-2-yl | 3,6^{[b]} |
| 110 | [3,5-Bis(difluormethyl)-1H-pyrazol-1-yl]acetyl | CH | O | O | Bindung | Naphthalen-1-yl | 3,82^{[b]} |
| 111 | [3,5-Bis(difluormethyl)-1H-pyrazol-1-yl]acetyl | CH | O | O | Bindung | Naphthalen-2-yl | 3,86^{[b]} |
| 112 | [3,5-Bis(difluormethyl)-1H-pyrazol-1-yl]acetyl | CH | O | S | Bindung | Cyclohexyl | 4,63^{[a]}; 4,65^{[c]} |
| 113 | [3,5-Bis(difluormethyl)-1H-pyrazol-1-yl]acetyl | CH | O | S | CH₂ | 2,6-Difluorphenyl | 4,12^{[a]} ; 4,13^{[c]} |
| 114 | [3,5-Bis(difluormethyl)-1H-pyrazol-1-yl]acetyl | CH | O | S | Bindung | Naphthalen-1-yl | 4,4^{[a]} ; 4,42^{[c]} |
| 115 | [3,5-Bis(difluormethyl)-1H-pyrazol-1-yl]acetyl | CH | O | O | Bindung | 2-Fluorcyclohexyl | 3,37^{[b]} |
| 116 | [5-Methyl-3-(trifluormethyl)-1H-pyrazol-1-yl]acetyl | CH | O | O | Bindung | 2-Fluorcyclohexyl | 3,43^{[b]} |
| 117 | [3,5-Bis(difluormethyl)-1H-pyrazol-1-yl]acetyl | N | O | O | Bindung | 1,2,3,4-Tetrahydronaphthalen-1-yl | 4,02^{[a]}; 4,07^{[c]} |
| 118 | [5-Methyl-3-(trifluormethyl)-1H-pyrazol-1-yl]acetyl | N | O | O | CH₂ | 2,4,6-Trifluorphenyl | 3,68^{[b]}; 3,71^{[c]} |
| 119 | [3,5-Bis(difluormethyl)-1H-pyrazol-1-yl]acetyl | N | O | S | Bindung | Cyclohexyl | 4,61^{[a]}; 4,65^{[c]} |
| 120 | [3,5-Bis(difluormethyl)-1H-pyrazol-1-yl]acetyl | N | O | S | Bindung | Naphthalen-1-yl | 4,25^{[a]}; 4,28^{[c]} |
| 121 | [3,5-Bis(difluormethyl)-1H-pyrazol-1-yl]acetyl | CH | O | S | Bindung | Phenyl | 3,8^{[a]}; 3,85^{[c]} |
| 122 | [3,5-Bis(difluormethyl)-1H-pyrazol-1-yl]acetyl | N | O | S | Bindung | Phenyl | 3,79^{[a]}; 3,81^{[c]} |
| 123 | [3,5-Bis(difluormethyl)-1H-pyrazol-1-yl]acetyl | CH | O | S | Bindung | Thiophen-2-yl | 3,76^{[a]}; 3,77^{[c]} |
| 124 | [3,5-Bis(difluormethyl)-1H-pyrazol-1-yl]acetyl | N | O | S | Bindung | Thiophen-2-yl | 3,73^{[a]}; 3,75^{[c]} |
| 125 | [3,5-Bis(difluormethyl)-1H-pyrazol-1-yl]acetyl | N | O | S | CH₂ | 2,6-Difluorphenyl | 4,11^{[a]}; 4,14^{[c]} |
| 126 | [3,5-Bis(difluormethyl)-1H-pyrazol-1-yl]acetyl | CH | O | S | CH₂ | 2-Chlorphenyl | 4,44^{[a]}; 4,49^{[c]} |
| 127 | [3,5-Bis(difluormethyl)-1H-pyrazol-1-yl]acetyl | N | O | S | CH₂ | 2-Chlorphenyl | 4,42^{[a]}; 4,44^{[c]} |
| 128 | [3,5-Bis(difluormethyl)-1H-pyrazol-1-yl]acetyl | CH | O | S | CH₂ | 4-Fluorphenyl | 4,1^{[a]}; 4,15^{[c]} |
| 129 | [3,5-Bis(difluormethyl)-1H-pyrazol-1-yl]acetyl | CH | O | S | CH₂CH₂CH₂ | Butyl | 5,43^{[a]}; 5,44^{[c]} |
| 130 | [3,5-Bis(difluormethyl)-1H-pyrazol-1-yl]acetyl | N | O | S | CH₂CH₂CH₂ | Butyl | 514^{[a]} ; 5,38^{[c]} |
| 131 | [3,5-Bis(difluormethyl)-1H-pyrazol-1-yl]acetyl | CH | O | O | CH₂ | CH₃ | 2,68^{[b]} |
| 132 | [5-Methyl-3-(trifluormethyl)-1H-pyrazol-1-yl]acetyl | CH | O | O | CH₂ | CH₃ | 2,73^{[b]} |
| 133 | [3,5-Bis(difluormethyl)-1H-pyrazol-1-yl]acetyl | CH | O | S | CH₂ | 2-Methylphenyl | 4,39^{[a]}; 4,41^{[c]} |
| 134 | [5-Methyl-3-(trifluormethyl)-1H-pyrazol-1-yl]acetyl | CH | O | O | CH₂ | CH₃ | 2,74^{[b]} |
| 135 | [3,5-Bis(difluormethyl)-1H-pyrazol-1-yl]acetyl | CH | O | O | Bindung | H | 1,55^{[b]} |
| 136 | [5-Methyl-3-(trifluormethyl)-1H-pyrazol-1-yl]acetyl | CH | O | O | Bindung | H | |
| 137 | [3,5-Bis(difluormethyl)-1H-pyrazol-1-yl]acetyl | N | O | O | CH₂ | 2-Fluorphenyl | 3,47^{[a]}; 3,49^{[c]} |
| 138 | [3,5-Bis(difluormethyl)-1H-pyrazol-1-yl]acetyl | N | O | O | CH₂ | 2,6-Difluorphenyl | 3,43^{[a]}; 3,43^{[c]} |
| 139 | [3,5-Bis(difluormethyl)-1H-pyrazol-1-yl]acetyl | N | O | O | Bindung | 5,6,7,8-Tetrahydronaphthalen-1-yl | 4,25^{[a]}; 4,29^{[c]} |
| 140 | [3,5-Bis(difluormethyl)-1H-pyrazol-1-yl]acetyl | N | O | O | Bindung | Cyclohexyl | 3,83^{[a]}, 3,86^{[c]} |
| 141 | [3,5-Bis(difluormethyl)-1H-pyrazol-1-yl]acetyl | N | O | O | Bindung | Naphthalen-1-yl | 3,88^{[c]} |
| 142 | [3,5-Bis(difluormethyl)-1H-pyrazol-1-yl]acetyl | N | O | O | Bindung | Cyclohex-2-en-1-yl | 3,56^{[a]}; 3,6^{[c]} |
| 143 | [3,5-Bis(difluormethyl)-1H-pyrazol-1-yl]acetyl | N | O | NCH₃ | Bindung | 1,2,3,4-Tetrahydronaphthalen-1-yl | 3,42^{[a]}; 3,45^{[c]} |
| 144 | [3,5-Bis(difluormethyl)-1H-pyrazol-1-yl]acetyl | N | O | NH | CH₂ | 2,4-Dichlorphenyl | 3,68^{[a]}; 3,7^{[c]} |
| 145 | [5-Methyl-3-(trifluormethyl)-1H-pyrazol-1-yl]acetyl | N | O | S | CH₂ | 2-Methylphenyl | 4,52^{[b]}; 4,55^{[c]} |
| 146 | [3,5-Bis(difluormethyl)-1H-pyrazol-1-yl]acetyl | N | O | NH | CH₂ | 2-Chlorphenyl | 3,24^{[a]}; 3,26^{[c]} |
| 147 | [5-Methyl-3-(trifluormethyl)-1H-pyrazol-1-yl]acetyl | N | O | NCH₃ | Bindung | 1,2,3,4-Tetrahydronaphthalen-1-yl | 3,55^{[b]}; 3,59^{[c]} |
| 148 | [3,5-Bis(difluormethyl)-1H-pyrazol-1-yl]acetyl | N | O | NCH₃ | CH₂ | 2-Chlorphenyl | 3,22^{[a]}; 3,25^{[c]} |
| 149 | [5-Methyl-3-(trifluormethyl)-1H-pyrazol-1-yl]acetyl | N | O | S | CH₂ | 2,6-Difluorphenyl | 4,11^{[b]}; 4,29^{[c]} |

**Die Beispiele sind mit Zahlen bezeichnet und sind im Text mit "I-und der entsprechenden Beispielzahl" wie z.B. I-5= Beispiel 5 in Tabelle I abgekürzt.**

Die Messung der logP Werte erfolgte gemäß EEC Directive 79/831 Annex V.A8 durch HPLC (High Performance Liquid Chromatography)
[a] Die Bestimmung erfolgt im sauren Bereich bei pH 2.3 mit 0,1% wässriger Phosphorsäure und Acetonitril als Eluenten; linearer Gradient von 10% Acetonitril bis 95% Acetonitril.
[b] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril
[c] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).Die lambda-maX-Werte wurden an Hand der UV-Spektren von 200 nm bis 400 nm in den Maxima der chromatographischen Signale ermittelt.

### NMR-Daten ausgewählter Beispiele

Die NMR-Daten ausgewählter Beispiele werden entweder in klassischer Form (δ-Werte, Anzahl der H-Atome, Multiplettaufspaltung) oder als NMR-Peak-Listen aufgeführt.

Wenn die 1H-NMR-Daten ausgewählter Beispiele in Form von 1H-NMR-Peaklisten notiert werden, wird zu jedem Signalpeak erst der δ-Wert in ppm und dann die Signalintensität durch ein Leerzeichen getrennt aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ₁ Intensität₁;δ₂ Intensität₂; ........ ;δᵢ Intensitätᵢ;.......; δₙ Intensitätₙ

Das Lösungsmittel, in welchem das NMR-Spektrum aufgenommen wurde, wird in eckigen Klammern vor der NMR-Peakliste aufgeführt.

| **Bsp.** | **NMR-Daten** |
|---|---|
| I-1 | ¹H NMR (DMSO-d₆, 400 MHz): dₚₚₘ : [7.91 (t), 7.83 (t)] (1H), [7.68 (d,) 7.55 (d)] (1H), 7.55-7.20 (m, 5H), 7.18 (t, 1H), 7.05 (t, 1H), 6.92 (s, 1H), [5.40 (q), 5.31 (s)] (2H), [4.73 (s), 4.64 (s)] (2H), [4.42 (d), 4.24 (d)] (1H), [4.01 (d), 3.80 (d)] (1H), 3.40-2.50 (m, 3H) [3.04 (s), 2.98 (s)] (3H), 2.02-1.50 (m, 3H), 1.45-1.09 (m, 1H) |
| I-2 | [DMSO-d₆] 7,8768 0,54;7,814 0,94;7,7928 0,59;7,6449 0,51;7,5739 0,89;7,5593 0,83;7,5216 1,14;7,5036 1,42;7,4367 1,31;7,434 1,35;7,4177 3,71;7,3997 3,61;7,3969 3,43;7,3754 2,97;7,356 1,49;7,3314 1;7,3115 0,85;7,2299 1,05;7,2142 1,02;6,4581 2,47;5,6893 14,21;5,2258 0,54;5,2223 0,59;5,2037 0,67;5,1781 0,74;5,1453 2,98;5,1228 0,38;4,7459 1,49;4,7105 0,58;4,6719 2,32;4,2859 0,34;4,272 0,39;4,2447 0,42;4,0643 1,25;4,0465 3,59;4,0287 3,64;4,0109 1,29;3,9991 0,33;3,8689 0,39;3,8301 0,4;3,3028 0,36;3,2952 0,37;3,2828 0,42;3,2495 0,54;3,2155 0,57;3,2038 0,63;3,1909 0,7;3,1706 0,93;3,146 1,69;3,1092 476,359985;3,0737 1,24;3,0323 4,64;2,974 2,25;2,8156 0,93;2,6894 0,33;2,6658 0,76;2,6614 1,15;2,6566 1,5;2,6521 1,12;2,6237 0,51;2,5909 0,57;2,526 3,41;2,5098 4,77;2,4969 72,150002;2,4923 141,779999;2,4876 194,380005;2,4829 135,910004;2,4782 65,370003;2,3192 0,79;2,3146 1,14;2,3097 0,8;2,2303 9,63;2,0404 0,73;1,9738 16;1,9475 0,68; 1,7693 0,34;1,7444 0,39;1,6796 0,8;1,6656 0,91;1,6501 0,91;1,6343 0,8;1,6288 0,74;1,4304 0,33;1,4038 1,24;1,3939 0,39;1,3617 0,32;1,2955 0,55;1,2447 0,69;1,2259 0,39;1,1949 4,56;1,1771 8,73;1,1593 4,22;-0,0001 11,98;-0,0084 0,49 |
| I-3 | [CD₃CN] 7,6943 0,63;7,6738 0,9;7,6499 0,95;7,6309 2,08;7,6101 1,87;7,6005 1,84;7,5914 1,61;7,5806 1,92;7,4377 3,38;7,4266 4,38;7,4119 0,96;7,3917 1,19;7,3842 1,13;7,3705 0,37;7,2515 0,8;7,2414 1,47;7,2296 1,41;7,2213 1,47;7,2094 0,77;6,9848 2,26;6,9666 2,15;6,9224 1,15;6,9045 1,09;6,87 1,16;6,8488 1,11;6,7722 2,27;6,7507 2,16;6,395 3,46;5,4456 2,62;5,0907 4,28;5,0018 8,43;4,7535 3,94;4,6509 7,47;4,0861 1,07;4,0683 3,2;4,0505 3,25;4,0327 1,13;3,701 1,09;3,6866 1,08;3,6652 1,18;3,6512 1,48;3,6239 2,35;3,3875 1,38;3,3759 2,1;3,3625 1,87;3,3268 1,96;3,3115 2,12;3,2776 2,16;3,2628 1,88;3,1956 1,79;3,1825 2,05;3,1702 1,33;3,0368 15;2,989 7,58;2,2349 7,05;2,2144 13,29;2,1442 140,580002;2,1063 0,84;2,0998 0,57;1,9712 14,78;1,951 19,549999;1,9449 35,98;1,9387 50,169998;1,9325 34,59;1,9264 17,74;1,4369 0,58;1,2756 0,42;1,2707 0,42;1,2581 0,47;1,2399 0,31;1,2214 3,74;1,2036 7,32;1,1858 3,6;-0,0001 29,389999;-0,0083 1,37 |
| I-4 | [DMSO-d₆] 9,2216 1,57;9,2055 3,07;9,1899 1,53;7,9757 2,45;7,9565 6,31;7,9375 4,96;7,9098 6,05;7,893 3,12;7,6413 4,15;7,6214 4,47;7,5636 4,45;7,5448 4,14;7,3777 1,56;7,3583 4,03;7,3401 3,3;7,2967 7,14;7,281 2,5;7,2776 2,4;7,2363 2,32;7,2319 2,06;7,2168 3,28;7,1982 1,67;7,1942 1,51;7,163 6,04;7,1459 3,04;7,0293 3,02;7,0098 6,38;6,8924 6,27;6,8737 3,18;5,7387 2,86;5,4292 0,85;5,3873 5,11;5,3607 5,05;5,3176 0,84;4,5779 |
| | 7,41;4,5622 7,43;4,4713 1,43;4,4359 1,45;4,0579 1,4;4,0402 4,49;4,0224 3,82;4,005 2,51;3,2822 693,52002;3,2583 57,169998;3,2281 1,32;3,2202 1,47;3,1785 0,43;3,1653 0,5;3,1183 1,07;3,1093 0,84;3,097 1,39;3,0895 2,15;3,0684 0,96;3,0605 1,17;3,0512 0,71;2,8177 0,97;2,7896 1,75;2,7597 1,02;2,6714 0,88;2,6675 1,12;2,6631 0,94;2,5846 0,31;2,5376 1,88;2,5029 128,520004;2,4986 162,830002;2,4944 114,690002;2,4403 0,47;2,4246 0,33;2,3253 1,15;2,0656 0,78;2,0457 1,25;2,0358 1,27;1,985 15;1,9579 1,74;1,9064 0,96;1,8932 0,67;1,8632 1,28;1,831 1,37;1,7967 1,38;1,7616 1,14;1,7345 0,52;1,2354 0,33;1,193 3,69;1,1752 7,12;1,1575 3,58;-0,0001 17,690001 |
| I-5 | [DMSO-d₆] 9,0541 0,59;9,0386 1,03;9,0238 0,59;7,9543 1,31;7,9353 3,55;7,9165 3,38;7,9041 2,99;7,9008 3,38;7,8849 1,38;7,8816 0,99;7,6255 1,87;7,6235 1,92;7,6042 2,3;7,5403 2,16;7,5371 2,13;7,5217 1,99;7,5184 1,84;7,3694 0,42;7,3666 0,42;7,35 1,9;7,3473 1,97;7,3374 2,69;7,3343 3,72;7,3317 5,09;7,3182 0,59;7,2262 1,01;7,2193 1,09;7,2104 0,9;7,2063 1,21;7,199 0,98;7,1911 0,68;7,1837 0,66;6,444 3,59;5,6895 7,12;5,2091 2,74;4,5975 4,59;4,5816 4,53;3,2581 0,33;3,2457 0,34;3,2387 0,32;3,2175 0,32;3,1086 181,279999;3,0863 1,95;3,0652 0,54;3,0561 0,72;3,0477 0,43;2,6613 0,42;2,6565 0,6;2,6521 0,43;2,526 1,31;2,5098 1,84;2,4969 31,620001;2,4922 62,970001;2,4875 87,209999;2,4828 61,57;2,4782 30,059999;2,3191 0,41;2,3144 0,49;2,3097 0,35;2,2182 15,94;2,2172 16;2,0405 0,51;2,0173 0,9;1,9864 1,07;1,9737 0,86;1,9624 0,39;1,8618 0,38;1,8102 0,63;-0,0001 5,58 |
| I-6 | [DMSO-d₆] 7,9697 0,78;7,9504 3,14;7,9388 3,69;7,9339 6,46;7,9196 0,84;7,6005 1,96;7,5946 1,86;7,5842 1,69;7,5783 1,68;6,4972 3,86;5,7469 1,64;5,3391 0,71;5,297 2,47;5,2522 2,53;5,2098 0,69;4,4586 0,71;4,4255 0,77;4,3787 2,18;4,3626 4,42;4,3464 2,18;4,0263 0,67;3,9934 0,73;3,3082 391,470001;3,2499 1,08;3,2209 0,57;3,1182 0,5;3,1089 0,37;3,0978 0,6;3,089 1,01;3,0797 0,59;3,0681 0,41;3,0596 0,55;2,8235 0,49;2,7923 0,86;2,7655 0,49;2,6742 0,36;2,6698 0,49;2,6653 0,37;2,5399 0,85;2,523 2,25;2,5096 29,34;2,5053 54,080002;2,5008 70,139999;2,4964 47,639999;2,492 22,690001;2,4725 1,61;2,4528 1,3;2,4437 1,38;2,4326 1,41;2,424 1,21;2,4179 0,81;2,4037 1,22;2,3952 0,48;2,3893 0,31;2,3748 0,44;2,332 0,38;2,3275 0,5;2,3228 0,39;2,2207 15;2,2009 0,54;1,9983 0,75;1,9819 1,94;1,962 2,31;1,9425 2,39;1,9263 1,82;1,9083 0,78;1,8928 0,78;1,8474 0,32;1,826 0,6;1,8183 0,61;1,7949 0,53;1,7854 0,5;1,6423 0,57;1,633 0,61;1,611 0,55;1,6015 0,56;-0,0001 2,37 |
| I-7 | ¹H NMR (DMSO-d₆, 400 MHz): dₚₚₘ: 1.57-1.66 (m, 1H), 1.75-1.84 (m, 1H), 1.88-1.98 (m, 2H), 2.75-2.81 (m, 1H), 3.05-3.11 (m, 1H), 3.24 (m, 1H), 4.01-4.04 (m, 1H), 4.39-4.43 (m, 1H), 5.34 (d, 1H), 5.42 (d, 1H), 5.45 (s, 2H), 6.90 (s, 1H), 7.02 (t, 1H), 7.17 (t, 1H), 7.43-7.56 (m, 3H), 7.59 (dd, 1H), 7.68 (dd, 1H), 7.89-7.97 (m, 2H) |
| I-8 | [DMSO-d₆] 7,9714 1,2;7,9522 3,38;7,9333 3;7,9253 0,43;7,9175 3;7,9144 3,31;7,8983 1,46;7,8952 1,14;7,7023 1,43;7,6987 1,59;7,6839 1,67;7,6797 1,84;7,61 2,22;7,607 2,24;7,5911 2,12;7,5882 1,92;7,5628 0,66;7,5583 0,68;7,5435 1,62;7,5389 1,55;7,5239 1,59;7,5193 1,39;7,481 1,29;7,478 1,75;7,4593 3,19;7,4532 1,71;7,4436 1,07;7,4404 1,2;7,437 0,95;7,4331 1,11;6,4929 3,94;5,7471 3,33;5,4534 9,8;5,3324 0,66;5,2894 2,49;5,2454 2,44;5,2023 0,69;4,4535 0,69;4,4198 0,74;4,0216 0,69;3,988 0,72;3,3141 331,75;3,2905 7,82;3,2714 1,07;3,2414 1,05;3,2099 0,63;3,1145 0,52;3,1054 0,41;3,0941 0,66;3,0854 1,04;3,0762 0,64;3,0651 0,46;3,0556 0,59;2,8107 0,48;2,7841 0,87;2,7795 0,88;2,7524 0,5;2,6708 0,3;2,5408 0,65;2,5238 1,01;2,5105 17,379999;2,5061 32,990002;2,5017 43,34;2,4973 30,110001;2,493 14,65;2,3284 0,34;2,2243 0,98;2,2099 15;2,1985 1,2;2,07 0,31;1,9513 0,57;1,9218 1,35;1,8833 0,81;1,8508 0,32;1,842 0,34;1,8203 0,61;1,8121 0,64;1,7906 0,57;1,7814 0,54;1,6695 0,3;1,6607 0,32;1,6394 0,62;1,6285 0,65;1,6078 0,6;1,5991 0,6;-0,0001 5,87 |
| I-9 | |
| I-10 | [DMSO-d₆] 7,7674 1,63;7,748 3,73;7,7287 2,29;7,6152 2,53;7,5959 1,94;7,4569 0,6;7,4447 0,56;7,4384 0,58;7,4332 0,81;7,4173 0,68;7,4084 0,62;7,4024 0,7;7,3936 0,72;7,3428 0,45;7,333 0,6;7,3266 0,56;7,3189 0,89;7,3069 0,64;7,3004 0,51;7,2943 0,67;7,283 0,89;7,2749 0,5;7,2658 1,62;7,2566 1,3;7,2512 1,3;7,242 1,34;7,2304 1,4;7,221 1,49;7,2103 1,02;7,198 2,48;7,1802 2,26;6,5293 3,34;5,7465 2,4;5,2513 1,81;5,2407 4,86;5,1886 0,81;4,3117 0,46;4,2956 0,6;4,0569 0,87;4,0391 2,58;4,0213 2,62;4,0035 0,89;3,8652 0,6;3,8468 0,45;3,3068 563,47998;3,2905 26,950001;3,1015 0,61;3,069 0,98;3,0433 1,78;2,8552 1,18;2,7357 0,51;2,7269 0,41;2,7156 0,62;2,7066 1,03;2,6974 0,67;2,6858 0,44;2,6743 0,82;2,6691 0,88;2,6652 0,65;2,6154 0,54;2,5846 0,98;2,5549 0,72;2,5393 2,99;2,5223 2,25;2,509 33,91;2,5047 63,220001;2,5002 81,910004;2,4958 55,900002;2,4915 26,41;2,3315 0,48;2,3269 0,63;2,3223 0,47;2,2634 15;2,2222 1,56;2,1937 1,16;1,9866 11,35;1,5397 |
| | 0,33;1,5031 0,72;1,4685 0,47;1,4336 0,41;1,3984 1,19;1,352 0,58;1,318 0,32;1,3092 0,31;1,2203 0,38;1,2127 0,38;1,1927 3,35;1,1749 6,26;1,1571 3,19;1,1404 0,39;1,1297 0,36;1,1087 0,35;1,0985 0,32;1,0141 0,34;1,0045 0,33;0,9813 0,31;-0,0001 4,57 |
| I-11 | [DMSO-d₆] 7,843 1,53;7,8236 3,28;7,8042 1,84;7,3602 1,29;7,3351 2,82;7,316 2,15;6,4446 4,01;5,6876 10,56;5,1938 1,47;4,4264 0,33;4,0646 0,38;4,0466 0,9;4,0289 0,91;4,0209 0,36;4,0109 0,52;4,0014 0,33;3,4317 0,4;3,2541 0,42;3,2397 0,46;3,2264 0,51;3,1127 300,720001;3,056 1,11;3,0374 0,99;3,0279 1,41;3,0199 0,89;3,0068 0,68;2,9987 0,77;2,9886 0,56;2,871 2,57;2,8217 0,64;2,7651 0,98;2,6618 0,53;2,657 0,69;2,6524 0,45;2,5264 1,45;2,5101 2,26;2,4973 37,790001;2,4927 74,620003;2,488 102,900002;2,4833 72,550003;2,4786 35,509998;2,3836 0,44;2,3193 0,48;2,3146 0,67;2,3099 0,48;2,26 0,33;2,2215 16;2,0399 0,37;1,9737 2,87;1,9398 1,13;1,9073 1,45;1,9007 1,54;1,8269 0,5;1,7895 0,72;1,6927 2,34;1,5537 1,32;1,5316 1,32;1,195 0,85;1,1773 1,59;1,1645 0,62;1,1595 0,9;1,0827 1,1;1,0569 0,41;0,9978 0,75;0,9866 0,7;0,9815 0,7;-0,0001 2,42 |
| I-12 | ¹H NMR (DMSO-d₆, 400 MHz): dₚₚₘ : 1.45-2.15 (m, 8H), 2.22 (s, 3H), 2.61 und 2.68 (s, 3H), 2.65-2.88 (m, 3H), 2.95-3.31 (m, 2H), 3.98 (bs, 1H), 4.38 (bs, 1H), 4.99 und 5.83 (m, 1H), 5.17 (bs, 2H), 6.45 (s, 1H), 7.05-7.25 (m, 4H), 7.38 (m, 1H), 7.50 (m, 1H), 7.86 (m, 1H) |
| I-13 | [DMSO-d₆] 7,8893 0,35;7,8491 0,6;7,8391 0,61;7,8262 0,67;7,6093 0,51;7,5421 0,63;7,4922 1,03;7,4514 1,04;7,4466 1,02;7,4309 2,5;7,4259 2,56;7,3993 2,08;7,35 0,66;6,4469 3,3;5,688 16;5,1692 1,86;4,7434 2,29;4,7086 0,49;4,0471 0,41;4,029 0,43;3,1091 291,630005;3,0406 1,66;3,01 3,54;2,9826 2,9;2,9156 0,66;2,8868 0,55;2,8213 0,7;2,8151 0,65;2,7267 0,32;2,662 0,52;2,657 0,7;2,6523 0,47;2,5263 1,69;2,4972 30,93;2,4925 68,470001;2,4878 100,620003;2,4832 75,510002;2,4785 40,799999;2,3946 0,91;2,3835 0,94;2,3477 0,6;2,3247 0,69;2,3198 0,83;2,3147 1,03;2,3099 0,9;2,3051 0,7;2,2787 0,44;2,2264 10,98;2,1814 0,68;2,1259 0,46;2,1082 0,42;2,1007 0,41;2,0813 0,39;2,0667 0,37;2,04 0,75;2,0371 0,58;2,0316 0,39;1,9738 2,33;1,9434 0,77;1,9008 0,47;1,8851 0,39;1,8737 0,4;1,8568 0,36;1,827 0,37;1,7916 0,46;1,7081 0,81;1,6931 0,78;1,6293 0,56;1,6007 0,46;1,5662 0,4;1,5481 0,44;1,5071 0,42;1,4868 0,43;1,4597 0,39;1,4545 0,4;1,4419 0,37;1,4341 0,35;1,404 0,46;1,3939 0,36;1,3328 0,38;1,3287 0,36;1,3156 0,4;1,296 0,52;1,243 0,41;1,195 0,66;1,1772 1,08;1,1596 0,64;-0,0001 6,57 |
| I-14 | [DMSO-d₆] 8,541 0,54;8,4784 0,81;7,9741 0,34;7,8644 0,49;7,8475 0,41;7,8117 0,72;7,7941 1,31;7,7746 1,01;7,7428 0,9;7,7256 0,47;7,4653 3,42;7,4634 3,63;7,446 3,18;7,4441 3,18;7,4025 0,53;7,3678 0,7;7,349 0,64;7,3203 1,09;7,2999 1,34;7,2863 1,44;7,2665 1,2;7,2427 0,7;7,2268 0,77;6,4563 2,31;5,6877 16;5,1709 2,23;4,7649 1,62;4,7047 2,98;4,0646 0,37;4,0467 0,83;4,0291 0,84;4,0112 0,44;3,207 0,38;3,1977 0,47;3,1123 348,380005;3,0267 18,4;2,8931 0,46;2,8883 0,48;2,865 0,69;2,8273 0,39;2,6878 0,34;2,662 0,66;2,6573 0,86;2,6524 0,68;2,6478 0,47;2,5266 1,37;2,5104 2,07;2,4975 35,860001;2,4928 71,870003;2,4882 99,709999;2,4835 70,410004;2,4788 34,419998;2,3197 0,43;2,3151 0,66;2,3103 0,44;2,2304 9,15;2,0398 1,19;1,9738 3,5;1,9471 0,66;1,9011 1,42;1,7367 0,85;1,7251 0,87;1,7176 0,84;1,2959 0,34;1,2438 0,41;1,195 1,17;1,1772 1,86;1,1595 0,83;0,008 0,74;-0,0001 16,370001;-0,0084 0,6 |
| I-15 | [DMSO-d₆] 7,9289 0,74;7,9093 1,58;7,8899 0,95;7,581 1,54;7,5621 1,44;7,4464 1,06;7,4269 1,04;7,0322 0,39;7,0143 0,81;6,9969 0,57;6,9155 1,72;6,8984 1,25;6,895 1,2;6,4869 3,19;5,7468 0,8;5,2499 1,51;5,2276 2,1;5,1859 0,36;4,3591 0,42;4,3529 0,44;4,3072 1,73;4,0568 1,19;4,039 3,38;4,0212 3,44;4,0033 1,2;3,9446 0,48;3,8851 1,31;3,3029 483,839996;3,2272 0,49;3,1956 0,69;3,1668 0,38;3,0219 0,3;3,0062 0,4;2,997 0,48;2,7829 0,41;2,7504 0,7;2,7207 0,43;2,6734 0,46;2,6687 0,62;2,6641 0,47;2,5389 3,54;2,522 2,55;2,5086 34,439999;2,5042 63,5;2,4998 81,970001;2,4954 55,18;2,491 25,66;2,3312 0,39;2,3264 0,54;2,3218 0,4;2,205 12,27;2,1883 0,34;2,1797 0,67;1,9866 15;1,8479 0,56;1,7969 0,37;1,4934 0,32;1,4852 0,32;1,3984 0,58;1,1926 4,14;1,1748 8,26;1,157 4,03;0,008 0,75;-0,0001 16,389999;-0,0085 0,55 |
| I-16 | [DMSO-d₆] 8,7649 1,1;8,745 1,04;7,9348 1,56;7,9156 3,77;7,8963 2,6;7,844 2,68;7,8417 2,8;7,825 1,78;7,8224 1,63;7,556 1,68;7,5518 1,75;7,5365 4,04;7,5335 3,79;7,5169 2,14;7,5147 2,03;7,4315 1,64;7,4279 1,59;7,4119 2,14;7,4084 2,13;7,3451 0,82;7,3297 1,92;7,3263 1,69;7,3108 1,27;7,3072 1;7,2878 1,49;7,2832 1,48;7,2686 1,62;7,2643 1,61;7,2495 0,68;7,2456 0,54;6,4562 3,69;5,6897 0,49;5,4873 1,13;5,468 1,41;5,4493 1,11;5,236 1,29;4,479 0,33;4,4703 0,33;4,0644 1,2;4,0466 3,28;4,0288 3,23;4,0111 1,15;3,3009 0,35;3,2707 0,41;3,2508 0,39;3,2437 0,38;3,1977 0,38;3,1152 306,679993;3,0824 1,11;2,8262 0,35;2,8194 0,33;2,6623 0,38;2,6575 0,58;2,6523 0,36;2,5266 2,89;2,5104 2,13;2,4975 33,73;2,4928 66,910004;2,4881 92,330002;2,4835 64,959999;2,4788 31,68;2,3198 0,42;2,3151 0,55;2,3103 |
| | 0,38;2,2356 16;2,0375 1,17;2,0109 1,15;1,9741 13,56;1,9649 0,4;1,8415 0,42;1,8112 0,54;1,7911 0,51;1,7684 0,5;1,7611 0,48;1,7553 0,48;1,7488 0,43;1,7382 0,41;1,5536 9,59;1,5361 9,55;1,195 3,77;1,1772 7,43;1,1594 3,65;-0,0001 9,71 |
| I-17 | [DMSO-d₆] 8,2111 1,33;8,1965 1,35;7,9421 1,74;7,9293 4,29;7,9165 2,93;7,8769 3,26;7,8754 3,39;7,8642 2,29;7,8626 2,16;7,5319 2,53;7,5308 2,56;7,5191 2,47;6,5157 4,73;5,3461 0,77;5,3382 0,73;5,3174 1,54;5,3097 1,52;5,2655 3;5,2371 1,35;4,4895 0,83;4,4676 0,86;4,0458 0,82;4,0377 0,83;4,034 1,68;4,0221 2,16;4,0191 1,53;4,0103 1,43;3,9964 0,75;3,9831 0,38;3,3843 0,47;3,3517 322,350006;3,3171 0,36;3,2648 0,59;3,261 0,67;3,2422 1,1;3,2395 1,12;3,2212 0,65;3,2174 0,55;3,0892 0,57;3,0832 0,37;3,075 0,68;3,0692 1,17;3,0634 0,67;3,0554 0,4;3,0493 0,61;3,0436 0,32;2,7931 0,55;2,7719 1,1;2,7502 0,57;2,6187 0,54;2,6157 0,74;2,6127 0,53;2,5432 2,42;2,5247 1,59;2,5216 2,07;2,5185 2,35;2,5096 43,450001;2,5067 91,019997;2,5037 122,970001;2,5007 88,709999;2,4978 40,5;2,3905 0,55;2,3876 0,74;2,3845 0,53;2,2214 16;2,0781 0,37;1,9906 4,65;1,9729 0,85;1,9523 0,64;1,9307 0,71;1,8491 0,33;1,8348 0,45;1,8282 0,77;1,8213 0,5;1,8141 0,46;1,8071 0,7;1,8005 0,41;1,7258 0,33;1,7188 0,38;1,7046 0,78;1,6978 0,8;1,6836 0,75;1,677 0,71;1,5803 0,65;1,5745 0,65;1,5676 0,56;1,5172 0,7;1,5069 0,89;1,4941 0,74;1,4834 0,51;1,2673 4,7;1,1881 5,3;1,1862 6,04;1,1772 5,47;1,1745 7,18;1,1626 1,27;0,8453 4,25;0,8388 4,07;0,8279 1,37;0,0053 0,48;0 12,84;-0,0055 0,41 |
| I-18 | [DMSO-d₆] 8,0927 0,84;8,0712 0,86;7,9265 1,29;7,9075 3,58;7,8887 3,35;7,8753 2,94;7,8719 3,4;7,8562 1,4;7,8528 1,08;7,5034 2,1;7,5002 2,14;7,4848 1,96;7,4815 1,91;6,4554 3,62;5,6878 10,32;5,2285 0,96;5,2117 0,97;4,0469 0,47;4,0292 0,49;3,8077 0,61;3,7977 0,6;3,7862 0,57;3,7768 0,46;3,1136 174,580002;3,0917 4,82;3,0728 1,68;3,0634 0,95;3,0526 0,61;3,0433 0,8;3,0336 0,48;2,5268 0,62;2,5107 0,92;2,4978 16,9;2,4931 34,049999;2,4884 47,43;2,4837 33,720001;2,479 16,610001;2,3153 0,34;2,2369 15,99;2,2358 16;2,0401 0,62;1,9969 0,94;1,9739 2,19;1,8649 1,24;1,8523 1,3;1,8405 1,94;1,8059 0,47;1,7345 1,67;1,7246 1,83;1,712 1,53;1,7015 1,53;1,6158 0,7;1,5849 0,73;1,4568 0,48;1,4495 0,36;1,4279 1,28;1,4212 1,06;1,399 2,84;1,3748 2,77;1,352 0,88;1,3456 1,26;1,3232 0,32;1,3159 0,51;1,266 0,39;1,2579 0,34;1,2447 0,58;1,2366 0,64;1,2148 0,48;1,207 0,5;1,1953 0,53;1,1775 0,77;1,1597 0,34;-0,0001 2,87 |
| I-19 | [DMSO-d₆] 8,5537 0,98;8,5331 1,02;7,9551 0,49;7,9458 7,4;7,9362 4,59;7,9331 4,65;7,914 0,4;7,5287 0,35;7,5184 1,94;7,5064 2,14;7,4962 1,76;7,2911 1,11;7,2724 1,75;7,2439 0,88;7,2406 0,91;7,2277 1,91;7,2231 2,19;7,2088 3,19;7,1969 1,89;7,1937 1,92;7,1808 1,14;7,1595 0,43;6,4223 3,61;5,6892 11,06;5,5779 0,51;5,5579 1,4;5,5372 1,38;5,5165 0,49;5,1883 1,29;4,0465 0,68;4,0287 0,74;4,011 0,43;3,2144 0,36;3,1934 0,33;3,11 181,839996;3,0741 0,95;3,0625 0,86;3,054 1,28;3,0442 1,44;3,0376 1,07;3,0213 1,16;3,0146 1,46;2,9987 0,93;2,991 0,89;2,9228 0,62;2,9031 1,25;2,8827 0,98;2,863 0,75;2,8425 0,49;2,6611 0,34;2,6568 0,49;2,652 0,34;2,5468 0,55;2,5389 0,59;2,5262 1,8;2,5186 1,29;2,5149 1,25;2,5052 2,66;2,4969 26,870001;2,4923 52,77;2,4876 73,559998;2,4829 51,639999;2,4782 25,469999;2,319 0,35;2,3145 0,46;2,3097 0,34;2,1883 15,89;2,1875 16;2,1101 0,51;2,0881 1,16;2,0788 0,51;2,0675 1,13;2,0566 1,07;2,0455 0,5;2,0403 0,65;2,0359 1,04;2,0137 0,45;1,9738 3,3;1,9395 1,12;1,9005 0,37;1,7963 0,33;1,7399 0,41;1,7292 0,43;1,6888 0,4;1,1949 0,69;1,1771 1,39;1,1593 0,69;-0,0001 4,81 |
| I-20 | [DMSO-d₆] 8,6172 0,59;8,6019 1,17;8,5865 0,63;7,934 1,29;7,9149 3,48;7,8959 2,77;7,8695 2,84;7,8667 3,14;7,8504 1,64;7,8476 1,43;7,513 2,08;7,5104 2,15;7,494 2,02;7,4914 1,94;6,501 3,86;5,7475 2,56;5,3235 0,46;5,2812 2,93;5,2586 2,87;5,2163 0,47;4,4969 0,67;4,462 0,71;4,0567 0,44;4,0393 1,1;4,0214 0,93;4,0105 0,73;4,0039 0,73;3,3244 1,93;3,3004 273,100006;3,2766 9,11;3,2402 1,14;3,2101 0,66;3,0832 0,52;3,074 0,38;3,0625 0,64;3,054 1,05;3,0441 0,76;3,0332 0,43;3,0242 0,59;2,8008 0,49;2,7742 0,88;2,7428 0,51;2,6737 0,39;2,669 0,53;2,6646 0,41;2,5392 1,42;2,5222 2,03;2,5088 31,110001;2,5044 57,610001;2,5 74,370003;2,4957 51,02;2,4915 24,549999;2,3313 0,42;2,3267 0,56;2,3222 0,44;2,2236 15;2,1996 0,76;2,0697 0,68;1,9869 2,74;1,9556 1,23;1,9176 0,84;1,8898 0,31;1,8791 0,35;1,8572 0,62;1,8477 0,64;1,826 0,57;1,8168 0,55;1,7951 0,48;1,7854 0,49;1,7709 0,8;1,7643 0,67;1,7543 0,66;1,733 0,57;1,7229 0,54;1,5555 1,2;1,5377 1,73;1,5209 1,22;1,3983 0,87;1,2978 5,18;1,2918 5,03;1,2698 3,56;1,2628 4,82;1,1928 0,71;1,175 1,3;1,1572 0,68;0,8722 2,47;0,8553 7,57;0,8378 2,75;0,008 0,33;-0,0001 7,26 |
| I-21 | [DMSO-d₆] 9,062 1,11;9,0462 0,65;7,9508 1,35;7,9319 3,53;7,913 3,15;7,8963 2,86;7,8931 3,2;7,877 1,44;7,874 1,08;7,5698 2,78;7,5651 2,93;7,5384 2,11;7,5354 2,13;7,5196 1,93;7,5166 1,82;7,3982 1,03;7,3934 0,86;7,3773 3,33;7,3724 3,47;7,3614 4,21;7,3403 1,05;6,4472 3,76;5,6898 7,15;5,2106 2,72;4,5987 4,47;4,5832 4,4;4,4557 0,34;4,4403 0,32;4,0642 |
| | 0,68;4,0466 1,54;4,0288 1,53;4,011 0,64;3,288 0,4;3,2496 0,48;3,2366 0,47;3,2237 0,48;3,1165 372,149994;3,0807 1,57;3,0712 0,75;3,0525 0,61;2,6623 0,45;2,6574 0,57;2,6531 0,47;2,5267 3,04;2,4976 35,049999;2,4929 69,699997;2,4883 96,309998;2,4836 68,110001;2,4789 33,299999;2,32 0,48;2,315 0,56;2,3104 0,37;2,2196 16;2,0373 0,6;2,0033 0,91;1,9742 6,44;1,8366 0,52;1,8282 0,68;1,8076 0,68;1,7974 0,64;1,762 0,4;1,4038 0,42;1,195 1,58;1,1772 3,16;1,1595 1,51;0,0081 0,46;-0,0001 10,38 |
| I-22 | [DMSO-d₆] 8,9842 0,59;8,9705 0,99;8,9558 0,59;7,9392 1,27;7,9201 3,55;7,9016 3,57;7,8911 3,12;7,8875 3,49;7,8718 1,36;7,8684 0,98;7,5202 2,23;7,5169 2,14;7,5017 1,97;7,4985 1,87;7,4425 0,72;7,4254 0,87;7,4206 1,4;7,4042 1,44;7,3826 0,69;7,1679 0,87;7,1616 0,87;7,1444 1,04;7,1418 1,08;7,1384 1,16;7,1354 1,1;7,1183 0,81;7,1119 0,86;7,0415 0,69;7,0392 0,7;7,0351 0,65;7,0201 1,24;7,0175 1,25;7,0136 1,16;6,9985 0,6;6,9957 0,62;6,9922 0,53;6,4469 3,71;5,6879 10,34;5,2085 2,38;4,563 3,56;4,5473 3,55;4,0472 0,45;4,0296 0,45;4,0115 0,33;3,2823 0,32;3,2641 0,37;3,2494 0,42;3,2424 0,41;3,2361 0,44;3,2198 0,4;3,1632 0,6;3,1109 312,809998;3,077 1,09;3,0678 1,48;3,0586 0,82;3,0477 0,6;3,0379 0,73;3,0288 0,4;2,6618 0,39;2,6571 0,51;2,6524 0,39;2,5264 2,52;2,5103 1,82;2,4974 29,35;2,4927 58,200001;2,488 80,199997;2,4833 56,470001;2,4787 27,540001;2,3197 0,39;2,3148 0,49;2,3104 0,35;2,2218 16;2,2208 16;2,2029 0,46;2,1917 0,34;2,04 0,48;2,0371 0,36;1,9942 0,99;1,9738 1,88;1,7934 0,7;1,1774 0,49;-0,0001 6,05 |
| I-23 | ¹H NMR (DMSO-d₆, 400 MHz): dₚₚₘ : 9.02 (t, 1H), 8.05-7.85 (m, 2H), 7.53 (dd, 1H), 7.44 (dd, 1H), 7.40-7.25 (m, 3H), 6.44 (s, 1H), 5.21 (bs, 2H), 4.62 (d, 2H), 4.55-4.35 (m, 1H), 4.10-3.95 m, 1H), 3.40-3.00 (m, 2H), 2.90-2.70 (m, 1H), 2.21 (s, 3H), 2.05-1.95 (m, 2H), 1.90-1.70 (m, 2H) |
| I-24 | ¹H NMR (DMSO-d₆, 400 MHz): dₚₚₘ : 8.72 (t, 1H), 7.91 (t, 1H), 7.87 (t, 1H), 7.50 (dd, 1H), 7.43-7.32 (m, 1H), 7.05 (t, 2H), 6.45 (s, 1H), 5.21 (bs, 2H), 4.64 (d, 2H), 4.52-4.45 (bs, 1H), 4.10-3.95 (bs, 1H), 3.35-3.00 (m, 2H), 2.90-2.70 (bs, 1H), 2.22 (t, 3H), 2.05-1.90 (m, 2H), 1.85-1.60 (m, 2H) |
| I-25 | [DMSO-d₆] 9,1657 0,62;9,1527 0,93;8,5274 1,32;8,5154 1,34;7,9745 0,49;7,9523 1,13;7,9334 3,26;7,9153 4,04;7,9112 3,26;7,907 3,79;7,892 1,18;7,8879 0,68;7,7643 1,05;7,7596 1,01;7,745 1,96;7,7405 1,87;7,7258 1,18;7,7213 1,21;7,5333 2,07;7,5294 2,07;7,5155 1,91;7,5114 1,86;7,3459 1,98;7,3266 1,85;7,267 1,03;7,2523 1,16;7,2485 1,12;7,2365 0,95;6,4553 0,41;6,4451 3,6;5,6879 3,74;5,2141 2,46;4,9318 0,43;4,6527 4,53;4,6378 4,49;4,4703 0,34;4,4591 0,34;4,4531 0,34;4,4312 0,34;4,4231 0,38;4,4084 0,34;4,0704 0,35;4,0466 0,55;4,0285 0,47;4,0113 0,32;3,2924 0,41;3,253 0,48;3,2391 0,5;3,2149 0,51;3,1754 0,9;3,1131 698,659973;3,0635 1,52;3,0541 1,1;2,8259 0,36;2,6665 0,47;2,6622 0,8;2,6574 1,17;2,6525 0,88;2,6479 0,51;2,5265 2,5;2,5104 3,69;2,4975 62,919998;2,4928 125,989998;2,4882 174,610001;2,4835 123,220001;2,4788 60,23;2,424 0,37;2,3199 0,77;2,315 1,17;2,3103 0,73;2,3056 0,43;2,2796 0,64;2,2199 16;2,2188 15,84;2,04 4,06;2,0252 0,8;2,0205 0,91;2,0144 0,96;1,9866 1,12;1,974 1,86;1,8282 0,6;1,8141 0,65;1,7877 0,53;1,2465 0,35;1,195 0,5;1,1773 0,74;1,1594 0,32;-0,0001 9,26 |
| I-26 | [DMSO-d₆] 9,2831 0,83;9,2725 1,69;9,2618 0,84;7,9515 1,45;7,9388 3,89;7,9261 3,11;7,9085 3,13;7,9066 3,36;7,8958 1,78;7,8938 1,54;7,5371 2,25;7,5353 2,28;7,5244 2,22;7,5226 2,08;7,3837 2,71;7,3816 2,75;7,3752 2,83;7,3731 2,75;7,035 1,92;7,0332 1,97;7,0293 2,28;7,0276 2,13;6,9681 2,71;6,9623 2,42;6,9596 2,58;6,9539 2,18;6,5089 4,29;5,7639 0,98;5,3204 0,99;5,2918 2,95;5,262 2,9;5,2336 0,98;4,8937 0,42;4,6724 2,29;4,6689 2,26;4,6621 2,21;4,6584 2,27;4,4915 0,74;4,4709 0,88;4,0458 0,91;4,0339 3,13;4,0283 0,77;4,022 3,15;4,0101 1,49;3,3508 317,170013;3,3274 0,65;3,2479 0,48;3,2438 0,55;3,2254 0,94;3,2223 0,96;3,2043 0,57;3,2004 0,47;3,0708 0,49;3,0648 0,32;3,0568 0,6;3,0509 1,04;3,0449 0,59;3,037 0,35;3,0311 0,55;2,7775 0,46;2,7734 0,53;2,7563 0,96;2,7521 0,96;2,7349 0,55;2,731 0,52;2,6185 0,45;2,6154 0,6;2,6124 0,43;2,5429 2,31;2,5244 1,24;2,5214 1,65;2,5182 1,9;2,5094 34,220001;2,5064 72,040001;2,5034 97,400002;2,5004 69,779999;2,4974 31,370001;2,3903 0,42;2,3873 0,57;2,3843 0,41;2,2593 0,68;2,2085 16;2,0779 0,79;1,9905 12,04;1,9714 0,6;1,9501 0,85;1,9253 0,63;1,9045 0,82;1,8677 0,32;1,8535 0,66;1,8471 0,7;1,8327 0,64;1,8262 0,61;1,8123 0,4;1,8071 0,45;1,7932 0,68;1,7864 0,7;1,7723 0,62;1,7656 0,61;1,3968 0,42;1,1862 3,29;1,1743 6,65;1,1624 3,24;0,0053 0,42;0 11,28;-0,0056 0,33 |
| I-27 | [DMSO-d₆] 9,0765 4,44;9,0749 4,27;8,6424 2,55;8,6386 2,71;8,6307 2,77;8,6269 2,62;8,4586 2,55;8,4548 2,51;8,4383 2,93;8,4346 2,55;8,3662 2,75;8,3627 2,89;8,083 3,3;8,081 3,46;8,0786 3,27;8,0516 0,36;8,0475 0,4;7,9939 0,39;7,9908 0,39;7,6641 2,56;7,6524 2,55;7,6439 2,45;7,6323 2,42;7,5262 0,42;7,473 8,02;7,063 0,35;6,0013 0,4;5,9136 10,93;4,0572 0,31;4,0395 0,93;4,0216 0,89;4,0039 0,33;3,4018 0,51;3,3104 335,649994;2,6744 |
| | 0,47;2,6703 0,61;2,6653 0,48;2,5401 2,22;2,5098 36,200001;2,5055 66,330002;2,501 85,220001;2,4967 57,950001;2,4924 27,18;2,4731 1,17;2,3321 0,41;2,3278 0,59;2,3233 0,43;2,3022 1,75;2,2944 0,8;2,2276 0,45;2,1366 1,72;2,0697 2,1;1,9871 3,76;1,7297 15;1,2367 0,38;1,193 1,05;1,1752 2,04;1,1575 0,96;0,8898 0,31 |
| I-28 | [DMSO-d₆] 10,2242 2,71;8,0185 1,03;7,9994 2,94;7,9806 2,68;7,9656 2,61;7,9626 2,81;7,9466 1,22;7,9435 0,99;7,6255 1,81;7,6228 1,83;7,6068 1,72;7,604 1,59;7,4471 0,64;7,4423 0,61;7,4262 1,26;7,4104 0,67;7,4049 0,83;7,389 0,35;7,2373 2,46;7,2171 3,8;7,1966 1,8;7,1883 0,39;6,4911 3,88;5,7474 1,03;5,3196 0,37;5,2785 3,09;5,262 3,04;5,2201 0,39;4,5143 0,68;4,4806 0,7;4,057 1,5;4,0392 3,42;4,0214 3,45;4,0036 1,08;3,3013 342,190002;3,2554 1,09;3,2259 0,54;3,1471 0,45;3,1386 0,35;3,1172 0,87;3,1088 0,56;3,0965 0,4;3,0895 0,48;2,814 0,45;2,7842 0,86;2,7551 0,49;2,6738 0,39;2,6688 0,52;2,6644 0,41;2,539 3,03;2,5221 2,1;2,5088 29,85;2,5044 55,360001;2,4999 71,589996;2,4956 48,560001;2,4912 22,75;2,3312 0,37;2,3268 0,5;2,3223 0,36;2,2155 15;2,0496 0,79;2,0178 0,93;1,9868 13,55;1,9654 1,03;1,9337 0,59;1,9235 0,63;1,9016 0,73;1,8915 0,75;1,8709 0,7;1,8621 0,7;1,8383 0,51;1,83 0,48;1,3984 0,71;1,1927 3,66;1,1749 7,2;1,1571 3,53;0,008 0,69;-0,0001 15,66;-0,0083 0,57 |
| I-29 | [CD₃CN] 10,7594 3,08;8,2171 3,16;8,2057 3,07;8,0861 6,29;7,9743 6,2;7,9689 6,3;7,794 3,2;7,7761 3,33;7,7203 0,61;7,6467 3,43;7,564 8,09;6,3822 4,17;5,1659 1,64;5,1243 5,93;5,0912 4,75;5,0563 1,88;4,9785 0,33;4,6516 2,29;4,6259 2,46;4,0502 3,74;4,012 2,86;3,7902 1,57;3,3263 2,47;3,1973 2,56;2,8498 2,54;2,8188 1,75;2,5164 1,05;2,424 1,12;2,2151 16;2,1535 14,96;2,0973 6,82;2,0592 6,13;1,9718 9,86;1,9457 15,14;1,9398 15,57;1,7908 0,81;1,7168 0,58;1,697 0,56;1,4346 0,6;1,264 2,1;1,2209 3,34;1,2102 4,05;1,2038 4,44;1,1866 3,09;1,0672 0,39;1,0595 0,38;1,0528 0,36;0,8722 0,54 |
| I-30 | [DMSO-d₆] 10,4443 1,67;8,2219 2,6;8,2176 2,55;7,8822 2,27;7,86 2,69;7,8457 1,82;7,8253 1,93;7,8038 1,8;7,7834 1,96;7,6127 2,03;7,6075 1,93;7,5904 1,74;7,5854 1,75;7,4866 0,89;7,4834 0,94;7,4696 1,58;7,4664 1,93;7,4495 1,44;7,4461 1,33;7,4268 1,45;7,4235 1,65;7,4065 2,03;7,4036 1,6;7,3894 0,85;7,3865 0,8;6,5083 3,51;5,1225 10,98;5,1068 0,48;3,1821 0,62;3,1269 289,040009;2,6625 0,51;2,658 0,7;2,653 0,49;2,5271 3,02;2,511 2,55;2,498 39,41;2,4934 77,989998;2,4887 107,190002;2,484 75,580002;2,4794 36,740002;2,3445 16;2,3434 15,98;2,32 0,53;2,3153 0,71;2,3111 0,46;2,2267 0,7;1,4034 0,32;1,2444 0,6;-0,0001 10,45 |
| I-31 | [DMSO-d₆] 12,4662 1,94;8,9497 2,1;8,9456 2,22;8,9393 2,18;8,9351 2,09;8,8306 1,99;8,8272 2,03;8,8118 2,08;8,8085 2,04;8,4402 1,88;8,4362 1,91;8,4193 2,09;8,4153 1,93;8,1002 0,9;8,0967 1,25;8,081 3,51;8,0775 3,22;8,0695 3,27;8,051 3,5;8,032 1,29;7,9764 0,39;7,7268 1,32;7,7234 1,51;7,706 2,75;7,7026 2,54;7,6683 3,94;7,6642 2,29;7,6494 4,57;7,6295 1,33;7,6204 2,02;7,61 2,01;7,5997 1,94;7,5893 1,87;6,4578 3,71;5,694 0,39;5,2872 2,44;4,5359 0,32;4,5243 0,33;4,1241 0,33;4,1174 0,33;3,4006 0,35;3,3914 0,35;3,2822 0,34;3,2717 0,63;3,2615 0,44;3,2547 0,72;3,2445 1,16;3,2346 0,76;3,2268 0,53;3,2169 0,74;3,2066 0,47;3,1295 220,970001;2,9894 0,36;2,6593 0,39;2,5285 1,64;2,5122 1,28;2,4993 21,559999;2,4947 42,860001;2,49 59,259998;2,4854 41,900002;2,4807 20,459999;2,3167 0,35;2,2285 16;2,1501 0,63;2,1246 0,84;2,0461 0,78;2,0353 0,86;2,0164 1,37;2,0066 1,45;1,9838 1,08;1,9758 1,09;1,9553 0,51;1,9446 0,38;1,403 0,37;-0,0001 4,78 |
| I-32 | [DMSO-d₆] 7,9865 0,69;7,9743 6,79;7,9675 3,43;7,9592 3,28;7,9399 0,73;7,661 1,6;7,6564 1,76;7,6419 1,91;7,6375 1,98;7,6208 1,85;7,6137 1,68;7,6056 1,71;7,5987 1,67;7,5063 1,57;7,5031 1,53;7,487 2,13;7,4835 2,15;7,428 0,59;7,4093 1,46;7,3899 1,09;7,3801 1,36;7,3753 1,34;7,3606 1,46;7,3563 1,39;7,342 0,55;7,3381 0,46;6,4958 4,04;6,3436 0,6;6,3276 2,02;6,3112 2,02;6,2951 0,6;5,7469 2,61;5,3447 0,62;5,3028 2,23;5,2543 2,46;5,2114 0,72;4,4589 0,71;4,4268 0,78;4,039 0,69;4,0362 0,68;4,022 0,54;4,0006 0,75;3,374 0,38;3,3561 0,7;3,3123 358,790009;3,2904 5,67;3,2628 1,18;3,229 0,59;3,1415 0,31;3,1333 0,51;3,1244 0,41;3,1135 0,63;3,1041 1,04;3,0951 0,68;3,0838 0,44;3,0748 0,58;2,8389 0,46;2,8063 0,87;2,7752 0,5;2,6703 0,39;2,5404 0,92;2,51 22,91;2,5057 42,700001;2,5012 55,369999;2,4969 38,07;2,4926 18,059999;2,3324 0,3;2,3279 0,4;2,2188 15;2,1978 0,73;2,0698 0,34;1,9872 1,05;1,9785 0,59;1,9449 1,26;1,9092 0,81;1,8326 0,57;1,8015 0,5;1,7915 0,39;1,673 0,33;1,6369 9,03;1,6205 9,17;1,1754 0,32;0,008 0,34;-0,0001 7,22 |
| I-33 | [DMSO-d₆] 7,9514 1,26;7,9323 3,34;7,9132 2,83;7,8905 2,85;7,8877 3,05;7,8714 1,5;7,5834 2,16;7,5807 2,12;7,5642 1,97;7,5616 1,87;6,4975 3,87;6,044 0,55;6,0347 0,96;6,0204 0,7;6,0115 1,15;6,0024 0,63;5,8189 1;5,8102 1,03;5,8048 0,62;5,7934 0,78;5,7849 0,82;5,7467 1,15;5,4357 1,04;5,3472 0,77;5,3044 2,37;5,2491 2,39;5,2056 0,79;4,458 0,69;4,4275 0,73;4,0276 0,71;3,9947 0,73;3,408 0,32;3,3977 0,39;3,3068 845,460022;3,283 |
| | 23,370001;3,2506 1,5;3,218 0,79;3,2105 0,71;3,1111 0,53;3,1009 0,42;3,0906 0,71;3,083 1,1;3,0749 0,69;3,063 0,47;3,0533 0,58;3,0437 0,37;2,8191 0,51;2,791 0,84;2,7581 0,54;2,6692 0,98;2,6644 0,66;2,5394 2,27;2,5223 3,58;2,5091 57,450001;2,5047 108,660004;2,5002 142,169998;2,4958 98,309998;2,4915 47,139999;2,4498 0,52;2,3315 0,8;2,3269 1;2,3226 0,76;2,2215 15;2,1996 0,49;2,1908 0,3;2,1442 0,37;2,1306 0,38;2,0922 0,79;2,0841 0,8;2,0693 1,21;2,0496 0,98;2,0382 0,79;1,9866 0,71;1,9719 0,84;1,9617 1,03;1,9502 1,12;1,9391 1,47;1,929 1,98;1,9077 1,03;1,8888 0,87;1,854 0,35;1,8415 0,58;1,821 1,29;1,8132 1,24;1,7982 1,19;1,7873 1,29;1,7712 0,95;1,7535 0,86;1,7394 0,64;1,7307 0,63;1,7158 0,39;1,687 0,61;1,6787 0,72;1,6655 0,91;1,6556 0,83;1,633 0,9;1,6241 0,88;1,6016 0,64;1,2366 0,32;-0,0001 18,1;-0,0083 0,85 |
| I-34 | [DMSO-d₆] 15,7097 0,01;14,017 0,01;13,2339 0,01;13,0845 0,01;10,467 0,01;7,9955 0,03;7,9568 0,28;7,9376 0,77;7,9186 0,66;7,8989 0,62;7,8959 0,74;7,8797 0,35;7,8766 0,29;7,5861 0,43;7,5831 0,45;7,5671 0,42;7,5641 0,4;7,5535 0,02;6,679 0,03;6,6053 0,01;6,5048 0,81;5,9782 0,01;5,7545 2,1;5,354 0,17;5,3115 0,48;5,2581 0,5;5,2157 0,17;5,2001 0,03;5,1553 0,01;4,9804 0,06;4,9708 0,12;4,9582 0,14;4,9486 0,23;4,9388 0,15;4,9266 0,11;4,917 0,06;4,4588 0,13;4,4266 0,14;4,1879 0,01;4,1623 0,01;4,0665 0,01;4,0379 0,09;4,0272 0,12;4,0205 0,12;3,9925 0,13;3,9195 0,04;3,8262 0,01;3,6015 0,01;3,5388 0,01;3,5219 0,01;3,5088 0,01;3,4344 0,19;3,385 0,16;3,3766 0,1;3,3348 188,910004;3,3112 2,07;3,2875 0,14;3,2845 0,3;3,2474 0,2;3,2351 0,09;3,2195 0,12;3,1626 0,02;3,1575 0,02;3,1206 0,05;3,1129 0,09;3,1041 0,07;3,0928 0,11;3,0835 0,18;3,0747 0,11;3,0639 0,07;3,0546 0,1;3,0076 0,01;2,8892 0,01;2,8207 0,09;2,7939 0,16;2,7892 0,16;2,7632 0,09;2,6807 0,04;2,676 0,1;2,6713 0,14;2,6668 0,1;2,6067 0,02;2,6026 0,02;2,5434 0,03;2,5248 0,36;2,5202 0,51;2,5115 6,91;2,5069 15,49;2,5023 21,860001;2,4977 15,84;2,4931 7,32;2,4518 0,05;2,4473 0,05;2,3804 0,03;2,3387 0,05;2,3338 0,1;2,3292 0,14;2,3246 0,1;2,3207 0,05;2,2752 0,01;2,2468 0,02;2,2214 2,99;2,2202 2,94;2,202 0,16;2,1963 0,06;2,1478 0,01;2,1006 0,01;2,0734 0,84;2,0583 0,02;1,9887 0,12;1,9597 0,1;1,9269 0,39;1,89 0,35;1,8588 0,06;1,8466 0,06;1,8254 0,11;1,8173 0,12;1,7946 0,1;1,7844 0,11;1,7541 0,2;1,7479 0,2;1,7317 0,22;1,7232 0,22;1,6689 0,05;1,6577 0,05;1,6371 0,11;1,627 0,12;1,6054 0,12;1,5958 0,19;1,5871 0,13;1,571 0,24;1,564 0,34;1,5485 0,25;1,5397 0,38;1,5176 0,22;1,509 0,19;1,461 0,08;1,4526 0,12;1,4369 0,14;1,4284 0,25;1,4202 0,18;1,4118 0,12;1,4035 0,22;1,3957 0,23;1,3794 0,09;1,3715 0,12;1,3638 0,1;1,3529 0,11;1,345 0,07;1,3289 0,11;1,324 0,12;1,306 0,08;1,2985 0,1;1,2744 0,04;1,2487 0,02;1,2347 0,05;1,1926 0,04;1,1748 0,07;1,1679 0,01;1,1646 0,01;1,157 0,04;1,1456 0,02;1,1207 0,01;1,0656 0,01;1,0524 0,01;0,8897 0,07;0,8704 0,01;0,852 0,01;0,8483 0,01;0,7232 0,01;0,1461 0,01;0,008 0,06;-0,0001 2,14;-0,0084 0,07;-2,6982 0,01 |
| I-35 | ¹H NMR (DMSO-d₆, 400 MHz): dₚₚₘ : 1.54-1.64 (m, 1H), 1.74-2.12 (m, 7H), 2.21 (s, 3H), 2.72-2.92 (m, 3H), 3.04-3.11 (m, 1H), 3.23 (m, 1H), 3.98-4.02 (m, 1H), 4.41-4.44 (m, 1H), 5.22 (d, 1H), 5.31 (d, 1H), 6.17 (t, 1H), 6.49 (s, 1H), 7.17-7.33 (m, 4H), 7.56 (dd, 1H), 7.85-7.93 (m, 2H) |
| I-36 | [DMSO-d₆] 8,0029 0,58;7,9836 3,01;7,975 3,42;7,9683 7,59;7,9558 0,83;7,637 1,83;7,63 1,85;7,6206 2,74;7,6148 1,9;7,6049 2,24;7,6007 2,26;7,5783 1,64;7,5756 1,74;7,558 2,22;7,5556 2,29;7,4674 0,98;7,463 1,07;7,4486 1,93;7,4442 1,79;7,4286 1,37;7,4241 1,21;7,3932 1,69;7,39 1,68;7,3743 2,31;7,3711 2,25;7,3555 0,92;7,3523 0,88;6,4881 3,83;5,7471 7,85;5,3503 0,77;5,3385 0,31;5,3048 12,29;5,252 2,39;5,2095 0,76;4,6291 0,42;4,6101 0,45;4,4725 0,69;4,4377 0,73;4,0574 0,36;4,0395 1,31;4,0219 1,29;4,0039 0,93;3,3085 325,380005;3,2851 4,52;3,2482 1,06;3,2174 0,58;3,1386 0,49;3,1287 0,36;3,1177 0,62;3,109 1,02;3,1004 0,61;3,0893 0,42;3,0797 0,57;3,0711 0,32;2,814 0,51;2,7871 0,89;2,7823 0,89;2,7563 0,5;2,6748 0,41;2,6701 0,54;2,6657 0,41;2,5402 0,93;2,5233 2,39;2,5099 29,09;2,5056 53,060001;2,5011 68,040001;2,4968 46,259998;2,4925 21,75;2,3324 0,35;2,3279 0,45;2,3232 0,33;2,2172 15;2,1966 1,08;2,1711 0,41;2,0297 0,61;1,9873 3,74;1,9698 0,64;1,9364 1,42;1,9087 1,51;1,8693 0,42;1,8482 0,61;1,8392 0,64;1,8172 0,55;1,8084 0,49;1,6748 0,32;1,6544 0,58;1,6447 0,62;1,6234 0,54;1,6133 0,56;1,4209 0,72;1,413 1,2;1,1932 0,94;1,1753 1,91;1,1576 0,94;0,7746 0,4;0,7551 0,81;0,7364 0,38;-0,0001 1,77 |
| I-37 | [DMSO-d₆] 7,9713 0,31;7,9522 0,87;7,9333 0,8;7,9189 0,78;7,9157 0,86;7,8997 0,38;7,5978 0,54;7,5947 0,53;7,5791 0,52;7,5759 0,46;6,4965 0,98;5,7469 0,89;5,2939 0,64;5,2492 0,63;4,0149 3,01;3,3071 119,559998;3,2838 1,37;2,5229 0,66;2,5095 8,77;2,5051 16,190001;2,5006 20,950001;2,4963 14,3;2,4919 6,83;2,2174 3,79;1,003 15;0,8309 0,96;-0,0001 1,55 |
| I-38 | [DMSO-d₆] 7,9598 1,16;7,9406 3,12;7,9216 2,6;7,9006 2,79;7,8979 3,03;7,8816 1,45;7,8788 |
| | 1,22;7,5888 2,04;7,5861 2,02;7,5697 1,91;7,5672 1,81;6,4979 3,86;5,7468 3,75;5,3395 0,75;5,2977 2,43;5,2517 2,59;5,2086 0,72;4,4535 0,71;4,4225 0,76;4,1379 4,1;4,1225 4,34;4,0394 0,51;4,022 0,82;3,9935 0,76;3,3089 409,480011;3,2856 5,5;3,2527 1,21;3,2232 0,66;3,1157 0,52;3,1072 0,41;3,096 0,62;3,0865 1,04;3,0782 0,65;3,0655 0,42;3,0573 0,55;2,8296 0,5;2,8002 0,89;2,7709 0,51;2,6743 0,38;2,6696 0,5;2,6653 0,38;2,5399 0,82;2,5094 30,799999;2,5053 55,790001;2,5009 71,300003;2,4967 49,639999;2,332 0,39;2,3276 0,5;2,3232 0,38;2,2214 15;2,2011 0,97;1,987 0,93;1,9653 0,59;1,9296 1,42;1,9084 0,69;1,8925 0,9;1,8551 0,31;1,8466 0,36;1,8236 0,65;1,8153 0,69;1,7783 1,98;1,7635 1,5;1,7513 2,59;1,7436 2,46;1,7262 1,79;1,7177 1,59;1,7014 1,49;1,6927 1,75;1,656 0,98;1,6432 1,13;1,6324 1,29;1,6027 0,7;1,5715 0,33;1,2968 0,38;1,2654 1,13;1,2349 1,74;1,2026 1,61;1,175 1,07;1,1656 0,58;1,1576 0,44;1,1425 0,59;1,1352 0,36;1,0905 0,59;1,0605 1,43;1,0301 1,17;1,0038 0,36;-0,0001 1,12 |
| I-39 | [DMSO-d₆] 7,9606 1,26;7,9415 3,46;7,9224 2,86;7,8999 2,91;7,897 3,15;7,8806 1,55;7,8779 1,28;7,5884 2,16;7,5856 2,11;7,5692 2,06;7,5665 1,85;6,498 3,85;5,8704 0,4;5,8539 0,77;5,8452 0,46;5,837 0,41;5,8281 1,18;5,8112 1,23;5,8021 0,48;5,7943 0,46;5,7855 0,95;5,769 0,46;5,7464 6,68;5,3392 0,7;5,2972 2,48;5,2513 2,47;5,209 0,68;5,061 0,67;5,0569 1,41;5,0518 1,54;5,0479 0,74;5,0179 0,6;5,014 1,29;5,0089 1,39;5,0049 0,66;4,9805 1,46;4,9778 1,34;4,9752 1,31;4,955 1,4;4,9523 1,25;4,9497 1,2;4,4593 0,68;4,4256 0,72;4,3263 2,74;4,3097 5,87;4,2932 2,8;4,0258 0,65;3,9922 0,71;3,3127 556,960022;3,289 12,16;3,2464 1,25;3,2171 0,69;3,1129 0,56;3,1034 0,43;3,0923 0,64;3,0838 1,04;3,0742 0,66;3,063 0,45;3,0536 0,59;3,045 0,34;2,8188 0,47;2,7911 0,87;2,7598 0,5;2,674 0,36;2,67 0,49;2,6658 0,37;2,5401 1,1;2,5232 1,71;2,5098 28,73;2,5054 54,419998;2,5009 71,5;2,4965 49,540001;2,4922 23,84;2,3321 0,43;2,3276 0,53;2,3232 0,37;2,2219 15;2,2018 0,7;2,1276 0,94;2,1096 2,33;2,0919 2,43;2,0741 1,1;2,0696 1,18;1,9624 0,59;1,9291 1,33;1,892 0,78;1,8453 0,32;1,8243 0,6;1,8174 0,64;1,7938 0,56;1,785 0,55;1,7639 0,77;1,7473 1,81;1,7295 2,17;1,7095 1,95;1,693 0,79;1,6651 0,34;1,6431 0,62;1,6321 0,64;1,6113 0,57;1,6028 0,6;1,5322 0,78;1,5125 2,02;1,4999 1,16;1,4941 2,59;1,4751 1,56;1,457 0,51;1,3981 0,32;0,008 0,33;-0,0001 8,86;-0,0082 0,4 |
| I-40 | [DMSO-d₆] 7,9596 1,58;7,9468 4,09;7,9339 2,87;7,9012 3,08;7,8996 3,32;7,8884 2,05;7,8868 1,95;7,5941 2,39;7,5927 2,46;7,5812 2,4;7,5798 2,28;7,5268 0,5;7,2074 0,34;6,5117 4,34;5,7617 1,29;5,3489 1,31;5,3204 2,66;5,2627 2,68;5,2342 1,3;4,4576 0,75;4,4356 0,77;4,3061 2,54;4,295 5,49;4,2839 2,62;4,0194 0,73;3,9965 0,75;3,4153 0,49;3,4095 0,56;3,3781 809,320007;3,3548 4,71;3,267 0,46;3,2632 0,55;3,2422 0,96;3,2242 0,69;3,2198 0,52;3,1025 0,49;3,0965 0,32;3,0888 0,58;3,0827 1,04;3,0767 0,6;3,0693 0,35;3,0629 0,56;2,8069 0,45;2,8026 0,53;2,7854 0,97;2,7814 0,97;2,7642 0,56;2,7601 0,46;2,6178 0,36;2,5268 0,69;2,5237 0,91;2,5205 1,05;2,5117 21,16;2,5087 45,279999;2,5057 62,029999;2,5027 44,830002;2,4997 20,32;2,3896 0,36;2,2195 16;2,2018 1,03;1,9509 0,59;1,9304 0,84;1,9119 0,72;1,8915 0,77;1,8199 0,65;1,8135 0,71;1,799 0,61;1,7926 0,59;1,7346 0,53;1,7233 1,76;1,7116 2,28;1,6989 1,92;1,6876 0,69;1,6471 0,35;1,6332 0,68;1,6264 0,72;1,6124 0,74;1,6057 0,7;1,5918 0,37;1,4107 0,33;1,4005 1,08;1,396 0,87;1,3869 1,66;1,3748 1,4;1,3629 0,81;1,3531 0,66;1,341 1,25;1,3304 1,4;1,3163 1,07;1,3053 0,53;1,3014 0,52;1,296 0,64;1,2862 1,31;1,2833 1,3;1,2759 2,63;1,2721 4,45;1,2669 3,46;1,2605 2,58;1,2504 0,72;0,8682 3,04;0,8569 8,31;0,8451 3,41;0 3,37 |
| I-41 | [DMSO-d₆] 7,9706 1,32;7,9514 3,65;7,9323 3,04;7,9096 2,92;7,9067 3,33;7,8903 1,69;7,8875 1,37;7,6002 2,18;7,5973 2,17;7,5811 2,07;7,5782 1,89;6,4979 3,84;5,7467 0,63;5,3477 0,74;5,3043 2,48;5,252 2,42;5,2092 0,75;4,4634 0,79;4,4438 3,63;4,4357 3,18;4,4323 4,33;4,4283 3,15;4,4204 3,91;4,0308 0,69;3,9989 0,7;3,6794 3,54;3,6714 2,49;3,6676 3,67;3,6641 2,51;3,656 3,26;3,4146 0,38;3,3883 0,63;3,3497 1,72;3,3068 906,27002;3,2515 1,57;3,218 0,83;3,1342 0,36;3,1201 0,62;3,0995 0,68;3,0911 1,09;3,082 0,69;3,0715 0,47;3,0613 0,59;2,8163 0,52;2,7833 0,89;2,7696 0,3;2,7572 0,53;2,6739 0,93;2,6695 1,19;2,6647 0,9;2,5804 0,4;2,5393 1,85;2,5225 5,11;2,5091 68,019997;2,5047 125,610001;2,5002 162,800003;2,4959 110,949997;2,4915 52,77;2,3316 0,82;2,327 1,11;2,3224 0,87;2,2438 0,32;2,2234 15;2,2021 0,75;2,0693 0,5;1,9869 0,31;1,9637 0,6;1,9281 1,38;1,8892 0,81;1,8507 0,32;1,8298 0,6;1,8201 0,64;1,7983 0,56;1,7893 0,51;1,6406 0,64;1,6292 0,61;1,6078 0,54;1,5994 0,55;-0,0001 5,51 |
| I-42 | [DMSO-d₆] 7,9745 0,43;7,9636 1,21;7,9445 3,28;7,9257 3,12;7,9135 3,04;7,9102 3,35;7,8943 1,4;7,891 1;7,6849 0,73;7,6681 0,92;7,6634 1,55;7,6469 1,56;7,6422 1;7,6253 0,8;7,606 2,17;7,6028 2,09;7,5873 2,02;7,5841 1,8;7,3492 0,81;7,3427 0,87;7,3254 1,04;7,3231 1,1;7,3191 1,15;7,2994 0,78;7,2931 0,8;7,1709 0,68;7,1667 0,65;7,1517 1,24;7,1495 |
| | 1,31;7,1455 1,15;7,1304 0,61;7,1283 0,61;7,124 0,54;6,4942 4,04;5,7468 1,28;5,4069 7,38;5,3392 0,86;5,2971 2,41;5,2451 2,42;5,2028 0,73;4,4516 0,7;4,4203 0,77;4,0216 0,72;3,9862 0,72;3,3139 408,269989;3,2903 8,86;3,2752 0,9;3,266 0,82;3,2377 0,95;3,207 0,51;3,1092 0,51;3,1009 0,38;3,0886 0,61;3,0795 0,98;3,07 0,6;3,0594 0,39;3,0503 0,53;2,8083 0,5;2,7756 0,88;2,7499 0,49;2,6706 0,37;2,5406 0,9;2,5236 1,43;2,5103 21,370001;2,506 39,959999;2,5015 51,98;2,4971 35,59;2,4927 16,84;2,328 0,35;2,2134 15;2,1991 1,02;2,0699 0,56;1,9873 0,34;1,9489 0,62;1,9162 1,37;1,8773 0,77;1,8378 0,35;1,8171 0,6;1,8073 0,61;1,786 0,54;1,7768 0,48;1,647 0,34;1,637 0,61;1,6205 0,95;1,5949 0,58;1,5857 0,55;0,008 0,31;-0,0001 7,22 |
| I-43 | ¹HNMR (DMSO-d₆, 400 MHz): dₚₚₘ : 1.57-1.60 (m, 1H), 1.77-1.80 (m, 1H), 1.86-1.92 (m, 2H), 2.21 (s, 3H), 2.74-2.79 (m, 1H), 3.04-3.09 (m, 1H), 3.20-3.25 (m, 1H), 3.98-4.00 (m, 1H), 4.42-4.44 (m, 1H), 5.23 (d, 1H), 5.32 (d, 1H), 5.40 (s, 2H), 6.50 (s, 1H), 7.30-7.33 (m, 2H), 7.59-7.61 (m, 1H), 7.87-7.95 (m,2H) 2H) |
| I-44 | [DMSO-d₆] 7,9637 0,88;7,9446 2,61;7,9263 3,49;7,923 3,23;7,9189 3,35;7,9036 1,04;7,8996 0,61;7,6004 1,8;7,5965 1,81;7,5825 1,72;7,5785 1,6;7,4377 1,52;7,4196 1,81;7,295 0,31;7,2914 0,32;7,2761 1,16;7,2729 1,18;7,2601 3,79;7,2565 4,05;7,2391 1,75;7,233 1,01;7,2208 1,29;7,2147 1,02;7,2045 0,46;7,1984 0,44;6,4943 3,56;5,7469 4,21;5,396 9,5;5,3351 0,68;5,2922 2,29;5,2466 2,28;5,2041 0,66;4,4505 0,64;4,4185 0,71;4,0214 0,76;3,9845 0,69;3,3089 268,51001;3,2855 3,52;3,2412 0,96;3,2112 0,55;3,1131 0,47;3,1045 0,35;3,0931 0,59;3,0839 0,96;3,0755 0,58;3,0639 0,39;3,0548 0,51;3,0459 0,3;2,8145 0,46;2,7831 0,82;2,7546 0,49;2,6699 0,37;2,5397 0,6;2,5093 21,549999;2,5051 38,93;2,5007 49,619999;2,4965 34,450001;2,3787 15;2,3275 0,39;2,2126 13,81;2,2009 1,15;1,987 0,75;1,9547 0,54;1,9213 1,29;1,9086 1,06;1,8854 0,75;1,8199 0,54;1,8107 0,58;1,7886 0,52;1,7797 0,49;1,6375 0,54;1,6284 0,57;1,6069 0,52;1,5975 0,52;1,1751 0,38;-0,0001 1,21 |
| I-45 | ¹HNMR (DMSO-d₆, 400 MHz): dₚₚₘ : 1.60-1.66 (m, 1H), 1.80-1.86 (m, 1H), 1.89-1.98 (m, 2H), 2.21 (s, 3H), 2.75-2.82 (m, 1H), 3.06-3.12 (m, 1H), 3.24 (m, 1H), 3.99-4.02 (m, 1H), 4.42-4.45 (m, 1H), 5.22 (d, 1H), 5.31 (d, 1H), 5.54 (s, 2H), 6.49 (s, 1H), 7.59-7.62 (m, 2H), 7.73-7.83 (m, 3H), 7.91-7.98 (m, 2H) |
| I-46 | [DMSO-d₆] 7,9801 0,83;7,9608 2,88;7,9462 3,99;7,9439 5,23;7,9416 4,54;7,9274 0,92;7,6415 1,41;7,6344 0,97;7,6302 1,5;7,6262 1,53;7,6191 3,21;7,6145 2,18;7,6025 1,8;7,5974 1,67;7,5492 1,43;7,5419 1,11;7,5379 1,52;7,5323 0,97;7,526 2,02;7,4389 0,59;7,4352 0,47;7,4275 3,43;7,4205 2,12;7,4168 2,61;7,4145 2,38;7,4105 2,02;7,4039 3,56;7,392 0,36;6,4933 3,9;5,7472 1,56;5,4637 10,59;5,3401 0,79;5,2979 2,45;5,2483 2,45;5,2057 0,71;4,4552 0,71;4,4226 0,74;4,0226 0,69;3,9907 0,72;3,3709 0,34;3,3061 395,619995;3,2823 12,49;3,2443 1,05;3,2163 0,58;3,1255 0,5;3,1174 0,37;3,1047 0,61;3,0954 1;3,0869 0,6;3,0762 0,41;3,066 0,53;2,8177 0,48;2,7867 0,9;2,7593 0,49;2,6742 0,37;2,6696 0,51;2,6647 0,37;2,5396 1,24;2,5093 31,139999;2,5049 58,419998;2,5004 76,18;2,4961 52,34;2,4917 24,84;2,3316 0,42;2,3274 0,53;2,3225 0,39;2,2122 15;2,1978 0,85;2,0696 1,4;2,0496 0,33;1,987 0,32;1,9646 0,57;1,9316 1,34;1,8935 0,79;1,8542 0,32;1,8319 0,6;1,8237 0,62;1,8014 0,54;1,7924 0,54;1,6639 0,31;1,6441 0,6;1,6338 0,61;1,6129 0,57;1,6041 0,55;1,2363 0,44;0,0079 0,62;-0,0001 15,04;-0,0084 0,67 |
| I-47 | ¹HNMR (DMSO-d₆, 400 MHz): dₚₚₘ : 1.56-1.65 (m, 1H), 1.78-1.84 (m, 1H), 1.88-1.95 (m, 2H), 2.21 (s, 3H), 2.74-2.80 (m, 1H), 3.05-3.11 (m, 1H), 3.23 (m, 1H), 3.98-4.01 (m, 1H), 4.41-4.45 (m, 1H), 5.22 (d, 1H), 5.31 (d, 1H), 5.44 (s, 2H), 6.49 (s, 1H), 7.24-7.29 (m, 2H), 7.41-7.47 (m, 1H), 7.56-7.60 (m, 2H), 7.90-7.96 (m, 2H) |
| I-48 | [DMSO-d₆] 7,9521 1,29;7,9328 3,36;7,9136 2,63;7,8818 2,78;7,8794 2,97;7,8627 1,63;7,8602 1,41;7,5994 2,2;7,5972 2,17;7,5801 2,12;7,5776 2,12;7,56 0,86;7,5556 0,81;7,5388 1,61;7,522 0,81;7,5177 0,92;7,5008 0,43;7,2209 0,45;7,2114 2,43;7,1914 3,73;7,1791 0,43;7,171 2,06;7,1616 0,3;6,4935 3,93;5,7476 2,08;5,4531 7,2;5,336 0,73;5,293 2,35;5,2407 2,35;5,1981 0,72;4,4452 0,67;4,413 0,71;4,0128 0,65;3,9778 0,7;3,3118 191,509995;3,2881 6,9;3,2604 0,65;3,2291 0,92;3,2013 0,52;3,0981 0,48;3,0889 0,37;3,0779 0,6;3,0687 0,97;3,0597 0,59;3,0486 0,38;3,0393 0,54;2,8015 0,46;2,7734 0,86;2,7411 0,5;2,541 0,55;2,5106 13,11;2,5063 24,59;2,5019 32;2,4975 22,16;2,4932 10,62;2,2119 15;1,9877 0,42;1,9381 0,56;1,9064 1,37;1,868 0,77;1,8246 0,31;1,8024 0,57;1,7944 0,62;1,7717 0,53;1,7634 0,49;1,6117 0,56;1,602 0,62;1,5814 0,55;1,5712 0,54;-0,0001 3,7 |
| I-49 | [DMSO-d₆] 8,3741 1,66;8,3724 1,72;8,3662 1,71;8,3645 1,68;7,9681 1,12;7,9554 3,23;7,943 3,55;7,9381 3,3;7,9357 3,68;7,9253 1,35;7,9229 0,92;7,6725 1,6;7,6713 1,62;7,6599 1,73;7,6586 1,7;7,613 2,07;7,6106 2,08;7,6007 2,06;7,5984 1,96;7,3093 1,77;7,3013 1,77;7,2966 1,7;7,2886 1,62;6,5085 4,22;5,7615 3,08;5,4852 10,75;5,3427 1,28;5,3141 |
| | 2,57;5,256 2,53;5,2275 1,26;4,567 0,34;4,5581 0,33;4,4508 0,72;4,4288 0,74;4,0101 0,7;3,9874 0,73;3,4428 0,41;3,427 0,41;3,4016 2,13;3,3764 1459,030029;3,3531 9,64;3,3448 0,83;3,3364 0,45;3,3277 0,32;3,2554 0,48;3,2514 0,56;3,2303 0,96;3,2122 0,58;3,2081 0,5;3,1033 0,49;3,0895 0,6;3,0834 1,04;3,0773 0,6;3,0696 0,35;3,0635 0,55;2,7926 0,43;2,7883 0,52;2,771 0,95;2,7672 0,96;2,7499 0,55;2,7458 0,45;2,6202 0,56;2,6171 0,75;2,6141 0,53;2,5261 1,64;2,523 2,19;2,5198 2,6;2,511 44,549999;2,5081 93,360001;2,5051 126,209999;2,5021 90,669998;2,4991 41,110001;2,3937 16;2,3063 1,03;2,2851 0,32;2,2095 15,62;2,2002 0,59;2,0772 0,62;1,9415 0,58;1,9215 0,84;1,9087 0,7;1,9044 0,71;1,8832 0,75;1,8322 0,32;1,8181 0,65;1,8117 0,69;1,7971 0,61;1,7907 0,58;1,6238 0,64;1,617 0,68;1,6029 0,65;1,5963 0,63;0,8891 0,42;0 6,03 |
| I-50 | [DMSO-d₆] 7,9649 1,19;7,9457 3,3;7,9269 3,1;7,9148 3,02;7,9116 3,28;7,8956 1,35;7,8923 0,92;7,5999 2,11;7,5967 2,06;7,5811 1,97;7,578 1,72;7,1453 2,13;7,1424 2,87;7,1395 2,09;7,0844 3,25;6,4956 3,84;5,7463 3,57;5,4866 10,13;5,3436 0,79;5,3012 2,34;5,2458 2,3;5,2034 0,73;4,4567 0,69;4,4229 0,72;4,0215 0,71;3,9866 0,73;3,4292 0,34;3,4254 0,31;3,3909 0,51;3,3127 684,559998;3,2893 12,01;3,2696 1,14;3,2658 1,08;3,2395 0,99;3,2063 0,55;3,1082 0,51;3,0994 0,37;3,0879 0,6;3,0781 0,99;3,0685 0,59;3,0581 0,43;3,0483 0,54;2,8032 0,47;2,7773 0,86;2,7471 0,49;2,6745 0,45;2,6698 0,57;2,6654 0,41;2,5399 1,45;2,5231 2,31;2,5096 35,41;2,5053 66,639999;2,5008 87,099998;2,4964 59,889999;2,492 28,610001;2,4485 0,3;2,3317 0,45;2,3273 0,61;2,3228 0,45;2,2175 15;2,1951 13,6;2,1929 13,46;2,1737 0,41;2,1592 0,41;2,0693 0,9;2,0497 0,33;1,9445 0,54;1,9084 1,53;1,8749 0,79;1,8488 0,34;1,8397 0,37;1,8165 0,6;1,8083 0,64;1,7874 0,55;1,7774 0,53;1,6518 0,31;1,6319 0,56;1,6221 0,6;1,6015 0,58;1,5909 0,59;1,3981 0,39;1,3889 1,53;1,3836 0,56;0,0077 0,41;-0,0001 11,02;-0,0081 0,5 |
| I-51 | [DMSO-d₆] 8,2422 2,35;8,241 2,44;8,2295 2,92;8,2282 2,89;8,1079 2,03;8,0949 3,76;8,082 1,97;8,0647 1,75;8,0627 1,98;8,0504 1,98;7,9458 2,28;7,932 2,51;7,9214 1,75;7,9079 2,06;7,7582 2,56;7,7461 2,46;7,7451 2,41;7,63 2,51;7,6171 4,78;7,6102 0,55;7,606 2,39;7,6037 5,45;7,6012 2,23;7,5923 0,93;7,59 1,76;7,5879 1,68;7,5789 0,87;7,5766 0,68;7,5275 2,82;7,5262 3,08;7,5151 2,24;7,5138 2,13;6,4963 4,39;5,7634 7,45;5,757 0,78;5,3641 1,37;5,3356 2,55;5,2674 2,6;5,239 1,36;4,4903 0,75;4,4681 0,78;4,0508 0,72;4,046 0,91;4,034 1,63;4,0279 0,78;4,0222 1,59;4,0103 0,41;3,3909 0,63;3,3798 1,18;3,3587 788,400024;3,3352 6,5;3,3016 0,46;3,2974 0,55;3,2763 0,94;3,2583 0,55;3,2543 0,44;3,2143 0,48;3,2008 0,57;3,1946 1,03;3,1887 0,59;3,1811 0,34;3,1748 0,54;2,8413 0,45;2,8376 0,53;2,8199 0,96;2,8162 0,97;2,7989 0,56;2,7945 0,46;2,6189 0,6;2,616 0,81;2,613 0,6;2,525 1,4;2,5219 1,77;2,5188 1,86;2,5098 45,02;2,5069 96,790001;2,5039 132,770004;2,5009 96,639999;2,498 44,439999;2,4036 1,08;2,3908 0,54;2,3879 0,76;2,3848 0,53;2,2008 16;2,1823 0,44;2,0778 0,81;2,0409 0,58;2,0202 0,87;2,0153 0,87;2,01 0,81;1,9906 5,4;1,9842 0,81;1,9204 0,62;1,9142 0,69; 1,8994 0,6;1,8932 0,58;1,7048 0,66;1,6979 0,71;1,684 0,66;1,6773 0,65;1,1863 1,33;1,1744 2,65;1,1626 1,3;0,8891 0,34;0 4,47 |
| I-52 | ¹H NMR (DMSO-d₆, 400 MHz): dₚₚₘ : 1.65-1.71 (m, 1H), 1.87-2.06 (m, 3H), 2.21 (s, 3H), 2.78-2.84 (m, 1H), 3.14-3.24 (m, 1H), 3.28 (m, 1H), 4.02-4.05 (m, 1H), 4.45-4.49 (m, 1H), 5.23 (d, 1H), 5.35 (d, 1H), 6.48 (s, 1H), 7.48 (dd, 1H), 7.53-7.60 (m, 2H), 7.70 (d, 1H), 7.85 (d, 1H), 7.94-8.07 (m, 4H), 8.15 (dd, 1H) |
| I-53 | [DMSO-d₆] 7,9897 1,65;7,9703 3,74;7,9509 2,3;7,7561 2,43;7,7542 2,7;7,737 2,27;7,735 2,25;7,6569 2,3;7,6551 2,33;7,6374 2,17;6,5032 3,84;5,3756 0,9;5,333 2,06;5,2451 2,06;5,2025 0,91;5,0285 0,64;4,456 0,7;4,4231 0,74;4,0396 0,76;4,0088 0,74;3,514 0,98;3,5058 0,87;3,4877 0,64;3,3228 1112,77002;3,2362 1,4;3,1707 0,39;3,1432 0,38;3,1333 0,53;3,1251 0,74;3,1157 0,59;3,105 0,83;3,0964 1,21;3,0884 0,81;3,0771 0,58;3,0676 0,73;3,0585 0,47;2,8507 0,56;2,8177 0,93;2,792 0,6;2,675 0,84;2,6705 1,11;2,666 0,84;2,6613 0,47;2,5237 4,81;2,5103 60,290001;2,5059 111,529999;2,5014 144,630005;2,497 98,989998;2,4926 47,119999;2,3375 0,44;2,3326 0,79;2,3282 1,06;2,3237 0,78;2,3191 0,45;2,2518 1,17;2,2295 15;2,2043 0,71;2,1918 0,31;2,0689 0,63;1,9983 0,64;1,9869 1,1;1,9535 1,72;1,9404 1,66;1,9181 2,49;1,8523 0,39;1,8309 0,64;1,8207 0,64;1,7986 0,6;1,7903 0,58;1,7685 0,37;1,7583 0,41;1,7206 1,61;1,7084 1,42;1,6956 1,42;1,6629 0,74;1,6534 0,73;1,6223 1,04;1,6002 0,82;1,5888 0,85;1,5109 0,51;1,4833 1,37;1,4577 3,01;1,4354 2,76;1,4108 1,31;1,3983 2,88;1,3834 0,49;1,332 0,3;1,3232 0,39;1,2979 0,68;1,2748 0,6;1,2371 0,4;1,1752 0,4;-0,0001 3,08 |
| I-54 | [DMSO-d₆] 7,9958 1,59;7,9765 3,66;7,957 2,24;7,7668 2,66;7,7495 2,18;7,7476 2,23;7,6649 2,4;7,647 2,16;6,4983 3,9;5,3675 0,82;5,325 2,13;5,2501 2,2;5,208 0,83;4,4524 0,66;4,4181 0,72;4,0396 0,67;4,0087 0,7;3,301 372,799988;3,2463 1,05;3,1365 0,63;3,1285 0,46;3,1174 |
| | 0,72;3,108 1,13;3,0994 0,72;3,0878 0,5;3,0787 0,65;3,0704 0,42;2,9606 2,51;2,9424 4,16;2,924 2,62;2,8901 0,37;2,8636 0,53;2,8318 0,9;2,8053 0,53;2,6736 0,55;2,669 0,69;2,6646 0,51;2,5392 3,73;2,5223 2,55;2,5089 38,990002;2,5046 72,800003;2,5001 94,620003;2,4957 64,830002;2,4914 30,879999;2,331 0,55;2,3267 0,69;2,3223 0,53;2,2459 0,93;2,2278 15;2,2022 0,56;2,1989 0,42;2,0497 0,39;2,01 0,62;1,9868 0,95;1,9714 1,19;1,9341 0,82;1,8681 0,32;1,8608 0,36;1,8368 0,61;1,83 0,65;1,8075 0,58;1,7983 0,54;1,7046 0,31;1,6946 0,35;1,6736 0,61;1,6644 0,68;1,6425 0,65;1,6307 0,91;1,6127 1,64;1,5937 2,28;1,5759 1,74;1,5567 0,75;1,3934 1;1,3745 1,55;1,3537 1,45;1,3382 1,34;1,3215 1,48;1,3067 2;1,2922 2,81;1,2857 2,78;1,2703 5,35;1,2536 2,64;0,8803 2,62;0,8636 8,07;0,8461 3,07;0,008 0,63;-0,0001 13,19;-0,0084 0,47 |
| I-55 | [DMSO-d₆] 7,5352 8,02;7,5298 8,51;7,5203 8,1;7,4995 10,36;7,3962 1,02;7,3916 0,82;7,3851 6,04;7,3797 5,68;7,3644 4,43;7,359 4,15;3,9416 1,4;3,808 15,74;3,7883 16;3,1254 38,93;2,8231 3,71;2,8034 7,91;2,7836 3,39;2,5294 0,4;2,5004 4,72;2,4958 9,28;2,4911 12,7;2,4864 8,95;2,4818 4,36;-0,0001 1,07 |
| I-56 | [DMSO-d₆] 8,0111 1,32;7,9918 2,94;7,9724 1,78;7,8092 2,24;7,7902 1,88;7,6747 2,04;7,655 1,84;7,3665 1,4;7,3503 1,65;7,3462 1,34;7,2065 0,7;7,1981 2,17;7,1947 2,72;7,1889 2,73;7,1831 3,1;7,1791 2,53;7,1718 1,72;7,1658 1,35;7,1611 1,31;7,1551 1,37;7,1476 1,02;7,1386 0,78;7,1323 0,53;6,5123 0,56;6,4621 3,23;5,7472 0,41;5,3497 0,79;5,3072 1,72;5,2139 1,71;5,1722 0,77;5,008 0,91;4,4255 0,62;4,394 0,7;4,2304 8,26;4,0572 0,88;4,0394 2,49;4,0216 2,75;4,004 1,35;3,9744 0,66;3,3038 196,100006;3,238 1,08;3,2078 0,59;3,1134 0,47;3,103 0,35;3,0942 0,62;3,0851 0,89;3,0755 0,57;3,0657 0,38;3,0558 0,5;2,8253 0,47;2,7986 0,81;2,7659 0,49;2,674 0,58;2,6694 0,72;2,6648 0,56;2,5394 1,27;2,5088 43,200001;2,5047 76,82;2,5003 96,32;2,4961 66,199997;2,3351 15;2,3038 0,4;2,282 0,44;2,2506 2,36;2,2121 0,35;2,2005 0,43;2,1894 0,69;2,1714 12,98;2,0695 0,59;1,9869 10,78;1,9424 1,22;1,9082 0,98;1,8029 0,56;1,7804 0,48;1,7717 0,47;1,6208 0,51;1,6115 0,53;1,5898 0,48;1,5808 0,48;1,3983 2,91;1,1928 2,79;1,175 5,54;1,1573 2,71;-0,0001 6,83;-0,008 0,33 |
| I-57 | ¹H NMR (DMSO-d₆, 400 MHz): dₚₚₘ: 1.54-1.68 (m, 1H), 1.71-1.86 (m, 1H), 1.90-2.01 (m, 2H), 2.19 (s, 3H), 2.75-2.84 (m, 1H), 3.04-3.16 (m, 1H), 3.95-4.06 (m, 1H), 4.32 (s, 2H), 4.39-4.46 (m, 1H), 5.20 (d, 1H), 5.33 (d, 1H), 6.47 (s, 1H), 7.28-7.37 (m, 2H), 7.45-7.51 (m, 1H), 7.56-7.61 (m, 1H), 7.67 (d, 1H), 7.80 (d, 1H), 8.00 (dd, 1H) |
| I-58 | [DMSO-d₆] 8,0208 1,64;8,0014 3,54;7,9821 2,15;7,8185 2,59;7,8166 2,56;7,7993 2,24;7,6909 2,39;7,6711 2,15;7,4519 0,36;7,4353 0,84;7,4315 0,77;7,4141 1,58;7,3978 0,88;7,3932 0,93;7,3762 0,41;7,1606 0,48;7,1511 2,5;7,1311 3,71;7,1222 0,68;7,1109 1,95;7,1012 0,38;6,517 0,49;6,4735 3,74;5,3601 0,87;5,3179 1,96;5,2193 1,91;5,1771 0,89;5,0255 0,97;4,4325 0,7;4,3994 0,74;4,2785 6,79;4,0392 0,42;4,0216 0,88;4,0045 0,53;3,9811 0,72;3,3022 366,459991;3,2405 1,19;3,2116 0,63;3,1199 0,53;3,0989 0,63;3,0908 1,02;3,0819 0,62;3,0702 0,42;3,061 0,54;2,8281 0,51;2,7994 0,88;2,7676 0,52;2,6739 0,75;2,6694 0,95;2,6648 0,75;2,5652 0,46;2,5225 4,54;2,5091 59,200001;2,5048 107,709999;2,5004 137,470001;2,496 94,580002;2,4918 45,470001;2,3317 0,75;2,3271 0,98;2,3225 0,74;2,2525 1,95;2,1846 15;2,0698 0,72;1,987 1,91;1,9464 1,38;1,9293 0,66;1,9081 1,15;1,8413 0,38;1,8369 0,38;1,8043 0,59;1,7787 0,53;1,7749 0,54;1,6172 0,58;1,6078 0,6;1,5858 0,56;1,5769 0,55;1,1931 0,46;1,1751 0,84;1,1572 0,45;0,008 0,61;-0,0001 14,2;-0,0083 0,67 |
| I-59 | [DMSO-d₆] 8,0097 1,61;8,002 0,33;7,9904 3,54;7,971 2,2;7,9521 0,43;7,8087 0,47;7,7981 2,67;7,7809 2,35;7,6766 2,34;7,6587 2,12;7,445 2,33;7,4311 2,85;7,4231 3,11;7,415 1,54;7,4094 2,86;7,1655 2,92;7,1603 1,44;7,1432 5,11;7,126 1,18;7,121 2,51;6,6595 0,36;6,5185 1,92;6,4805 3,61;5,3506 0,9;5,3083 2,12;5,2304 2,06;5,1877 0,88;5,0297 5,81;4,4341 0,74;4,3977 0,9;4,2148 8,98;4,1909 0,57;4,057 0,61;4,0393 1,72;4,0214 2,18;4,0037 0,9;3,9996 0,6;3,9862 0,83;3,7243 0,42;3,4793 0,44;3,4547 0,53;3,4368 0,66;3,3792 1,57;3,3045 643,530029;3,2188 1,17;3,1682 0,35;3,1233 0,64;3,0942 1,15;3,0837 0,77;3,0653 0,77;2,8905 2,33;2,8407 0,57;2,8091 0,96;2,7841 0,61;2,7319 1,9;2,6735 1,54;2,669 2;2,6647 1,52;2,5888 0,49;2,5223 9,6;2,5089 118,849998;2,5046 218,559998;2,5002 282,119995;2,4958 194,5;2,4915 93,57;2,4132 0,49;2,3989 0,43;2,3897 0,36;2,3527 0,41;2,3314 1,47;2,327 2,03;2,322 1,51;2,2528 8,24;2,196 15;2,1706 0,42;2,1435 0,35;2,0695 1,46;1,9868 7,12;1,9506 1,36;1,9078 1,11;1,8493 0,41;1,8455 0,51;1,8141 0,68;1,8064 0,67;1,7823 0,62;1,7639 0,35;1,681 0,38;1,6706 0,36;1,6471 0,71;1,6384 0,65;1,6139 0,65;1,6058 0,62;1,5818 0,34;1,3984 1,48;1,236 0,47;1,1928 1,83;1,175 3,54;1,1572 1,76;0,8898 0,36;0,0079 1,55;-0,0001 35,349998;-0,0084 1,56 |
| I-60 | ¹H NMR (DMSO-d₆, 400 MHz): dₚₚₘ : 1.70-1.81 (m, 1H), 1.89-1.98 (m, 1H), 2.02-2.14 (m, 2H), 2.23 (s, 3H), 2.85-2.96 (m, 1H), 4.02-4.11 (m, 1H), 4.46-4.54 (m, 1H), 5.25 (d, 1H), 5.37 (d, 1H), 6.49 (s, 1H), 7.55-7.64 (m, 3H), 7.70-7.84 (m, 3H), 8.00-8.17 (m, 4H) |
| I-61 | [DMSO-d₆] 10,0221 0,33;8,1672 3,41;8,0589 1,9;8,0551 0,36;8,046 4,11;8,0399 3,08;8,0331 2,72;8,0253 3,43;8,0204 2,15;8,0071 2,03;7,9954 1,79;7,9826 1,87;7,9525 0,37;7,8063 2,73;7,805 2,98;7,7936 2,61;7,7923 2,67;7,7676 2,57;7,7665 2,52;7,7545 2,6;7,6452 0,94;7,6427 0,99;7,6337 2,23;7,6314 2;7,6209 3,44;7,6177 3,46;7,6072 1,77;7,605 1,9;7,5959 0,88;7,5935 0,73;7,5783 2,35;7,5754 2,23;7,5642 2,3;7,5613 2,28;6,5232 0,48;6,5114 4,49;5,7644 0,4;5,4049 1,44;5,3764 2,39;5,2857 2,37;5,2572 1,45;4,4892 0,74;4,4674 0,79;4,0801 0,71;4,057 0,76;4,046 1,2;4,0341 3,29;4,0222 3,32;4,0104 1,09;3,3513 293,450012;3,3278 6,66;3,3184 1,41;3,2974 0,73;3,2118 0,54;3,2055 0,39;3,1985 0,65;3,1925 1,1;3,1864 0,68;3,1794 0,42;3,1729 0,63;3,1674 0,35;2,8967 0,46;2,8908 1,34;2,8758 0,95;2,8719 0,99;2,8546 0,78;2,8506 0,52;2,7314 0,87;2,7306 0,86;2,6188 0,61;2,6157 0,84;2,6127 0,61;2,5248 1,56;2,5217 2,1;2,5186 2,43;2,5097 52,360001;2,5068 111,389999;2,5037 151,720001;2,5007 109,730003;2,4978 50,18;2,3936 0,35;2,3906 0,69;2,3876 1;2,3848 0,75;2,2551 0,63;2,2474 2,16;2,2331 16;2,2203 0,5;2,1957 1,19;2,1916 0,33;2,0783 0,91;2,0605 0,85;2,035 0,72;2,0139 0,8;1,9953 0,51;1,9907 14,64;1,9387 0,33;1,9247 0,63;1,9183 0,7;1,9105 0,81;1,9038 0,63;1,8978 0,6;1,756 0,33;1,7418 0,63;1,7354 0,68;1,7213 0,65;1,7148 0,62;1,3968 0,73;1,2332 0,53;1,1863 3,96;1,1745 7,96;1,1626 3,9;0,8889 0,37;0,0053 0,37;0 10,79;-0,0056 0,36 |
| I-62 | [DMSO-d₆] 8,0443 1,66;8,0249 3,58;8,0056 2,22;7,7822 2,75;7,7649 2,29;7,7403 2,49;7,7207 2,24;7,5267 1,48;7,5089 11,54;7,5049 12,24;7,4955 1,98;7,4865 1,26;6,4975 3,84;5,3819 0,85;5,3402 2,21;5,2638 2,1;5,2206 0,91;4,4812 0,74;4,4453 0,82;4,0704 0,77;4,0668 0,7;4,057 1,02;4,0393 2,52;4,0214 1,96;4,0037 0,67;3,622 0,33;3,5932 0,36;3,549 0,47;3,4748 0,69;3,4591 0,77;3,3172 2088,639893;3,2284 0,73;3,2065 0,68;3,1963 0,75;3,1889 0,69;3,1764 0,91;3,168 1,31;3,1571 0,81;3,1379 0,72;3,13 0,44;2,891 0,55;2,8864 0,4;2,8605 0,9;2,8351 0,57;2,6745 1,42;2,6699 1,89;2,6656 1,43;2,5393 2,21;2,5231 8,84;2,5097 112,940002;2,5054 208,210007;2,501 268,640015;2,4966 185,160004;2,4923 88,660004;2,4149 0,43;2,3458 0,3;2,3366 0,8;2,3323 1,37;2,3278 1,94;2,3232 1,33;2,2512 0,51;2,2298 15;2,1963 0,68;2,0849 0,99;2,0689 1,67;2,0301 1,23;1,9868 8,63;1,9278 0,35;1,9214 0,43;1,9081 0,67;1,892 0,68;1,87 0,57;1,7514 0,32;1,7468 0,31;1,7257 0,6;1,7158 0,7;1,6947 0,6;1,683 0,58;1,3984 0,5;1,2355 0,33;1,1928 2,1;1,175 4,43;1,1573 2,19;0,8899 0,37;0,008 0,36;-0,0001 8,21 |
| I-63 | ¹H NMR (DMSO-d₆, 400 MHz): dₚₚₘ: 1.63-1.77 (m, 1H), 1.80-1.93 (m, 1H), 1.97-2.09 (m, 2H), 2.23 (s, 3H), 2.80-2.91 (m, 1H), 3.13-3.21 (m, 1H), 4.01-4.09 (m, 1H), 4.41-4.49 (m, 1H), 5.24 (d, 1H), 5.36 (d, 1H), 6.51 (s, 1H), 7.20-7.25 (m, 1H), 7.32-7.36 (m, 1H), 7.75 (d, 1H), 7.79 (d, 1H), 7.88-7.92 (m, 1H), 8.04 (dd, 1H) |
| I-64 | [CD₃CN] 7,643 1,14;7,6247 1,4;7,6216 1,8;7,6033 1,24;6,816 1,65;6,8013 0,85;6,7946 1,63;6,7802 0,68;6,7553 1,84;6,7372 1,76;6,3977 3,28;5,4459 1,89;5,0811 9,19;3,6526 2,7;3,6409 4,64;3,6288 2,62;3,6144 2,88;3,5995 2,12;3,5821 1,91;3,5737 1,88;3,5667 1,96;3,5238 0,38;3,5026 0,44;3,4941 0,72;3,4859 0,44;3,4646 0,41;2,8852 11,79;2,7805 4,45;2,2311 15;2,1577 95,870003;1,9713 0,86;1,9629 0,48;1,9511 6,77;1,945 12,7;1,9388 18,129999;1,9326 12,53;1,9265 6,48;1,8539 0,43;1,8217 0,47;1,746 1,35;1,728 2,25;1,6912 0,84;1,6574 0,3;1,6289 0,35;1,5905 0,78;1,5699 0,81;1,5567 0,94;1,5461 0,96;1,5263 1,08;1,4399 0,45;1,4078 0,39;1,2035 0,43;1,1857 0,33;1,0801 1,09;1,0686 1,57;1,045 0,54;-0,0001 1 |
| I-65 | [CD₃CN] 8,5608 0,71;7,7333 1,65;7,7149 2,07;7,712 2;7,6936 1,91;7,4371 3,22;7,4307 1,2;7,4188 3,5;7,4121 1,81;7,3998 0,33;7,3892 1,05;7,3823 1,15;7,3659 1,57;7,3117 0,45;7,3068 0,7;7,2931 2;7,2883 1,95;7,2849 1,89;7,2772 3,26;7,27 1,5;7,2665 1,48;7,2613 1,18;7,2477 0,41;6,9833 2,59;6,9619 2,45;6,401 3,23;5,0955 10,18;5,0018 0,37;4,6662 4,51;4,6501 4,44;4,0685 0,49;4,0507 0,46;3,7585 1,38;3,7464 2,17;3,7323 2,06;3,6566 8,02;3,6388 3,05;3,6309 2,24;3,6251 2,38;3,613 1,42;3,6005 0,67;3,0292 0,66;2,8985 0,54;2,2718 0,33;2,2367 15;2,2136 1,3;2,1556 377,309998;2,1129 1,25;2,1065 1,14;2,1004 0,9;2,0722 0,44;1,9715 3,01;1,9515 26,77;1,9453 49,98;1,9392 70,379997;1,933 48,59;1,9268 25,1;1,7739 0,36;1,7678 0,49;1,7616 0,3;1,2707 0,79;1,2216 0,66;1,2037 1,17;1,186 0,59;0,0078 1,98;-0,0001 36,900002;-0,0083 1,74 |
| I-66 | [CD₃CN] 8,3549 0,65;7,6997 2,17;7,6876 2,49;7,6855 2,4;7,6734 2,46;7,4128 2,78;7,4119 2,83;7,4007 2,64;7,3998 2,47;7,3577 0,4;7,3468 0,78;7,3437 0,79;7,3358 0,47;7,3328 1,54;7,3298 0,51;7,3219 0,77;7,3188 0,87;7,3078 0,41;6,9948 1,98;6,9884 0,37;6,9812 |
| | 3,52;6,974 0,4;6,9675 1,84;6,9476 2,65;6,9342 2,62;6,4049 3,34;5,4493 0,52;5,0965 10,94;4,6663 3,84;4,6561 3,86;4,0652 0,65;4,0534 0,69;3,7191 1,39;3,7108 1,99;3,7073 1,55;3,7018 1,99;3,6578 0,93;3,652 1,91;3,6467 1,55;3,6415 2,24;3,6189 3,89;3,6095 3,39;3,6021 2,19;2,235 16;2,234 15,75;2,2154 0,61;2,2128 0,37;2,2081 0,83;2,2002 2,38;2,1877 906,52002;2,1569 0,66;2,1544 0,53;2,1465 0,41;2,0566 0,52;2,0526 0,75;2,0484 0,53;1,9733 3,07;1,9663 1,41;1,9582 1,44;1,954 2,05;1,9502 47,91;1,9461 94,529999;1,942 140,320007;1,9379 95,580002;1,9338 48,73;1,925 1,35;1,8313 0,52;1,8272 0,77;1,8231 0,5;1,2161 0,83;1,2043 1,72;1,1924 0,85;0 1,02 |
| I-67 | [CD₃CN] 8,5455 0,66;7,7204 1,66;7,7021 2,13;7,6991 2,08;7,6808 2;7,4433 2,7;7,4252 2,33;7,2691 1,77;7,266 1,87;7,2563 1,97;7,2532 1,96;7,0342 1,42;7,0321 1,52;7,0259 1,79;7,0236 1,74;6,9639 2,34;6,9591 3;6,9554 2,22;6,9512 2,09;6,9424 1,84;6,9381 2,64;6,3998 3,24;5,0927 10,41;4,7285 4,42;4,7125 4,34;3,7412 1,32;3,7298 2,1;3,7157 2,08;3,6452 5,05;3,6412 5,18;3,628 3,64;3,6199 2,59;3,6138 2,55;3,6019 1,42;2,2334 15;2,1507 225,710007;2,1123 0,61;2,1063 0,59;2,1001 0,45;2,0939 0,32;1,963 1,02;1,9512 15,17;1,9451 28,6;1,9389 41;1,9327 28,370001;1,9265 14,67;-0,0001 2,75 |
| I-68 | [CD₃CN] 7,6945 1,56;7,676 2,04;7,6731 1,98;7,6547 1,86;7,3922 2,77;7,3741 2,39;6,9795 2,62;6,9581 2,48;6,4009 3,3;6,0462 0,48;6,0376 0,82;6,0277 0,54;6,0241 0,6;6,0212 0,62;6,0143 1,03;6,0054 0,59;6,0028 0,55;5,8321 0,46;5,8267 0,9;5,8227 0,84;5,8181 0,94;5,8125 0,52;5,807 0,42;5,8015 0,72;5,7978 0,65;5,7928 0,74;5,7874 0,38;5,4464 4,98;5,093 10,07;3,7153 1,22;3,7046 2,08;3,697 1,84;3,6914 2,02;3,6626 2,12;3,6476 2,9;3,6334 2,66;3,621 5,18;2,3375 0,68;2,2642 0,63;2,2358 15;2,1674 99,370003;2,1219 1,28;2,1171 1,28;2,1127 1,24;2,1073 1,31;2,1027 1;2,088 0,59;2,0826 0,57;2,0736 0,77;2,0685 0,83;2,0643 0,84;2,0604 0,83;2,0516 0,72;2,0424 0,58;2,0372 0,43;2,019 0,44;2,0099 0,64;2,0022 0,62;1,9971 0,57;1,986 0,66;1,9766 1,08;1,9718 1,41;1,9641 1,54;1,9519 11,31;1,9457 20,530001;1,9396 28,68;1,9334 19,76;1,9273 10,19;1,877 0,45;1,8694 0,59;1,8645 0,6;1,8564 1,01;1,8493 0,95;1,8364 1,06;1,8277 0,96;1,8235 1,02;1,8154 0,9;1,809 0,99;1,8015 0,87;1,794 0,6;1,777 0,37;1,7684 0,4;1,7411 0,32;1,7369 0,32;1,7262 0,64;1,7175 0,7;1,7112 0,63;1,7055 0,71;1,6973 0,61;1,6922 0,56;1,6844 0,53;1,6705 0,41;1,6639 0,31;1,4256 0,41;1,2745 0,3;1,2571 0,34;0,0078 0,81;-0,0001 14,45;-0,0082 0,63 |
| I-69 | [CD₃CN] 7,6996 1,68;7,6813 2,18;7,6782 1,77;7,6599 1,8;7,4011 2,68;7,3828 2,27;6,9818 2,52;6,9603 2,28;6,402 2,97;5,4468 1,38;5,1335 0,3;5,0943 9,8;4,9934 0,35;4,9843 0,62;4,972 0,69;4,9622 1,09;4,9526 0,67;4,9403 0,47;4,0868 0,47;4,0689 1,33;4,0511 1,31;4,0332 0,45;3,721 1,37;3,7103 2,11;3,703 1,77;3,6967 2,07;3,6644 2,01;3,6492 2,9;3,6358 2,83;3,6259 4,43;3,6121 2,27;2,2638 0,81;2,2371 15;2,2358 14,28;2,1696 194,880005;1,9719 6,43;1,9637 1,42;1,952 16,58;1,9459 30,700001;1,9397 42,950001;1,9335 29,32;1,9273 15,12;1,9009 1;1,8147 0,5;1,8076 0,66;1,7995 0,81;1,7914 0,9;1,7808 0,97;1,7744 1,17;1,7681 1,19;1,762 0,88;1,6227 0,47;1,6141 0,5;1,5986 1,2;1,5911 1,51;1,5827 1,08;1,5752 1,22;1,568 1,78;1,5595 1,33;1,5447 0,94;1,5373 0,8;1,4993 0,46;1,4911 0,59;1,4755 0,75;1,4672 1,33;1,4586 0,84;1,4513 0,59;1,4428 1,21;1,4348 1,21;1,4184 0,47;1,4096 0,77;1,4047 0,74;1,394 0,37;1,3786 0,54;1,3721 0,54;1,3626 0,3;1,3558 0,32;1,348 0,41;1,2217 1,61;1,2039 3,16;1,1861 1,56;-0,0001 0,85 |
| I-70 | [CD₃CN] 7,6784 1,67;7,66 2,13;7,6569 2,07;7,6385 2;7,3686 3,59;7,3499 3,31;7,2717 0,48;7,2683 0,5;7,2516 1,18;7,235 1,48;7,2313 1,3;7,1948 2,32;7,1804 1,76;7,1784 1,8;7,1632 0,64;6,9765 2,48;6,9556 2,37;6,3985 3,13;6,2034 0,85;6,1923 1,77;6,1811 0,92;5,4463 9,51;5,0837 9,83;3,7019 1,17;3,6919 1,98;3,6845 1,76;3,6786 1,95;3,6503 2,05;3,635 2,73;3,6202 2,54;3,6085 4,54;3,5962 2,5;2,9525 0,32;2,9388 0,61;2,926 0,35;2,9097 0,63;2,8964 1,08;2,8841 0,51;2,8375 0,6;2,8156 0,81;2,8017 0,63;2,7733 0,41;2,2654 0,56;2,231 15;2,2297 14,62;2,2211 1,69;2,1709 322,059998;2,1204 0,91;2,1131 1,2;2,1069 1,02;2,1005 2,54;2,0888 3,32;2,0817 1,8;2,0785 1,89;2,0655 0,71;2,0629 0,69;2,0508 0,76;2,0422 0,68;2,0292 0,77;2,0222 0,4;2,0164 0,43;2,0083 0,46;1,9951 0,35;1,9718 1,2;1,9636 1,26;1,9518 18,07;1,9457 33,990002;1,9395 48,080002;1,9333 32,689999;1,9271 16,52;1,9075 0,67;1,9041 0,56;1,8943 0,92;1,882 0,74;1,8741 0,48;1,8686 0,51;1,8586 0,37;1,8518 0,36;1,7679 0,37;1,4369 0,33;1,2038 0,37;-0,0001 0,87 |
| I-71 | [CD₃CN] 7,6955 2,14;7,6833 2,47;7,6813 2,44;7,6691 2,45;7,3982 2,88;7,3975 2,95;7,3861 2,73;7,3853 2,63;6,9844 2,79;6,9707 2,72;6,4045 3,44;5,8951 0,39;5,884 0,8;5,8781 0,43;5,8729 0,41;5,8669 1,22;5,8556 1,23;5,8496 0,46;5,8444 0,45;5,8385 0,9;5,8273 0,44;5,4489 2,26;5,0958 11,15;5,0614 0,58;5,0588 1,36;5,0552 1,48;5,0526 0,7;5,0329 0,56;5,0303 1,28;5,0267 1,38;5,0241 0,65;4,9762 0,69;4,9742 1,32;4,9724 1,21;4,9706 1,27;4,9686 0,66;4,9592 0,63;4,9572 1,29;4,9554 1,17;4,9536 1,22;4,9516 0,63;4,2992 |
| | 3,51;4,2883 7,4;4,2774 3,61;3,7132 1,31;3,7052 2,01;3,7013 1,52;3,6959 2,09;3,666 0,97;3,6591 1,98;3,6539 1,57;3,6486 2,14;3,6281 2,38;3,622 3,75;3,6112 3,21;3,6048 1,19;2,2363 15,97;2,2353 16;2,1791 99,779999;2,154 0,95;2,1518 1,19;2,1496 0,83;2,1396 2,19;2,1275 2,2;2,118 0,7;2,1159 1,05;2,1136 0,67;1,966 0,38;1,9577 0,34;1,9537 0,39;1,9499 10,85;1,9458 21,5;1,9417 32,009998;1,9376 21,870001;1,9335 11,18;1,7788 0,67;1,7678 1,84;1,7633 0,49;1,7559 1,86;1,7537 1,58;1,7424 2,07;1,7315 0,88;1,5659 0,78;1,553 1,93;1,5447 0,88;1,5407 2,79;1,5314 0,65;1,5279 1,62;1,5156 0,62;0 0,73 |
| I-72 | [CD₃CN] 7,7112 1,74;7,6928 2,23;7,6898 2,1;7,6714 2,11;7,6131 1,12;7,6059 0,89;7,6021 1,17;7,5957 0,82;7,5899 1,34;7,489 1,2;7,4833 0,75;7,4764 1,23;7,4727 1,07;7,4658 1,78;7,4564 0,33;7,4387 2,83;7,4209 2,48;7,3977 0,56;7,3852 2,64;7,3831 2,52;7,3791 1,8;7,3724 3,21;7,3661 1,46;7,362 1,91;7,3599 1,66;7,3474 0,3;7,0106 2,68;6,9894 2,53;6,4 3,26;5,4461 1,7;5,4369 10,67;5,0903 10,48;4,0865 0,48;4,0686 1,44;4,0508 1,47;4,033 0,49;3,728 1,26;3,7167 2,08;3,7097 1,74;3,703 2,15;3,6492 3,21;3,6332 5,13;3,6101 1,97;2,2637 0,32;2,2343 15;2,2333 14,79;2,1631 160,529999;2,1133 0,36;2,1069 0,37;1,9717 6,81;1,9635 0,87;1,9518 10,14;1,9456 19,1;1,9395 27,18;1,9333 18,709999;1,9271 9,63;1,2216 1,76;1,2038 3,44;1,186 1,71;-0,0001 1,47 |
| I-73 | [CD₃CN] 7,7 1,54;7,6815 2,03;7,6787 1,84;7,6602 1,72;7,5755 0,71;7,5567 1,32;7,5373 0,69;7,4388 0,45;7,4341 0,48;7,4125 3,14;7,3997 1,21;7,3943 2,68;7,386 0,48;7,3816 0,36;7,2517 1,08;7,233 1,73;7,2138 0,77;7,1936 1,01;7,1685 1,12;7,147 0,75;6,9995 2,65;6,9781 2,38;6,3985 3,23;5,4458 2,26;5,4052 7,6;5,0863 9,83;4,0862 0,4;4,0684 1,05;4,0506 1,08;4,0327 0,36;3,7127 1,41;3,702 2,25;3,6949 1,95;3,6886 2,24;3,6567 2,14;3,6417 3,12;3,6177 4,94;3,604 2,75;2,2644 0,5;2,2316 15;2,2093 0,63;2,144 198,509995;2,1063 0,78;2,1002 0,63;1,9713 5,43;1,9512 22,66;1,9451 42,16;1,9389 59,060001;1,9327 40,669998;1,9265 20,790001;1,2712 0,3;1,2215 1,25;1,2037 2,41;1,1859 1,16;-0,0001 34,84;-0,0083 1,88 |
| I-74 | [CD₃CN] 7,6805 1,75;7,6621 2,19;7,6591 2,11;7,6407 2,07;7,4939 0,37;7,4774 0,78;7,4727 0,77;7,4608 0,54;7,4563 1,55;7,4517 0,58;7,4397 0,78;7,4351 0,9;7,4186 0,45;7,3661 2,75;7,3484 2,44;7,0796 0,39;7,0701 2,15;7,0621 0,45;7,0584 0,5;7,05 3,29;7,0412 0,53;7,0375 0,43;7,0296 1,91;7,02 0,35;6,984 2,67;6,9626 2,53;6,3967 3,24;5,446 4,21;5,4294 7,29;5,0805 10,39;4,0686 0,78;4,0508 0,8;3,6929 1,24;3,6821 2,09;3,6738 1,83;3,6686 1,89;3,653 1,33;3,6435 2,17;3,6282 2,73;3,6144 2,32;3,608 2,37;3,599 4,06;3,5904 2,73;3,5816 1,98;3,572 0,99;2,2649 0,41;2,2291 15;2,2281 14,7;2,1566 161,330002;1,9716 3,86;1,9634 0,89;1,9517 10,4; 1,9456 19,41;1,9394 27,459999;1,9332 18,780001;1,927 9,5;1,2216 0,95;1,2038 1,86;1,1859 0,92;-0,0001 1,46 |
| I-75 | [CD₃CN] 8,0035 1,32;8,0012 1,66;7,989 1,59;7,9809 1,4;7,9677 1,48;7,9659 1,32;7,8866 1,87;7,8728 2,04;7,8303 1,73;7,8181 2,19;7,816 2;7,8038 2,23;7,7093 3,18;7,6973 2,58;7,6054 0,55;7,603 0,72;7,5921 3,16;7,5798 4,41;7,5784 4,22;7,5686 0,77;7,5661 3,41;7,5551 0,69;7,5528 0,58;7,4331 2,21;7,4317 2,34;7,4207 1,9;7,4192 1,9;7,1308 2,82;7,1166 2,73;6,4034 3,45;5,1025 11,05;4,0768 1,28;4,065 3,89;4,0531 3,93;4,0413 1,32;3,8026 1,48;3,7944 2,04;3,7911 1,56;3,7852 1,96;3,6972 14,32;3,6769 0,45;3,6695 2,06;3,6637 1,56;3,6604 2,08;3,652 1,5;2,2562 0,62;2,2376 16;2,2298 0,95;2,1889 47,09;1,973 17,540001;1,9659 0,34;1,9575 0,56;1,9533 0,58;1,9496 12,03;1,9455 23,690001;1,9414 35;1,9373 23,969999;1,9332 12,15;1,2158 4,61;1,204 9,24;1,1921 4,59;0 1,57 |
| I-76 | [CD₃CN] 7,9981 2,35;7,9833 2,45;7,9653 1,42;7,9521 1,49;7,914 1,48;7,9008 1,59;7,7988 1,99;7,7867 2,46;7,7845 2,24;7,7723 2,47;7,7417 2,3;7,738 2,35;7,6415 3,02;7,641 3,07;7,6295 2,66;7,5836 0,63;7,5812 0,75;7,5721 1,58;7,5698 1,49;7,5593 1,77;7,5567 2,65;7,554 1,92;7,5454 0,79;7,5436 1,51;7,5413 1,54;7,5322 0,72;7,5298 0,59;7,4184 2,02;7,4145 1,97;7,4036 1,92;7,3997 1,88;7,0946 2,74;7,0941 2,74;7,0803 2,71;6,4044 3,42;5,4485 2,77;5,1029 10,93;4,0768 0,79;4,065 2,36;4,0531 2,39;4,0413 0,8;3,7776 1,39;3,7693 1,97;3,766 1,53;3,7602 1,87;3,6962 0,65;3,6826 4,92;3,6805 4,97;3,6664 0,86;3,6599 2,22;3,6539 1,58;3,6507 2,05;3,6424 1,4;2,2387 16;2,2377 15,54;2,231 0,53;2,1877 205,589996;2,1704 0,39;1,973 11,04;1,9575 0,34;1,9532 0,52;1,9495 10,74;1,9454 21,02;1,9413 31,040001;1,9372 20,91;1,9331 10,45;1,2157 3,09;1,2038 6,17;1,192 3,04;0 0,66 |
| I-77 | [DMSO-d₆] 7,9572 0,34;7,9403 0,32;7,9197 0,32;7,8143 0,32;7,767 6,7;7,7477 15;7,7283 9,35;7,6104 8,82;7,591 6,66;7,568 0,49;7,5463 0,49;7,5265 0,47;7,4921 0,59;7,478 0,5;7,471 0,71;7,4443 2,48;7,4348 2,73;7,43 3,07;7,4207 3,58;7,4071 2,68;7,3922 2,79;7,3832 3,05;7,3448 6,76;7,3305 2,72;7,3239 4,21;7,3104 2,97;7,2953 3,22;7,2557 6,32;7,2418 6,09;7,2319 10,69;7,2226 6,55;7,2109 14,24;7,197 10,65;7,1834 7,76;7,1774 10,01;7,1483 0,39;7,0775 5,68;7,0475 13,97;7,0274 0,62;6,9337 12,78;6,9117 7;5,7474 2,18;5,4531 |
| | 0,67;5,4217 1,47;5,4028 0,72;5,3776 5,66;5,3568 11,6;5,3101 1,55;4,2714 2,43;4,0392 0,43;4,0215 0,36;4,0018 0,37;3,8746 2,05;3,8529 2,32;3,5195 0,32;3,4854 0,32;3,4652 0,79;3,4219 0,6;3,3947 0,91;3,3118 313,070007;3,2928 100,93;3,2029 0,61;3,1822 0,73;3,1258 0,56;3,102 2,36;3,0678 4,03;3,0371 2,97;2,9435 0,49;2,8499 0,88;2,8114 0,44;2,7847 0,41;2,7524 0,4;2,7307 2,09;2,7212 1,64;2,7103 2,55;2,7022 4,24;2,6928 2,55;2,6733 3,7;2,6688 3,33;2,664 2,68;2,6224 2,24;2,5901 4,18;2,564 2,67;2,5388 11,41;2,5219 7,7;2,5084 118,080002;2,5041 222,740005;2,4996 292,51001;2,4953 202,589996;2,4909 97,57;2,4468 1,15;2,418 0,79;2,3793 0,58;2,3354 0,99;2,3312 1,52;2,3266 2,01;2,322 1,62;2,2907 0,42;2,2371 0,35;2,0843 0,31;2,0693 2,93;2,0493 1,05;2,0081 0,38;1,9866 1,3;1,9581 0,45;1,9067 0,33;1,6641 0,34;1,6408 0,35;1,6173 0,33;1,5282 1,5;1,5025 3;1,4756 2,17;1,4394 1,73;1,4075 3,15;1,3983 3,3;1,3749 2,29;1,3345 1,61;1,3111 1,44;1,292 1,15;1,271 1,08;1,2598 0,96;1,2366 1,88;1,2072 1,34;1,1992 1,36;1,1748 1,41;1,1624 1,09;1,1381 1,41;1,1308 1,34;1,1109 1,72;1,0983 1,32;1,0703 1,21;1,0564 0,88;1,0494 0,78;1,0263 1,28;1,0172 1,4;0,9927 1,23;0,9853 1,31;0,9628 0,64;0,9534 0,49;0,8904 0,44;0,008 1,42;-0,0001 32,599998;-0,0085 1,33;-2,2704 0,34 |
| I-78 | [DMSO-d₆] 7,8679 1,41;7,8617 0,69;7,8486 2,98;7,8423 1,37;7,8292 1,72;7,8229 0,75;7,3891 2,05;7,3691 2,66;7,3527 2,53;7,3477 2,01;7,3353 2,17;7,3287 1,13;7,3121 0,54;7,2984 1,12;7,1786 1,06;7,1649 2,58;7,1538 1,33;7,0451 0,53;7,0315 1,32;7,0242 1,38;7,0176 2,79;6,8979 3,78;6,8817 1,39;5,747 6,02;5,458 0,31;5,4158 0,91;5,3834 2,21;5,3543 3;5,3119 0,74;4,4274 0,88;4,3946 0,93;4,3199 0,4;4,057 0,45;4,0392 1,3;4,0214 1,66;4,0038 1,15;3,9766 0,93;3,3892 0,5;3,3808 0,45;3,3602 1,05;3,3028 353,709991;3,2809 3,34;3,2638 1,22;3,2263 1,34;3,1983 0,73;3,0598 0,6;3,0513 0,49;3,0305 1,14;3,0115 0,48;3,0018 0,64;2,8726 15;2,7953 0,71;2,7618 1,42;2,7508 6,34;2,7369 0,82;2,6736 0,46;2,6693 0,53;2,6645 0,4;2,5391 1,21;2,5221 2,27;2,5088 33,290001;2,5045 61,48;2,5 79,449997;2,4957 54,490002;2,4914 26,040001;2,3311 0,43;2,3266 0,56;2,3222 0,44;2,0694 0,32;1,9867 5,57;1,9339 0,72;1,9076 1,78;1,9028 1,79;1,8673 1,03;1,8245 0,93;1,7953 1,35;1,7638 0,9;1,754 0,83;1,7452 0,81;1,7088 2,31;1,6801 2,37;1,6539 1,3;1,614 0,95;1,6048 1,2;1,5737 1,79;1,5424 1,59;1,5039 1,16;1,4673 0,95;1,3768 0,56;1,3449 0,51;1,1927 1,56;1,1749 3,17;1,1571 1,61;1,1404 0,33;1,0504 0,42;1,0222 0,64;0,9923 1,22;0,9632 1,12;0,9304 0,82;0,8906 0,32;-0,0001 4,17 |
| I-79 | ¹H NMR (DMSO-d₆, 400 MHz): dₚₚₘ : [7.90 (t), 7.84 (t)] (1H), [7.67 (d), 7.58 (d)] (1H), 7.55-7.30 (m, 4H), 7.20-7.15 (m, 1H), 7.03 (t, 1H), 6.94-6.86 (m, 1H), [5.40 (q), 5.34 (s)] (2H), 4.80-4.60 (m, 2H), [4.42 (d), 4.29 (d)] (1H), [4.01 (d), 3.88 (d)] (1H), 3.40-2.40 (m, 3H), [3.02 (s), 2.97 (s)] (3H), 2.01-1.20 (m, 4H) |
| I-80 | [DMSO-d₆] 8,5633 0,86;8,5531 0,79;8,4858 1,25;8,4759 1,21;7,9137 0,74;7,8942 1,57;7,8747 0,92;7,8363 1,72;7,8267 0,4;7,8169 3,43;7,7976 1,95;7,7884 0,88;7,7841 0,78;7,7695 1,47;7,7649 1,42;7,7504 0,84;7,7462 0,81;7,4873 1,41;7,477 2,07;7,4692 1,3;7,4594 1,92;7,4294 1,18;7,4114 1,09;7,3706 1,07;7,3511 1,14;7,3407 1,96;7,3191 2,95;7,3096 2,81;7,2895 1,58;7,256 0,91;7,2455 1;7,2379 0,99;7,2271 0,89;7,1854 3,4;7,1773 1,54;7,1604 0,71;7,0498 3,14;7,0434 0,98;7,0244 1,49;6,9189 2,47;6,9005 1,48;6,8882 0,76;5,7463 0,67;5,4629 0,38;5,4196 1,05;5,4013 0,51;5,358 2,96;5,3251 1,98;5,2813 0,42;4,7705 3,38;4,69 5,81;4,438 0,42;4,4091 0,39;4,3041 0,59;4,2727 0,6;4,0393 0,72;4,0215 0,9;4,0036 0,38;3,9856 0,48;3,8907 0,62;3,8526 0,65;3,464 0,32;3,4549 0,36;3,4281 0,44;3,3941 0,7;3,3109 1092,949951;3,2872 78,389999;3,2445 1,09;3,2165 0,52;3,2019 0,48;3,195 0,33;3,1339 0,51;3,1005 0,95;3,0705 0,77;3,0621 0,86;3,0534 0,56;3,0277 15;3,0203 10,04;2,8908 0,5;2,8585 0,79;2,8306 0,5;2,8195 0,46;2,7854 0,51;2,778 0,54;2,7495 0,35;2,6742 0,96;2,6695 1,38;2,665 1,2;2,6266 0,82;2,5983 0,58;2,5397 2,17;2,5225 4,87;2,5092 73,919998;2,5049 138,350006;2,5005 180,330002;2,4961 124,330002;2,4918 59,540001;2,4339 0,46;2,3319 0,97;2,3268 1,22;2,3226 0,94;2,0691 1,01;2,0497 0,44;1,9867 2,92;1,9365 0,73;1,9066 0,5;1,8233 0,42;1,7952 0,35;1,7842 0,31;1,7418 0,55;1,7066 0,69;1,6699 0,66;1,6328 0,75;1,5991 0,38;1,5099 0,53;1,4888 0,45;1,4816 0,47;1,3981 0,45;1,2923 0,33;1,2591 0,58;1,2364 0,84;1,1926 0,79;1,175 1,54;1,1571 0,84;0,0078 0,91;-0,0001 21,77;-0,0086 0,99 |
| I-81 | [DMSO-d₆] 7,9305 1,18;7,9111 2,47;7,8918 1,46;7,5805 2,36;7,5614 2,19;7,4442 1,78;7,425 1,77;7,3909 0,36;7,2964 1,82;7,1629 4,1;7,1518 2,02;7,0295 2,79;7,0158 5,64;6,998 0,99;6,9195 2,66;6,9164 2,85;6,8951 5,41;6,8799 2,4;6,8419 0,4;6,7688 0,39;6,7646 0,4;6,7512 0,4;6,7467 0,4;5,747 10,97;5,4114 0,47;5,3693 2,44;5,3444 3,18;5,303 0,59;4,3064 2,99;4,0568 1,14;4,039 3,42;4,0212 3,45;4,0034 1,21;3,9336 0,76;3,8847 2,16;3,3006 528,530029;3,2769 15,56;3,2312 0,99;3,1978 1,22;3,1689 0,72;3,0262 0,59;3,0007 0,82;2,7841 0,62;2,754 |
| | 1,08;2,7261 0,62;2,6774 0,38;2,6732 0,69;2,6687 0,92;2,6641 0,67;2,5387 2,37;2,5219 3,35;2,5085 54,509998;2,5041 101,230003;2,4996 130,699997;2,4953 89,199997;2,491 42,59;2,4104 0,3;2,3356 0,46;2,3309 0,76;2,3263 1;2,3217 0,76;2,0692 1,05;2,0493 0,39;1,9866 15;1,907 0,43;1,8501 0,93;1,8234 0,82;1,8109 0,73;1,8072 0,72;1,764 0,39;1,7089 0,48;1,6584 0,31;1,5016 0,5;1,4822 0,49;1,3984 0,66;1,1925 4,13;1,1748 8,13;1,157 4,02;0,0078 0,44;-0,0001 10,13;-0,0084 0,41 |
| I-82 | [DMSO-d₆] 8,922 1,21;8,9026 1,23;7,9555 1,52;7,9362 3,68;7,9169 2,45;7,8435 3,09;7,8262 2,18;7,5688 1,83;7,565 2,08;7,5587 2,81;7,55 2,28;7,5462 2,34;7,5397 2,53;7,4464 1,7;7,427 2,18;7,3572 0,69;7,3384 1,54;7,3222 2,43;7,2964 1,39;7,2776 1,65;7,2588 0,63;7,1889 3,41;7,1651 1,66;7,0555 1,7;7,0291 3,78;6,9105 3,49;6,8932 1,87;5,7468 0,56;5,5048 0,32;5,487 1,22;5,4683 2,1;5,4496 1,3;5,4303 1,98;5,3808 2,59;5,3382 0,75;4,4932 0,79;4,4592 0,82;4,0574 1,79;4,0396 3,75;4,0218 3,98;4,004 1,19;3,3102 325,160004;3,2677 1,49;3,2374 0,7;3,15 0,55;3,1293 0,72;3,1208 1,13;3,1127 0,7;3,1002 0,46;3,0911 0,61;2,8347 0,55;2,805 1,01;2,775 0,56;2,6701 0,4;2,54 0,89;2,5096 22,719999;2,5053 41,68;2,5009 53,59;2,4966 36,98;2,3275 0,38;2,0696 0,61;2,0501 0,78;2,019 1,34;1,9872 15;1,9 0,32;1,8698 0,69;1,8386 0,64;1,779 0,32;1,7474 0,69;1,7158 0,63;1,5468 7,04;1,5293 7,01;1,193 3,91;1,1752 7,84;1,1574 3,81;-0,0001 2,21 |
| I-83 | [DMSO-d₆] 8,1932 4,31;8,1784 4,42;7,9457 4,71;7,9329 11,59;7,9201 7,93;7,8799 8,52;7,8783 8,86;7,8671 6,02;7,8655 5,64;7,8462 0,35;7,8446 0,34;7,5272 6,54;7,5258 6,6;7,5144 6,45;7,5129 6,09;7,2847 3,28;7,1957 7,29;7,1348 3,81;7,1069 3,65;7,0442 9,52;6,9536 4,54;6,9234 8,96;5,7641 1,45;5,4623 1,83;5,4542 1,73;5,4336 3,49;5,4257 3,46;5,3771 6,81;5,3486 3,19;5,3195 0,39;4,4643 2,06;4,4425 2,12;4,0459 1,45;4,0341 5,85;4,0222 5,74;4,0104 5,62;4,0006 2,45;3,9862 1,08;3,3995 0,36;3,386 0,62;3,372 1,65;3,3511 918,869995;3,3276 2,16;3,3181 0,66;3,2633 1,41;3,2594 1,62;3,2382 2,75;3,2197 1,58;3,2158 1,32;3,1005 0,75;3,0948 1,38;3,0891 0,9;3,0808 1,66;3,0749 2,91;3,0691 1,66;3,061 0,97;3,0551 1,52;3,0492 0,81;3,0369 1,1;2,891 0,33;2,8487 0,86;2,795 1,37;2,7739 2,7;2,7529 1,4;2,6216 0,61;2,6188 1,3;2,6157 1,77;2,6127 1,28;2,6097 0,61;2,5432 7,01;2,5248 3,5;2,5217 4,53;2,5186 4,94;2,5097 100,57;2,5067 216,080002;2,5037 295,079987;2,5007 212,289993;2,4977 95,790001;2,3936 0,62;2,3906 1,29;2,3876 1,75;2,3846 1,26;2,3816 0,57;2,0782 3,04;2,0532 0,43;1,9956 1,91;1,9907 16;1,9752 2,1;1,9508 1,64;1,9292 1,86;1,8402 0,65;1,8358 0,74;1,8196 1,65;1,8151 1,7;1,7991 1,51;1,7942 1,46;1,778 0,56;1,7728 0,51;1,7089 0,7;1,7046 0,62;1,689 1,65;1,6728 1,28;1,6679 1,55;1,6632 1,21;1,6524 0,52;1,6483 0,58;1,6425 0,45;1,6004 0,79;1,5961 0,86;1,5865 1,38;1,5773 1,73;1,5736 1,68;1,5643 1,56;1,5534 0,75;1,5197 1,74;1,5091 2,23;1,4967 1,8;1,4859 1,23;1,4743 0,58;1,3969 0,69;1,2682 11,74;1,2634 10,77;1,2345 1,49;1,1903 12,62;1,1866 14,36;1,1793 13,43;1,1763 13,3;1,1746 14,18;1,1684 1,68;1,1627 4,46;1,1577 0,79;1,0669 0,32;0,8495 6,03;0,8444 10,99;0,8385 10,87;0,8335 5,4;0,8275 3,61;0,0054 1,17;0 35,220001;-0,0056 1,1 |
| I-84 | [DMSO-d₆] 16,9338 0,52;16,757601 0,52;16,381001 0,58;8,7368 2,49;8,7169 2,45;7,9996 0,58;7,9852 1,31;7,9662 6,89;7,9589 7,84;7,9514 15;7,9397 1,52;7,5527 0,91;7,5405 3,9;7,5334 3,85;7,5265 3,77;7,519 3,55;7,339 0,54;7,3012 3,27;7,2845 5,67;7,2628 3,25;7,2549 1,87;7,2436 2,85;7,2333 3,63;7,2222 2,44;7,2146 2,75;7,197 11,97;7,165 0,61;7,1448 2,98;7,1298 5,86;7,0083 5,91;6,9965 3,13;6,8845 7,62;6,872 3,15;5,7468 4,31;5,6044 1,09;5,5817 3,27;5,5604 3,43;5,5378 1,15;5,4167 1,57;5,374 5,71;5,3339 5,39;5,3077 0,68;5,2902 1,49;4,4306 2,09;4,3992 2,13;4,0568 1,23;4,0391 3,38;4,0212 3,57;4,0039 2,7;3,9692 2,02;3,9647 2,13;3,6742 0,55;3,5839 0,57;3,5377 0,71;3,5252 0,57;3,4963 0,71;3,4731 0,76;3,408 1,27;3,3858 1,74;3,3033 2034,709961;3,2368 2,56;3,2 2,98;3,1801 1,23;3,1691 1,75;3,1566 0,92;3,1415 0,61;3,1358 0,6;3,0815 1,09;3,0751 1,45;3,044 3,91;3,0228 2,41;3,0121 3,36;3,0057 3;2,99 2,22;2,9831 2,25;2,9201 1,39;2,8998 2,86;2,8789 2,26;2,8603 1,81;2,841 1,02;2,7681 1,43;2,7363 2,52;2,7081 1,48;2,6682 2,81;2,6489 0,59;2,6421 0,56;2,5389 8,32;2,5 426,429993;2,4403 1,93;2,4054 0,8;2,3574 0,63;2,3261 2,84;2,1474 1,19;2,1238 2,56;2,1169 1,35;2,1023 2,55;2,0931 2,36;2,0847 1,75;2,0696 3,36;2,0599 0,67;2,0493 1,98;1,9867 14,85;1,9427 3,05;1,9078 2,67;1,8239 1,36;1,8007 1,86;1,7792 1,3;1,7679 1,1;1,7329 0,67;1,6803 1,6;1,6675 1,34;1,6485 1,39;1,6364 1,21;1,6154 0,77;1,3998 0,66;1,2917 0,76;1,2362 0,89;1,1926 3,57;1,1749 7,03;1,157 3,59;1,0456 1,33;1,0292 1,25;-0,0001 77,949997;-3,6563 0,61 |
| I-85 | [DMSO-d₆] 8,6033 1,62;8,5882 2,85;8,5719 1,42;7,9373 2,65;7,9181 6,81;7,8991 5,33;7,8714 5,66;7,8687 6,18;7,8524 3,18;7,8497 2,81;7,5104 4,4;7,5078 4,39;7,4913 4,08;7,4889 3,89;7,317 2,69;7,1835 6,06;7,1651 2,93;7,05 3,05;7,029 6,76;6,9076 6,07;6,8931 |
| | 3,25;5,4462 1,11;5,4262 0,41;5,4194 0,4;5,4029 5,09;5,373 5,08;5,3316 1,03;4,4731 1,43;4,4701 1,46;4,4378 1,43;4,0569 0,47;4,0393 2,19;4,0216 1,78;4,0036 1,73;3,4495 0,35;3,4334 0,37;3,3753 1,05;3,312 134,529999;3,2758 4,55;3,2386 2,17;3,2077 1,18;3,0876 0,99;3,0778 0,78;3,0673 1,34;3,0585 2,07;3,0494 1,37;3,0373 1,27;3,0288 1,16;3,0195 0,71;2,8503 0,45;2,8054 0,92;2,7727 1,84;2,7441 1,04;2,6736 0,9;2,6691 1,1;2,5659 0,3;2,539 5,95;2,5085 65,870003;2,5043 125,209999;2,4999 165,910004;2,4956 118,529999;2,4118 0,4;2,3312 0,9;2,3264 1,15;2,3226 0,85;2,0697 0,69;2,0495 0,57;1,9868 5,46;1,9625 2,17;1,9173 1,68;1,908 1,44;1,8776 0,6;1,868 0,67;1,8485 1,18;1,8379 1,31;1,8143 1,19;1,8044 1,14;1,7862 0,88;1,7738 0,96;1,7534 1,25;1,7442 1,36;1,7217 1,15;1,713 1,17;1,6906 0,47;1,6805 0,44;1,5576 2,36;1,54 3,49;1,5229 2,52;1,3985 0,33;1,2986 10,41;1,2922 10,14;1,2702 7,42;1,2628 9,95;1,1927 1,53;1,175 2,8;1,1572 1,59;1,1109 0,32;1,1014 0,32;1,0707 0,45;0,872 4,81;0,8552 15;0,8376 5,7;0,0082 0,64;-0,0001 18,17;-0,0081 0,91 |
| I-86 | [DMSO-d₆] 9,2669 0,94;9,2516 1,95;9,236 0,96;7,9744 1,54;7,9552 4,14;7,9361 3,2;7,9058 3,4;7,9033 3,68;7,8867 2,02;7,8841 1,75;7,625 4,06;7,6198 4,23;7,5646 2,61;7,5624 2,65;7,5457 2,52;7,5432 2,33;7,4236 1,89;7,4183 1,79;7,4027 2,85;7,3973 2,74;7,3234 4;7,3029 4,06;7,1702 3,79;7,1554 1,88;7,0368 1,87;7,0194 4,18;6,9025 3,66;6,8836 2,03;5,7463 1,17;5,439 0,61;5,3958 3,08;5,3673 3,05;5,3251 0,64;4,5811 4,29;4,5654 4,25;4,4765 0,87;4,4463 0,87;4,0572 1,26;4,0395 4,01;4,0217 3,59;4,0039 1,9;3,3138 479,829987;3,2902 10,54;3,2448 1,3;3,2154 0,73;3,1145 0,62;3,1053 0,44;3,0944 0,76;3,0847 1,22;3,0765 0,74;3,0641 0,51;3,0554 0,66;3,047 0,39;3,038 0,4;2,8505 0,31;2,8119 0,58;2,78 1,05;2,754 0,61;2,6746 0,32;2,6701 0,41;2,6654 0,32;2,5401 1;2,5098 25,139999;2,5055 46,779999;2,501 60,709999;2,4967 41,98;2,4925 20,18;2,3321 0,32;2,3276 0,42;2,0697 0,31;2,0496 0,36;2,0305 0,73;1,9871 15;1,9499 0,99;1,8918 0,35;1,8667 0,71;1,8595 0,77;1,8284 0,9;1,7989 0,91;1,7899 0,85;1,7688 0,66;1,7591 0,62;1,3977 0,38;1,193 3,73;1,1752 7,31;1,1574 3,64;0,0078 0,33;-0,0001 7,08 |
| I-87 | [DMSO-d₆] 9,2143 1,96;9,1984 4,04;9,1827 1,94;7,9639 3,2;7,9448 8,54;7,9257 6,67;7,8981 7,31;7,8958 7,34;7,8791 4,05;7,8767 3,24;7,5483 5,56;7,5461 5,28;7,5294 5,29;7,527 4,67;7,4201 1,6;7,3984 3,44;7,3816 3,46;7,377 2,12;7,3598 1,73;7,3097 3,44;7,2527 2,06;7,2462 2,16;7,2287 2,68;7,2262 2,78;7,2225 2,88;7,2029 2,09;7,1965 2,19;7,1763 7,96;7,1663 3,85;7,1542 0,42;7,0785 1,78;7,072 1,74;7,0571 3,3;7,0505 3,29;7,0431 4,23;7,0302 9,91;7,021 0,69;6,9109 7,46;6,8945 4,37;5,755 16;5,4472 1,68;5,4046 6,44;5,3701 6,37;5,3274 1,71;5,3134 0,87;4,5477 7,66;4,532 7,63;4,4768 1,77;4,4446 1,83;4,0562 0,51;4,0383 2,45;4,0207 1,44;4,0028 2,06;3,4316 1,38;3,3809 1,73;3,3312 1209,810059;3,3087 3,99;3,2648 1,64;3,2328 2,6;3,205 1,36;3,1075 0,66;3,0987 1,17;3,09 0,82;3,078 1,46;3,0692 2,45;3,0604 1,45;3,0486 0,95;3,0376 2,84;2,8497 1,58;2,799 1,18;2,7726 2,2;2,767 2,17;2,741 1,31;2,6759 0,85;2,6714 1,13;2,6668 0,81;2,6626 0,37;2,5414 4,41;2,5245 4,18;2,5196 6,03;2,5112 60,82;2,5068 124,940002;2,5024 168,600006;2,4979 120,279999;2,4935 55,740002;2,3336 0,86;2,3292 1,16;2,3249 0,82;2,3202 0,41;2,0735 0,48;2,0105 1,4;1,9888 5,03;1,9738 2,49;1,9301 2,03;1,8893 0,67;1,88 0,77;1,8579 1,49;1,8488 1,59;1,826 1,59;1,8184 1,87;1,7882 1,88;1,7783 1,69;1,7569 1,42;1,7472 1,37;1,7256 0,52;1,7153 0,42;1,2357 0,42;1,1926 1,01;1,1748 1,96;1,1571 0,99;-0,0001 6,78 |
| I-88 | [DMSO-d₆] 16,7486 0,41;14,2673 0,39;13,1161 0,41;12,3798 0,41;9,2083 2,89;9,1974 1,68;9,0798 0,41;7,9992 0,43;7,9762 1,62;7,957 3,65;7,938 2,94;7,9119 4,78;7,8923 2,29;7,6743 0,39;7,6408 0,46;7,5648 3,59;7,5451 3,4;7,5213 0,52;7,4952 0,63;7,4736 2,84;7,4578 3,27;7,3887 0,41;7,3602 0,62;7,3104 15;7,1671 3,83;7,1168 0,43;7,0337 2,22;7,0173 3,81;6,9027 5,87;6,8815 1,95;5,7468 2,68;5,4377 1,27;5,3958 4,41;5,3675 4,46;5,3262 1,14;5,3049 0,42;4,6157 6,55;4,6013 6,14;4,4771 2,05;4,4475 2,14;4,0398 3,12;4,0207 2,54;4,0084 2,34;3,7329 0,46;3,4608 0,39;3,4394 0,46;3,3098 371,700012;3,3049 420,179993;3,3006 555,710022;3,2577 3,58;3,217 1,89;3,1816 0,55;3,1661 0,62;3,1547 0,52;3,1473 0,57;3,1155 1,5;3,0873 2,27;3,0668 1,42;2,9673 0,39;2,9155 0,43;2,8484 0,6;2,8194 1,4;2,7858 2,36;2,7534 1,36;2,7425 0,48;2,6736 1,69;2,6294 0,42;2,609 0,43;2,5588 1,09;2,5389 4,03;2,5041 267,200012;2,436 0,98;2,403 0,72;2,3803 0,53;2,3606 0,53;2,3586 0,55;2,3318 1,76;2,3043 0,45;2,069 1,1;2,0356 1,82;1,9904 6,48;1,9866 7,58;1,9548 2,5;1,9071 0,99;1,868 1,89;1,8371 1,89;1,8195 1,4;1,7941 2;1,7889 1,66;1,7644 1,49;1,7196 0,63;1,6829 0,4;1,292 0,54;1,2361 0,64;1,1927 1,58;1,1752 2,89;1,1572 1,64;1,1353 0,39;1,0391 0,4;0,8991 0,52;0,873 0,4;0,0089 20,620001;0,0036 23,48;-0,0001 31,66;-0,1512 0,41;-3,1868 0,44 |
| I-89 | ¹H NMR (DMSO-d₆, 400 MHz): dₚₚₘ : 8.89 (t, 1H), 7.93 (t, 1H), 7.87 (t, 1H), 7.51 (dd, 1H), 7.45-7.32 (m, 1H), 7.17 (t, 1H), 7.08 (t, 2H), 7.02 (t, 1H), 6.90 (s, 1H), 5.38 (q, 2H), 4.62 (d, 2H), 4.44 (d, 1H), 4.00 (d, 1H), 3.30-3.16 (m, 1H), 3.10-3.00 (m, 1H), 2.77 (t, 1H), 2.05-1.89 (m, 2H), |
| | 1.87-1.62 (m, 2H) |
| I-90 | [DMSO-d₆] 16,369101 0,34;9,3029 2,5;9,2879 4,68;9,2736 2,42;8,5306 6,08;8,52 5,96;7,9723 4,13;7,9532 11,68;7,9343 10,12;7,9147 12,02;7,8981 5,1;7,7776 3,59;7,7735 3,7;7,7584 7,12;7,7542 7,23;7,7394 4,11;7,7348 4,16;7,5528 8,27;7,5364 7,62;7,3341 8,43;7,3144 7,75;7,3016 5,52;7,2825 4,64;7,2698 4,93;7,2648 4,73;7,2522 4,09;7,1681 11,44;7,148 5,51;7,0346 5,68;7,0119 12,06;6,8955 11,98;6,8761 5,9;5,739 4,25;5,4395 1,78;5,3961 9,72;5,3668 9,53;5,3237 1,68;4,6472 13,75;4,6325 15;4,4723 2,83;4,4386 2,84;4,4057 0,35;4,0403 3,12;4,0214 1,91;4,0051 2,89;3,4358 0,32;3,4235 0,32;3,3976 0,38;3,3752 0,61;3,2827 706,349976;3,2609 39,23;3,2273 3,23;3,1678 0,54;3,1211 2,16;3,1008 2,61;3,0915 4,2;3,0839 2,59;3,071 1,65;3,0628 2,35;2,8275 1,93;2,7992 3,48;2,7696 1,86;2,6679 1,43;2,6636 1,27;2,6124 0,31;2,5945 0,42;2,5693 0,56;2,5377 2,58;2,503 178,309998;2,4988 226,889999;2,4947 162,949997;2,4262 0,5;2,4211 0,42;2,3914 0,38;2,365 0,32;2,3252 1,53;2,0653 1,44;2,0351 2,36;1,9997 4,16;1,9852 5,24;1,9533 3,21;1,9076 2,02;1,9 1,37;1,8696 2,51;1,8452 2,31;1,8392 2,2;1,8125 1,97;1,7919 2,5;1,7823 2,5;1,7615 2,17;1,7284 0,85;1,7199 0,71;1,2378 0,48;1,1931 0,49;1,1752 1,14;1,1577 0,65;0,8897 0,47;0,0193 0,4;-0,0001 23,35 |
| I-91 | [DMSO-d₆] 9,2229 2,03;9,2077 3,69;9,1925 1,86;7,9587 2,76;7,9396 7,96;7,921 8,15;7,9121 7,69;7,9087 8,44;7,8929 3,08;7,8895 2,24;7,5324 5,26;7,529 5,24;7,5141 4,94;7,5107 4,59;7,3782 5,2;7,3754 5,16;7,3656 5,59;7,3627 5,22;7,305 3,57;7,1715 8,06;7,1618 4,03;7,038 8,72;7,026 12,82;6,9699 6,1;6,9612 5,21;6,9573 5,82;6,9488 4,49;6,9039 7,91;6,8898 4,19;5,7463 12,55;5,4397 1,53;5,422 0,42;5,3974 6,41;5,3611 6,26;5,319 1,51;4,715 0,34;4,6818 9,45;4,666 9,27;4,4715 1,83;4,4381 1,93;4,0633 0,41;4,0571 1,22;4,0393 4,07;4,0216 4,81;4,004 2,32;3,9954 1,98;3,5562 0,36;3,53 0,34;3,5166 0,33;3,4914 0,41;3,4187 0,73;3,4034 0,88;3,3116 1540,969971;3,288 36,91;3,2616 2,62;3,2275 2,75;3,1964 1,54;3,0854 1,34;3,0763 1,01;3,0638 1,73;3,0556 2,81;3,049 1,55;3,0375 1,22;3,0263 1,49;2,7924 1,23;2,762 2,28;2,7353 1,37;2,7325 1,37;2,6697 1,53;2,6654 1,14;2,5395 3,47;2,5092 93,239998;2,505 172,770004;2,5006 222,860001;2,4964 154,990005;2,3791 0,32;2,3271 1,51;2,3227 1,07;2,0693 0,95;2,0497 0,69;1,9869 15;1,9593 2,96;1,9162 2,23;1,8949 0,48;1,8645 0,81;1,8354 2,01;1,8038 1,96;1,7773 1,94;1,7679 1,89;1,7457 1,5;1,7356 1,44;1,714 0,62;1,3987 1;1,2921 0,39;1,2359 0,57;1,1929 3,6;1,1749 7,14;1,1572 3,56;0,891 0,3;0,0077 1,13;-0,0001 25,040001;-0,0159 0,36 |
| I-92 | [DMSO-d₆] 9,1147 0,65;9,099 1,24;9,0839 0,57;7,9573 1,02;7,9383 2,75;7,9196 2,74;7,9084 2,55;7,905 2,76;7,8892 1,13;7,8858 0,78;7,5316 1,77;7,5282 1,74;7,513 1,66;7,5097 1,5;7,3036 1,18;7,2604 2,7;7,2478 2,82;7,1701 2,65;7,1613 1,31;7,0367 1,38;7,0254 2,99;6,9034 2,55;6,8895 1,47;6,8254 2,79;6,8127 2,6;5,7473 5,03;5,4396 0,52;5,3966 2,05;5,3606 1,99;5,3179 0,48;4,6125 3,21;4,5968 3,14;4,4702 0,58;4,436 0,61;4,0569 0,42;4,0391 1,45;4,0214 1,53;4,0035 0,85;3,3012 339,890015;3,2681 0,89;3,2297 0,82;3,2007 0,45;3,0876 0,41;3,0671 0,52;3,0583 0,87;3,0488 0,5;3,037 0,45;3,0284 0,48;2,7968 0,39;2,7648 0,72;2,738 0,42;2,6738 0,36;2,6691 0,51;2,6645 0,37;2,5389 2,94;2,5219 1,9;2,5087 30,209999;2,5043 56,48;2,4998 73,379997;2,4955 50,130001;2,4911 23,84;2,3306 0,39;2,3267 0,51;2,3219 0,41;2,2471 15;1,9867 5,22;1,9621 0,96;1,9188 0,71;1,9077 0,61;1,8396 0,5;1,8326 0,53;1,8105 0,5;1,7997 0,54;1,7826 0,4;1,7622 0,58;1,7538 0,58;1,7323 0,48;1,7214 0,44;1,398 0,35;1,1926 1,36;1,1748 2,69;1,157 1,35;0,008 0,54;-0,0001 15,01;-0,0083 0,65 |
| I-93 | [DMSO-d₆] 10,242 4,36;8,0238 1,86;8,0047 5,24;7,9957 0,33;7,9858 4,7;7,9686 4,18;7,9656 4,95;7,9495 2,22;7,9464 1,87;7,6265 2,82;7,6235 3,06;7,6076 2,76;7,6046 2,72;7,4679 0,46;7,452 0,99;7,4465 0,92;7,4361 0,76;7,4308 1,99;7,4144 1;7,4097 1,38;7,3939 0,6;7,3068 2,23;7,2563 0,41;7,2518 0,69;7,2438 4,11;7,2237 6,14;7,2033 2,89;7,1949 0,65;7,1735 5,24;7,1592 2,56;7,0402 2,52;7,0232 6,25;6,9052 4,64;6,8875 2,92;5,7548 16;5,4512 0,87;5,4085 3,85;5,3795 3,82;5,3368 0,9;4,4906 0,94;4,4577 1,02;4,0559 1,39;4,0381 1,83;4,0203 2,48;4,0026 0,64;3,4326 1,57;3,3831 2,5;3,3616 0,89;3,3326 1026,800049;3,284 2,14;3,2784 0,67;3,2498 1,38;3,2205 0,79;3,1589 0,39;3,1501 0,66;3,1413 0,49;3,1296 0,8;3,1206 1,34;3,1123 0,81;3,1004 0,53;3,0915 0,74;3,0369 0,49;2,8493 0,36;2,8143 0,67;2,7877 1,2;2,7823 1,21;2,7569 0,69;2,6757 0,57;2,6709 0,77;2,6664 0,56;2,5525 0,39;2,5479 0,35;2,5413 3,21;2,5246 2;2,5199 2,79;2,5112 39,689999;2,5066 89,610001;2,502 127,580002;2,4973 92,459999;2,4927 42,41;2,3335 0,57;2,3288 0,76;2,3242 0,6;2,0734 1,35;2,0571 0,77;2,0513 0,88;2,0268 1,2;1,9886 7,49;1,9701 1,16;1,9414 0,48;1,9199 0,84;1,911 0,9;1,8902 1,02;1,8815 1,07;1,8613 1,02;1,8515 1,07;1,8303 0,77;1,8207 |
| | 0,74;1,1924 2,05;1,1746 4,21;1,1568 2,03;-0,0001 7,15 |
| I-94 | [DMSO-d₆] 10,8799 5,86;8,0813 0,95;8,062 7,78;8,0578 7,8;8,0489 15;8,0388 1,54;8,0234 7,97;8,0041 12,57;7,9832 4,22;7,8561 4,34;7,8356 5,06;7,7914 0,3;7,6862 0,83;7,6762 3,61;7,6671 3,68;7,6633 3,44;7,654 3,8;7,648 2,25;7,6343 3,78;7,6309 3,79;7,6143 3,37;7,6098 3,81;7,6045 7,67;7,5845 9,01;7,5653 4,63;7,2972 3,14;7,1637 7,01;7,1366 3,37;7,0302 3,52;7,0005 7,69;6,898 6,72;6,8646 3,62;5,7471 0,33;5,4671 1,09;5,425 5,76;5,3994 5,72;5,3572 1,02;4,5144 1,51;4,4828 1,55;4,0929 1,54;4,0584 1,61;4,0394 0,68;4,0218 0,55;3,4443 0,32;3,4166 0,35;3,4112 0,39;3,3988 0,43;3,3654 1,08;3,3064 899,190002;3,2645 1,59;3,2535 1,27;3,2408 1,38;3,2337 1,17;3,2115 2,34;3,2027 1,52;3,183 1,26;3,0725 1,37;3,0382 0,3;2,8795 1,09;2,8503 2,07;2,8213 1,1;2,674 0,73;2,6694 1,07;2,6651 0,75;2,66 0,41;2,5646 0,44;2,5394 4,15;2,5224 3,97;2,5092 63,27;2,5049 118,889999;2,5004 155,089996;2,4961 107,169998;2,4917 51,209999;2,4065 0,31;2,3317 0,78;2,3273 1,09;2,3222 0,79;2,1373 1,24;2,1041 2,05;2,0695 1,69;2,0499 2,15;2,0124 0,69;1,987 3,08;1,9608 1,18;1,9505 1,12;1,9279 0,56;1,9085 1,69;1,8781 1,29;1,8713 1,25;1,8485 1,18;1,8374 1,06;1,8173 0,46;1,806 0,41;1,3979 0,45;1,2929 0,41;1,2351 0,48;1,1933 0,58;1,1751 0,97;1,1575 0,54;0,8895 0,44;0,0079 1,04;-0,0001 25,360001;-0,0081 1,05 |
| I-95 | [DMSO-d₆] 10,5642 7,41;8,5177 5,95;8,06 1,46;8,0523 16;8,0473 7,98;8,0426 7,25;8,0299 1,33;7,9662 2;7,9515 8,76;7,9447 6,76;7,9416 6,07;7,93 1,53;7,9268 1,65;7,9102 4,22;7,9044 4,13;7,8974 4,3;7,8912 4,49;7,6512 0,52;7,6435 3,76;7,6385 3,31;7,6338 3,59;7,6288 3,59;7,6209 0,48;7,5358 2,05;7,5338 2,05;7,5244 3,21;7,5225 4,12;7,5203 2,26;7,5107 2,79;7,5089 2,74;7,4735 3,08;7,4718 2,97;7,4621 2,51;7,46 4,39;7,4582 3,12;7,4487 2,04;7,4468 1,99;7,3117 2,46;7,2227 5,77;7,1431 2,77;7,1339 2,83;7,0525 6,84;6,9619 3,15;6,9357 6,5;5,7624 2,13;5,492 2,29;5,4633 4,99;5,4143 4,98;5,3857 2,29;4,5148 1,52;4,4931 1,54;4,0803 1,44;4,0578 1,54;4,046 0,44;4,0341 0,73;4,0222 0,7;3,5053 0,34;3,4883 0,33;3,4862 0,34;3,476 0,42;3,4699 0,43;3,4615 0,45;3,4581 0,51;3,4541 0,55;3,4479 0,69;3,4414 0,85;3,4341 1,17;3,431 1,09;3,423 1,29;3,4049 2,2;3,4009 2,56;3,3965 3,46;3,3679 3527,939941;3,3445 18,950001;3,3366 1,14;3,3061 0,68;3,3003 0,96;3,283 1,73;3,2804 1,75;3,2623 1,02;3,2577 0,77;3,2065 0,47;3,2006 0,86;3,1944 0,57;3,1865 1,14;3,1806 1,96;3,1748 1,09;3,167 0,65;3,1608 0,94;3,1551 0,47;2,84 0,93;2,8363 1,01;2,8188 1,94;2,8149 1,92;2,7975 1,04;2,7933 0,9;2,6225 0,82;2,6196 1,69;2,6166 2,28;2,6136 1,63;2,6106 0,76;2,5256 4,75;2,5225 6,28;2,5193 7,33;2,5105 133,360001;2,5076 281,079987;2,5045 380,359985;2,5015 271,429993;2,4985 121,480003;2,3942 0,68;2,3914 1,57;2,3884 2,15;2,3854 1,51;2,0775 3,09;2,0568 1,73;2,0343 1,33;2,0127 1,6;1,9989 0,74;1,9906 3,4;1,9846 1,31;1,978 1,37;1,9636 1,17;1,9573 1,13;1,943 0,4;1,8432 0,5;1,8359 0,58;1,822 1,31;1,8151 1,37;1,801 1,29;1,7943 1,23;1,7801 0,48;1,7732 0,39;1,2324 0,57;1,1864 0,85;1,1745 1,71;1,1626 0,84;0,889 1,43;0,0054 0,64;0 18,02;-0,0056 0,55 |
| I-96 | [DMSO-d₆] 12,5396 9,47;8,9656 6,6;8,9615 7,39;8,9552 7,32;8,951 7,29;8,8455 6,43;8,8421 7,25;8,8267 7,13;8,8233 7,3;8,4803 5,95;8,4762 6,49;8,4594 6,61;8,4553 6,39;8,116 1,1;8,1109 3,16;8,0968 14,72;8,0942 16;8,0919 13,85;8,0774 11,16;8,0582 3,62;7,7576 4,38;7,7542 5,13;7,7368 8,54;7,7334 8,22;7,7183 0,46;7,7024 6,29;7,6972 6,59;7,6932 8,56;7,6855 6,21;7,6803 6,21;7,674 8,92;7,6574 7,03;7,6538 5,29;7,647 6,68;7,6367 6,56;7,6262 6,65;7,3362 4,42;7,2028 10,29;7,163 4,95;7,0695 4,95;7,0271 12,15;6,9337 9,2;6,8912 5,61;5,7559 1,65;5,5286 2,12;5,4855 7,45;5,4473 7,33;5,4045 2,11;4,5325 2,01;4,4991 2,15;4,1307 1,9;4,0975 2,06;3,4355 2,53;3,4051 1,34;3,3926 0,61;3,3847 3,14;3,3758 3,58;3,3733 3,58;3,3698 3,51;3,3353 2097,399902;3,2844 2,91;3,2736 2,02;3,2646 1,52;3,2553 2,08;3,2458 3,2;3,2362 2,17;3,2276 1,33;3,218 1,73;3,2084 0,95;3,0382 0,52;2,982 1,4;2,9572 2,36;2,9514 2,45;2,9261 1,47;2,8502 0,35;2,6816 0,51;2,6772 1,12;2,6725 1,55;2,6677 1,12;2,6631 0,56;2,5426 6,66;2,5259 4,48;2,5212 6,04;2,5126 80,190002;2,508 179,720001;2,5034 254,350006;2,4988 184,600006;2,4942 84,769997;2,4575 0,43;2,4514 0,48;2,4476 0,47;2,3395 0,53;2,3349 1,14;2,3303 1,62;2,3257 1,2;2,3208 0,55;2,1694 1,53;2,1403 2,26;2,1 1,5;2,0748 6,31;2,0524 1,64;2,0414 0,98;2,0202 1,72;2,0117 2,01;1,9929 2,12;1,9785 2,25;1,9644 2,03;1,9433 1,55;1,9357 1,42;1,9135 0,64;1,9028 0,5;1,2924 0,44;1,2312 0,51;0,008 0,35;-0,0001 12,34;-0,0084 0,39 |
| I-97 | ¹H NMR (DMSO-d₆, 400 MHz): dₚₚₘ : 0.91 (d, 3H), 1.11-2.05 (m, 13H), 2.75-2.85 (m, 1H), 3.04-3.12 (m, 1H), 3.25 (m, 1H), 3.98-4.02 (m, 1H), 4.40-4.43 (m, 1H), 4.56-4.62 (m, 1H), 5.34 (d, 1H), 5.43 (d, 1H), 6.90 (s, 1H), 7.03 (t, 1H), 7.18 (t, 1H), 7.56 (d, 1H), 7.87-7.97 (m, 2H) |
| I-98 | ¹H NMR (DMSO-d₆, 400 MHz): dₚₚₘ: 1.54-2.15 (m, 10H), 2.72-2.83 (m, 1H), 3.03-3.12 (m, 1H), 3.24 (m, 1H), 3.97-4.02 (m, 1H), 4.40-4.43 (m, 1H), 5.34 (d, 1H), 5.41-5.46 (m, 2H), 5.78-5.82 (m, 1H), 6.00-6.04 (m, 1H), 6.90 (s, 1H), 7.02 (t, 1H), 7.18 (t, 1H), 7.56 (dd, 1H), 7.87-7.95 (m, |
| | 2H) |
| I-99 | ¹H NMR (DMSO-d₆, 400 MHz): dₚₚₘ : 1.27-1.98 (m, 14H), 2.76-2.84 (m, 1H), 3.02-3.11 (m, 1H), 3.25 (m, 1H), 3.98-4.03 (m, 1H), 4.38-4.43 (m, 1H), 4.91-4.98 (m, 1H), 5.34 (d, 1H), 5.44 (d, 1H), 6.90 (s, 1H), 7.03 (t, 1H), 7.18 (t, 1H), 7.56 (dd, 1H), 7.87-7.95 (m, 2H) |
| I-100 | ¹H NMR (DMSO-d₆, 400 MHz): dₚₚₘ: 1.54-1.64 (m, 1H), 1.74-2.12 (m, 7H), 2.72-2.92 (m, 3H), 3.04-3.10 (m, 1H), 3.23 (m, 1H), 3.97-4.00 (m, 1H), 4.39-4.42 (m, 1H), 5.33 (d, 1H), 5.42 (d, 1H), 6.17 (t, 1H), 6.90 (s, 1H), 7.02 (t, 1H), 7.04-7.33 (m, 5H), 7.56 (dd, 1H), 7.85-7.93 (m, 2H) |
| I-101 | [DMSO-d₆] 7,9631 3,09;7,9439 8,57;7,9248 6,95;7,9014 7,49;7,8993 8,19;7,8825 3,94;7,8802 3,48;7,5826 5,82;7,5806 5,89;7,5636 5,49;7,5615 5,19;7,3158 3,67;7,1823 8,23;7,1652 4,12;7,0489 4,08;7,0291 8,98;6,9051 8,27;6,8933 4,77;5,8713 0,91;5,8547 1,89;5,8458 1,09;5,8382 1,06;5,8289 2,93;5,812 3,01;5,8029 1,23;5,7952 1,18;5,7863 2,25;5,7697 1,12;5,7465 9,5;5,4563 2,44;5,4144 6,3;5,3671 6,44;5,3247 1,85;5,0569 3,65;5,0525 3,91;5,0486 1,85;5,018 1,53;5,014 3,33;5,0096 3,55;5,0057 1,68;4,9807 3,69;4,9783 3,55;4,976 3,36;4,9552 3,48;4,9529 3,35;4,9506 3,15;4,4383 1,93;4,406 2,01;4,3278 7,05;4,3113 15;4,2947 7,34;4,0213 1,88;3,9864 1,97;3,921 0,43;3,3142 500,690002;3,2907 19,27;3,2473 2,8;3,2177 1,56;3,1248 0,8;3,116 1,41;3,1071 1,03;3,0957 1,7;3,0866 2,81;3,078 1,68;3,0664 1,1;3,0574 1,51;3,0485 0,84;3,0383 0,44;2,9421 0,55;2,8509 0,35;2,8221 1,32;2,7945 2,39;2,7899 2,4;2,7635 1,36;2,6754 0,38;2,6707 0,51;2,6665 0,38;2,5407 1,13;2,506 56,150002;2,5017 71,639999;2,4975 50,34;2,3951 0,31;2,3327 0,4;2,3282 0,52;2,3243 0,38;2,1278 2,42;2,1099 6,28;2,0923 6,54;2,0744 2,72;2,0709 2,1;1,9872 0,78;1,9664 1,58;1,9303 3,48;1,9069 1,35;1,8921 2,17;1,8529 0,77;1,8433 0,86;1,822 1,67;1,8133 1,73;1,7907 1,54;1,7817 1,48;1,7655 1,88;1,7488 5,12;1,7313 6,03;1,7112 5,37;1,6947 2,19;1,6697 0,75;1,6601 0,86;1,639 1,66;1,6293 1,74;1,6079 1,58;1,5982 1,53;1,5775 0,68;1,5672 0,57;1,5325 2,06;1,5132 5,36;1,4948 6,92;1,4759 4,22;1,4576 1,35;1,2755 0,31;1,2362 0,45;0,8571 0,44;-0,0001 2,46 |
| I-102 | |
| I-103 | [DMSO-d₆] 7,9657 1,97;7,9465 5,56;7,9276 5,13;7,9138 5,28;7,9109 5,75;7,8947 2,43;7,8918 1,87;7,6849 1,25;7,6677 1,66;7,6634 2,68;7,6468 2,7;7,6423 1,67;7,6253 1,36;7,6 3,85;7,5972 3,79;7,5813 3,63;7,5784 3,32;7,3485 1,44;7,3421 1,52;7,3223 2,14;7,3183 2,37;7,3161 2,42;7,3117 2,83;7,2989 1,47;7,2926 1,46;7,1781 5,81;7,1623 3,28;7,1497 2,45;7,1451 2,21;7,1283 1,21;7,1241 1,09;7,0448 2,8;7,026 5,97;6,9027 5,57;6,8901 3,09;5,7469 3,14;5,4556 1,71;5,408 15;5,3598 4,08;5,3171 1,32;4,4309 1,3;4,3983 1,33;4,0394 0,65;4,0214 1,37;4,0128 1,24;3,9792 1,32;3,5682 0,95;3,3095 558,380005;3,2858 25,43;3,2372 1,89;3,2072 1,02;3,1201 0,53;3,1116 0,93;3,1028 0,69;3,0909 1,15;3,0821 1,86;3,0734 1,12;3,0619 0,72;3,0531 1,01;2,9416 0,4;2,81 0,9;2,7782 1,61;2,7507 0,93;2,6747 0,53;2,67 0,7;2,6656 0,53;2,54 1,53;2,5053 76,669998;2,501 97,25;2,4968 67,599998;2,332 0,51;2,3277 0,67;2,3233 0,52;2,0698 0,65;1,9871 2,33;1,954 1,07;1,9183 2,42;1,8819 1,44;1,8427 0,5;1,8345 0,58;1,811 1,09;1,8032 1,15;1,7803 1;1,7723 0,94;1,7509 0,39;1,6541 0,44;1,6421 0,55;1,621 1,12;1,6123 1,14;1,5913 1,06;1,5816 1,02;1,5613 0,39;1,55 0,32;1,3976 0,3;1,236 0,3;1,1928 0,63;1,1752 1,17;1,1574 0,61;1,1118 1,17;-0,0001 4,89 |
| I-104 | [DMSO-d₆] 7,9631 0,38;7,9556 2,05;7,9447 0,61;7,9363 5,52;7,917 4,07;7,881 4,66;7,8789 4,67;7,8619 2,78;7,8596 2,37;7,614 0,34;7,5956 3,85;7,5763 3,48;7,3278 0,44;7,3113 5,03;7,2914 4,72;7,2891 4,59;7,2687 3,1;7,2524 0,32;7,177 5,09;7,1647 2,53;7,0436 2,53;7,0286 5,47;6,9024 5,24;6,8928 3,03;5,747 2,76;5,5478 0,48;5,4524 1,33;5,4088 15;5,3573 3,68;5,3147 1,17;4,4253 1,19;4,3927 1,24;4,0575 0,5;4,0396 1,45;4,0218 1,86;4,0042 1,52;3,9746 1,22;3,3945 0,35;3,31 561,539978;3,2864 23,34;3,2691 1,59;3,2317 1,66;3,2024 0,92;3,1016 0,87;3,0924 0,65;3,0808 1,04;3,0722 1,66;3,063 1,01;3,0515 0,66;3,0421 0,91;2,9423 0,32;2,8046 0,84;2,7783 1,46;2,7463 0,83;2,6749 0,49;2,6701 0,64;2,6654 0,48;2,5404 1,48;2,5056 72,480003;2,5012 89,940002;2,497 60,939999;2,3325 0,48;2,328 0,62;2,3234 0,46;2,0701 0,63;1,9873 6,07;1,9436 1,04;1,9066 2,3;1,8717 1,28;1,8296 0,48;1,8212 0,54;1,7991 1,01;1,7896 1,02;1,7679 0,91;1,7597 0,87;1,738 0,35;1,6404 0,43;1,63 0,51;1,609 0,99;1,5987 1,04;1,5775 0,95;1,5677 0,88;1,5474 0,36;1,2367 0,31;1,1932 1,63;1,1754 3,23;1,1576 1,59;1,112 0,52;-0,0001 4,46 |
| I-105 | ¹H NMR (DMSO-d₆, 400 MHz): dₚₚₘ: 1.57-1.67 (m, 1H), 1.76-1.85 (m, 1H), 1.89-1.98 (m, 2H), 2.76-2.82 (m, 1H), 3.06-3.12 (m, 1H), 3.24 (m, 1H), 4.02-4.03 (m, 1H), 4.40-4.43 (m, 1H), 5.34 (d, 1H), 5.43 (d, 1H), 5.46 (s, 2H), 6.89 (s, 1H), 7.02 (t, 1H), 7.17 (t, 1H), 7.39-7.44 (m, 2H), 7.51-7.55 (m, 1H), 7.59-7.64 (m, 2H), 7.92-7.98 (m, 2H) |
| I-106 | ¹H NMR (DMSO-d₆, 400 MHz): dₚₚₘ : 1.55-1.65 (m, 1H), 1.74-1.84 (m, 1H), 1.88-1.98 (m, 2H), 2.75-2.81 (m, 1H), 3.04-3.11 (m, 1H), 3.23 (m, 1H), 3.98-4.03 (m, 1H), 4.40-4.43 (m, 1H), 5.34 (d, 1H), 5.43 (d, 1H), 5.44 (s, 2H), 6.90 (s, 1H), 7.02 (t, 1H), 7.17 (t, 1H), 7.24-7.31 (m, 2H), 7.42-7.47 (m, 1H), 7.56-7.60 (m, 2H), 7.90-7.97 (m, 2H) |
| I-107 | ¹H NMR (DMSO-d₆, 400 MHz): dₚₚₘ: 1.53-1.64 (m, 1H), 1.72-1.82 (m, 1H), 1.87-1.94 (m, 2H), 2.74-2.80 (m, 1H), 3.04-3.10 (m, 1H), 3.18-3.26 (m, 1H), 3.97-4.00 (m, 1H), 4.39-4.42 (m, 1H), 5.33 (d, 1H), 5.42 (d, 1H), 5.45 (s, 2H), 6.90 (s, 1H), 7.03 (t, 1H), 7.06-7.31 (m+t, 3H), 7.50-7.59 (m, 2H), 7.86-7.95 (m, 2H) |
| I-108 | (m, 2H), 2.39 (s, 3H), 2.75-2.82 (m, 1H), 3.05-3.12 (m, 1H), 3.24 (m, 1H), 3.98-4.01 (m, 1H), 4.40-4.43 (m, 1H), 5.29 (d, 1H), 5.37 (d, 1H), 5.45 (s, 2H), 6.90 (s, 1H), 7.02 (t, 1H), 7.17 (t, 1H), 7.27-7.30 (m, 1H), 7.58 (dd, 1H), 7.65 (d, 1H), 7.90-7.97 (m, 2H), 8.36 (d, 1H) |
| I-109 | [DMSO-d₆] 7,9667 1,21;7,9476 3,45;7,9287 3,17;7,9152 3,32;7,9123 3,61;7,8961 1,52;7,8933 1,17;7,5939 2,36;7,5911 2,36;7,5752 2,23;7,5723 2,07;7,3144 1,55;7,181 3,51;7,1657 1,78;7,1417 3,21;7,0841 3,75;7,0476 1,74;7,0297 3,82;6,9032 3,55;6,8939 2,17;5,7471 3,06;5,4873 11,47;5,4602 1,01;5,4179 2,52;5,361 2,51;5,3182 0,85;4,4361 0,83;4,4039 0,84;4,0393 0,33;4,0211 0,84;4,013 0,78;3,9806 0,85;3,3044 253,399994;3,2807 15,25;3,2364 1,16;3,2051 0,64;3,1184 0,34;3,11 0,58;3,1018 0,43;3,0899 0,71;3,0807 1,16;3,0718 0,73;3,0597 0,47;3,0508 0,62;3,0419 0,36;2,8058 0,57;2,779 1,02;2,7477 0,59;2,6739 0,35;2,6692 0,47;2,6648 0,33;2,5393 1,01;2,5045 50,09;2,5002 63,139999;2,496 43,830002;2,3316 0,34;2,327 0,44;2,3225 0,33;2,1955 15;2,1747 0,36;2,0696 0,37;1,9868 1,15;1,9476 0,68;1,9153 1,57;1,8791 0,93;1,8345 0,36;1,8145 0,69;1,8053 0,72;1,7827 0,62;1,774 0,59;1,648 0,32;1,6279 0,68;1,6176 0,72;1,596 0,67;1,587 0,65;1,1929 0,31;1,175 0,59;-0,0001 2,68 |
| 1-110 | [DMSO-d₆] 10,0508 0,5;8,2376 5,26;8,22 6,91;8,11 4,75;8,0905 8,33;8,071 4,41;8,0625 3,92;8,0578 4,14;8,0398 4,12;7,9426 5,38;7,9301 4,36;7,9228 7,09;7,9129 4,07;7,9078 3,75;7,8957 0,43;7,7448 5,9;7,7264 5,55;7,6323 5,1;7,6173 5,65;7,6133 10,79;7,6056 5,14;7,5998 8,68;7,5933 8,53;7,5853 3,79;7,5816 3,92;7,5682 1,55;7,5553 0,33;7,5241 6,55;7,5223 6,55;7,5054 4,67;7,3042 3,25;7,2908 0,33;7,1707 7,53;7,148 3,56;7,0375 3,64;7,0285 0,67;7,012 8,05;6,9141 0,42;6,8923 7,76;6,876 3,92;5,7476 15;5,4714 1,86;5,4547 0,93;5,4294 5,38;5,3703 5,38;5,3281 1,93;4,4716 1,79;4,4386 1,92;4,057 2,02;4,039 2,35;4,0213 3,31;4,0037 1,02;3,9204 0,42;3,3931 0,37;3,3808 0,45;3,3009 489,959991;3,2779 32,869999;3,2527 2,25;3,2275 1,64;3,2072 1,8;3,1977 2,65;3,1892 1,64;3,1776 1,15;3,1678 1,54;3,0364 0,35;2,8561 1,26;2,8241 2,24;2,7979 1,26;2,6731 1,19;2,6689 1,54;2,6642 1,2;2,5389 3,26;2,5041 175,110001;2,4998 221,259995;2,4956 153,389999;2,4569 1,39;2,3948 0,5;2,3878 0,39;2,3308 1,19;2,3266 1,53;2,322 1,15;2,0695 1,89;2,0499 1,66;2,0206 3,47;1,9867 8,48;1,9517 0,76;1,9413 0,84;1,9188 1,55;1,9096 1,71;1,8863 1,39;1,8567 0,63;1,7444 0,73;1,7334 0,79;1,7116 1,49;1,7025 1,53;1,6816 1,39;1,6717 1,36;1,6509 0,55;1,2356 0,48;1,1926 1,8;1,1749 3,48;1,1571 1,7;1,1113 0,3;0,8893 0,32;-0,0001 11,24 |
| I-111 | |
| I-112 | [DMSO-d₆] 7,9914 0,95;7,9721 2,21;7,9618 0,31;7,9528 1,65;7,7551 1,56;7,738 1,4;7,736 1,31;7,6543 1,35;7,6525 1,37;7,6346 1,28;7,3186 0,77;7,1849 1,86;7,1651 0,95;7,0516 1,04;7,0291 2,1;6,9062 1,86;6,8932 1,17;5,4913 0,5;5,4481 1,12;5,3621 1,12;5,3198 0,49;4,4357 0,41;4,4034 0,42;4,0335 0,38;4,0004 0,44;3,9668 0,76;3,6827 0,75;3,5166 0,59;3,5079 0,56;3,2997 485,100006;3,2363 0,7;3,1266 0,42;3,1176 0,36;3,1064 0,49;3,0985 0,71;3,0891 0,51;3,0785 0,38;3,0686 0,46;3,0597 0,36;2,8902 1,78;2,8501 0,4;2,8249 0,57;2,794 0,37;2,7317 1,39;2,6735 0,75;2,6687 1;2,6642 0,76;2,6602 0,43;2,5389 3,1;2,5219 4,64;2,5086 56,009998;2,5042 102,040001;2,4998 130,990005;2,4954 89,459999;2,4911 42,91;2,3311 0,72;2,3265 0,92;2,3219 0,68;2,3179 0,39;2,0693 2,34;2,0493 0,46;2,0063 0,44;1,9864 0,59;1,9549 1,14;1,9408 1,17;1,9192 1,76;1,9077 1,34;1,8294 0,41;1,8217 0,43;1,7985 0,39;1,7924 0,39;1,7593 0,32;1,7222 1,19;1,71 1,09;1,698 1,05;1,6572 0,56;1,6488 0,54;1,6182 0,82;1,5971 0,68;1,5871 0,71;1,5113 0,39;1,4808 0,97;1,4584 2,04;1,4364 1,96;1,3984 15;1,3161 0,41;1,2926 0,6;1,2669 0,53;1,2364 0,76;0,0079 0,65;-0,0001 13,57;-0,0084 0,56 |
| I-113 | [DMSO-d₆] 8,0478 1,13;8,0344 0,66;8,0234 3,1;8,015 1,53;8,0041 6,81;7,9958 1,14;7,9847 4,32;7,9658 0,3;7,9516 0,36;7,8209 4,88;7,8189 4,96;7,8018 4,31;7,6886 4,78;7,6709 4,2;7,669 4,11;7,5361 1,7;7,4473 0,91;7,4304 1,94;7,4265 1,95;7,4094 3,59;7,3922 2,31;7,3886 2,44;7,3714 1,32;7,3627 0,63;7,3541 0,42;7,2883 2,59;7,2116 0,36;7,1897 0,52;7,1844 0,74;7,1547 7,18;7,1503 4,63;7,1461 5,51;7,1258 8,02;7,1054 5,63;7,0834 1,76;7,0706 |
| | 0,76;7,0543 0,94;7,049 1,45;7,0314 0,93;7,0212 3,33;7,0142 6,48;6,9898 1,12;6,9593 0,4;6,9494 0,99;6,9301 0,53;6,9186 0,68;6,9141 0,78;6,8908 5,37;6,8783 3,19;6,8161 0,49;5,4693 1,52;5,4268 3,35;5,3881 0,44;5,3647 0,32;5,341 3,4;5,2984 1,43;4,4608 0,44;4,4418 0,44;4,4116 1,34;4,3803 1,35;4,3213 0,34;4,2807 15;4,2604 1,04;4,2216 2,12;4,1995 0,58;4,173 0,69;4,16 0,51;4,0112 1,44;3,9766 1,53;3,9673 3,89;3,919 0,72;3,8284 0,33;3,8228 0,37;3,7194 0,47;3,7089 0,42;3,7003 0,36;3,6831 3,15;3,6319 0,41;3,5316 0,34;3,5252 0,34;3,3023 1356,869995;3,2435 2,42;3,2105 1,3;3,1861 0,59;3,1824 0,58;3,1469 0,55;3,1175 1,27;3,098 1,75;3,0898 2,34;3,0814 1,46;3,0689 1,13;3,0608 1,38;2,8904 2,06;2,8296 0,95;2,8033 1,64;2,7987 1,63;2,7723 1,02;2,7318 1,74;2,6738 1,97;2,6692 2,63;2,6647 1,97;2,6599 1,11;2,5393 8,28;2,5223 12,71;2,509 154,240005;2,5046 280,290009;2,5002 358,350006;2,4958 244,440002;2,4915 116,559998;2,3848 0,32;2,3315 1,94;2,3269 2,55;2,3223 1,86;2,1581 0,35;2,0696 4,13;2,0497 1,13;1,9869 1,62;1,9568 2,65;1,9241 1,57;1,908 1,26;1,879 0,32;1,8459 0,57;1,8368 0,66;1,8051 1,14;1,7824 1,01;1,7737 1,01;1,7528 0,51;1,7428 0,5;1,7044 0,38;1,6769 0,57;1,6709 0,55;1,6412 0,83;1,6125 1,19;1,6025 1,21;1,581 1,08;1,5703 1,01;1,5513 0,5;1,3983 9,48;1,292 0,31;1,2363 1,35;1,1752 0,45;1,159 0,32;0,0079 1,57;-0,0001 35,639999;-0,0084 1,65 |
| I-114 | [DMSO-d₆] 9,6017 0,33;8,1606 5,17;8,1352 0,41;8,1095 0,44;8,0615 2,97;8,0419 7,49;8,0224 6,02;8,0165 6,75;7,9962 4,52;7,9771 2,69;7,9717 2,48;7,9517 0,56;7,9265 0,41;7,9156 0,39;7,8933 0,47;7,8786 0,35;7,8063 4,36;7,8045 4,66;7,7872 4,01;7,7852 3,8;7,7577 4,11;7,7384 3,65;7,6801 0,3;7,6484 1,04;7,6445 1,28;7,6315 3,33;7,6274 3,02;7,621 3,31;7,6145 6,1;7,6076 3,15;7,6019 2,84;7,5975 2,78;7,5795 4,2;7,5751 3,51;7,5581 3,39;7,5538 3,31;7,5315 0,47;7,5146 0,38;7,4141 1,13;7,3286 2,31;7,2815 2,33;7,2084 0,34;7,1949 5,14;7,1778 1,34;7,1595 2,74;7,1484 1,41;7,1394 0,36;7,0617 2,45;7,0418 2,61;7,0235 5,86;6,9655 0,32;6,926 2,15;6,9057 4,99;6,9028 5,06;6,8876 2,81;5,5081 1,34;5,4946 0,33;5,4654 3,27;5,4123 0,34;5,3886 3,63;5,3464 1,33;5,1344 6,42;4,4717 1,25;4,435 1,26;4,0839 1,23;4,0684 0,86;4,0461 1,29;4,0034 0,34;3,853 0,98;3,8147 0,3;3,786 0,34;3,7719 0,3;3,7084 0,8;3,6824 0,44;3,6768 0,4;3,6204 0,49;3,5967 0,51;3,5769 0,52;3,5508 0,62;3,5071 0,7;3,4947 0,79;3,4095 1,61;3,3006 2024,209961;3,2254 2,1;3,2057 1,84;3,1948 2,26;3,1671 1,31;3,1466 0,85;3,1352 0,42;3,0366 0,45;2,9438 0,45;2,9144 0,9;2,8898 3,64;2,8495 1,06;2,7963 0,3;2,782 0,37;2,7306 2,13;2,6778 2,16;2,6733 3,88;2,6688 5,15;2,6642 3,93;2,6206 0,83;2,5388 16,76;2,5219 24,73;2,5085 303,920013;2,5042 554,929993;2,4997 714,030029;2,4953 487,720001;2,4909 234,240005;2,3986 0,89;2,3938 0,77;2,386 0,73;2,3358 2,07;2,3309 3,95;2,3264 5,07;2,3217 3,77;2,3171 2,05;2,2808 0,34;2,2694 0,38;2,2541 0,33;2,0969 1,14;2,0843 1,39;2,0692 15;2,0493 3,48;2,0189 1,35;1,9867 1,77;1,9554 0,85;1,9251 1,03;1,9192 1,03;1,8967 1;1,862 0,45;1,7687 0,55;1,7479 1,05;1,7401 1,1;1,7144 1,01;1,685 0,48;1,6737 0,42;1,3984 1,99;1,2918 0,45;1,2592 0,4;1,2486 0,48;1,2367 1,79;1,193 0,5;1,1748 1,1;1,1571 0,62;0,0081 2,37;-0,0001 58,860001;-0,0084 2,72 |
| I-115 | [DMSO-d₆] 7,9623 2,3;7,9431 6,31;7,9239 4,96;7,897 5,4;7,8949 6,01;7,878 2,98;7,8758 2,75;7,5815 4,19;7,5794 4,34;7,5625 3,96;7,5603 3,85;7,3155 2,65;7,1821 5,94;7,1644 2,95;7,0487 2,93;7,0284 6,46;6,9042 5,95;6,8925 3,46;5,7467 7,25;5,4573 1,53;5,4151 4,53;5,3676 4,59;5,325 1,33;4,4391 1,38;4,4066 1,44;4,3137 4,95;4,307 1,63;4,2971 10,78;4,2805 5,61;4,04 0,36;4,0218 1,41;3,9869 1,4;3,3945 0,7;3,3784 1,5;3,3652 1,58;3,3621 1,31;3,3487 1,46;3,3138 354,920013;3,29 14,59;3,2463 2,01;3,2181 1,12;3,1239 0,56;3,1153 1,01;3,1062 0,73;3,0951 1,23;3,086 2;3,0771 1,22;3,066 0,79;3,0567 1,09;3,0484 0,61;2,9418 0,47;2,8217 0,96;2,7952 1,72;2,7909 1,71;2,7634 0,97;2,6707 0,36;2,5409 0,79;2,5061 40,939999;2,5017 53,060001;2,4975 37,619999;2,3284 0,36;1,9874 0,78;1,9671 1,11;1,931 2,5;1,894 1,55;1,8528 0,54;1,8429 0,62;1,8206 1,17;1,8122 1,23;1,7897 1,07;1,7815 1,05;1,75 1,23;1,733 3,14;1,7157 4,41;1,6971 3,6;1,6803 1,37;1,6719 0,64;1,6601 0,64;1,6387 1,19;1,6289 1,25;1,608 1,12;1,5984 1,09;1,5772 0,47;1,5676 0,39;1,4111 2,01;1,4045 2,28;1,3946 3,16;1,39 3,1;1,3709 3,19;1,357 3,01;1,3498 3,11;1,3359 2,87;1,3116 2,76;1,2752 9,03;1,268 8,69;1,2583 6,79;1,1757 0,41;0,8744 5,55;0,8575 15;0,8401 5,76;-0,0001 2,29 |
| I-116 | [DMSO-d₆] 7,9759 1,08;7,9568 3,1;7,9381 3,15;7,928 3,03;7,9246 3,36;7,9088 1,27;7,9053 0,9;7,6076 2,08;7,6043 2,04;7,5891 1,99;7,5857 1,82;6,4983 3,89;5,7474 3,2;5,3495 0,73;5,3075 2,33;5,2525 2,4;5,2103 0,77;5,0948 0,34;5,0801 0,64;5,0733 0,54;5,0682 0,7;5,0588 0,73;5,0468 0,7;5,033 0,32;5,0205 0,3;4,7714 0,3;4,7595 0,33;4,7506 0,39;4,7459 0,43;4,7386 0,41;4,7243 0,34;4,6322 0,36;4,6191 0,44;4,6115 0,4;4,6072 0,4;4,4653 0,68;4,4331 0,71;4,039 0,62;4,0319 0,65;3,9972 0,69;3,3025 200,820007;3,2787 8,31;3,2532 1,08;3,2221 0,57;3,1226 0,49;3,1139 0,36;3,1017 0,63;3,0931 0,98;3,0836 0,6;3,0736 |
| | 0,41;3,0637 0,56;2,8218 0,47;2,7906 0,84;2,7638 0,49;2,6695 0,33;2,5394 0,76;2,5223 1,28;2,5091 21,08;2,5047 39,849998;2,5002 52,16;2,4958 36,189999;2,4915 17,42;2,3272 0,38;2,2236 15;2,2126 1,02;2,2018 0,45;2,1982 0,41;2,1311 0,41;2,1102 0,64;2,0697 1,21;2,0616 0,6;2,0498 0,75;2,0351 0,6;2,0261 0,65;1,987 0,65;1,9692 0,58;1,9338 1,36;1,8964 0,79;1,8589 0,32;1,8495 0,36;1,8292 0,61;1,82 0,66;1,7977 0,55;1,7879 0,54;1,6908 1,09;1,6784 1,17;1,6692 1,15;1,6461 0,85;1,6358 0,85;1,6155 0,9;1,6039 1,01;1,5739 0,63;1,5453 0,44;1,5224 0,58;1,5158 0,62;1,4898 0,63;1,4651 0,36;1,4568 0,32;1,4026 0,73;1,3799 1,16;1,3587 1,04;1,3339 0,48;0,008 0,43;-0,0001 11,11;-0,0081 0,5 |
| I-117 | [DMSO-d₆] 7,7213 0,38;7,7028 0,51;7,7 0,48;7,6816 0,44;7,3196 1,14;7,3016 1,29;7,2576 0,49;7,2394 0,43;7,2356 0,34;7,2024 0,42;7,1871 0,96;7,1718 1,07;7,1604 0,42;7,1324 0,62;7,111 0,58;7,0388 0,37;7,0243 0,8;6,9091 0,75;6,8884 0,39;6,1265 0,51;5,7466 0,32;5,4148 1,7;4,0394 0,32;4,0216 0,32;3,6729 0,54;3,6403 0,56;3,6273 0,52;3,5715 2,19;3,304 59,509998;3,2806 0,86;2,8565 0,31;2,5087 5,73;2,5046 10,22;2,5002 12,91;2,4959 8,94;2,0467 0,34;2,0365 0,31;1,9867 1,57;1,3982 15;1,1929 0,39;1,175 0,76;1,1573 0,38;-0,0001 2,11 |
| I-118 | [DMSO-d₆] 7,7293 1,55;7,7107 2,07;7,7079 1,98;7,6894 1,82;7,3091 3,88;7,3049 2,46;7,291 3,62;7,2842 3,11;7,2613 1,92;7,1454 2,54;7,1239 2,4;6,5001 3,92;5,3644 7,03;5,2968 7,9;3,6674 2,44;3,6394 2,53;3,5737 9,94;3,4286 0,32;3,3057 570,400024;2,6739 0,64;2,6694 0,82;2,6648 0,62;2,5395 3,63;2,5087 50,369999;2,5047 87,110001;2,5004 107,690002;2,4962 74,57;2,3316 0,63;2,3271 0,8;2,3226 0,61;2,2586 0,55;2,2128 15;2,0694 0,53;2,0497 0,34;1,9869 0,78;1,2926 0,32;1,2373 0,38;1,175 0,42;1,0455 0,31;-0,0001 4,64 |
| I-119 | [DMSO-d₆] 7,7752 2,4;7,7567 3,16;7,7538 3,14;7,7355 2,91;7,3125 2,02;7,2006 4,82;7,1954 5,82;7,1785 11,49;7,1652 2,56;7,0461 2,5;7,0291 5,07;6,9152 4,74;6,8932 2,52;5,7463 2,86;5,4289 10,86;5,3885 0,47;4,0394 0,8;4,0217 0,8;4,0037 0,32;3,7126 3,62;3,6836 3,66;3,6023 15;3,5432 0,34;3,5311 0,34;3,4683 1,55;3,4605 1,35;3,4348 0,78;3,4169 0,59;3,406 0,66;3,3905 0,83;3,3082 654,059998;3,2035 0,41;2,6742 0,73;2,6694 0,93;2,6651 0,69;2,5395 4,73;2,5089 57,009998;2,5048 100,760002;2,5004 126,889999;2,4962 88,5;2,3315 0,71;2,3273 0,9;2,3224 0,67;2,0691 1,32;2,0495 0,41;1,9868 3,43;1,9352 1,38;1,9235 2,03;1,9117 2,65;1,9034 2,8; 1,7131 2,57;1,7036 2,35;1,6912 1,87;1,656 0,31;1,6096 1,09;1,579 1,24; 1,485 0,83;1,4785 0,75;1,4539 2,8;1,429 5,46;1,3984 5,14;1,3726 0,76;1,2919 1,23;1,262 1,04;1,2365 0,93;1,1929 1,06;1,1751 2,03;1,1575 1,13;-0,0001 6,46 |
| I-120 | [DMSO-d₆] 8,1249 1,46;8,1056 1,8;8,0876 0,9;8,0613 0,96;8,0503 0,93;8,0381 1,01;7,8293 1,06;7,808 2,4;7,7895 2,38;7,6313 1,46;7,6261 0,68;7,6133 3,22;7,6021 1,87;7,5931 2,28;7,3302 0,89;7,3118 1,6;7,2903 1,49;7,1969 1,91;7,1798 2,1;7,1727 1,06;7,1622 1,94;7,0636 0,95;7,0366 1,91;6,9228 1,88;6,9009 0,94;5,4608 4,25;4,0392 0,73;4,0214 0,73;3,8508 1,35;3,7419 1,89;3,7303 1,97;3,7201 1,59;3,6808 1,51;3,4192 0,69;3,4085 0,8;3,4029 0,87;3,3095 650,429993;3,2127 0,32;3,1995 0,3;2,6694 0,84;2,6651 0,63;2,5394 2,24;2,5089 56,82;2,5048 99,25;2,5004 123,519997;2,4962 85,459999;2,3315 0,69;2,3271 0,9;2,3226 0,67;2,0691 0,54;1,9867 2,87;1,3984 15;1,2366 0,34;1,1928 0,84;1,1749 1,57;1,1571 0,81;-0,0001 14,7 |
| I-121 | [DMSO-d₆] 8,0457 2,09;8,0263 4,62;8,007 2,81;7,7842 3,53;7,7669 3,06;7,7651 2,92;7,7366 3,26;7,717 3,01;7,5274 2,3;7,5198 3,5;7,5104 13,7;7,5057 15;7,4875 2,13;7,4146 1,21;7,3985 0,32;7,3951 0,32;7,3238 1,84;7,2819 2,2;7,1904 3,98;7,178 1,27;7,1597 1,96;7,1491 1,18;7,057 2;7,0422 2,39;7,0238 4,35;6,9263 2,03;6,906 4,34;6,8879 2,19;5,7467 4,55;5,4993 1,07;5,4568 2,64;5,3802 2,58;5,3375 1,08;5,1329 6,09;4,4596 0,96;4,4268 0,98;4,0667 0,94;4,0575 1,1;4,0395 2,3;4,0218 1,94;4,004 0,6;3,4728 0,34;3,3084 46,98;3,2069 0,9;3,1987 1,16;3,1788 1,14;3,1698 1,6;3,1616 1,05;3,1496 0,74;3,1411 0,9;2,8906 0,8;2,8688 1,23;2,8364 0,71;2,7323 0,31;2,6743 0,41;2,6699 0,5;2,6651 0,38;2,5398 2,85;2,5093 28,290001;2,5051 49,52;2,5007 61,889999;2,4964 42,369999;2,3321 0,32;2,3274 0,42;2,323 0,31;2,0694 0,97;2,0494 1,2;2,037 1,42;1,987 7,21;1,9204 0,4;1,9082 0,47;1,8996 0,79;1,89 0,81;1,8689 0,7;1,8598 0,68;1,7531 0,34;1,7438 0,45;1,7219 0,79;1,7135 0,81;1,6916 0,73;1,6828 0,69;1,398 8,28;1,2927 0,31;1,2365 0,38;1,1929 1,77;1,1752 3,47;1,1574 1,76;-0,0001 5,57 |
| I-122 | [DMSO-d₆] 7,8212 0,82;7,8029 1,06;7,7999 1,07;7,7816 0,97;7,5083 0,57;7,4898 15;7,3831 0,31;7,3228 0,73;7,2763 1,41;7,2548 1,32;7,2141 1,75;7,196 1,79;7,1896 1,7;7,1696 0,81;7,0563 0,83;7,0336 1,69;6,9204 1,63;6,8977 0,85;5,7462 2,19;5,4482 3,71;4,0396 0,36;4,0218 0,36;3,7912 1,11;3,7845 1,1;3,7069 1,22;3,6635 1,58;3,6473 1,55;3,3119 78,5;3,1933 0,39;2,5399 0,69;2,5053 16,85;2,501 21,1;2,4968 14,76;1,9871 1,48;1,193 0,42;1,1752 0,8;1,1574 0,42;-0,0001 0,98 |
| I-123 | [DMSO-d₆] 8,0598 1,57;8,0403 3,39;8,0209 2,08;7,9034 2,25;7,9003 2,29;7,8902 |
| | 2,33;7,8871 2,12;7,8037 2,61;7,7864 2,22;7,7572 2,42;7,7379 2,19;7,7261 0,33;7,3383 2,32;7,3352 2,4;7,3293 2,88;7,3263 2,68;7,3203 1,52;7,2536 0,41;7,2386 2,61;7,2296 2,36;7,2253 2,46;7,2164 2,13;7,187 2,94;7,1586 1,47;7,1134 0,33;7,068 0,35;7,0537 1,46;7,0224 3,17;6,9954 0,32;6,9024 2,84;6,8864 1,57;5,4945 0,76;5,4511 1,98;5,3762 1,91;5,3346 0,82;4,4475 0,81;4,4146 0,75;4,0575 0,78;4,0217 0,92;3,9035 0,31;3,7432 0,32;3,7219 0,35;3,7124 0,33;3,6743 0,36;3,6448 0,41;3,6259 0,43;3,5902 0,5;3,5826 0,48;3,5722 0,51;3,555 0,58;3,459 1,15;3,3095 2849,459961;3,2861 38,669998;3,203 1,37;3,1966 1,29;3,1775 1,1;3,1682 1,4;3,1401 0,83;3,1034 0,34;3,0946 0,31;3,0832 0,31;3,0365 0,42;2,8902 0,87;2,86 0,98;2,83 0,65;2,7307 0,44;2,6739 2,18;2,6692 2,86;2,6648 2,13;2,5394 5,95;2,509 170,830002;2,5048 302,220001;2,5003 379,850006;2,496 260,809998;2,4247 0,85;2,4009 0,53;2,3316 2,07;2,3272 2,74;2,3225 1,96;2,2102 0,43;2,0689 1,93;2,0568 0,58;2,0492 1;2,0281 1,1;1,9866 1,49;1,9074 1,32;1,8782 0,66;1,8568 0,58;1,8264 0,34;1,7418 0,33;1,697 0,7;1,6742 0,66;1,646 0,3;1,3984 15;1,2925 0,66;1,2359 1,03;1,1753 0,42;1,1573 0,36;1,0704 0,3;0,8902 1,14;0,0079 3,47;-0,0001 68,889999;-0,0083 3,35 |
| I-124 | [DMSO-d₆] 8,5809 0,36;8,5708 0,34;7,8847 3,08;7,8818 3;7,8716 3,13;7,8686 2,94;7,8298 2,16;7,8115 2,87;7,8083 2,81;7,7901 2,61;7,7746 0,68;7,7696 0,7;7,7635 0,64;7,7583 0,61;7,7256 1,18;7,4147 1,71;7,4025 0,43;7,3916 0,33;7,3882 0,41;7,3834 0,38;7,3689 0,37;7,32 2,17;7,3111 3,32;7,3081 3,39;7,3022 4,28;7,2991 4,42;7,2945 4,37;7,2818 3,91;7,2729 3,59;7,2439 0,45;7,226 6,14;7,2161 3,85;7,2116 5,14;7,2096 5,25;7,203 3,39;7,1867 4,19;7,1779 2,23;7,1682 2,3;7,1491 2,01;7,1302 1,16;7,1139 2,33;7,1091 1,15;7,1024 1,08;7,0934 0,43;7,0669 0,37;7,0536 2,1;7,0421 3,8;7,0322 4,66;6,9947 0,51;6,9196 4,76;6,9065 2,16;6,8963 2,21;5,7464 0,55;5,4445 9,32;5,1351 9,57;4,0572 1,3;4,0395 3,65;4,0217 3,63;4,0039 1,31;3,8527 0,32;3,7797 2,94;3,7731 2,91;3,701 3,24;3,6568 4,25;3,6415 4,15;3,5731 0,38;3,5451 0,33;3,5214 0,36;3,4963 0,41;3,307 81,07;3,2036 3,34;3,0374 0,62;2,8506 0,53;2,6738 0,6;2,6695 0,78;2,6648 0,59;2,5395 3,28;2,5047 74,510002;2,5004 90,870003;2,4963 62,459999;2,3317 0,46;2,3272 0,6;2,3226 0,43;2,211 2,69;2,0692 0,36;1,9869 15;1,9084 0,44;1,3983 1,24;1,2926 0,34;1,2371 0,68;1,1929 4,22;1,1751 8,17;1,1574 4,06;-0,0001 4,5 |
| I-125 | [DMSO-d₆] 7,8007 2,47;7,7825 3,34;7,7794 2,98;7,7611 2,84;7,4421 0,77;7,4247 1,38;7,4201 1,26;7,4038 2,53;7,3872 1,36;7,3829 1,52;7,3661 0,74;7,2978 2,12;7,2511 5,11;7,2332 8,43;7,2117 3,81;7,1645 4,91;7,156 2,89;7,1435 3,92;7,1237 5,74;7,1034 3,05;7,0315 2,47;7,0199 4,92;6,9062 4,61;6,8841 2,34;5,4028 10,71;4,2406 10,93;4,0393 0,35;3,8587 0,31;3,8225 0,32;3,8 0,34;3,7821 0,34;3,771 0,37;3,7665 0,35;3,6845 4,07;3,6608 4,04;3,5816 15;3,509 0,69;3,4531 0,94;3,3093 1444,550049;3,2089 0,47;2,6738 1,33;2,6695 1,7;2,665 1,32;2,5396 4,75;2,5091 113,230003;2,5049 199,759995;2,5005 250,399994;2,4962 172,940002;2,3806 0,34;2,3708 0,33;2,3316 1,42;2,3274 1,81;2,3227 1,31;2,0692 1,25;1,9868 1,01;1,3983 13,56;1,2359 0,75;1,1928 0,42;1,175 0,59;1,157 0,33;-0,0001 46,560001;-0,0084 2,6;-0,1508 0,31 |
| I-126 | [DMSO-d₆] 8,0182 0,56;7,999 1,25;7,9796 0,86;7,8112 0,88;7,794 0,83;7,7736 0,31;7,6848 0,85;7,6654 0,79;7,5829 0,65;7,5741 0,73;7,5687 0,65;7,5596 0,75;7,4907 0,59;7,4811 0,57;7,476 0,75;7,4674 0,75;7,3435 0,32;7,3339 1,7;7,3255 1,49;7,3192 1,48;7,3105 1,52;7,3031 0,53;7,2931 0,54;7,1593 1,08;7,1509 0,57;7,026 0,59;7,0146 1,12;6,8927 1,06;6,8789 0,6;5,468 0,32;5,4243 0,65;5,3423 0,65;5,3001 0,3;4,3211 3,65;4,3084 1,18;3,9809 0,31;3,4079 0,54;3,3001 641,619995;3,2767 10,26;3,0928 0,41;3,0373 0,33;2,8493 0,31;2,8094 0,43;2,7779 0,32;2,6733 1,04;2,6687 1,33;2,6641 0,99;2,6056 0,32;2,5933 0,38;2,5915 0,4;2,5388 2,8;2,5084 82,190002;2,5041 145,470001;2,4997 182,970001;2,4954 125,709999;2,3308 0,97;2,3265 1,31;2,322 0,94;2,318 0,54;2,0693 0,73;1,9865 0,4;1,9646 0,47;1,9234 0,36;1,9072 0,4;1,8036 0,35;1,7766 0,33;1,3985 15;1,2923 0,33;1,2369 0,62;0,8903 0,36;0,0079 1,74;-0,0001 35,150002;-0,0083 1,8 |
| I-127 | [DMSO-d₆] 7,7962 2,13;7,7777 3,02;7,7753 3,02;7,7566 2,64;7,5678 2,27;7,559 2,47;7,5547 2,22;7,5445 2,76;7,4854 2,11;7,4753 2,01;7,4709 2,53;7,4622 2,86;7,3385 1,12;7,3294 6,08;7,3206 4,8;7,3151 4,86;7,306 5,43;7,3002 3,09;7,2704 0,4;7,2645 0,39;7,246 4,69;7,2278 8,03;7,206 3,84;7,1666 4,41;7,1573 2,45;7,048 0,33;7,0335 2,29;7,0212 4,52;6,9072 4,72;6,8853 2,43;5,7467 1,89;5,407 10,47;5,308 0,36;4,2838 15;4,0575 0,81;4,0398 2,33;4,022 2,35;4,0043 0,82;3,9628 0,49;3,6936 3,75;3,6648 3,76;3,5863 14,92;3,5123 0,5;3,4248 0,52;3,3101 218,339996;3,0381 0,78;2,8511 0,66;2,6749 0,37;2,6702 0,46;2,6659 0,36;2,5401 1,9;2,5052 49,459999;2,5012 60,130001;2,3277 0,45;1,9873 9,62;1,3977 0,33;1,2357 0,48;1,1932 2,68;1,1754 5,16;1,1576 2,62;-0,0001 1,82 |
| I-128 | [DMSO-d₆] 8,0129 2,61;8,0057 1,07;7,9935 5,1;7,9866 0,6;7,9741 3,1;7,9536 0,32;7,803 |
| | 4,15;7,7841 3,46;7,7657 0,3;7,7463 0,34;7,6779 3,8;7,6583 3,21;7,5241 1,06;7,4455 3,99;7,4398 2,33;7,4316 4,71;7,4239 4,5;7,4156 2,56;7,41 3,72;7,3826 0,35;7,3022 2,05;7,2726 0,38;7,2587 0,5;7,236 0,49;7,1687 6,03;7,1644 5,12;7,1552 3,4;7,1422 7,6;7,1255 2,24;7,1201 3,66;7,1049 0,48;7,0816 0,36;7,0583 0,56;7,0355 2,26;7,0192 4,89;6,9502 0,41;6,8989 4,2;6,8833 1,96;5,7475 0,48;5,467 1,22;5,424 2,89;5,3493 2,81;5,3076 1,17;4,4123 1,16;4,3823 1,13;4,2177 13,71;4,1995 0,99;4,1558 1,23;4,0577 1,27;4,0398 3,6;4,022 4,15;4,0043 1,8;3,9802 1,09;3,6443 0,31;3,306 563,599976;3,2836 5,67;3,2511 1,33;3,2238 0,68;3,1242 0,74;3,0955 1,58;3,0866 1,04;3,0666 1;3,0385 0,45;2,8506 0,85;2,813 1,23;2,7869 0,68;2,6701 0,79;2,6655 0,56;2,5398 1,43;2,5095 53,189999;2,5053 95,830002;2,501 121,959999;2,4967 85,239998;2,3321 0,66;2,3274 0,9;2,0697 0,31;1,9873 15;1,9619 1,88;1,9226 1,4;1,8356 0,56;1,8064 1,05;1,7835 0,93;1,6637 0,58;1,6349 1,14;1,6137 1,14;1,5823 0,55;1,3981 1,65;1,236 0,8;1,1931 3,88;1,1754 7,74;1,1576 3,95;0,008 0,53;-0,0001 19,790001 |
| I-129 | [DMSO-d₆] 7,9982 2,53;7,9789 5,61;7,9596 3,43;7,769 4,12;7,7514 3,74;7,6638 3,9;7,6446 3,54;7,601 0,89;7,3179 2,06;7,2817 0,36;7,223 0,36;7,1841 4,96;7,1634 2,51;7,1428 0,33;7,0877 0,64;7,0506 3,18;7,0275 5,22;6,9505 0,41;6,9055 4,95;6,8915 2,76;5,4809 1,31;5,4381 3,29;5,3659 3,33;5,324 1,38;5,1352 0,56;4,431 1,18;4,3985 1,2;4,2899 0,37;4,2629 0,35;4,2473 0,37;4,0368 1,1;4,0048 1,18;3,5736 0,38;3,4753 0,47;3,4263 0,68;3,4075 0,94;3,3051 1051,73999;3,2462 2,1;3,1968 0,7;3,1912 0,66;3,1405 1,23;3,1199 1,41;3,1103 2;3,0894 0,98;3,0832 1,17;3,0371 0,46;2,9617 4,1;2,9434 6,82;2,9252 4,55;2,8914 0,96;2,8737 1,24;2,8394 1,58;2,808 1,04;2,7781 0,35;2,7719 0,35;2,7453 0,31;2,7374 0,36;2,7237 0,35;2,7096 0,39;2,6894 0,56;2,6783 0,95;2,6738 1,57;2,6695 1,88;2,6648 1,42;2,6372 0,41;2,624 0,46;2,6016 0,61;2,5393 3,54;2,509 103,199997;2,5049 182,690002;2,5005 229,910004;2,4962 159,270004;2,3869 0,42;2,3771 0,4;2,3518 0,37;2,3274 1,74;2,3226 1,34;2,2703 0,35;2,2527 0,48;2,2359 0,33;2,0697 1,08;2,0495 0,49;2,0181 1,18;2,0128 1,16;1,983 2,02;1,9372 1,42;1,8569 0,62;1,837 1,15;1,828 1,15;1,7963 1,03;1,7664 0,66;1,7457 0,52;1,727 0,52;1,7011 0,81;1,6948 0,85;1,69 0,83;1,6676 1,37;1,6599 1,49;1,6313 2,06;1,6128 3,17;1,5951 4,47;1,5765 3,58;1,5585 1,76;1,536 0,76;1,4846 0,49;1,4672 0,47;1,3758 3,46;1,3536 3,49;1,3383 3,36;1,2923 6,99;1,2707 12,81;1,238 6,64;1,1123 0,38;1,0707 0,31;0,8803 5,34;0,864 15;0,8466 7,25;0,8215 1,95;0,804 0,9;0,1466 0,36;0,0305 0,4;0,0079 3,52;-0,0001 61,439999;-0,0083 2,9 |
| I-130 | [DMSO-d₆] 9,6025 0,45;7,7813 3,03;7,7624 4,02;7,7418 3,35;7,3129 2,67;7,2907 0,31;7,2833 0,32;7,2069 8,55;7,1882 12,69;7,1798 6,44;7,165 3,02;7,0464 3,29;7,0291 6,2;6,9634 0,41;6,9552 0,31;6,9157 6,3;6,8932 3,09;6,2886 0,33;5,7463 0,67;5,4306 14,4;5,389 0,78;3,7201 4,57;3,6828 4,72;3,6718 4,45;3,6115 15;3,5422 0,57;3,4923 0,54;3,4764 0,6;3,4286 0,83;3,3054 1108,430054;3,1645 0,45;3,1479 0,44;3,1233 0,3;3,0899 0,31;2,9257 4,7;2,9076 7,99;2,8892 4,89;2,6957 0,58;2,674 1,56;2,6693 1,87;2,6472 0,4;2,6429 0,38;2,6255 0,45;2,5936 0,69;2,5393 8,51;2,5046 199,830002;2,5004 250,910004;2,4964 181,169998;2,4062 0,54;2,3988 0,52;2,3579 0,39;2,3445 0,36;2,331 1,43;2,3273 1,81;2,3231 1,35;2,0691 1,44;2,05 0,74;1,9871 1,03;1,761 0,39;1,6097 0,98;1,5929 2,9;1,5742 4,54;1,5559 3,59;1,5374 1,43;1,4553 0,31;1,4219 0,4;1,3971 1,09;1,3669 3,29;1,3444 3,15;1,3306 2,99;1,2894 6,18;1,2662 11,51;1,1756 0,8;1,1586 0,88;1,109 0,31;1,0704 0,4;0,8793 5,51;0,8626 14,81;0,8451 6,44;-0,0001 8,31 |
| I-131 | [DMSO-d₆] 7,935 1,21;7,9159 3,38;7,8969 2,98;7,8797 2,58;7,8765 2,98;7,8605 1,31;7,8573 1,04;7,5468 1,9;7,5436 1,93;7,5278 1,77;7,5247 1,7;7,2873 1,38;7,1535 2,89;7,1114 1,55;7,0196 1,45;6,9815 0,38;6,9751 3,28;6,8686 0,34;6,8562 2,31;6,8457 0,4;6,8388 1,72;5,4078 0,45;5,3618 0,75;5,3387 0,75;5,0738 0,53;4,3828 2,07;4,365 6,05;4,3473 6,16;4,3296 2,02;4,0653 1,22;4,0475 3,53;4,0298 3,62;4,012 1,36;3,116 5,44;3,089 1,79;3,0795 1,02;3,0692 0,64;3,0597 0,73;3,0504 0,44;2,5113 0,33;2,4984 5,18;2,4937 10,27;2,489 14,17;2,4843 9,92;2,4796 4,78;1,9746 16;1,9351 0,88;1,3543 6,16;1,3366 12,71;1,3189 6,02;1,1955 4,41;1,1777 8,82;1,16 4,32;-0,0001 0,7 |
| I-132 | [DMSO-d₆] 7,9336 1,39;7,9145 3,84;7,8955 3,34;7,878 2,94;7,8748 3,37;7,8588 1,48;7,8556 1,17;7,5505 2,15;7,5474 2,15;7,5315 1,98;7,5285 1,9;6,4666 0,54;6,4507 3,37;5,2826 0,6;5,2367 0,8;5,2125 0,83;4,9763 1,54;4,3816 2,35;4,3638 7;4,3461 7,13;4,3284 2,34;4,0653 1,04;4,0476 2,8;4,0298 2,83;4,0121 1,11;3,9191 0,34;3,6401 0,37;3,6236 0,93;3,607 1,23;3,5906 0,95;3,5738 0,42;3,2438 0,41;3,1211 10,63;3,1025 4,7;3,0853 2,64;3,077 1,51;3,0662 0,98;3,0565 1,04;3,0472 0,68;2,8889 0,54;2,7351 0,4;2,7339 0,39;2,5123 0,42;2,5073 0,62;2,4994 6,88;2,4947 13,79;2,49 19,15;2,4853 13,46;2,4806 6,51;2,2594 2,17;2,2578 2,11;2,2364 16;2,235 15,43;2,209 0,97;2,2019 1,4;1,9747 12,22;1,9313 1,08;1,9032 0,48;1,8115 0,32;1,6561 0,32;1,353 7,01;1,3353 14,7;1,3175 7,6;1,3104 |
| | 4,69;1,3041 7,89;1,2921 8,28;1,2877 7,97;1,2738 2,87;1,1956 3,42;1,1778 6,63;1,1601 3,29 |
| I-133 | [DMSO-d₆] 8,0141 1,26;7,9945 2,68;7,975 1,61;7,9486 0,41;7,811 1,95;7,7923 1,75;7,7697 0,41;7,7558 0,35;7,6748 1,76;7,6551 1,58;7,6232 0,32;7,5071 0,49;7,3666 1,42;7,35 1,96;7,3462 1,57;7,3182 0,33;7,2847 1,16;7,2674 0,36;7,2621 0,37;7,2581 0,37;7,2431 0,37;7,1907 3,08;7,1858 3,26;7,1791 3,84;7,175 3,25;7,1689 2,65;7,1516 4,37;7,1464 2,95;7,1086 1,12;7,0983 0,81;7,0857 0,65;7,0663 0,55;7,0569 0,53;7,0409 0,5;7,0348 0,49;7,0185 1,4;7,0105 2,35;6,9927 0,37;6,9838 0,46;6,9408 0,41;6,8883 2,35;6,8745 1,41;6,8168 0,34;5,462 0,65;5,4201 1,45;5,3351 1,33;5,3058 0,42;5,294 0,65;4,4066 0,66;4,3736 0,6;4,2322 7,52;4,205 1,19;4,1646 0,64;4,145 0,33;4,1298 0,31;4,1145 0,32;4,0599 0,32;4,0037 0,68;3,9804 0,51;3,9705 0,7;3,8099 0,31;3,7728 0,38;3,7361 0,38;3,5109 0,33;3,4825 0,33;3,4709 0,37;3,4639 0,46;3,4592 0,43;3,4147 0,67;3,3774 1,35;3,3021 1350,109985;3,2427 2,19;3,2232 1,26;3,1767 0,71;3,1642 0,69;3,1451 0,55;3,1107 0,71;3,093 0,84;3,0843 1,04;3,0556 0,82;3,0372 1,02;3,0062 0,49;2,9834 0,38;2,951 0,32;2,9243 0,4;2,9023 0,32;2,8904 0,33;2,8497 0,61;2,8292 0,57;2,8206 0,48;2,797 0,85;2,7723 0,62;2,7408 0,33;2,6734 1,73;2,669 2,19;2,6645 1,68;2,6598 1,02;2,6422 0,41;2,6337 0,44;2,5389 4,55;2,5085 138,130005;2,5043 243,25;2,4999 304,350006;2,4956 209,149994;2,4057 0,73;2,3909 0,68;2,3784 0,73;2,3711 0,69;2,3365 15;2,3054 0,86;2,2874 0,87;2,263 1,45;2,2496 0,68;2,2316 0,5;2,2139 0,57;2,1897 0,81;2,1782 0,74;2,1484 0,38;2,1001 0,3;2,0692 1,06;2,0495 0,48;2,0087 0,4;1,9955 0,67;1,9866 1,17;1,9569 1,21;1,9234 0,9;1,915 0,86;1,8808 0,36;1,863 0,4;1,8421 0,56;1,8124 0,68;1,7819 0,56;1,7512 0,4;1,7448 0,35;1,637 0,55;1,6286 0,49;1,6066 0,7;1,5827 0,75;1,5546 0,42;1,5302 0,33;1,5175 0,32;1,2922 0,47;1,2369 1,05;1,1918 0,36;1,1755 0,49;1,158 0,37;0,1458 0,34;0,0079 4,28;-0,0001 78,559998;-0,0084 3,78;-0,1497 0,31 |
| I-134 | [DMSO-d₆] 7,9329 1,44;7,9137 4,02;7,8948 3,59;7,8783 3;7,875 3,53;7,8591 1,51;7,8558 1,18;7,5498 2,16;7,5466 2,2;7,5308 1,99;7,5277 1,93;6,4511 3,39;5,2797 0,62;5,2353 0,75;5,2076 0,76;4,3818 2,39;4,3641 7,41;4,3464 7,39;4,3286 2,44;4,0655 0,46;4,0477 1,14;4,03 1,17;4,0122 0,52;3,1211 70,699997;3,0948 0,89;3,0853 1,21;3,0757 0,7;3,0656 0,45;3,0559 0,64;3,0467 0,37;2,4992 5,25;2,4945 10,96;2,4898 15,57;2,4851 10,96;2,4804 5,33;2,2363 15,98;2,2348 16;2,2019 0,4;1,9748 4,82;1,9612 0,78;1,9484 0,78;1,9321 1;1,9028 0,4;1,3531 7,41;1,3354 15,56;1,3177 7,29;1,1957 1,29;1,1779 2,52;1,1602 1,28 |
| I-135 | [DMSO-d₆] 7,9292 3,08;7,9102 8,73;7,892 10,95;7,8876 9,26;7,8835 10,43;7,8684 3,35;7,8643 1,95;7,5328 5,88;7,5288 5,78;7,5147 5,28;7,5107 5,09;7,3779 0,54;7,2937 4,43;7,2448 1,11;7,1598 9,4;7,1235 0,85;7,1161 4,85;7,0259 4,72;6,9873 1,58;6,9797 10,3;6,8855 0,96;6,8624 7,73;6,8514 1,29;6,8434 5,22;5,4154 1,22;5,3619 3,41;5,3523 3,38;5,1052 3,1;4,426 0,73;4,0676 1,47;4,0499 3,67;4,0321 3,8;4,0143 1,73;3,9226 0,78;3,2536 0,71;3,1226 0,87;3,1133 1,47;3,1037 1,04;3,0936 1,75;3,0843 2,89;3,0748 1,69;3,0648 1,12;3,0552 1,6;3,0457 0,92;2,8208 0,69;2,5158 0,37;2,5032 4,18;2,4985 8,1;2,4938 11;2,4891 7,66;2,4845 3,71;1,9758 16;1,945 2,93;1,9072 13,84;1,8247 0,8;1,8126 0,84;1,7795 0,68;1,7449 0,77;1,7088 0,82;1,1963 3,83;1,1786 7,5;1,1608 3,71;-0,0001 0,92 |
| I-136 | [DMSO-d₆] 7,9269 1,21;7,9078 3,4;7,8893 3,75;7,8817 3,19;7,878 3,68;7,8625 1,31;7,8588 0,86;7,5358 2,1;7,5322 2,11;7,5175 1,9;7,5138 1,84;6,4688 0,53;6,4502 3,48;5,282 0,48;5,2281 1,26;5,2181 1,27;4,9845 1,89;4,0663 0,65;4,0485 1,57;4,0307 1,59;4,013 0,7;3,9197 0,34;3,1169 0,35;3,1075 0,58;3,0982 0,41;3,0881 0,67;3,0786 1,12;3,0691 0,68;3,0591 0,45;3,0495 0,65;3,0402 0,39;2,5007 2,64;2,496 5,24;2,4914 7,23;2,4867 5,1;2,482 2,51;2,2622 2,54;2,2607 2,47;2,238 16;2,2367 15,37;2,2118 0,42;2,2029 1,09;1,9749 6,63;1,966 0,97;1,9378 1,14;1,9118 0,45;1,9042 5,89;1,8117 0,32;1,1959 1,69;1,1781 3,32;1,1604 1,65;-0,0001 0,52 |
| I-137 | [DMSO-d₆] 7,7489 2,82;7,7304 3,88;7,7275 3,72;7,7091 3,42;7,5856 1,42;7,5817 1,52;7,567 2,82;7,5627 2,99;7,5473 1,65;7,5434 1,61;7,4682 0,72;7,4637 0,75;7,4544 0,97;7,4497 1,83;7,4348 1,69;7,4292 2,03;7,4247 1,21;7,4153 1,17;7,411 1,02;7,3665 5,81;7,3484 5,39;7,3083 2,49;7,2851 2,49;7,2715 2,83;7,2618 3,1;7,2585 3,42;7,2549 4,97;7,237 3,22;7,175 5,63;7,1613 4,04;7,1574 5,25;7,1358 4,58;7,0418 2,77;7,0257 6;6,9116 5,63;6,8899 3,07;5,7465 3,95;5,4185 13,64;5,4016 14,9;5,308 0,32;4,0576 0,48;4,0399 1,41;4,0221 1,43;4,0043 0,47;3,6975 3,69;3,6837 4,09;3,6481 4,16;3,6357 3,94;3,5821 15;3,3131 284,929993;3,2901 6,77;3,0382 0,63;2,8516 0,53;2,6704 0,35;2,5403 0,98;2,5099 21,950001;2,5058 37,91;2,5014 46,73;2,4972 32,07;2,328 0,32;1,9874 5,99;1,3976 1,03;1,2366 0,39;1,1932 1,67;1,1754 3,24;1,1576 1,62;0,0078 0,55;-0,0001 8,14;-0,0085 0,35 |
| I-138 | [DMSO-d₆] 7,7304 1,77;7,7119 2,34;7,7091 2,28;7,6907 2,08;7,5662 0,45;7,5494 0,98;7,5451 0,94;7,5283 1,81;7,5115 0,96;7,5073 1,09;7,4906 0,48;7,3141 3,72;7,3057 |
| | 1,81;7,2959 3,43;7,2105 0,55;7,2013 2,85;7,1813 4,62;7,1724 4,19;7,1609 4,17;7,1447 3,08;7,1231 2,78;7,0392 1,71;7,0247 3,68;6,9109 3,47;6,8888 1,84;5,7457 2,25;5,4129 15;4,0582 0,3;4,0403 0,9;4,0225 0,91;4,0047 0,31;3,6804 2,18;3,6671 2,64;3,638 2,69;3,6248 2,35;3,5672 10,91;3,3246 233,389999;2,5418 0,5;2,5114 11,3;2,5072 20,24;2,5029 25,620001;2,4986 17,93;1,9879 3,86;1,1937 1,06;1,1759 2,08;1,1581 1,04;-0,0001 3 |
| I-139 | [DMSO-d₆] 7,8288 3,1;7,8103 4,24;7,8075 4,16;7,789 3,93;7,5543 6,82;7,5361 6,12;7,3115 2,78;7,2404 5,38;7,2188 5,13;7,1956 2,47;7,1776 9,93;7,1617 3,84;7,1568 4,09;7,0449 7,54;7,0258 10,35;7,0053 4,7;6,9858 3,84;6,9113 6,56;6,8899 3,52;5,4244 14,9;4,0392 0,44;4,0215 0,45;3,7389 4,32;3,7309 4,3;3,6582 5,2;3,646 4,94;3,6252 6,54;3,6087 6,26;3,5312 0,33;3,5226 0,33;3,5148 0,31;3,4305 0,52;3,3034 1135,209961;3,1479 0,39;3,1283 0,34;3,0371 0,32;2,8503 0,3;2,7744 5,44;2,6733 1,44;2,669 1,91;2,6645 1,45;2,6329 0,44;2,6294 0,45;2,5388 8,36;2,5218 13,16;2,5085 108,489998;2,5044 192,050003;2,5 242,100006;2,4957 169,029999;2,3312 1,36;2,3267 1,74;2,3221 1,27;2,0845 4,99;2,069 1,38;1,9866 1,77;1,7166 12,6;1,3984 15;1,2365 0,68;1,1927 0,55;1,1748 0,97;1,1571 0,52;-0,0001 31,129999;-0,008 1,48 |
| I-140 | [DMSO-d₆] 7,7416 2,28;7,7228 3,26;7,7019 2,73;7,3436 4,97;7,3254 4,65;7,3102 2,16;7,1769 4,63;7,1626 2,34;7,1319 4,14;7,1103 3,94;7,0437 2,3;7,0266 4,86;6,9117 4,9;6,8908 2,49;5,4225 11,58;4,937 1,03;4,925 1,34;4,9157 1,94;4,9066 1,33;4,8941 1,04;4,8858 0,5;3,6884 3,74;3,6538 3,72;3,6417 3,56;3,5841 15;3,3052 1212,689941;3,3045 1226,640015;3,0378 0,32;2,6735 1,47;2,6694 1,9;2,6649 1,48;2,6454 0,38;2,5391 4,87;2,5046 205,119995;2,5005 257,420013;2,4964 185,020004;2,327 1,8;2,0844 0,44;2,0691 1,21;1,8665 2,07;1,8567 2,11;1,849 2,01;1,7332 1,91;1,7177 2,17;1,7106 2,11;1,5784 1,05;1,5561 2,53;1,5324 3,39;1,51 2,14;1,5016 2,07;1,4518 1,13;1,429 2,35;1,3987 12,57;1,3709 1,84;1,3377 1,14;1,3151 0,87;1,2367 0,78;0,0006 29,299999;-0,0001 30,09 |
| I-141 | [DMSO-d₆] 8,3834 0,31;8,0644 0,94;8,0555 3,24;8,0495 2,7;8,0423 2;8,0321 3,43;7,9289 4,85;7,9084 7,34;7,8952 3,34;7,8902 5,87;7,8719 4,72;7,8689 4,02;7,8505 3,96;7,7283 0,31;7,6749 7,17;7,657 6,15;7,628 1,96;7,6212 4,66;7,616 5,14;7,6109 7,43;7,6018 13,29;7,592 6,34;7,5867 3,95;7,5819 4,55;7,5744 1,17;7,492 5,61;7,4733 4,18;7,3341 0,32;7,3132 3,3;7,3032 5,51;7,2816 5,06;7,18 6,34;7,1614 3,03;7,0468 3,2;7,0253 6,85;6,9108 6,5;6,8894 3,42;5,4594 0,41;5,429 15;5,3057 0,37;4,3025 0,37;4,2841 0,32;4,0567 0,99;4,0391 2,67;4,0214 2,74;4,0034 1,02;3,902 0,32;3,8615 0,35;3,7858 4,7;3,6807 8,7;3,6312 5,22;3,5732 0,69;3,559 0,71;3,5312 0,71;3,5274 0,73;3,5137 0,83;3,4853 0,93;3,3061 2356,01001;3,1754 0,43;3,1679 0,37;3,1599 0,32;3,1472 0,36;3,037 0,86;2,8496 0,75;2,6738 2,4;2,6692 3,06;2,6647 2,25;2,6497 0,44;2,6144 0,63;2,5392 8,1;2,5085 189,410004;2,5045 330,869995;2,5002 411,950012;2,4959 284,890015;2,3315 2,39;2,3268 2,96;2,3225 2,15;2,0691 2,92;2,0491 0,4;1,9867 11,14;1,9078 1,33;1,4114 0,47;1,3984 5,95;1,3756 0,33;1,2532 0,37;1,2365 1,22;1,2182 0,31;1,1927 3,12;1,1749 6,08;1,1571 3,09;0,0078 3,17;-0,0001 60,380001;-0,0082 3,35 |
| I-142 | [DMSO-d₆] 7,7382 2,31;7,7197 3,21;7,7168 3,06;7,6985 2,78;7,3376 4,73;7,3196 4,58;7,3095 2,15;7,2888 0,44;7,1761 4,61;7,1622 2,32;7,148 0,32;7,1329 3,98;7,1114 3,73;7,0431 2,3;7,026 4,91;6,9509 0,41;6,9114 4,76;6,8902 2,45;6,0223 1,51;6,0056 1,2;5,9967 1,92;5,9896 1,06;5,804 1,67;5,7954 1,7;5,7783 1,3;5,7744 1,23;5,7699 1,28;5,4203 11,05;5,3846 1,89;5,3767 1,85;5,2495 0,98;3,6833 3,71;3,6539 3,8;3,5807 15;3,4644 0,56;3,307 1035,680054;3,1925 0,53;3,1679 0,4;3,1428 0,34;2,8896 0,36;2,7315 0,39;2,6736 1,18;2,6692 1,49;2,6649 1,15;2,5721 0,82;2,5393 9,38;2,5085 93,220001;2,5045 161,199997;2,5002 199,649994;2,496 138,639999;2,4342 0,77;2,4253 0,62;2,4161 0,6;2,4064 0,45;2,3798 0,35;2,3662 0,35;2,3491 0,34;2,3312 1,18;2,3268 1,47;2,3223 1,1;2,1415 0,43;2,1313 0,63;2,1226 0,62;2,0691 3,21;2,0493 1,31;2,0321 1,37;2,028 1,36;2,0195 1,17;1,9965 0,58;1,9864 0,67;1,9547 0,73;1,9383 0,79;1,929 1,23;1,9167 1,27;1,9069 1,13;1,8956 1,09;1,8843 0,77;1,8192 0,67;1,8097 0,92;1,7973 1,58;1,7905 1,47;1,7845 1,44;1,7773 1,82;1,7635 1,9;1,7497 1,59;1,7423 1,48;1,7265 1;1,6979 0,63; 1,6826 0,99; 1,6743 1,22;1,6619 1,2;1,6412 0,85;1,6285 0,66;1,5959 0,32;1,5799 0,32;1,2924 0,35;1,2368 0,35;0,89 0,33;-0,0001 5,26 |
| I-143 | [DMSO-d₆] 7,9082 0,3;7,7288 0,66;7,7239 0,51;7,7104 0,85;7,7073 0,88;7,7032 0,58;7,695 1,51;7,6891 0,89;7,6766 1,84;7,6737 1,83;7,6553 1,62;7,3543 0,56;7,3363 0,53;7,3136 0,4;7,3085 0,61;7,294 1,28;7,2818 0,31;7,2595 1,22;7,2408 2,18;7,2131 1,49;7,2065 1,08;7,1952 2,37;7,1751 3,5;7,1696 2,77;7,1606 4,46;7,1537 4,6;7,1379 2,02;7,1272 1,13;7,1203 1,3;7,1043 2,31;7,0868 1,4;7,0557 0,37;7,0476 0,62;7,0422 0,91;7,0275 2,31;7,0242 1,96;7,0178 3,09;6,9783 1,04;6,9566 1,14;6,9445 2,6;6,923 3,18;6,9159 |
| | 4,33;6,9079 4,7;6,8984 4,07;6,888 1,3;6,882 1,7;5,807 0,37;5,7961 0,34;5,782 0,34;5,7467 9,2;5,421 2,79;5,3834 6,12;5,0404 0,73;5,0238 0,8;5,015 0,91;4,9994 0,72;4,0572 0,41;4,0394 1,13;4,0216 1,14;4,004 0,41;3,7204 0,49;3,7094 0,53;3,6619 2,22;3,6038 3;3,5749 4,25;3,5134 3,22;3,4887 2,51;3,3033 152,5;3,1916 0,31;3,1465 0,86;2,9507 0,42;2,8902 1,23;2,811 0,66;2,7685 1,39;2,7592 1,19;2,7319 1,84;2,6909 0,63;2,6688 1,78;2,6642 1,89;2,6459 15;2,6258 1,39;2,6027 6,45;2,5392 3,9;2,5086 33,369999;2,5046 57,439999;2,5003 71,279999;2,4961 49,580002;2,4604 0,45;2,3314 0,32;2,327 0,45;2,3223 0,33;2,0692 0,45;2,0497 0,72;2,0141 1,32;1,9868 5,69;1,9568 1,15;1,9272 1,6;1,9081 1,96;1,9005 1,18;1,894 1,07;1,865 0,59;1,8379 0,46;1,8218 0,37;1,8082 0,33;1,5695 0,44;1,5587 0,56;1,5508 0,48;1,5279 0,5;1,398 0,74;1,2365 0,54;1,1928 1,29;1,175 2,48;1,1573 1,23;-0,0001 4,4 |
| I-144 | [DMSO-d₆] 19,3265 0,36;16,4666 0,36;16,4389 0,37;16,0506 0,36;11,5296 0,36;11,1847 0,5;10,5635 0,36;9,1659 1,85;9,1496 3,47;9,1331 1,73;8,8326 0,39;7,793 0,35;7,7628 3,39;7,7445 4,72;7,7415 4,37;7,7232 4,13;7,6439 0,55;7,6248 7,53;7,6195 7,65;7,4773 0,44;7,4657 0,38;7,4268 4,27;7,4216 4,08;7,4062 5,13;7,4009 4,86;7,3729 0,71;7,3539 7,13;7,336 6,31;7,3124 3,41;7,2925 6,88;7,2716 5,18;7,2406 0,71;7,213 0,38;7,179 6,74;7,1622 3,32;7,1099 5,58;7,0886 5,15;7,0461 3,25;7,026 7,26;6,9138 7,08;6,8902 3,87;5,9352 0,35;5,7466 1,25;5,4351 15;5,3047 0,52;5,0854 1,33;4,6341 0,37;4,5491 7,71;4,5331 7,38;4,5088 0,48;4,5029 0,41;4,3632 0,84;4,3501 0,77;4,2599 0,38;4,057 0,88;4,039 2,72;4,0212 2,72;4,0034 1,03;3,9441 0,41;3,9264 0,42;3,9022 0,47;3,8802 0,4;3,8612 0,45;3,7599 4,77;3,7067 1,44;3,6664 5,32;3,6554 5,72;3,5995 5,04;3,5466 0,79;3,5353 0,82;3,5029 0,85;3,4905 1,02;3,4853 1,07;3,4323 1,62;3,3049 2472,110107;3,1905 0,89;3,1462 0,71;3,1286 0,53;3,1233 0,53;3,071 0,36;3,0369 1,35;2,944 0,65;2,8896 0,45;2,8497 1,05;2,8159 0,37;2,7854 0,51;2,7514 0,46;2,7306 0,59;2,7206 0,43;2,7136 0,39;2,6931 0,86;2,6735 2,82;2,6689 3,72;2,6642 2,81;2,6483 0,72;2,6347 0,68;2,591 1,3;2,5391 22,059999;2,5086 214,75;2,5044 385,980011;2,5 491,359985;2,4957 341,299988;2,4197 1,16;2,4028 0,85;2,3634 0,58;2,3586 0,63;2,331 2,75;2,3265 3,43;2,3223 2,62;2,2918 0,42;2,2767 0,37;2,2558 0,38;2,2162 0,39;2,1878 0,37;2,1752 0,35;2,0692 1,74;2,0493 1,91;2,0096 0,42;1,9971 0,6;1,9866 11,35;1,9561 0,66;1,9076 0,83;1,3984 6,77;1,2922 2,42;1,2792 0,54;1,2382 1,64;1,2058 0,63;1,1927 3,35;1,1749 6,41;1,1572 3,71;1,1084 0,42;1,0906 0,46;1,0699 0,87;0,8904 1,45;0,8551 0,7;0,8388 0,5;0,8136 0,37;0,018 0,56;0,008 2,55;-0,0001 50,349998;-0,0085 2,57;-0,0212 0,45;-1,2287 0,36;-3,2941 0,36;-3,5272 0,41 |
| I-145 | [DMSO-d₆] 7,7926 1,35;7,7741 1,86;7,7716 1,79;7,753 1,57;7,3515 1,43;7,3356 1,67;7,3309 1,42;7,243 2,89;7,2249 3,02;7,2179 2,61;7,1963 2,77;7,1901 2,45;7,1868 2,78;7,1817 2,75;7,1747 3,11;7,1708 2,4;7,1647 1,74;7,1576 1,44;7,1477 1,31;7,1405 0,82;7,131 0,49;7,1242 0,31;6,4938 3,55;5,7472 5,82;5,2796 6,94;4,1924 9,01;4,0394 0,47;4,022 0,49;3,687 2,16;3,6568 2,23;3,587 8,98;3,2998 129,899994;2,6691 0,35;2,539 1,69;2,5044 40,240002;2,5001 49,77;2,4961 35,189999;2,3255 15;2,2035 13,54;1,987 1,96;1,3982 1,83;1,193 0,59;1,1751 1,1;1,1576 0,59;-0,0001 7,12 |
| I-146 | [DMSO-d₆] 9,1367 1,79;9,1209 3,4;9,1044 1,66;7,7785 0,32;7,7661 3,32;7,7477 4,39;7,7265 3,78;7,626 0,42;7,5737 0,32;7,5661 0,4;7,5589 0,33;7,5487 0,35;7,4716 2,41;7,4674 3,85;7,4531 2,9;7,4485 4,53;7,4315 0,36;7,3625 6,85;7,3444 6,53;7,3363 1,53;7,3224 3,3;7,3124 5,86;7,3057 7,82;7,301 9,84;7,2869 9,45;7,2732 2,92;7,2656 2,01;7,1938 0,37;7,179 6,27;7,162 3,01;7,1109 5,52;7,0895 5,09;7,0458 3,16;7,0258 6,6;6,9132 6,58;6,8899 3,34;5,7469 3,54;5,4349 15;5,3071 0,34;4,5854 7,86;4,5692 7,73;4,057 0,7;4,0394 1,87;4,0215 1,97;4,0041 0,67;3,8098 0,35;3,7641 4,57;3,6796 4,87;3,6661 5,35;3,6576 5,58;3,646 5,15;3,6064 4,82;3,5493 0,39;3,5275 0,37;3,4927 0,41;3,485 0,42;3,4627 0,47;3,4362 0,62;3,4222 0,65;3,3065 772,119995;3,2834 15,87;3,0368 0,79;2,8496 0,64;2,6695 1,2;2,6648 0,93;2,6167 0,3;2,5813 0,55;2,5391 3,12;2,5046 130,369995;2,5004 164,139999;2,4962 116,32;2,3275 1,18;2,0695 0,69;1,9869 7,74;1,9083 1,1;1,2367 0,81;1,1928 2,16;1,1751 4,05;1,1572 2,01;-0,0001 16,549999 |
| I-147 | [DMSO-d₆] 7,7269 0,44;7,7081 0,64;7,7055 0,65;7,6928 1,19;7,6869 0,64;7,6746 1,43;7,6716 1,33;7,6532 1,19;7,2592 0,91;7,2403 1,53;7,2237 0,37;7,2121 0,99;7,1943 1,55;7,1743 1,51;7,1694 1,33;7,1524 2,02;7,1382 1,15;7,1207 0,46;7,1053 1,44;7,0874 0,8;6,9763 0,68;6,9548 0,8;6,9425 1,72;6,9217 2,19;6,913 2,21;6,905 0,95;6,8949 1,94;6,4939 2,8;5,7468 0,64;5,3025 2,08;5,2659 5,03;5,0442 0,54;5,0275 0,61;5,0184 0,65;5,0036 0,51;4,0569 1,14;4,0391 3,32;4,0213 3,32;4,0035 1,16;3,663 1,69;3,6031 2,18;3,5771 2,71;3,5177 2,38;3,5059 2,09;3,4977 2,08;3,4695 0,58;3,4527 0,38;3,4399 0,31;3,3002 344,73999;2,8158 0,36;2,7699 0,87;2,7382 0,51;2,7276 0,53;2,6687 1,5;2,6642 1,43;2,6595 |
| | 1,32;2,6451 11,39;2,6027 4,84;2,5388 3,95;2,5083 46,98;2,5041 82,290001;2,4997 102,830002;2,4954 70,989998;2,4289 0,33;2,3352 0,38;2,3309 0,61;2,3267 0,74;2,3218 0,56;2,2151 4,29;2,2024 10,03;2,069 0,32;2,0493 0,71;2,0146 0,93;1,9866 15;1,9557 0,8;1,9263 1,1;1,9076 0,97;1,8995 0,82;1,8654 0,36;1,8349 0,3;1,5751 0,35;1,563 0,43;1,53 0,39;1,2373 0,3;1,1926 3,95;1,1749 7,88;1,1571 3,98;-0,0001 4,01 |
| I-148 | [DMSO-d₆] 7,7303 0,83;7,7118 1,18;7,6904 1,06;7,6718 1,43;7,653 1,88;7,6319 1,66;7,5134 1,21;7,4939 1,38;7,4514 1,67;7,4318 2,6;7,4239 2,02;7,4056 2,49;7,3845 3,84;7,3685 3,38;7,3541 2,71;7,3357 2,08;7,3083 1,12;7,2896 1,31;7,1748 1,75;7,1562 3,98;7,0413 0,95;7,0233 3,72;7,02 3,72;6,9985 1,53;6,977 1,49;6,9532 2,8;6,935 2,89;6,9105 6,6;6,8887 3,4;5,4188 4,06;5,3454 6,27;4,7453 4,59;4,6897 6,02;4,6611 0,65;4,0572 0,31;4,0395 0,85;4,0217 0,85;4,0038 0,31;3,6641 3,1;3,5839 3,98;3,4022 3,47;3,3251 1403,030029;3,1427 2,45;3,0373 0,68;2,9933 15;2,9694 10,12;2,9443 0,66;2,8908 2,22;2,8501 0,44;2,7321 1,72;2,675 0,79;2,6707 1,04;2,6579 0,71;2,5406 4,38;2,5102 55,810001;2,506 100,169998;2,5016 127,290001;2,4973 88,690002;2,3329 0,71;2,3282 0,94;2,1962 0,36;2,0686 0,9;2,0494 0,35;1,9869 3,61;1,9086 1,4;1,3981 0,8;1,2925 0,47;1,2363 0,75;1,193 1,01;1,1752 1,98;1,1575 1,04;-0,0001 3,35 |
| I-149 | [DMSO-d₆] 7,7989 1,61;7,7805 2,08;7,7776 2,07;7,7592 1,84;7,4416 0,38;7,4248 0,84;7,4207 0,8;7,4038 1,58;7,387 0,85;7,3828 0,97;7,3661 0,43;7,2491 3,32;7,2313 5,65;7,2098 2,53;7,1526 0,5;7,1432 2,54;7,1233 3,87;7,1031 2,07;7,0933 0,38;6,4966 4;5,7469 6,8;5,2869 7,85;4,2412 7,31;4,0398 0,68;4,0221 0,68;3,687 2,64;3,6645 2,69;3,5901 9,75;3,3094 232,960007;2,6704 0,32;2,5404 2,09;2,5098 19,540001;2,5057 34,619999;2,5013 43,700001;2,4971 30,610001;2,3276 0,32;2,2079 15;2,1925 0,62;1,9874 2,84;1,1933 0,82;1,1756 1,6;1,1578 0,78;-0,0001 1,12 |

Eine detaillierte Beschreibung der Darstellung von NMR-Daten in Form von Peaklisten kann der Publikation "Citation of NMR Peaklist Data within Patent Applications":
http://www.rdelectronic.co.uk/rd/free/RD 564025.pdf
entnommen werden.

Die chemischen NMR-Verschiebungen in ppm wurden bei 400 MHz falls nicht anders angegeben im Lösungsmittel DMSO-d₆mit Tetramethylsilan als internem Standard gemessen.

Folgende Abbkürzungen beschreiben die Signalaufspaltung:
b = Breite, s = Singulett, d = Dublett, t = Triplett, q = Quadruplett, m = Multiplett

### Verwendungsbeispiele

### Beispiel A

### Phytophthora-Test (Tomate) / protektiv

| | |
|---|---|
| Lösungsmittel: | 49 Gewichtsteile N, N - Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Tomatenpflanzen mit der Wirkstoffzubereitung in der angegebenen Aufwandmenge besprüht. 1 Tag nach der Behandlung werden die Pflanzen mit einer Sporensuspension von Phytophthora infestans inokuliert und stehen dann 24h bei 100 rel. Feuchte und 22°C. Anschließend werden die Pflanzen in einer Klimazelle bei ca. 96% relativer Luftfeuchtigkeit und einer Temperatur von ca. 20°C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigen die nachfolgenden erfindungsgemäßen Verbindungen bei einer Konzentration an Wirkstoff von 500ppm einen Wirkungsgrad von 70% oder mehr. Im Einzelnen zeigten folgende Verbindungen aus Tabelle I einen Wirkungsgrad von 70% und mehr, wobei für ausgewählte Beispiele der genaue Wirkungsgrad in runden Klammern aufgeführt wird:
I-1, I-3 (88%), I-7, I-8, I-9, I-12, I-23, I-24, I-32, I-33, I-34, I-35, I-38, I-39, I-40, I-43, I-45, I-46, I-47, I-48, I-49, I-50, I-51, I-52, I-57, I-60, I-63, I-68 (94%), I-69 (71%), I-70 (97%), I-71 (76%), I-73 (71%), I-75 (71%), I-79, I-89, I-97, I-98, I-99, I-100, I-101, I-102, I-103, I-104, I-105, I-106, I-107,1-108,1-109,1-110,1-111,1-115 I-116, I-117 (93%), I-118 (93%), I-120 (93%), I-123 (93%), I-137 (93%), I-138 (93%), I-140 (93%), I-141 (71%), I-142 (83%), I-143 (71%).

### Plasmopara-Test (Rebe) / protektiv

| | |
|---|---|
| Lösungsmittel: | 24,5 Gewichtsteile Aceton |
| | 24,5 Gewichtsteile Dimethylacetamid |
| Emulgator : | 1 Gewichtsteil Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100% relativer Luft¬feuchtigkeit. Anschließend werden die Pflanzen 4 Tage im Gewächshaus bei ca. 21°C und ca. 90% Luftfeuchtigkeit auf¬gestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

In diesem Test zeigen die nachfolgenden erfindungsgemäßen Verbindungen bei einer Konzentration an Wirkstoff von 100ppm einen Wirkungsgrad von 70% oder mehr. Im Einzelnen zeigten folgende Verbindungen aus Tabelle I einen Wirkungsgrad von 70% und mehr, wobei für ausgewählte Beispiele der genaue Wirkungsgrad in runden Klammern aufgeführt wird:

I-7, I-9, I-33, I-34, I-35, I-43, I-45, I-47, I-48, I-52, I-97, I-98, I-99, I-100, I-102, I-105, I-106, I-107, I-108, I-111, I-115, I-117 (93%), I-140 (71%).

## Patentansprüche

1. Verbindungen der Formel (I), 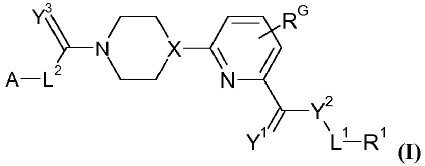 in welcher die Restedefinitionen folgende Bedeutungen haben:
A steht für Phenyl, das bis zu drei Substituenten enthalten kann,
wobei die Substituenten unabhängig voneinander ausgewählt aus Z^{A-1} sind,
oder
A steht für ein gegebenenfalls benzokondensiertes, unsubstituiertes oder substituiertes 5-oder 6-gliedriges Heteroaryl, wobei die Substituenten am Kohlenstoff unabhängig voneinander ausgewählt aus Z^{A-2} sind und die Substituenten am Stickstoff unabhängig voneinander ausgewählt sind aus Z^{A-3},
L¹ steht für (C(R^{L1})₂)ₚ,
p steht für 1, 2 oder 3,
R^{L1} ist gleich oder verschieden unabhängig voneinander Wasserstoff, Halogen, C₁-C₄-Alkyl, C₂-C₄-Alkenyl, C₂-C₄-Alkinyl, C₁-C₄-Alkoxy, C₃-C₆-Cycloalkyl, C₁-C₄-Halogenalkyl oder Phenyl
unter der Voraussetzung, dass L¹ maximal zwei R^{L1} enthalten kann, die von Wasserstoff verschieden sind,
L² steht für NR^{L21} oder C(R^{L22})₂,
R^{L21} steht für Wasserstoff, C₁-C₄-Alkyl, C₂-C₄-Alkenyl, C₂-C₄-Alkinyl, C₁-C₄-Halogenalkyl, C₂-C₄-Halogenalkenyl, C₂-C₄-Halogenalkinyl, C₂-C₄-Alkoxyalkyl, C₂-C₄-Alkylthioalkyl, C₂-C₄-Alkylsulfinylalkyl, C₂-C₄-Alkylsulfonylalkyl, C₂-C₄-Alkylcarbonyl, C₂-C₄-Halogenalkylcarbonyl, C₂-C₅-Alkoxycarbonyl, C₃-C₅-Alkoxycarbonylalkyl, C₂-C₅-Alkylaminocarbonyl, C₃-C₅-Dialkylaminocarbonyl, C₁-C₄-Alkylsulfonyl oder C₁-C₄-Halogenalkylsulfonyl,
R^{L22} steht gleich oder verschieden unabhängig voneinander für Wasserstoff, C₁-C₄-Alkyl, C₁-C₄-Halogenalkyl, Cyclopropyl, Halogen,
oder die beiden Reste R^{L22} bilden zusammen mit dem Kohlenstoffatom, an das sie gebunden sind, einen Cyclopropylring,
Y¹ und Y³ stehen gleich oder verschieden unabhängig voneinander für Schwefel oder Sauerstoff,
Y² steht für -(NR^{Y2})-, Schwefel oder Sauerstoff,
R^{Y2} steht für Wasserstoff, C₁-C₆-Alkyl, C₂-C₆-Alkenyl, C₂-C₆-Alkinyl, C₁-C₆-Halogenalkyl, C₃-C₆-Cycloalkyl, C₃-C₆-Halogencycloalkyl, Benzyl, Phenyl, NR³R⁴, C₁-C₄-Alkoxy, C₁-C₄-Halogenalkoxy, Phenoxy oder Benzyloxy,
oder
die Reste R^{Y2}, L¹ und R¹ bilden zusammen mit dem Stickstoffatom von Y² ein 5 bis 15-gliedrigen, unsubstituierten oder substituierten, gesättigten oder teilweise gesättigten oder ungesättigten mono-, bi- oder tricyclisches Ringsystem, der bis zu zwei weitere Heteroatome, ausgewählt aus N, O und S enthalten kann, wobei zwei Sauerstoffatome nicht benachbart sind, wobei mögliche Substituenten am Kohlenstoff unabhängig voneinander sind ausgewählt aus Z^{Y-1} und die Substituenten am Stickstoff unabhängig voneinander sind ausgewählt aus Z^{Y-2},
R³ und R⁴ stehen gleich oder verschieden unabhängig voneinander für Wasserstoff, C₁-C₄-Alkyl, C₃-C₆-Alkenyl, C₃-C₆-Alkinyl, C₁-C₃-Halogenalkyl, C₃-C₆-Cycloalkyl, Benzyl oder Phenyl,
X steht für -CR^{X1-} oder Stickstoff,
R^{X1} steht für Wasserstoff, Halogen, Cyano, Hydroxy, C₁-C₄-Alkyl, C₂-C₄-Alkenyl, C₂-C₄-Alkinyl, C₁-C₄-Halogenalkyl, C₁-C₄-Alkoxy, C₁-C₄-Halogenalkoxy, C₂-C₄-Carbonylalkoxy, OC(=O)H, C(=O)H, C(=O)OH, C₂-C₄-Alkoxycarbonyl oder C₁-C₃-Alkylcarbonyl,
R^{G} steht für Wasserstoff, Halogen oder C₁-C₃-Alkyl,
R¹ steht für Wasserstoff, C₁-C₆-Alkyl, C₁-C₆-Halogenalkyl, C₂-C₆-Alkenyl, C₂-C₆-Alkinyl, C₂-C₈-Alkoxyalkyl oder C₅-C₉-Cycloalkoxyalkyl,
R¹ steht für unsubstituiertes oder substituiertes C₃-C₁₀-Cycloalkyl,
wobei die Substituenten unabhängig voneinander ausgewählt sind aus -Q oder aus Z¹,
oder
R¹ steht für unsubstituiertes oder substituiertes C₅-C₁₀-Cycloalkenyl,
wobei die Substituenten unabhängig voneinander ausgewählt sind aus Z²,
oder
R¹ steht für substituiertes Phenyl,
wobei die Substituenten unabhängig voneinander ausgewählt sind aus -L³Q oder aus Z³
oder
R¹ steht für unsubstituiertes oder substituiertes, Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-1-yl, 5,6,7,8-Tetrahydronaphthalen-2-yl, Decalin-1-yl, Decalin-2-yl, 1H-Inden-1-yl, 2,3-Dihydro-1H-inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl,
wobei die Substituenten unabhängig voneinander ausgewählt sind aus Z⁴,
oder
R¹ steht für einen unsubstituierten oder substituierten 5- oder 6-gliedrigen Heteroarylrest, wobei die Substituenten am Kohlenstoff unabhängig voneinander ausgewählt sind aus - L³Q oder aus Z⁵ und die Substituenten am Stickstoff unanhängig voneinander ausgewählt sind aus Z⁶,
oder
R¹ steht für benzokondensiertes unsubstituiertes oder substituiertes 5- oder 6- gliedriges Heteroaryl, wobei die Substituenten am Kohlenstoff unabhängig voneinander ausgewählt sind aus Z⁷ sind und die Substituenten am Stickstoff unanhängig voneinander ausgewählt aus Z⁸ sind,
oder
R¹ steht für unsubstituiertes oder substituiertes C₅-C₁₅-Heterocyclyl, wobei die Substituenten am Kohlenstoff unabhängig voneinander ausgewählt sind aus Z⁹ sind und die Substituenten am Stickstoff unanhängig voneinander ausgewählt sind aus Z¹⁰,
³ steht für eine direkte Bindung, -O, -C(=O), -S(O)ₘ, -CHR^{L31} oder -NR^{L32},
m steht für 0, 1 oder 2,
R^{L31} steht für Wasserstoff, C₁-C₄-Alkyl oder C₁-C₄-Halogenalkyl,
R^{L32} steht für Wasserstoff, C₁-C₆-Alkyl, C₁-C₆-Halogenalkyl, C₃-C₈-Cycloalkyl, C₂-C₆-Alkylcarbonyl, C₂-C₆-Halogenalkylcarbonyl, C₂-C₆-Alkoxycarbonyl oder C₂-C₆-Halogenalkoxycarbonyl,
Q steht für ein Phenyl, das bis zu zwei Substituenten enthalten kann, wobei die Substituenten gleich oder verschieden unabhängig voneinander ausgewählt sind aus folgender Liste:
Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Phenyl,
oder
Q steht für ein 5- oder 6-gliedrigen Heteroarylrest, das bis zu zwei Substituenten enthalten kann, wobei die Substituenten gleich oder verschieden unabhängig voneinander ausgewählt sind aus folgender Liste:
Substituenten am Kohlenstoff: Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl, C₁-C₄-Halogenalkylsulfonyl oder Phenyl,
Substituenten am Stickstoff: C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₁-C₄-Alkylsulfonyl, C(=O)H, C(=O)Me, C(=O)OMe oder Phenyl,
Z^{A-1} und Z³ stehen gleich oder verschieden unabhängig voneinander für Halogen, Cyano, Hydroxy, SH, Amino, Nitro, C(=O)H, C(=O)OH, CONR³R⁴, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₈-Cycloalkyl, C₃-C₈-Halogencycloalkyl, C₄-C₁₀-Alkylcycloalkyl, C₄-C₁₀-Cycloalkylalkyl, C₆-C₁₄-Cycloalkylcycloalkyl, C₄-C₁₀-Halogencycloalkylalkyl, C₅-C₁₀-Alkylcycloalkylalkyl, C₃-C₈-Cycloalkenyl, C₃-C₈-Halogencycloalkenyl, C₂-C₆-Alkoxyalkyl, C₄-C₁₀-Cycloalkoxyalkyl, C₃-C₈-Alkoxyalkoxyalkyl, C₂-C₆-Alkylthioalkyl, C₂-C₆-Alkylsulfinylalkyl, C₂-C₆-Alkylsulfonylalkyl, C₂-C₆-Alkylaminoalkyl, C₃-C₈-Dialkylaminoalkyl, C₂-C₆-Halogenalkylaminoalkyl, C₄-C₁₀-Cycloalkylaminoalkyl, C₂-C₆-Alkylcarbonyl, C₂-C₆-Halogenalkylcarbonyl, C₄-C₈-Cycloalkylcarbonyl, C₂-C₆-Alkoxycarbonyl, C₄-C₈-Cycloalkoxycarbonyl, C₅-C₁₀-Cycloalkylalkoxycarbonyl, C₂-C₆-Alkylaminocarbonyl, C₃-C₅-Dialkylaminocarbonyl, C₄-C₈-Cycloalkylaminocarbonyl, C₂-C₆-Halogenalkoxyalkyl, C₁-C₆-Hydroxyalkyl, C₁-C₆-Alkoxy, C₁-C₆-Halogenalkoxy, C₃-C₈-Cycloalkoxy, C₃-C₈-Halogencycloalkoxy, C₄-C₁₀-cloalkylalkoxy, C₃-C₆-Alkenyloxy, C₃-C₆-Halogenalkenyloxy, C₃-C₆-Alkinyloxy, C₃-C₆-Halogenalkinyloxy, C₂-C₆-Alkoxyalkoxy, C₂-C₆-Alkylcarbonyloxy, C₂-C₆-Halogenalkylcarbonyloxy, C₄-C₈-Cycloalkylcarbonyloxy, C₃-C₆-Alkylcarbonylalkoxy, C₁-C₆-Alkylthio, C₁-C₆-Halogenalkylthio, C₃-C₆-Cycloalkylthio, C₁-C₆-Alkylsulfinyl, C₁-C₆-Halogenalkylsulfinyl, C₁-C₆-Alkylsulfonyl, C₁-C₆-Halogenalkylsulfonyl, C₃-C₈-Cycloalkylsulfonyl, Tri(C₁-C₄-alkyl)silyl, C₁-C₆-Alkylsulfonylamino oder C₁-C₆-Halogenalkylsulfonylamino oder SF₅,
Z¹ und Z² stehen gleich oder verschieden unabhängig voneinander für Cyano, Halogen, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Hydroxy, Oxo, C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio, C₂-C₄-Alkylcarbonyl, oder C₂-C₆-Alkylcarbonyloxy,
Z⁴ und Z⁷ stehen gleich oder verschieden unabhängig voneinander für Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₅-Dialkylaminocarbonyl, C₁-C₄-Hydroxyalkyl, C₁-C₄-Alkoxy, C₁-C₄-Halogenalkoxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
Z^{A-2} und Z⁵ stehen gleich oder verschieden unabhängig voneinander für Halogen, Cyano, Hydroxy, SH, Amino, Nitro, NR³R⁴, C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, C₆-C₁₄-Cycloalkylcycloalkyl, C₅-C₁₀-Alkylcycloalkylalkyl, C₂-C₄-Alkoxyalkyl, C₂-C₄-Alkylcarbonyl, C₂-C₆-Alkoxycarbonyl, C₂-C₆-Alkylaminocarbonyl, C₃-C₈-Dialkylaminocarbonyl, C₁-C₄-Hydroxyalkyl, C₁-C₄-Alkoxy, C₁-C₄-Halogenalkoxy, C₃-C₆-Alkenyloxy, C₃-C₆-Alkinyloxy, C₂-C₆-Alkylcarbonyloxy, C₂-C₆-Alkylcarbonylthio, C₁-C₄-Alkylthio, C₁-C₄-Halogenalkylthio, C₁-C₄-Alkylsulfinyl, C₁-C₄-Halogenalkylsulfinyl, C₁-C₄-Alkylsulfonyl oder C₁-C₄-Halogenalkylsulfonyl,
Z^{A-3}, Z^{Y-2}, Z⁶, Z⁸ und Z¹⁰ stehen gleich oder verschieden unabhängig voneinander für C₁-C₆-Alkyl, C₂-C₆-Alkenyl, C₂-C₆-Alkinyl, C₁-C₆-Halogenalkyl, C₂-C₆-Halogenalkenyl, C₂-C₆-Halogenalkinyl, C₃-C₆-Cycloalkyl, C₃-C₆-Halogencycloalkyl, C₄-C₁₀-Alkylcycloalkyl, C₄-C₁₀-Cycloalkylalkyl, Phenyl, Benzyl, C₁-C₄-Alkylsulfonyl, C(=O)H, C₂-C₄-Alkylcarbonylalkoxy oder C₁-C₃-Alkylcarbonyl,
Z^{Y-1} und Z⁹ stehen gleich oder verschieden unabhängig voneinander für Hydroxy, Cyano, Halogen, SH, Amino, Nitro, Oxo, NR³R⁴, C₁-C₆-Alkyl, C₁-C₆-Halogenalkyl, C₃-C₆-Cycloalkyl, C₂-C₆-Alkenyl, C₂-C₆-Halogenalkenyl, C₂-C₆-Alkinyl, C₂-C₆-Halogenalkinyl, Phenyl, , C₁-C₆-Alkoxy, C₁-C₆-Halogenalkoxy, C₂-C₆-Alkenyloxy, C₂-C₆-Alkinyloxy, C₁-C₆-Alkylthio oder C₁-C₆-Halogenalkylthio, C₂-C₄-Alkoxyalkyl, C₂-C₄-Alkylcarbonyl, C₂-C₆-Alkoxycarbonyl, C₂-C₆-Alkylaminocarbonyl, C₃-C₅-Dialkylaminocarbonyl, C₁-C₄-Hydroxyalkyl, C₂-C₆-Alkylcarbonyloxy, C₂-C₆-Alkylcarbonylthio, C₁-C₄-Alkylsulfinyl, C₁-C₄-Halogenalkylsulfinyl, C₁-C₄-Alkylsulfonyl oder C₁-C₄-Halogenalkylsulfonyl, sowie deren Salze, Metallkomplexe und N-Oxide.

2. Verbindungen der Formel (I) nach Anspruch 1, in welcher die Restedefinition folgende Bedeutung haben:
A steht für Phenyl, das bis zu zwei Substituenten enthalten kann, wobei die Substituenten unabhängig voneinander ausgewählt sind aus folgender Liste: Fluor, Brom, Iod, Chlor, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1 -Methylethoxy, Propoxy, Methoxy, Trifluormethoxy, Difluormethoxy, 1-Methylethylthio, Methylthio, Ethylthio, Propylthio, Difluormethylthio oder Trifluormethylthio, oder
A steht für einen heteroaromatischen Rest ausgewählt aus folgender Gruppe: Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,3-Triazol-1-yl, 1,2,4-Triazol-1-yl, Pyridin-2-yl, Pyridin-3-yl, Pyridin-4-yl, Pyridazin-3-yl, Pyridazin-4-yl, Pyrimidin-2-yl, Pyrimidin-4-yl oder Pyrimidin-5-yl, der bis zu zwei Substituenten enthalten kann, wobei die Substituenten gleich oder verschieden unabhängig voneinander ausgewählt sind aus folgender Liste:
Substituenten am Kohlenstoff:
Fluor, Chlor, Brom, Iod, Cyano, Nitro, Methyl, Ethyl, Propyl, 1-Methylethyl, 1,1-Dimethylethyl, Chlorfluormethyl, Dichlormethyl, Dichlorfluormethyl, Difluormethyl, Trichlormethyl, Trifluormethyl, Cyclopropyl, Ethoxy, 1-Methylethoxy, Propoxy, Methoxy, Trifluormethoxy, Difluormethoxy, 1-Methylethylthio, Methylthio, Ethylthio, Propylthio, Difluormethylthio Trifluormethylthio oder Phenyl,
Substituenten am Stickstoff:
Methyl, Ethyl, Propyl, 1-Methylethyl, 2,2-Trifluorethyl, 2,2-Difluorethyl, 2,2-Dichlor-2-fluorethyl, 2-Chlor-2-difluorethyl oder 2-Chlor-2-fluorethyl,
L ² steht für CH₂
Y¹ steht für Sauerstoff,
R^{Y2} steht für Wasserstoff, Methyl, Ethyl, Propyl, 1-Methylethyl, Prop-2-enyl, 1-Mehylprop-2-enyl, Ethinyl, Prop-2-inyl, 2,2,2-Trifluorethyl, Cyclopropyl, 1-Chlorcyclopropyl, Benzyl oder Phenyl, oder
die Reste R^{Y2}, L¹ und R¹ bilden zusammen mit dem Stickstoffatom von Y², Piperidin, Morpholin, Thiomorpholin, 2,3-Dihydro-4H-1,4-oxazin, 2,3-Dihydro-4H-1,4-benzoxazin, oder 1,2,3,4-Tetrahydrochinolin,
X steht für -CH-, -CF- oder Stickstoff,
R^{G} steht für Wasserstoff,
R¹ steht für Wasserstoff, 1,1-Dimethylethyl, 3,3-Dimethylbutyl, 2,2-Dimethylpropyl, 1,2,2-Trimethylpropyl, Pentyl, 1-Ethylpropyl, Butyl, 2-Methylpropyl, 1-Methylethyl, Ethyl, Propyl, 4-Methylpentyl, Hexyl, Trifluoromethyl, Methoxymethyl, Ethoxymethyl, Ethenyl, Prop-2-en-1-yl oder But-3-en-1-yl, oder
R¹ steht für Cyclopentyl, Cyclohexyl, Cycloheptyl oder Cyclooctyl, welche jeweils bis zu zwei Substituenten enthalten kann, wobei die Substituenten unabhängig voneinander ausgewählt sind aus folgender Liste:
Cyano, Chlor, Fluor, Brom, Iod, Methyl, Ethyl, Propyl, 1-Methylethyl, 1,1-Dimethylethyl, Ethenyl, 2-Propenyl, 2-Propinyloxy, Phenyl, Methoxy, Ethoxy, Propyloxy, Trifluormethoxy, Ethinyl, 2-Propinyloxy, Methylthio, Ethylthio oder Trifluomethylthio, oder
R¹ steht für Cyclopentenyl, Cyclohexenyl oder Cycloheptenyl, welche jeweils bis zu zwei Substituenten enthalten kann, wobei die Substituenten unabhängig voneinander ausgewählt sind aus folgender Liste: Methyl, Ethyl, Methoxy, Ethoxy, Trifluormethoxy, Ethinyl, 2-Propinyloxy, Methylthio, Ethylthio oder Trifluormethylthio, oder
R¹ steht für Phenyl, das bis zu drei Substituenten enthält, wobei die Substituenten unabhängig voneinander ausgewählt sind aus folgender Liste:
Fluor, Chlor, Brom, Iod, Cyano, Nitro, Methyl, Ethyl, Propyl, 1-Methylethyl, Butyl, 1,1-Dimethylethyl, 1,2-Dimethylethyl, Ethenyl, Ethinyl, Trifluoromethyl, Difluormethyl, Trichlormethyl, Dichlormethyl, Cyclopropyl, Methoxy, Ethoxy, Propoxy, 1-Methylethoxyl, 1,1 -Dimethylethoxyl, Methylcarbonyl, Ethylcarbonyl, Trifluormethylcarbonyl, Methoxycarbonyl, Ethoxycarbonyl, Propoxycarbonyl, 1-Methylethoxycarbonyl, 1,1 -Dimethylethoxycarbonyl, 2-Propinyloxy, Methylthio, Ethylthio, Methylsulfinyl oder Methylsulfonyl oder -L³Q, oder
R¹ steht für Naphthalen-1-yl, Naphthalen-2-yl, 1,2,3,4-Tetrahydronaphthalen-1-yl, 1,2,3,4-Tetrahydronaphthalen-2-yl, 5,6,7,8-Tetrahydronaphthalen-l-yl, 5,6,7,8-Tetrahydronaphthalen-2-yl, Decalin-1-yl, Decalin-2-yl, Dihydro-1H-inden-1-yl, 1H-Inden-1-yl, 1H-Inden-2-yl, 1H-Inden-3-yl, 1H-Inden-4-yl, 1H-Inden-5-yl, 1H-Inden-6-yl, 1H-Inden-7-yl, Indan-1-yl, Indan-2-yl, Indan-3-yl, Indan-4-yl oder Indan-5-yl, das bis zu drei Substituenten enthalten kann, wobei die Substituenten unabhängig voneinander aus folgender Liste ausgewählt sind: Methyl, Methoxy, Cyano, Fluor, Chlor, Brom, Iod, oder
R¹ steht für Furan-2-yl, Furan-3-yl, Thiophen-2-yl, Thiophen-3-yl, Isoxazol-3-yl, Isoxazol-4-yl, Isoxazol-5-yl, Pyrrol-1-yl, Pyrrol-2-yl, Pyrrol-3-yl, Oxazol-2-yl, Oxazol-4-yl, Oxazol-5-yl, Thiazol-2-yl, Thiazol-4-yl, Thiazol-5-yl, Isothiazol-3-yl, Isothiazol-4-yl, Isothiazol-5-yl, Pyrazol-1-yl, Pyrazol-3-yl, Pyrazol-4-yl, Imidazol-1-yl, Imidazol-2-yl, Imidazol-4-yl, 1,2,4-Oxadiazol-3-yl, 1,2,4-Oxadiazol-5-yl, 1,3,4-Oxadiazol-2-yl, 1,2,4-Thiadiazol-3-yl, 1,2,4-Thiadiazol-5-yl, 1,3,4-Thiadiazol-2-yl, 1,2,3-Triazol-1-yl, 1,2,3-Triazol-2-yl, 1,2,3-Triazol-4-yl, 1,2,4-Triazol-1-yl, 1,2,4-Triazol-3-yl, 1,2,4-Triazol-4-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl oder Pyrazin-2-yl, welche jeweils bis zu zwei Substituenten enthalten können, wobei die Substituenten unabhängig voneinander aus folgender Liste ausgewählt sind:
Substituenten am Kohlenstoff: Chlor, Fluor, Brom, Iod, Cyano, Nitro, Methyl, Ethyl, Propyl, 1-Methylethyl, Butyl, 1,1-Dimethylethyl, Ethenyl, Ethinyl, Trifluormethyl, Difluormethyl, Cyclopropyl, Cyclopentyl, Cyclohexyl, Methylcarbonyl, Ethylcarbonyl, Methoxylcarbonyl, Ethoxycarbonyl, Methoxy, Ethoxy, Propoxy, 1-Methylethoxy, 2-Propinyoxy, Trifluormethoxyl, Methylcabonyloxy, Methylcarbonylthio, Methylthio, Ethylthio, Trifluomethylthio, Methylsulfinyl, Ethylsulfinyl, Trifluoromethylsulfinyl, Methylsulfonyl, Ethylsulfonyl, oder Trifluormethylsulfonyl,
Substituenten am Stickstoff: Methyl, Ethyl, Propyl, Cyclopropyl, Cyclohexyl, Phenyl oder 2-Propinyl, oder
R¹ steht für Indol-1-yl, Indol-2-yl, Indol-3-yl, Indol-4-yl, Indol-5-yl, Indol-6-yl, Indol-7-yl, Benzimidazol-1-yl, Benzimidazol-2-yl, Benzimidazol-4-yl, Benzimidazol-5-yl, Indazol-1-yl, Indazol-3-yl, Indazol-4-yl, Indazol-5-yl, Indazol-6-yl, Indazol-7-yl, Indazol-2-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, l,3-Benzothiazol-2-yl, l,3-Benzothiazol-4-yl, 1l,3-Benzothiazol-5-yl, 1,3-Benzothiazol-6-yl, 1,3-Benzothiazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, Chinolin-2-yl, Chinolin-3-yl, Chinolin-4-yl, Chinolin-5-yl, Chinolin-6-yl, Chinolin-7-yl, Chinolin-8-yl, Isochinolin-1-yl, Isochinolin-3-yl, Isochinolin-4-yl, Isochinolin-5-yl, Isochinolin-6-yl, Isochinolin-7-yl oder Isochinolin-8-yl, welche jeweils bis zu zwei Substituenten enthalten können, wobei die Substituenten unabhängig voneinander aus folgender Liste ausgewählt sind:
Substituenten am Kohlenstoff: Fluor, Chlor, Brom, Iod, Methyl, Methoxy, 2-Propinyloxy, 2- Propenyloxy,
Substituenten am Stickstoff: Methyl, Ethyl, Propyl, Cyclopropyl, Cyclohexyl, Phenyl oder 2-Propinyl, oder
R¹ steht für Piperidin-1-yl, Piperidin-2-yl, Piperidin-3-yl, Piperidin-4-yl, Piperazin-1-yl, Piperazin-2-yl, Piperazin-3-yl, Morpholin-1-yl, Morpholin-2-yl, Morpholin-3-yl, Tetrahydropyran-2-yl, Tetrahydropyran-3-yl, Tetrahydropyran-4-yl, 1,2,3,4-Tetrahydrochinolin-1-yl, 1,2,3,4-Tetrahydroisochinolin-2-yl, 1,2,3,4-Tetrahydrochinoxalin-1-yl, Indolin-1-yl, Isoindolin-2-yl, Decahydrochinolin-1-yl oder Decahydroisochinolin-2-yl, welche jeweils bis zu zwei Substituenten enthalten kann, wobei die Substituenten unabhängig voneinander ausgewählt sind aus folgender Liste:
Substituenten am Kohlenstoff: Fluor, Chlor, Brom, Iod, Methyl, Methoxy, 2-Propinyloxy, 2- Propenyloxy,
Substituenten am Stickstoff: Methyl, Ethyl, Propyl, Cyclopropyl, Cyclohexyl, Phenyl oder 2-Propinyl,
Q steht für Phenyl.

3. Verfahren zur Bekämpfung von pflanzenpathogenen Schadpilzen, **dadurch gekennzeichnet, dass** man Verbindungen der Formel (I) gemäß einem der Ansprüche 1 oder 2, auf die pflanzenpathogenen Schadpilze und/oder deren Lebensraum ausbringt.

4. Mittel zur Bekämpfung von pflanzenpathogenen Schadpilzen, **gekennzeichnet durch** einen Gehalt an mindestens einer Verbindung der Formel (I) gemäß einem der Ansprüche 1 oder 2, neben Streckmitteln und/oder oberflächenaktiven Stoffen.

5. Verwendung von mindestens einer Verbindung der Formel (I), gemäß einem der Ansprüche 1 oder 2 bzw. eines Mittels gemäß Anspruch 4 zur Bekämpfung von pflanzenpathogenen Schadpilzen.

6. Verfahren zur Herstellung von Mitteln zur Bekämpfung von pflanzenpathogenen Schadpilzen gemäß Anspruch 4, **dadurch gekennzeichnet, dass** Verbindungen der Formel (I) gemäß einem der Ansprüche 1 oder 2 mit Streckmitteln und/oder oberflächenaktiven Stoffen vermischt werden.

7. Verwendung von Verbindungen der Formel (I) gemäß einem der Ansprüche 1 oder 2 zur Behandlung von Saatgut.

8. Verwendung von Verbindungen der Formel (I) gemäß einem der Ansprüche 1 oder 2 zur Behandlung von transgenen Pflanzen.

9. Verwendung von Verbindungen der Formel (I) gemäß einem der Ansprüche 1 oder 2 zur Behandlung von transgenem Saatgut.

## Claims

1. Compounds of the formula (I) 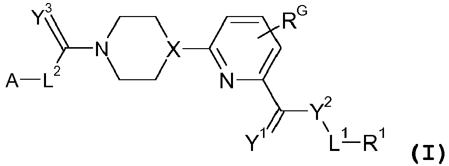 in which the radicals are each defined as follows:
A is phenyl which may contain up to three substituents,
where the substituents are each independently selected from Z^{Å-1}_{"} or
A is an optionally benzofused, unsubstituted or substituted 5- or 6-membered heteroaryl, where the substituents on the carbon are each independently selected from Z^{A-2} and the substituents on the nitrogen are each independently selected from Z^{A-3},
L¹ is (C(R^{L1})₂)ₚ,
P is 1, 2 or 3,
R^{L1} are the same or different and are each independently hydrogen, halogen, C₁-C₄-alkyl, C₂-C₄-alkenyl, C₂-C₄-alkynyl, C₁-C₄-alkoxy, C₃-C₆-cycloalkyl, C₁-C₄-haloalkyl or phenyl, with the proviso that L¹ may contain not more than two R^{L1} other than hydrogen,
L² is NR^{L21} or C(R^{L22})₂,
R^{L21} is hydrogen, C₁-C₄-alkyl, C₂-C₄-alkenyl, C₂-C₄-alkynyl, C₁-C₄-haloalkyl, C₂-C₄-haloalkenyl, C₂-C₄-haloalkynyl, C₂-C₄-alkoxyalkyl, C₂-C₄-alkylthioalkyl, C₂-C₄-alkylsulphinylalkyl, C₂-C₄-alkylsulphonylalkyl, C₂-C₄-alkylcarbonyl, C₂-C₄-haloalkylcarbonyl, C₂-C₅-alkoxycarbonyl, C₃-C₅-alkoxycarbonylalkyl, C₂-C₅-alkylaminocarbonyl, C₃-C₅-dialkylaminocarbonyl, C₁-C₄-alkylsulphonyl or C₁-C₄-haloalkylsulphonyl,
R^{L22} ₐᵣₑ the same or different and are each independently hydrogen, C₁-C₄-alkyl, C₁-C₄-haloalkyl, cyclopropyl, halogen, or the two R^{L22} radicals, together with the carbon atom to which they are bonded, form a cyclopropyl ring,
Y¹ and Y³ are the same or different and are each independently sulphur or oxygen,
Y² is -(NR^{Y2})-, sulphur or oxygen,
R^{Y2} is hydrogen, C₁-C₆-alkyl, C₂-C₆-alkenyl, C₂-C₆-alkynyl, C₁-C₆-haloalkyl, C₃-C₆-cycloalkyl, C₃-C₆-halocycloalkyl, benzyl, phenyl, NR³R⁴, C₁-C₄-alkoxy, C₁-C₄-haloalkoxy, phenoxy or benzyloxy,
or
the R^{Y2}, L¹ and R¹ radicals form, together with the nitrogen atom of Y², a 5- to 15-membered, unsubstituted or substituted, saturated or partly saturated or unsaturated mono-, bi- or tricyclic ring system which may contain up to two further heteroatoms selected from N, O and S, where no two oxygen atoms are adjacent, where possible substituents on the carbon are each independently selected from Z^{Y-1} and the substituents on the nitrogen are each independently selected from Z^{Y-2},
R³ and R⁴ are the same or different and are each independently hydrogen, C₁-C₄-alkyl, C₃-C₆-alkenyl, C₃-C₆-alkynyl, C₁-C₃-haloalkyl, C₃-C₆-cycloalkyl, benzyl or phenyl,
X is -CR^{X1}- or nitrogen,
R^{X1} is hydrogen, halogen, cyano, hydroxyl, C₁-C₄-alkyl, C₂-C₄-alkenyl, C₂-C₄-alkynyl, C₁-C₄-haloalkyl, C₁-C₄-alkoxy, C₁-C₄-haloalkoxy, C₂-C₄-carbonylalkoxy, OC(=O)H, C(=O)H, C(=O)OH, C₂-C₄-alkoxycarbonyl or C₁-C₃-alkylcarbonyl,
R^{G} is hydrogen, halogen or C₁-C₃-alkyl,
R¹ is hydrogen, C₁-C₆-alkyl, C₁-C₆-haloalkyl, C₂-C₆-alkenyl, C₂-C₆-alkynyl, C₂-C₈-alkoxyalkyl or C₅-C₉-cycloalkoxyalkyl,
R¹ is unsubstituted or substituted C₃-C₁₀-cycloalkyl, where the substituents are each independently selected from -Q or from Z¹,
or
R¹ is unsubstituted or substituted C₅-C₁₀-cycloalkenyl,
where the substituents are each independently selected from Z²,
or
R¹ is substituted phenyl,
where the substituents are each independently selected from -L³Q or from Z³,
or
R¹ is unsubstituted or substituted naphthalen-1-yl, naphthalen-2-yl, 1,2,3,4-tetrahydronaphthalen-1-yl, 1,2,3,4-tetrahydronaphthalen-2-yl, 5,6,7,8-tetrahydronaphthalen-1-yl, 5,6,7,8-tetrahydronaphthalen-2-yl, decalin-1-yl, decalin-2-yl, 1H-inden-1-yl, 2,3-dihydro-1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl,
where the substituents are each independently selected from Z⁴,
or
R¹ is an unsubstituted or substituted 5- or 6-membered heteroaryl radical, where the substituents on the carbon are each independently selected from -L³Q or from Z⁵ and the substituents on the nitrogen are each independently selected from Z⁶,
or
R¹ is benzofused unsubstituted or substituted 5- or 6-membered heteroaryl, where the substituents on the carbon are each independently selected from Z⁷ and the substituents on the nitrogen are each independently selected from Z⁸,
or
R¹ is unsubstituted or substituted C₅-C₁₅-heterocyclyl, where the substituents on the carbon are each independently selected from Z⁹ and the substituents on the nitrogen are each independently selected from Z¹⁰,
L³ is a direct bond, -O, -C(=O), -S(O)ₘ, -CHR^{L31} or - NR^{L32},
m is 0, 1 or 2,
R^{L31} is hydrogen, C₁-C₄-alkyl or C₁-C₄-haloalkyl,
R^{L32} is hydrogen, C₁-C₆-alkyl, C₁-C₆-haloalkyl, C₃-C₈-cycloalkyl, C₂-C₆-alkylcarbonyl, C₂-C₆-haloalkylcarbonyl, C₂-C₆-alkoxycarbonyl or C₂-C₆-haloalkoxycarbonyl,
Q is a phenyl which may contain up to two substituents, where the substituents are the same or different and are each independently selected from the following list:
halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkyl-cycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl or phenyl,
or
Q is a 5- or 6-membered heteroaryl radical which may contain up to two substituents, where the substituents are the same or different and are each independently selected from the following list:
substituents on carbon: halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl, C₁-C₄-haloalkylsulphonyl or phenyl, substituents on nitrogen: C₁-C₆-alkyl, C₂-C₆-alkenyl, C₂-C₆-alkynyl, C₁-C₆-haloalkyl, C₂-C₆-halo-alkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₁-C₄-alkylsulphonyl, C(=O)H, C(=O)Me, C(=O)OMe or phenyl,
Z^{A-1} and Z³ are the same or different and are each independently halogen, cyano, hydroxyl, SH, amino, nitro, C(=O)H, C(=O)OH, CONR³R⁴, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₈-cycloalkyl, C₃-C₈-halocycloalkyl, C₄-C₁₀-alkylcycloalkyl, C₄-C₁₀-cycloalkylalkyl, C₆-C₁₄-cycloalkylcycloalkyl, C₄-C₁₀-halocycloalkylalkyl, C₅-C₁₀-alkylcycloalkylalkyl, C₃-C₈-cycloalkenyl, C₃-C₈-halocycloalkenyl, C₂-C₆-alkoxyalkyl, C₄-C₁₀-cycloalkoxyalkyl, C₃-C₈-alkoxyalkoxyalkyl, C₂-C₆-alkylthioalkyl, C₂-C₆-alkylsulphinylalkyl, C₂-C₆-alkylsulphonylalkyl, C₂-C₆-alkylaminoalkyl, C₃-C₈-dialkylaminoalkyl, C₂-C₆-haloalkylaminoalkyl, C₄-C₁₀-cycloalkylaminoalkyl, C₂-C₆-alkylcarbonyl, C₂-C₆-haloalkylcarbonyl, C₄-C₈-cycloalkylcarbonyl, C₂-C₆-alkoxycarbonyl, C₄-C₈-cycloalkoxycarbonyl, C₅-C₁₀-cycloalkylalkoxycarbonyl, C₂-C₆-alkylaminocarbonyl, C₃-C₈-dialkylaminocarbonyl, C₄-C₈-cycloalkylaminocarbonyl, C₂-C₆-haloalkoxyalkyl, C₁-C₆-hydroxyalkyl, C₁-C₆-alkoxy, C₁-C₆-haloalkoxy, C₃-C₈-cycloalkoxy, C₃-C₈-halocycloalkoxy, C₄-C₁₀-cycloalkylalkoxy, C₃-C₆-alkenyloxy, C₃-C₆-haloalkenyloxy, C₃-C₆-alkynyloxy, C₃-C₆-haloalkynyloxy, C₂-C₆-alkoxyalkoxy, C₂-C₆-alkylcarbonyloxy, C₂-C₆-haloalkylcarbonyloxy, C₄-C₈-cycloalkylcarbonyloxy, C₃-C₆-alkylcarbonylalkoxy, C₁-C₆-alkylthio, C₁-C₆-haloalkylthio, C₃-C₆-cycloalkylthio, C₁-C₆-alkylsulphinyl, C₁-C₆-haloalkylsulphinyl, C₁-C₆-alkylsulphonyl, C₁-C₆-haloalkylsulphonyl, C₃-C₈-cycloalkylsulphonyl, tri(C₁-C₄-alkyl)silyl, C₁-C₆-alkylsulphonylamino, C₁-C₆-haloalkylsulphonylamino or SF₅,
Z¹ and Z² are the same or different and are each independently cyano, halogen, C₁-C₆-alkyl, C₁-C₆-haloalkyl, C₃-C₆-cycloalkyl, C₂-C₆-alkenyl, C₂-C₆-haloalkenyl, C₂-C₆-alkynyl, C₂-C₆-haloalkynyl, hydroxyl, oxo, C₁-C₆-alkoxy, C₁-C₆-haloalkoxy, C₂-C₆-alkenyloxy, C₂-C₆-alkynyloxy, C₁-C₆-alkylthio or C₁-C₆-haloalkylthio, C₂-C₄-alkylcarbonyl, or C₂-C₆-alkylcarbonyloxy,
Z⁴ and Z⁷ are the same or different and are each independently halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl or C₁-C₄-haloalkylsulphonyl,
Z^{A-2} and Z⁵ are the same or different and are each independently halogen, cyano, hydroxyl, SH, amino, nitro, NR³R⁴, C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, C₆-C₁₄-cycloalkylcycloalkyl, C₅-C₁₀-alkylcycloalkylalkyl, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₁-C₄-alkoxy, C₁-C₄-haloalkoxy, C₃-C₆-alkenyloxy, C₃-C₆-alkynyloxy, C₂-C₆-alkylcarbonyloxy, C₂-C₆-alkylcarbonylthio, C₁-C₄-alkylthio, C₁-C₄-haloalkylthio, C₁-C₄-alkylsulphinyl, C₁-C₄-haloalkylsulphinyl, C₁-C₄-alkylsulphonyl or C₁-C₄-haloalkylsulphonyl,
Z^{A-3}, Z^{Y-2}, Z⁶, Z⁸ and Z¹⁰ are the same or different and are each independently C₁-C₆-alkyl, C₂-C₆-alkenyl, C₂-C₆-alkynyl, C₁-C₆-haloalkyl, C₂-C₆-haloalkenyl, C₂-C₆-haloalkynyl, C₃-C₆-cycloalkyl, C₃-C₆-halocycloalkyl, C₄-C₁₀-alkylcycloalkyl, C₄-C₁₀-cycloalkylalkyl, phenyl, benzyl, C₁-C₄-alkylsulphonyl, C(=O)H, C₂-C₄-alkylcarbonylalkoxy or C₁-C₃-alkylcarbonyl,
Z^{Y-1} and Z⁹ are the same or different and are each independently hydroxyl, cyano, halogen, SH, amino, nitro, oxo, NR³R⁴, C₁-C₆-alkyl, C₁-C₆-haloalkyl, C₃-C₆-Cycloalkyl, C₂-C₆-alkenyl, C₂-C₆-haloalkenyl, C₂-C₆-alkynyl, C₂-C₆-haloalkynyl, phenyl, C₁-C₆-alkoxy, Cᵢ-C₆-haloalkoxy, C₂-C₆-alkenyloxy, C₂-C₆-alkynyloxy, C₁-C₆-alkylthio or C₁-C₆-haloalkylthio, C₂-C₄-alkoxyalkyl, C₂-C₄-alkylcarbonyl, C₂-C₆-alkoxycarbonyl, C₂-C₆-alkylaminocarbonyl, C₃-C₈-dialkylaminocarbonyl, C₁-C₄-hydroxyalkyl, C₂-C₆-alkylcarbonyloxy, C₂-C₆-alkylcarbonylthio, C₁-C₄-alkylsulphinyl, C₁-C₄-haloalkylsulphinyl, C₁-C₄-alkylsulphonyl or C₁-C₄-haloalkylsulphonyl,
and the salts, metal complexes and N-oxides thereof.

2. Compounds of the formula (I) according to Claim 1, in which the radicals are each defined as follows:
A is phenyl which may contain up to two substituents, where the substituents are each independently selected from the following list: fluorine, bromine, iodine, chlorine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio or trifluoromethylthio, or
A is a heteroaromatic radical selected from the following group: furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,3-triazol-1-yl, 1,2,4-triazol-1-yl, pyridin-2-yl, pyridin-3-yl, pyridin-4-yl, pyridazin-3-yl, pyridazin-4-yl, pyrimidin-2-yl, pyrimidin-4-yl or pyrimidin-5-yl, which may contain up to two substituents, where the substituents are the same or different and are each independently selected from the following list:
substituents on carbon:
fluorine, chlorine, bromine, iodine, cyano, nitro, methyl, ethyl, propyl, 1-methylethyl, 1,1-dimethylethyl, chlorofluoromethyl, dichloromethyl, dichlorofluoromethyl, difluoromethyl, trichloromethyl, trifluoromethyl, cyclopropyl, ethoxy, 1-methylethoxy, propoxy, methoxy, trifluoromethoxy, difluoromethoxy, 1-methylethylthio, methylthio, ethylthio, propylthio, difluoromethylthio, trifluoromethylthio or phenyl,
substituents on nitrogen:
methyl, ethyl, propyl, 1-methylethyl, 2,2-trifluoroethyl, 2,2-difluoroethyl, 2,2-dichloro-2-fluoroethyl, 2-chloro-2-difluoroethyl or 2-chloro-2-fluoroethyl,
L² is CH₂
Y¹ is oxygen,
R^{Y2} is hydrogen, methyl, ethyl, propyl, 1-methylethyl, prop-2-enyl, 1-methylprop-2-enyl, ethynyl, prop-2-ynyl, 2,2,2-trifluoroethyl, cyclopropyl, 1-chlorocyclopropyl, benzyl or phenyl, or
the R^{Y2}, L¹ and R¹ radicals form, together with the nitrogen atom of Y², piperidine, morpholine, thiomorpholine, 2,3-dihydro-4H-1,4-oxazine, 2,3-dihydro-4H-1,4-benzoxazine, or 1,2,3,4-tetrahydroquinoline,
X is -CH-, -CF- or nitrogen,
R^{G} is hydrogen,
R¹ is hydrogen, 1,1-dimethylethyl, 3,3-dimethylbutyl, 2,2-dimethylpropyl, 1,2,2-trimethylpropyl, pentyl, 1-ethylpropyl, butyl, 2-methylpropyl, 1-methylethyl, ethyl, propyl, 4-methylpentyl, hexyl, trifluoromethyl, methoxymethyl, ethoxymethyl, ethenyl, prop-2-en-1-yl or but-3-en-1-yl, or
R¹ is cyclopentyl, cyclohexyl, cycloheptyl or cyclooctyl, each of which may contain up to two substituents, where the substituents are each independently selected from the following list: cyano, chlorine, fluorine, bromine, iodine, methyl, ethyl, propyl, 1-methylethyl, 1,1-dimethylethyl, ethenyl, 2-propenyl, 2-propynyloxy, phenyl, methoxy, ethoxy, propyloxy, trifluoromethoxy, ethynyl, methylthio, ethylthio or trifluoromethylthio, or
R¹ is cyclopentenyl, cyclohexenyl or cycloheptenyl, each of which may contain up to two substituents, where the substituents are each independently selected from the following list: methyl, ethyl, methoxy, ethoxy, trifluoromethoxy, ethynyl, 2-propynyloxy, methylthio, ethylthio or trifluoromethylthio, or
R¹ is phenyl which contains up to three substituents, where the substituents are each independently selected from the following list: fluorine, chlorine, bromine, iodine, cyano, nitro, methyl, ethyl, propyl, 1-methylethyl, butyl, 1,1-dimethylethyl, 1,2-dimethylethyl, ethenyl, ethynyl, trifluoromethyl, difluoromethyl, trichloromethyl, dichloromethyl, cyclopropyl, methoxy, ethoxy, propoxy, 1-methylethoxy, 1,1-dimethylethoxy, methylcarbonyl, ethylcarbonyl, trifluoromethylcarbonyl, methoxycarbonyl, ethoxycarbonyl, propoxycarbonyl, 1-methylethoxycarbonyl, 1,1-dimethylethoxycarbonyl, 2-propynyloxy, methylthio, ethylthio, methylsulphinyl or methylsulphonyl or -L³Q, or
R¹ is naphthalen-l-yl, naphthalen-2-yl, 1,2,3,4-tetrahydronaphthalen-l-yl, 1,2,3,4-tetrahydronaphthalen-2-yl, 5,6,7,8-tetrahydronaphthalen-l-yl, 5,6,7,8-tetrahydronaphthalen-2-yl, decalin-l-yl, decalin-2-yl, dihydro-1H-inden-1-yl, 1H-inden-1-yl, 1H-inden-2-yl, 1H-inden-3-yl, 1H-inden-4-yl, 1H-inden-5-yl, 1H-inden-6-yl, 1H-inden-7-yl, indan-1-yl, indan-2-yl, indan-3-yl, indan-4-yl or indan-5-yl, which may contain up to three substituents, where the substituents are each independently selected from the following list: methyl, methoxy, cyano, fluorine, chlorine, bromine, iodine, or
R¹ is furan-2-yl, furan-3-yl, thiophen-2-yl, thiophen-3-yl, isoxazol-3-yl, isoxazol-4-yl, isoxazol-5-yl, pyrrol-1-yl, pyrrol-2-yl, pyrrol-3-yl, oxazol-2-yl, oxazol-4-yl, oxazol-5-yl, thiazol-2-yl, thiazol-4-yl, thiazol-5-yl, isothiazol-3-yl, isothiazol-4-yl, isothiazol-5-yl, pyrazol-1-yl, pyrazol-3-yl, pyrazol-4-yl, imidazol-1-yl, imidazol-2-yl, imidazol-4-yl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 1,2,3-triazol-1-yl, 1,2,3-triazol-2-yl, 1,2,3-triazol-4-yl, 1,2,4-triazol-1-yl, 1,2,4-triazol-3-yl, 1,2,4-triazol-4-yl, pyridin-2-yl, pyridin-3-yl, pyridin-4-yl, pyridazin-3-yl, pyridazin-4-yl, pyrimidin-2-yl, pyrimidin-4-yl, pyrimidin-5-yl or pyrazin-2-yl, each of which may contain up to two substituents, where the substituents are each independently selected from the following list:
substituents on carbon:
chlorine, fluorine, bromine, iodine, cyano, nitro, methyl, ethyl, propyl, 1-methylethyl, butyl, 1,1-dimethylethyl, ethenyl, ethynyl, trifluoromethyl, difluoromethyl, cyclopropyl, cyclopentyl, cyclohexyl, methylcarbonyl, ethylcarbonyl, methoxylcarbonyl, ethoxycarbonyl, methoxy, ethoxy, propoxy, 1-methylethoxy, 2-propynyloxy, trifluoromethoxy, methylcarbonyloxy, methylcarbonylthio, methylthio, ethylthio, trifluoromethylthio, methylsulphinyl, ethylsulphinyl, trifluoromethylsulphinyl, methylsulphonyl, ethylsulphonyl or trifluoromethylsulphonyl,
substituents on nitrogen: methyl, ethyl, propyl, cyclopropyl, cyclohexyl, phenyl or 2-propynyl, or
R¹ is indol-l-yl, indol-2-yl, indol-3-yl, indol-4-yl, indol-5-yl, indol-6-yl, indol-7-yl, benzimidazol-1-yl, benzimidazol-2-yl, benzimidazol-4-yl, benzimidazol-5-yl, indazol-1-yl, indazol-3-yl, indazol-4-yl, indazol-5-yl, indazol-6-yl, indazol-7-yl, indazol-2-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, quinolin-2-yl, quinolin-3-yl, quinolin-4-yl, quinolin-5-yl, quinolin-6-yl, quinolin-7-yl, quinolin-8-yl, isoquinolin-1-yl, isoquinolin-3-yl, isoquinolin-4-yl, isoquinolin-5-yl, isoquinolin-6-yl, isoquinolin-7-yl or isoquinolin-8-yl, each of which may contain up to two substituents, where the substituents are each independently selected from the following list:
substituents on carbon: fluorine, chlorine, bromine, iodine, methyl, methoxy, 2-propynyloxy, 2-propenyloxy,
R¹ substituents on nitrogen: methyl, ethyl, propyl, cyclopropyl, cyclohexyl, phenyl or 2-propynyl, or is piperidin-l-yl, piperidin-2-yl, piperidin-3-yl, piperidin-4-yl, piperazin-l-yl, piperazin-2-yl, piperazin-3-yl, morpholin-l-yl, morpholin-2-yl, morpholin-3-yl, tetrahydropyran-2-yl, tetrahydropyran-3-yl, tetrahydropyran-4-yl, 1,2,3,4-tetrahydroquinolin-1-yl, 1,2,3,4-tetrahydroisoquinolin-2-yl, 1,2,3,4-tetrahydroquinoxalin-l-yl, indolin-1-yl, isoindolin-2-yl, decahydroquinolin-1-yl or decahydroisoquinolin-2-yl, each of which may contain up to two substituents, where the substituents are each independently selected from the following list:
substituents on carbon: fluorine, chlorine, bromine, iodine, methyl, methoxy, 2-propynyloxy, 2-propenyloxy,
substituents on nitrogen: methyl, ethyl, propyl, cyclopropyl, cyclohexyl, phenyl or 2-propynyl,
Q is phenyl.

3. Method for controlling phytopathogenic harmful fungi, **characterized in that** compounds of the formula (I) according to either of Claims 1 and 2 are applied to the phytopathogenic harmful fungi and/or their habitat.

4. Composition for controlling phytopathogenic harmful fungi, **characterized by** a content of at least one compound of the formula (I) according to either of Claims 1 and 2, in addition to extenders and/or surfactants.

5. Use of at least one compound of the formula (I) according to either of Claims 1 and 2 or of a composition according to Claim 4 for controlling phytopathogenic harmful fungi.

6. Process for producing compositions for controlling phytopathogenic harmful fungi according to Claim 4, **characterized in that** compounds of the formula (I) according to either of Claims 1 and 2 are mixed with extenders and/or surfactants.

7. Use of compounds of the formula (I) according to either of Claims 1 and 2 for treatment of seed.

8. Use of compounds of the formula (I) according to either of Claims 1 and 2 for treatment of transgenic plants.

9. Use of compounds of the formula (I) according to either of Claims 1 and 2 for treatment of transgenic seed.

## Revendications

1. Composés de formule (I) 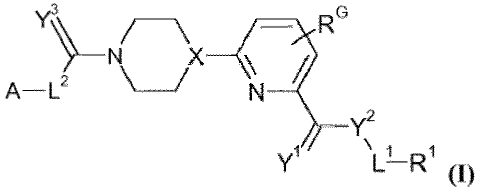 dans lesquels les radicaux ont les significations suivantes :
A représente phényle, qui peut contenir jusqu'à trois substituants,
les substituants étant choisis indépendamment les uns des autres parmi Z^{A-1},
ou
A représente un hétéroaryle à 5 ou 6 chaînons, non substitué ou substitué, éventuellement benzocondensé, les substituants sur le carbone étant choisis indépendamment les uns des autres parmi Z^{A-2} et les substituants sur l'azote étant choisis indépendamment les uns des autres parmi Z^{A-3},
L¹ représente (C(R^{L1})₂)ₚ,
p représente 1, 2 ou 3,
les R^{L1} représentent indépendamment les uns des autres, de manière identique ou différente, hydrogène, halogène, alkyle en C₁-C₄, alcényle en C₂-C₄, alcynyle en C₂-C₄, alcoxy en C₁-C₄, cycloalkyle en C₃-C₆, halogénoalkyle en C₁-C₄ ou phényle,
à condition que L¹ puisse contenir au plus deux R^{L1} qui sont différents de l'hydrogène,
L² représente NR^{L21} ou C(R^{L22})₂,
R^{L21} représente hydrogène, alkyle en C₁-C₄, alcényle en C₂-C₄, alcynyle en C₂-C₄, halogénoalkyle en C₁-C₄, halogénoalcényle en C₂-C₄, halogénoalcynyle en C₂-C₄, alcoxyalkyle en C₂-C₄, alkylthioalkyle en C₂-C₄, alkylsulfinylalkyle en C₂-C₄, alkylsulfonylalkyle en C₂-C₄, alkylcarbonyle en C₂-C₄, halogénoalkylcarbonyle en C₂-C₄, alcoxycarbonyle en C₂-C₅, alcoxycarbonylalkyle en C₃-C₅, alkylaminocarbonyle en C₂-C₅, dialkylaminocarbonyle en C₃-C₅, alkylsulfonyle en C₁-C₄ ou halogénoalkylsulfonyle en C₁-C₄,
les R^{L22} représentent indépendamment l'un de l'autre, de manière identique ou différente, hydrogène, alkyle en C₁-C₄, halogénoalkyle en C₁-C₄, cyclopropyle, halogène,
ou les deux radicaux R^{L22} forment ensemble avec l'atome de carbone auquel ils sont reliés un cycle cyclopropyle,
Y¹ et Y³ représentent indépendamment l'un de l'autre, de manière identique ou différente, soufre ou oxygène,
Y² représente - (NR^{Y2}) -, soufre ou oxygène,
R^{Y2} représente hydrogène, alkyle en C₁-C₆, alcényle en C₂-C₆, alcynyle en C₂-C₆, halogénoalkyle en C₁-C₆, cycloalkyle en C₃-C₆, halogénocycloalkyle en C₃-C₆, benzyle, phényle, NR³R⁴, alcoxy en C₁-C₄, halogénoalcoxy en C₁-C₄, phénoxy ou benzyloxy,
ou
les radicaux R^{Y2}, L¹ et R¹ forment ensemble avec l'atome d'azote de Y² un système cyclique mono-, bi- ou tricyclique, saturé ou partiellement saturé ou insaturé, non substitué ou substitué, de 5 à 15 chaînons, qui peut contenir jusqu'à deux hétéroatomes supplémentaires, choisis parmi N, 0 et S, deux atomes d'oxygène n'étant pas voisins, des substituants possibles sur le carbone étant choisis indépendamment les uns des autres parmi Z^{Y-1} et les substituants sur l'azote étant choisis indépendamment les uns des autres parmi Z^{Y-2},
R³ et R⁴ représentent indépendamment l'un de l'autre, de manière identique ou différente, hydrogène, alkyle en C₁-C₄, alcényle en C₃-C₆, alcynyle en C₃-C₆, halogénoalkyle en C₁-C₃, cycloalkyle en C₃-C₆, benzyle ou phényle,
X représente -CR^{X1}- ou azote,
R^{X1} représente hydrogène, halogène, cyano, hydroxy, alkyle en C₁-C₄, alcényle en C₂-C₄, alcynyle en C₂-C₄, halogénoalkyle en C₁-C₄, alcoxy en C₁-C₄, halogénoalcoxy en C₁-C₄, carbonylalcoxy en C₂-C₄, OC(=O)H, C(=O)H, C(=0)0H, alcoxycarbonyle en C₂-C₄ ou alkylcarbonyle en Ci-Cs,
R^{G} représente hydrogène, halogène ou alkyle en C₁-C₃,
R¹ représente hydrogène, alkyle en C₁-C₆, halogénoalkyle en C₁-C₆, alcényle en C₂-C₆, alcynyle en C₂-C₆, alcoxyalkyle en C₂-C₈ ou cycloalcoxyalkyle en C₅-C₉,
R¹ représente cycloalkyle en C₃-C₁₀ non substitué ou substitué,
les substituants étant choisis indépendamment les uns des autres parmi -Q ou Z ,
ou
R¹ représente cycloalcényle en C_{S}-C₁₀ non substitué ou substitué,
les substituants étant choisis indépendamment les uns des autres parmi Z²,
ou
R¹ représente phényle substitué,
les substituants étant choisis indépendamment les uns des autres parmi -L³Q ou Z³,
ou
R¹ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, 1H-indén-1-yle, 2,3-dihydro-1H-indén-1-yle, 1H-indén-2-yle, 1H-indén-3-yle, 1H-indén-4-yle, 1H-indén-5-yle, 1H-indén-6-yle, 1H-indén-7-yle, indan-1-yle, indan-2-yle, indan-3-yle, indan-4-yle ou indan-5-yle, non substitué ou substitué,
les substituants étant choisis indépendamment les uns des autres parmi Z⁴,
ou
R¹ représente un radical hétéroaryle à 5 ou 6 chaînons non substitué ou substitué,
les substituants sur le carbone étant choisis indépendamment les uns des autres parmi -L³Q ou Z⁵, et les substituants sur l'azote étant choisis indépendamment les uns des autres parmi Z⁶,
ou
R¹ représente un hétéroaryle à 5 ou 6 chaînons non substitué ou substitué, benzocondensé, les substituants sur le carbone étant choisis indépendamment les uns des autres parmi Z⁷, et les substituants sur l'azote étant choisis indépendamment les uns des autres parmi Z⁸,
ou
R¹ représente hétérocyclyle en C₅-C₁₅ non substitué ou substitué, les substituants sur le carbone étant choisis indépendamment les uns des autres parmi Z⁹, et les substituants sur l'azote étant choisis indépendamment les uns des autres parmi Z¹⁰,
L³ représente une liaison directe, -O, -C(=O), -S(O)ₘ, -CHR^{L31} ou -NR^{L32},
m représente 0, 1 ou 2,
_{RL}31 représente hydrogène, alkyle en C₁-C₄ ou halogénoalkyle en C₁-C₄,
_{RL}32 représente hydrogène, alkyle en C₁-C₆, halogénoalkyle en C₁-C₆, cycloalkyle en C₃-C₈, alkylcarbonyle en C₂-C₆, halogénoalkylcarbonyle en C₂-C₆, alcoxycarbonyle en C₂-C₆ ou halogénoalcoxycarbonyle en C₂-C₆,
Q représente un phényle, qui peut contenir jusqu'à deux substituants, les substituants étant choisis indépendamment les uns des autres, de manière identique ou différente, dans la liste suivante :
halogène, cyano, hydroxy, SH, amino, nitro, NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄ ou phényle,
ou
Q représente un radical hétéroaryle à 5 ou 6 éléments, qui peut contenir jusqu'à deux substituants, les substituants étant choisis indépendamment l'un de l'autre, de manière identique ou différente, dans la liste suivante :
substituants sur le carbone : halogène, cyano, hydroxy, SH, amino, nitro, NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄, halogénoalkylsulfonyle en C₁-C₄ ou phényle,
substituants sur l'azote : alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, alkylsulfonyle en C₁-C₄, C(=O)H, C(=O)Me, C(=O)OMe ou phényle,
Z^{A-1} et Z³ représentent indépendamment l'un de l'autre, de manière identique ou différente, halogène, cyano, hydroxy, SH, amino, nitro, C(=O)H, C(=O)OH, CONR³R⁴, NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₈, halogénocycloalkyle en C₃-C₈, alkylcycloalkyle en C₄-C₁₀, cycloalkylalkyle en C₄-C₁₀, cycloalkylcycloalkyle en C₆-C₁₄, halogénocycloalkylalkyle en C₄-C₁₀, alkylcycloalkylalkyle en C₅-C₁₀, cycloalcényle en C₃-C₈, halogénocycloalcényle en C₃-C₈, alcoxyalkyle en C₂-C₆, cycloalcoxyalkyle en C₄-C₁₀, alcoxyalcoxyalkyle en C₃-C₈, alkylthioalkyle en C₂-C₆, alkylsulfinylalkyle en C₂-C₆, alkylsulfonylalkyle en C₂-C₆, alkylaminoalkyle en C₂-C₆, dialkylaminoalkyle en C₃-C₈, halogénoalkylaminoalkyle en C₂-C₆, cycloalkylaminoalkyle en C₄-C₁₀, alkylcarbonyle en C₂-C₆, halogénoalkylcarbonyle en C₂-C₆, cycloalkylcarbonyle en C₄-C₈, alcoxycarbonyle en C₂-C₆, cycloalcoxycarbonyle en C₄-C₈, cycloalkylalcoxycarbonyle en C₅-C₁₀, alkylaminocarbonyle en C₂-C₆, dialkylaminocarbonyle en C₃-C₈, cycloalkylaminocarbonyle en C₄-C₈, halogénoalcoxyalkyle en C₂-C₆, hydroxyalkyle en C₁-C₆, alcoxy en C₁-C₆, halogénoalcoxy en C₁-C₆, cycloalcoxy en C₃-C₈, halogénocycloalcoxy en C₃-C₈, cycloalkylalcoxy en C₄-C₁₀, alcényloxy en C₃-C₆, halogénoalcényloxy en C₃-C₆, alcynyloxy en C₃-C₆, halogénoalcynyloxy en C₃-C₆, alcoxyalkoxy en C₂-C₆, alkylcarbonyloxy en C₂-C₆, halogénoalkylcarbonyloxy en C₂-C₆, cycloalkylcarbonyloxy en C₄-C₈, alkylcarbonylalcoxy en C₃-C₆, alkylthio en C₁-C₆, halogénoalkylthio en C₁-C₆, cycloalkylthio en C₃-C₆, alkylsulfinyle en C₁-C₆, halogénoalkylsulfinyle en C₁-C₆, alkylsulfonyle en C₁-C₆, halogénoalkylsulfonyle en C₁-C₆, cycloalkylsulfonyle en C₃-C₈, tri(alkyle en C₁-C₄)silyle, alkylsulfonylamino en C₁-C₆ ou halogénoalkylsulfonylamino en C₁-C₆ ou SF₅,
Z¹ et Z² représentent indépendamment l'un de l'autre, de manière identique ou différente, cyano, halogène, alkyle en C₁-C₆, halogénoalkyle en C₁-C₆, cycloalkyle en C₃-C₆, alcényle en C₂-C₆, halogénoalcényle en C₂-C₆, alcynyle en C₂-C₆, halogénoalcynyle en C₂-C₆, hydroxy, oxo, alcoxy en C₁-C₆, halogénoalcoxy en C₁-C₆, alcényloxy en C₂-C₆, alcynyloxy en C₂-C₆, alkylthio en C₁-C₆ ou halogénoalkylthio en C₁-C₆, alkylcarbonyle en C₂-C₄ ou alkylcarbonyloxy en C₂-C₆,
Z⁴ et Z⁷ représentent indépendamment l'un de l'autre, de manière identique ou différente, halogène, cyano, hydroxy, SH, amino, nitro, NR³R⁴, alkyle en C₁-C₆, alcényle en C₂-C₆, alcynyle en C₂-C₆, halogénalkyle en C₁-C₆, halogénoalcényle en C₂-C₆, halogénoalcynyle en C₂-C₆, cycloalkyle en C₃-C₆, halogénocycloalkyle en C₃-C₆, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄ ou halogénoalkylsulfonyle en C₁-C₄,
Z^{A-2} et Z⁵ représentent indépendamment l'un de l'autre, de manière identique ou différente, halogène, cyano, hydroxy, SH, amino, nitro, NR³R⁴, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, cycloalkylcycloalkyle en C₆-C₁₄, alkylcycloalkylalkyle en C₅-C₁₀, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alcoxy en C₁-C₄, halogénoalcoxy en C₁-C₄, alcényloxy en C₃-C₆, alcynyloxy en C₃-C₆, alkylcarbonyloxy en C₂-C₆, alkylcarbonylthio en C₂-C₆, alkylthio en C₁-C₄, halogénoalkylthio en C₁-C₄, alkylsulfinyle en C₁-C₄, halogénoalkylsulfinyle en C₁-C₄, alkylsulfonyle en C₁-C₄ ou halogénoalkylsulfonyle en C₁-C₄,
Z^{A-3}, Z^{Y-2}, Z⁶, Z⁸ et Z¹⁰ représentent indépendamment les uns des autres, de manière identique ou différente, alkyle en C₁-C₆, alcényle en C₂-C₆, alcynyle en C₂-C₆, halogénoalkyle en C₁-C₆, halogénoalcényle en C₂-C₆, halogénoalcynyle en C₂-C₆, cycloalkyle en C₃-C₆, halogénocycloalkyle en C₃-C₆, alkylcycloalkyle en C₄-C₁₀, cycloalkylalkyle en C₄-C₁₀, phényle, benzyle, alkylsulfonyle en C₁-C₄, C (=0) H, alkylcarbonylalcoxy en C₂-C₄ ou alkylcarbonyle en C₁-C₃,
Z^{Y-1} et Z⁹ représentent indépendamment l'un de l'autre, de manière identique ou différente, hydroxy, cyano, halogène, SH, amino, nitro, oxo, NR³R⁴, alkyle en C₁-C₆, halogénoalkyle en C₁-C₆, cycloalkyle en C₃-C₆, alcényle en C₂-C₆, halogénoalcényle en C₂-C₆, alcynyle en C₂-C₆, halogénoalcynyle en C₂-C₆, phényle, alcoxy en C₁-C₆, halogénoalcoxy en C₁-C₆, alcényloxy en C₂-C₆, alcynyloxy en C₂-C₆, alkylthio en C₁-C₆ ou halogénoalkylthio en C₁-C₆, alcoxyalkyle en C₂-C₄, alkylcarbonyle en C₂-C₄, alcoxycarbonyle en C₂-C₆, alkylaminocarbonyle en C₂-C₆, dialkylaminocarbonyle en C₃-C₈, hydroxyalkyle en C₁-C₄, alkylcarbonyloxy en C₂-C₆, alkylcarbonylthio en C₂-C₆, alkylsulfinyle en C₁-C₄, halogénoalkylsulfinyle en C₁-C₄, alkylsulfonyle en C₁-C₄ ou halogénoalkylsulfonyle en C₁-C₄,
ainsi que leurs sels, complexes métalliques et N-oxydes.

2. Composés de formule (I) selon la revendication 1, dans lesquels les radicaux ont la signification suivante :
A représente phényle, qui peut contenir jusqu'à deux substituants, les substituants étant choisis indépendamment l'un de l'autre dans la liste suivante : fluor, brome, iode, chlore, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio ou trifluorométhylthio, ou A représente un radical hétéroaromatique choisi dans le groupe suivant : furan-2-yle, furan-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,3-triazol-1-yle, 1,2,4-triazol-1-yle, pyridin-2-yle, pyridin-3-yle, pyridin-4-yle, pyridazin-3-yle, pyridazin-4-yle, pyrimidin-2-yle, pyrimidin-4-yle ou pyrimidin-5-yle, qui peut contenir jusqu'à deux substituants, les substituants étant choisis indépendamment l'un de l'autre, de manière identique ou différente, dans la liste suivante:
substituants sur le carbone:
fluor, chlore, brome, iode, cyano, nitro, méthyle, éthyle, propyle, 1-méthyléthyle, 1,1-diméthyléthyle, chlorofluorométhyle, dichlorométhyle, dichlorofluorométhyle, difluorométhyle, trichlorométhyle, trifluorométhyle, cyclopropyle, éthoxy, 1-méthyléthoxy, propoxy, méthoxy, trifluorométhoxy, difluorométhoxy, 1-méthyléthylthio, méthylthio, éthylthio, propylthio, difluorométhylthio trifluorométhylthio ou phényle,
substituants sur l'azote :
méthyle, éthyle, propyle, 1-méthyléthyle, 2,2-trifluoroéthyle, 2,2-difluoroéthyle, 2,2-dichloro-2-fluoroéthyle, 2-chloro-2-difluoroéthyle ou 2-chloro-2-fluoréthyle,
L² représente CH₂,
Y¹ représente oxygène,
R^{Y2} représente hydrogène, méthyle, éthyle, propyle, 1-méthyléthyle, prop-2-ényle, 1-méthylprop-2-ényle, éthynyle, prop-2-ynyle, 2,2,2-trifluoroéthyle, cyclopropyle, 1-chlorocyclopropyle, benzyle ou phényle, ou
les radicaux R^{Y2}, L¹ et R¹ forment ensemble avec l'atome d'azote de Y², pipéridine, morpholine, thiomorpholine, 2,3-dihydro-4H-1,4-oxazine, 2,3-dihydro-4H-1,4-benzoxazine ou 1,2,3,4-tétrahydroquinoline,
X représente -CH-, -CF- ou azote,
R^{G} représente hydrogène,
R¹ représente hydrogène, 1,1-diméthyléthyle, 3,3-diméthylbutyle, 2,2-diméthylpropyle, 1,2,2-triméthylpropyle, pentyle, 1-éthylpropyle, butyle, 2-méthylpropyle, 1-méthyléthyle, éthyle, propyle, 4-méthylpentyle, hexyle, trifluorométhyle, méthoxyméthyle, éthoxyméthyle, éthényle, prop-2-én-1-yle ou but-3-én-1-yle, ou
R¹ représente cyclopentyle, cyclohexyle, cycloheptyle ou cyclooctyle, qui peuvent chacun contenir jusqu'à deux substituants, les substituants étant choisis indépendamment l'un de l'autre dans la liste suivante : cyano, chlore, fluor, brome, iode, méthyle, éthyle, propyle, 1-méthyléthyle, 1,1-diméthyléthyle, éthényle, 2-propényle, 2-propynyloxy, phényle, méthoxy, éthoxy, propyloxy, trifluorométhoxy, éthynyle, 2-propynyloxy, méthylthio, éthylthio ou trifluorométhylthio, ou R¹ représente cyclopentényle, cyclohexényle ou cycloheptényle, qui peuvent chacun contenir jusqu'à deux substituants, les substituants étant choisis indépendamment l'un de l'autre dans la liste suivante : méthyle, éthyle, méthoxy, éthoxy, trifluorométhoxy, éthynyle, 2-propynyloxy, méthylthio, éthylthio ou trifluorométhylthio, ou
R¹ représente phényle, qui contient jusqu'à trois substituants, les substituants étant choisis indépendamment les uns des autres dans la liste suivante :
fluor, chlore, brome, iode, cyano, nitro, méthyle, éthyle, propyle, 1-méthyléthyle, butyle, 1,1-diméthyléthyle, 1,2-diméthyléthyle, éthényle, éthynyle, trifluorométhyle, difluorométhyle, trichlorométhyle, dichlorométhyle, cyclopropyle, méthoxy, éthoxy, propoxy, 1-méthyléthoxyle, 1,1-diméthyléthoxyle, méthylcarbonyle, éthylcarbonyle, trifluorométhylcarbonyle, méthoxycarbonyle, éthoxycarbonyle, propoxycarbonyle, 1-méthyléthoxycarbonyle, 1,1-diméthyléthoxycarbonyle, 2-propynyloxy, méthylthio, éthylthio, méthylsulfinyle ou méthylsulfonyle ou -L³Q, ou
R¹ représente naphtalén-1-yle, naphtalén-2-yle, 1,2,3,4-tétrahydronaphtalén-1-yle, 1,2,3,4-tétrahydronaphtalén-2-yle, 5,6,7,8-tétrahydronaphtalén-1-yle, 5,6,7,8-tétrahydronaphtalén-2-yle, décalin-1-yle, décalin-2-yle, dihydro-1H-indén-1-yle, 1H-indén-1-yle, 1H-indén-2-yle, 1H-indén-3-yle, 1H-indén-4-yle, 1H-indén-5-yle, 1H-indén-6-yle, 1H-indén-7-yle, indan-1-yle, indan-2-yle, indan-3-yle, indan-4-yle ou indan-5-yle, qui peut contenir jusqu'à trois substituants, les substituants étant choisis indépendamment les uns des autres dans la liste suivante : méthyle, méthoxy, cyano, fluor, chlore, brome, iode, ou
R¹ représente furan-2-yle, furan-3-yle, thiophén-2-yle, thiophén-3-yle, isoxazol-3-yle, isoxazol-4-yle, isoxazol-5-yle, pyrrol-1-yle, pyrrol-2-yle, pyrrol-3-yle, oxazol-2-yle, oxazol-4-yle, oxazol-5-yle, thiazol-2-yle, thiazol-4-yle, thiazol-5-yle, isothiazol-3-yle, isothiazol-4-yle, isothiazol-5-yle, pyrazol-1-yle, pyrazol-3-yle, pyrazol-4-yle, imidazol-1-yle, imidazol-2-yle, imidazol-4-yle, 1,2,4-oxadiazol-3-yle, 1,2,4-oxadiazol-5-yle, 1,3,4-oxadiazol-2-yle, 1,2,4-thiadiazol-3-yle, 1,2,4-thiadiazol-5-yle, 1,3,4-thiadiazol-2-yle, 1,2,3-triazol-1-yle, 1,2,3-triazol-2-yle, 1,2,3-triazol-4-yle, 1,2,4-triazol-1-yle, 1,2,4-triazol-3-yle, 1,2,4-triazol-4-yle, pyridin-2-yle, pyridin-3-yle, pyridin-4-yle, pyridazin-3-yle, pyridazin-4-yle, pyrimidin-2-yle, pyrimidin-4-yle, pyrimidin-5-yle ou pyrazin-2-yle, qui peuvent chacun contenir jusqu'à deux substituants, les substituants étant choisis indépendamment l'un de l'autre dans la liste suivante :
substituants sur le carbone : chlore, fluor, brome, iode, cyano, nitro, méthyle, éthyle, propyle, 1-méthyléthyle, butyle, 1,1-diméthyléthyle, éthényle, éthynyle, trifluorométhyle, difluorométhyle, cyclopropyle, cyclopentyle, cyclohexyle, méthylcarbonyle, éthylcarbonyle, méthoxylcarbonyle, éthoxycarbonyle, méthoxy, éthoxy, propoxy, 1-méthyléthoxy, 2-propynyloxy, trifluorométhoxyle, méthylcarbonyloxy, méthylcarbonylthio, méthylthio, éthylthio, trifluorométhylthio, méthylsulfinyle, éthylsulfinyle, trifluorométhylsulfinyle, méthylsulfonyle, éthylsulfonyle ou trifluorméthylsulfonyle,
substituants sur l'azote : méthyle, éthyle, propyle, cyclopropyle, cyclohexyle, phényle ou 2-propynyle, ou R¹ représente indol-1-yle, indol-2-yle, indol-3-yle, indol-4-yle, indol-5-yle, indol-6-yle, indol-7-yle, benzimidazol-1-yle, benzimidazol-2-yle, benzimidazol-4-yle, benzimidazol-5-yle, indazol-1-yle, indazol-3-yle, indazol-4-yle, indazol-5-yle, indazol-6-yle, indazol-7-yle, indazol-2-yle, 1-benzofuran-2-yle, 1-benzofuran-3-yle, 1-benzofuran-4-yle, 1-benzofuran-5-yle, 1-benzofuran-6-yle, 1-benzofuran-7-yle, 1-benzothiophén-2-yle, 1-benzothiophén-3-yle, 1-benzothiophén-4-yle, 1-benzothiophén-5-yle, 1-benzothiophén-6-yle, 1-benzothiophén-7-yle, 1,3-benzothiazol-2-yle, 1,3-benzothiazol-4-yle, 1,3-benzothiazol-5-yle, 1,3-benzothiazol-6-yle, 1,3-benzothiazol-7-yle, 1,3-benzoxazol-2-yle, 1,3-benzoxazol-4-yle, 1,3-benzoxazol-5-yle, 1,3-benzoxazol-6-yle, 1,3-benzoxazol-7-yle, quinolin-2-yle, quinolin-3-yle, quinolin-4-yle, quinolin-5-yle, quinolin-6-yle, quinolin-7-yle, quinolin-8-yle, isoquinolin-1-yle, isoquinolin-3-yle, isoquinolin-4-yle, isoquinolin-5-yle, isoquinolin-6-yle, isoquinolin-7-yle ou isoquinolin-8-yle, qui peuvent chacun contenir jusqu'à deux substituants, les substituants étant choisis indépendamment l'un de l'autre dans la liste suivante :
substituants sur le carbone : fluor, chlore, brome, iode, méthyle, méthoxy, 2-propynyloxy, 2-propényloxy, substituants sur l'azote : méthyle, éthyle, propyle, cyclopropyle, cyclohexyle, phényle ou 2-propynyle, ou R¹ représente pipéridin-1-yle, pipéridin-2-yle, pipéridin-3-yle, pipéridin-4-yle, pipérazin-1-yle, pipérazin-2-yle, pipérazin-3-yle, morpholin-1-yle, morpholin-2-yle, morpholin-3-yle, tétrahydropyran-2-yle, tétrahydropyran-3-yle, tétrahydropyran-4-yle, 1,2,3,4-tétrahydroquinolin-1-yle, 1,2,3,4-tétrahydroisoquinolin-2-yle, 1,2,3,4-tétrahydroquinoxalin-1-yle, indolin-1-yle, isoindolin-2-yle, décahydroquinolin-1-yle ou décahydroisoquinolin-2-yle, qui peuvent chacun contenir jusqu'à deux substituants, les substituants étant choisis indépendamment l'un de l'autre dans la liste suivante : substituants sur le carbone : fluor, chlore, brome, iode, méthyle, méthoxy, 2-propynyloxy, 2-propényloxy, substituants sur l'azote : méthyle, éthyle, propyle, cyclopropyle, cyclohexyle, phényle ou 2-propynyle, Q représente phényle.

3. Procédé de lutte contre des champignons phytopathogènes, **caractérisé en ce que** des composés de formule (I) selon l'une quelconque des revendications 1 ou 2 sont appliqués sur les champignons phytopathogènes et/ou leur habitat.

4. Agent de lutte contre des champignons phytopathogènes, **caractérisé par** une teneur en au moins un composé de formule (I) selon l'une quelconque des revendications 1 ou 2, en plus d'extendeurs et/ou de substances tensioactives.

5. Utilisation d'au moins un composé de formule (I) selon l'une quelconque des revendications 1 ou 2 ou d'un agent selon la revendication 4 pour lutter contre des champignons phytopathogènes.

6. Procédé de fabrication d'agents de lutte contre des champignons phytopathogènes selon la revendication 4, **caractérisé en ce que** des composés de formule (I) selon l'une quelconque des revendications 1 ou 2 sont mélangés avec des extendeurs et/ou des substances tensioactives.

7. Utilisation de composés de formule (I) selon l'une quelconque des revendications 1 ou 2 pour le traitement de graines.

8. Utilisation de composés de formule (I) selon l'une quelconque des revendications 1 ou 2 pour le traitement de plantes transgéniques.

9. Utilisation de composés de formule (I) selon l'une quelconque des revendications 1 ou 2 pour le traitement de graines transgéniques.
